# EUROPEAN PATENT SPECIFICATION

(11) **EP 2 834 371 B1**
(45) Date of publication and mention of the grant of the patent: **09.01.2019**
(21) Application number: 13717134.4
(22) Date of filing: 05.04.2013
(51) Int. Cl.: C12Q 1/6886

(54) **GENE EXPRESSION PANEL FOR BREAST CANCER PROGNOSIS**
GENEXPRESSIONSPANEEL FÜR BRUSTKREBSPROGNOSE
PANNEAU D'EXPRESSION GÉNIQUE POUR LE PRONOSTIC DU CANCER DU SEIN

(30) Priority: 05.04.2012 US 201261620907 P; 15.03.2013 US 201361789071 P
(43) Date of publication of application: 11.02.2015
(73) Proprietor: The Regents of the University of California, Oakland, CA 94607 (US); Oregon Health & Science University, Portland, OR 97239 (US)
(72) Inventor: GRIFFITH, Obi L., St. Louis, Missouri 63105 (US); ENACHE, Oana M., Palo Alto, California 94306 (US); PEPIN, Francois, San Bruno, California 94066 (US); SPELLMAN, Paul T., Portland, Oregon 97219 (US); GRAY, Joe W., Lake Oswego, Oregon 97034 (US)
(74) Representative: J A Kemp
(86) International application number: PCT/US2013/035482
(87) International publication number: WO 2013/152307

(56) References cited:
- WO-A1-2010/029440
- US-A1- 2009 197 259
- M. FILIPITS ET AL: "A New Molecular Predictor of Distant Recurrence in ER-Positive, HER2-Negative Breast Cancer Adds Independent Information to Conventional Clinical Risk Factors", CLINICAL CANCER RESEARCH, vol. 17, no. 18, 1 August 2011 (2011-08-01), pages 6012-6020, XP055069701, ISSN: 1078-0432, DOI: 10.1158/1078-0432.CCR-11-0926 -& Filipits, M et al.: "A New Molecular Predictor of Distant Recurrence in ER-Positive, HER2-Negative Breast Cancer Adds Independent Information to Conventional Clinical Risk Factors", Supplementary data online Clinical Cancer Research, 1 August 2011 (2011-08-01), pages 1-32, XP002700104, Retrieved from the Internet: URL:http://clincancerres.aacrjournals.org/ content/suppl/2011/08/01/1078-0432.CCR-11- 0926.DC1/CCR-11-0926_Meth.T8.F9_rev.pdf [retrieved on 2013-07-04]
- AFFYMETRIX: "GeneChip TM Human Genome U133 Arrays", DATASHEET AFFYMETRIX,, 1 January 2003 (2003-01-01), pages 1-8, XP007921348,
- M. CRONIN ET AL: "Analytical Validation of the Oncotype DX Genomic Diagnostic Test for Recurrence Prognosis and Therapeutic Response Prediction in Node-Negative, Estrogen Receptor-Positive Breast Cancer", CLINICAL CHEMISTRY, vol. 53, no. 6, 19 April 2007 (2007-04-19) , pages 1084-1091, XP055069698, ISSN: 0009-9147, DOI: 10.1373/clinchem.2006.076497
- RODY ACHIM ET AL: "T-cell metagene predicts a favorable prognosis in estrogen receptor-negative and HER2-positive breast cancers", BREAST CANCER RESEARCH, CURRENT SCIENCE, LONDON, GB, vol. 11, no. 2, 9 March 2009 (2009-03-09), page R15, XP021053451, ISSN: 1465-5411, DOI: 10.1186/BCR2234
- VIJVER VAN DE M J ET AL: "A GENE-EXPRESSION SIGNATURE AS A PREDICTOR OF SURVIVAL IN BREAST CANCER", NEW ENGLAND JOURNAL OF MEDICINE, THE - NEJM, MASSACHUSETTS MEDICAL SOCIETY, US, vol. 347, no. 25, 19 December 2002 (2002-12-19), pages 1999-2009, XP008032093, ISSN: 1533-4406, DOI: 10.1056/NEJMOA021967
- PAIK SOONMYUNG ET AL: "A multigene assay to predict recurrence of tamoxifen-treated, node-negative breast cancer", NEW ENGLAND JOURNAL OF MEDICINE, MASSACHUSETTS MEDICAL SOCIETY, US, vol. 351, no. 27, 30 December 2004 (2004-12-30), pages 2817-2826, XP002578486, ISSN: 0028-4793
- WANG Y ET AL: "Gene-expression profiles to predict distant metastasis of lymph-node-negative primary breast cancer", THE LANCET, THE LANCET PUBLISHING GROUP, GB, vol. 365, no. 9460, 19 February 2005 (2005-02-19), pages 671-679, XP027763936, ISSN: 0140-6736 [retrieved on 2005-02-19]

### SEQUENCE LISTING

<110> Griffith, Obi L.
   Enache, Oana M.
   Pepin, Francois
   Spellman, Paul T.
   Gray, Joe W.
   The Regents of the University of California
   Oregon Health and Science University
<120> GENE EXPRESSION PANEL FOR BREAST CANCER
   PROGNOSIS
<130> 77429-872094
<150> US 61/620,907
   <151> 2012-04-05
<150> US 61/789,071
   <151> 2013-03-15
<160> 1760
<170> FastSEQ for Windows Version 4.0
<210> 1
   <211> 25
   <212> DNA
   <213> Artificial Sequence
<220>
   <223> synthetic nucleotide probe
<400> 1
   atggagctga ctctcatcga ctatg 25
<210> 2
   <211> 25
   <212> DNA
   <213> Artificial Sequence
<220>
   <223> synthetic nucleotide probe
<400> 2
   atatggtgca ttatcatcct tctaa 25
<210> 3
   <211> 25
   <212> DNA
   <213> Artificial Sequence
<220>
   <223> synthetic nucleotide probe
<400> 3
   agtcctctgg tctatctcat gaaac 25
<210> 4
   <211> 25
   <212> DNA
   <213> Artificial Sequence
<220>
   <223> synthetic nucleotide probe
<400> 4
   cttgcctccc cactgatagg aaggt 25
<210> 5
   <211> 25
   <212> DNA
   <213> Artificial Sequence
<220>
   <223> synthetic nucleotide probe
<400> 5
   caaaagccgt caaagacctt gcctc 25
<210> 6
   <211> 25
   <212> DNA
   <213> Artificial Sequence
<220>
   <223> synthetic nucleotide probe
<400> 6
   gattttgtac atagtcctct ggtct 25
<210> 7
   <211> 25
   <212> DNA
   <213> Artificial Sequence
<220>
   <223> synthetic nucleotide probe
<400> 7
   gccactacac ttcttaaggc gagca 25
<210> 8
   <211> 25
   <212> DNA
   <213> Artificial Sequence
<220>
   <223> synthetic nucleotide probe
<400> 8
   gataggaagg tcctaggctg ccgtg 25
<210> 9
   <211> 25
   <212> DNA
   <213> Artificial Sequence
<220>
   <223> synthetic nucleotide probe
<400> 9
   atccttctaa ggtagcagca gctgc 25
<210> 10
   <211> 25
   <212> DNA
   <213> Artificial Sequence
<220>
   <223> synthetic nucleotide probe
<400> 10
   actccgtaac agattctgga ccaac 25
<210> 11
   <211> 25
   <212> DNA
   <213> Artificial Sequence
<220>
   <223> synthetic nucleotide probe
<400> 11
   gaccaacctt caactatctt cttga 25
<210> 12
   <211> 25
   <212> DNA
   <213> Artificial Sequence
<220>
   <223> synthetic nucleotide probe
<400> 12
   gaaagatgaa ctctgcaggc taaga 25
<210> 13
   <211> 25
   <212> DNA
   <213> Artificial Sequence
<220>
   <223> synthetic nucleotide probe
<400> 13
   acaagatgaa caagtcggac ttcct 25
<210> 14
   <211> 25
   <212> DNA
   <213> Artificial Sequence
<220>
   <223> synthetic nucleotide probe
<400> 14
   tggctcctag gaatgcttgg tgctg 25
<210> 15
   <211> 25
   <212> DNA
   <213> Artificial Sequence
<220>
   <223> synthetic nucleotide probe
<400> 15
   gatatgattc ggatcctgtg aaggc 25
<210> 16
   <211> 25
   <212> DNA
   <213> Artificial Sequence
<220>
   <223> synthetic nucleotide probe
<400> 16
   aaagaaagag tccatcagat ttgtg 25
<210> 17
   <211> 25
   <212> DNA
   <213> Artificial Sequence
<220>
   <223> synthetic nucleotide probe
<400> 17
   gaataatcag gctcgcttta tctta 25
<210> 18
   <211> 25
   <212> DNA
   <213> Artificial Sequence
<220>
   <223> synthetic nucleotide probe
<400> 18
   cttggtgctg aatctgctaa actga 25
<210> 19
   <211> 25
   <212> DNA
   <213> Artificial Sequence
<220>
   <223> synthetic nucleotide probe
<400> 19
   aagaacaaga gctggacaca ttaaa 25
<210> 20
   <211> 25
   <212> DNA
   <213> Artificial Sequence
<220>
   <223> synthetic nucleotide probe
<400> 20
   gagacttttt tgaactcaga cttaa 25
<210> 21
   <211> 25
   <212> DNA
   <213> Artificial Sequence
<220>
   <223> synthetic nucleotide probe
<400> 21
   gtacaactcg tatttatctc tgatg 25
<210> 22
   <211> 25
   <212> DNA
   <213> Artificial Sequence
<220>
   <223> synthetic nucleotide probe
<400> 22
   gaatgtttga cttcgtattg accct 25
<210> 23
   <211> 25
   <212> DNA
   <213> Artificial Sequence
<220>
   <223> synthetic nucleotide probe
<400> 23
   ggatgctgaa atttttccca tggaa 25
<210> 24
   <211> 25
   <212> DNA
   <213> Artificial Sequence
<220>
   <223> synthetic nucleotide probe
<400> 24
   acttcgtatt gacccttatc tgtaa 25
<210> 25
   <211> 25
   <212> DNA
   <213> Artificial Sequence
<220>
   <223> synthetic nucleotide probe
<400> 25
   caatatatca tcctttggca tccca 25
<210> 26
   <211> 25
   <212> DNA
   <213> Artificial Sequence
<220>
   <223> synthetic nucleotide probe
<400> 26
   cgctctcgtt tcattttctg cagcg 25
<210> 27
   <211> 25
   <212> DNA
   <213> Artificial Sequence
<220>
   <223> synthetic nucleotide probe
<400> 27
   tattcttctc tttagacgac ctctt 25
<210> 28
   <211> 25
   <212> DNA
   <213> Artificial Sequence
<220>
   <223> synthetic nucleotide probe
<400> 28
   tctctttaga cgacctcttc caaaa 25
<210> 29
   <211> 25
   <212> DNA
   <213> Artificial Sequence
<220>
   <223> synthetic nucleotide probe
<400> 29
   ctacctccat ttagggattt gcttg 25
<210> 30
   <211> 25
   <212> DNA
   <213> Artificial Sequence
<220>
   <223> synthetic nucleotide probe
<400> 30
   gtgtctcaga gctgttaagg gctta 25
<210> 31
   <211> 25
   <212> DNA
   <213> Artificial Sequence
<220>
   <223> synthetic nucleotide probe
<400> 31
   ccctcaatct agaacgctac acaag 25
<210> 32
   <211> 25
   <212> DNA
   <213> Artificial Sequence
<220>
   <223> synthetic nucleotide probe
<400> 32
   gaggccatgt gtctcagagc tgtta 25
<210> 33
   <211> 25
   <212> DNA
   <213> Artificial Sequence
<220>
   <223> synthetic nucleotide probe
<400> 33
   ttagggattt gcttgggata cagaa 25
<210> 34
   <211> 25
   <212> DNA
   <213> Artificial Sequence
<220>
   <223> synthetic nucleotide probe
<400> 34
   gtgctctacc tccatttagg gattt 25
<210> 35
   <211> 25
   <212> DNA
   <213> Artificial Sequence
<220>
   <223> synthetic nucleotide probe
<400> 35
   aaataggaac acgtgctcta cctcc 25
<210> 36
   <211> 25
   <212> DNA
   <213> Artificial Sequence
<220>
   <223> synthetic nucleotide probe
<400> 36
   gggatacaga agaggccatg tgtct 25
<210> 37
   <211> 25
   <212> DNA
   <213> Artificial Sequence
<220>
   <223> synthetic nucleotide probe
<400> 37
   gaagaggcca tgtgtctcag agctg 25
<210> 38
   <211> 25
   <212> DNA
   <213> Artificial Sequence
<220>
   <223> synthetic nucleotide probe
<400> 38
   cagagctgtt aagggcttat ttttt 25
<210> 39
   <211> 25
   <212> DNA
   <213> Artificial Sequence
<220>
   <223> synthetic nucleotide probe
<400> 39
   cattggagtc atagcatgtg tgtaa 25
<210> 40
   <211> 25
   <212> DNA
   <213> Artificial Sequence
<220>
   <223> synthetic nucleotide probe
<400> 40
   gaacttgcta tgtaatttgt gtcta 25
<210> 41
   <211> 25
   <212> DNA
   <213> Artificial Sequence
<220>
   <223> synthetic nucleotide probe
<400> 41
   gatggtgatg ttcacctggc aatca 25
<210> 42
   <211> 25
   <212> DNA
   <213> Artificial Sequence
<220>
   <223> synthetic nucleotide probe
<400> 42
   gagccaccag gaaggcgcat cttag 25
<210> 43
   <211> 25
   <212> DNA
   <213> Artificial Sequence
<220>
   <223> synthetic nucleotide probe
<400> 43
   ttgacccacc ttgagagaga gccac 25
<210> 44
   <211> 25
   <212> DNA
   <213> Artificial Sequence
<220>
   <223> synthetic nucleotide probe
<400> 44
   ggacactaag tccattgtta catga 25
<210> 45
   <211> 25
   <212> DNA
   <213> Artificial Sequence
<220>
   <223> synthetic nucleotide probe
<400> 45
   gaaatgattt cctggctggc caact 25
<210> 46
   <211> 25
   <212> DNA
   <213> Artificial Sequence
<220>
   <223> synthetic nucleotide probe
<400> 46
   acactggttt tcatgcgctg ttttt 25
<210> 47
   <211> 25
   <212> DNA
   <213> Artificial Sequence
<220>
   <223> synthetic nucleotide probe
<400> 47
   actgattact ggctgtgtct tgggt 25
<210> 48
   <211> 25
   <212> DNA
   <213> Artificial Sequence
<220>
   <223> synthetic nucleotide probe
<400> 48
   tggacctaga ctgtgctttt ctgtc 25
<210> 49
   <211> 25
   <212> DNA
   <213> Artificial Sequence
<220>
   <223> synthetic nucleotide probe
<400> 49
   ttgggtgggc agaaactcga acttg 25
<210> 50
   <211> 25
   <212> DNA
   <213> Artificial Sequence
<220>
   <223> synthetic nucleotide probe
<400> 50
   acctggcaat cagctgagtt gagac 25
<210> 51
   <211> 25
   <212> DNA
   <213> Artificial Sequence
<220>
   <223> synthetic nucleotide probe
<400> 51
   gaaggcactg ctgggagcca ttaga 25
<210> 52
   <211> 25
   <212> DNA
   <213> Artificial Sequence
<220>
   <223> synthetic nucleotide probe
<400> 52
   caggctcatg ggcaggcaca agaag 25
<210> 53
   <211> 25
   <212> DNA
   <213> Artificial Sequence
<220>
   <223> synthetic nucleotide probe
<400> 53
   gtcttagtcg tgaccacatg gctgt 25
<210> 54
   <211> 25
   <212> DNA
   <213> Artificial Sequence
<220>
   <223> synthetic nucleotide probe
<400> 54
   cacagataca agagaagcca ggagg 25
<210> 55
   <211> 25
   <212> DNA
   <213> Artificial Sequence
<220>
   <223> synthetic nucleotide probe
<400> 55
   aaggggctgt gtgaaggcac tgctg 25
<210> 56
   <211> 25
   <212> DNA
   <213> Artificial Sequence
<220>
   <223> synthetic nucleotide probe
<400> 56
   agaagaactg aggagtggtg gacca 25
<210> 57
   <211> 25
   <212> DNA
   <213> Artificial Sequence
<220>
   <223> synthetic nucleotide probe
<400> 57
   gccaggaggt ctatgatggt gacga 25
<210> 58
   <211> 25
   <212> DNA
   <213> Artificial Sequence
<220>
   <223> synthetic nucleotide probe
<400> 58
   cccacctggc atatactggc tggcc 25
<210> 59
   <211> 25
   <212> DNA
   <213> Artificial Sequence
<220>
   <223> synthetic nucleotide probe
<400> 59
   acatggctgt tgtcaggtcg tgctg 25
<210> 60
   <211> 25
   <212> DNA
   <213> Artificial Sequence
<220>
   <223> synthetic nucleotide probe
<400> 60
   tctatgggga tgtgctctac ttcct 25
<210> 61
   <211> 25
   <212> DNA
   <213> Artificial Sequence
<220>
   <223> synthetic nucleotide probe
<400> 61
   gcatggaaac catcacagct tgcct 25
<210> 62
   <211> 25
   <212> DNA
   <213> Artificial Sequence
<220>
   <223> synthetic nucleotide probe
<400> 62
   gagtggtgga ccaggctcga acact 25
<210> 63
   <211> 25
   <212> DNA
   <213> Artificial Sequence
<220>
   <223> synthetic nucleotide probe
<400> 63
   ttggagggac aaagctaatt gatct 25
<210> 64
   <211> 25
   <212> DNA
   <213> Artificial Sequence
<220>
   <223> synthetic nucleotide probe
<400> 64
   gatgccaagg ccacaaaagc caaga 25
<210> 65
   <211> 25
   <212> DNA
   <213> Artificial Sequence
<220>
   <223> synthetic nucleotide probe
<400> 65
   ccaggctcga acactggccg aggag 25
<210> 66
   <211> 25
   <212> DNA
   <213> Artificial Sequence
<220>
   <223> synthetic nucleotide probe
<400> 66
   tgacagagca ccgcgacgga ttcca 25
<210> 67
   <211> 25
   <212> DNA
   <213> Artificial Sequence
<220>
   <223> synthetic nucleotide probe
<400> 67
   gggagccatt agaacacaga tacaa 25
<210> 68
   <211> 25
   <212> DNA
   <213> Artificial Sequence
<220>
   <223> synthetic nucleotide probe
<400> 68
   tttgtcttca tgaatgccct gtggc 25
<210> 69
   <211> 25
   <212> DNA
   <213> Artificial Sequence
<220>
   <223> synthetic nucleotide probe
<400> 69
   ggagaccaag ccttcttatc tcaac 25
<210> 70
   <211> 25
   <212> DNA
   <213> Artificial Sequence
<220>
   <223> synthetic nucleotide probe
<400> 70
   tgcagtgtgt gggttcattc acctg 25
<210> 71
   <211> 25
   <212> DNA
   <213> Artificial Sequence
<220>
   <223> synthetic nucleotide probe
<400> 71
   ctccgcttca ttctacagct tgtgg 25
<210> 72
   <211> 25
   <212> DNA
   <213> Artificial Sequence
<220>
   <223> synthetic nucleotide probe
<400> 72
   tgtgtcagag cactgagctc caccc 25
<210> 73
   <211> 25
   <212> DNA
   <213> Artificial Sequence
<220>
   <223> synthetic nucleotide probe
<400> 73
   tacaagttcg gctttgagct tcctc 25
<210> 74
   <211> 25
   <212> DNA
   <213> Artificial Sequence
<220>
   <223> synthetic nucleotide probe
<400> 74
   aaaggatgcg gactcatcct cagcc 25
<210> 75
   <211> 25
   <212> DNA
   <213> Artificial Sequence
<220>
   <223> synthetic nucleotide probe
<400> 75
   actttgttca ctgtcctgtg tcaga 25
<210> 76
   <211> 25
   <212> DNA
   <213> Artificial Sequence
<220>
   <223> synthetic nucleotide probe
<400> 76
   gaaagggttg ctgctgtcag ccttg 25
<210> 77
   <211> 25
   <212> DNA
   <213> Artificial Sequence
<220>
   <223> synthetic nucleotide probe
<400> 77
   agatagggat attggcccct cactg 25
<210> 78
   <211> 25
   <212> DNA
   <213> Artificial Sequence
<220>
   <223> synthetic nucleotide probe
<400> 78
   ggcaatctcc tgggccttaa aggat 25
<210> 79
   <211> 25
   <212> DNA
   <213> Artificial Sequence
<220>
   <223> synthetic nucleotide probe
<400> 79
   cttagcctct gacttcctaa tgtag 25
<210> 80
   <211> 25
   <212> DNA
   <213> Artificial Sequence
<220>
   <223> synthetic nucleotide probe
<400> 80
   gcaaaggggt ttcctcgatt tggag 25
<210> 81
   <211> 25
   <212> DNA
   <213> Artificial Sequence
<220>
   <223> synthetic nucleotide probe
<400> 81
   aaatggcctc ctggcgtaag ctttt 25
<210> 82
   <211> 25
   <212> DNA
   <213> Artificial Sequence
<220>
   <223> synthetic nucleotide probe
<400> 82
   aaaccaactc agttccatca tggtg 25
<210> 83
   <211> 25
   <212> DNA
   <213> Artificial Sequence
<220>
   <223> synthetic nucleotide probe
<400> 83
   ttccaccgtc atttctaact cttaa 25
<210> 84
   <211> 25
   <212> DNA
   <213> Artificial Sequence
<220>
   <223> synthetic nucleotide probe
<400> 84
   ggttttctct tcatgtaagt ccttg 25
<210> 85
   <211> 25
   <212> DNA
   <213> Artificial Sequence
<220>
   <223> synthetic nucleotide probe
<400> 85
   cggagtacct gcgctatgaa gacac 25
<210> 86
   <211> 25
   <212> DNA
   <213> Artificial Sequence
<220>
   <223> synthetic nucleotide probe
<400> 86
   ccctgcatcc tcaacaacaa tgtgc 25
<210> 87
   <211> 25
   <212> DNA
   <213> Artificial Sequence
<220>
   <223> synthetic nucleotide probe
<400> 87
   gtgttctcct actgcaaata ttttc 25
<210> 88
   <211> 25
   <212> DNA
   <213> Artificial Sequence
<220>
   <223> synthetic nucleotide probe
<400> 88
   aattgaggcc ttttcgatag tttcg 25
<210> 89
   <211> 25
   <212> DNA
   <213> Artificial Sequence
<220>
   <223> synthetic nucleotide probe
<400> 89
   ggaggtggtc agcaggcaat ctcct 25
<210> 90
   <211> 25
   <212> DNA
   <213> Artificial Sequence
<220>
   <223> synthetic nucleotide probe
<400> 90
   ccagcgccca tgttgtgata caggg 25
<210> 91
   <211> 25
   <212> DNA
   <213> Artificial Sequence
<220>
   <223> synthetic nucleotide probe
<400> 91
   gaaaaactca ggcccatcca ttttc 25
<210> 92
   <211> 25
   <212> DNA
   <213> Artificial Sequence
<220>
   <223> synthetic nucleotide probe
<400> 92
   tgaggtggtc tttaacgacc ctgaa 25
<210> 93
   <211> 25
   <212> DNA
   <213> Artificial Sequence
<220>
   <223> synthetic nucleotide probe
<400> 93
   tgttcttagc actttaattc ctgtc 25
<210> 94
   <211> 25
   <212> DNA
   <213> Artificial Sequence
<220>
   <223> synthetic nucleotide probe
<400> 94
   agctccaccc ttttctgaga gttat 25
<210> 95
   <211> 25
   <212> DNA
   <213> Artificial Sequence
<220>
   <223> synthetic nucleotide probe
<400> 95
   cactctcagc catagcactt tgttc 25
<210> 96
   <211> 25
   <212> DNA
   <213> Artificial Sequence
<220>
   <223> synthetic nucleotide probe
<400> 96
   gaagcagctt tacctacttg tttct 25
<210> 97
   <211> 25
   <212> DNA
   <213> Artificial Sequence
<220>
   <223> synthetic nucleotide probe
<400> 97
   gaagttactc gtgtcaaagc cgtta 25
<210> 98
   <211> 25
   <212> DNA
   <213> Artificial Sequence
<220>
   <223> synthetic nucleotide probe
<400> 98
   ggtggatgtc aatacccctg attta 25
<210> 99
   <211> 25
   <212> DNA
   <213> Artificial Sequence
<220>
   <223> synthetic nucleotide probe
<400> 99
   ccgagccagc caactcaaga gacaa 25
<210> 100
   <211> 25
   <212> DNA
   <213> Artificial Sequence
<220>
   <223> synthetic nucleotide probe
<400> 100
   tggatgcagg gatcccagca gtgca 25
<210> 101
   <211> 25
   <212> DNA
   <213> Artificial Sequence
<220>
   <223> synthetic nucleotide probe
<400> 101
   gatcctggct tgcggagtgg ctaaa 25
<210> 102
   <211> 25
   <212> DNA
   <213> Artificial Sequence
<220>
   <223> synthetic nucleotide probe
<400> 102
   gctgaaactg gtctactgtg tctct 25
<210> 103
   <211> 25
   <212> DNA
   <213> Artificial Sequence
<220>
   <223> synthetic nucleotide probe
<400> 103
   tttctaagac tttttcacat ccaaa 25
<210> 104
   <211> 25
   <212> DNA
   <213> Artificial Sequence
<220>
   <223> synthetic nucleotide probe
<400> 104
   gttttactcc aatcagctgg caatt 25
<210> 105
   <211> 25
   <212> DNA
   <213> Artificial Sequence
<220>
   <223> synthetic nucleotide probe
<400> 105
   ggcaccctta gctgatggaa acaat 25
<210> 106
   <211> 25
   <212> DNA
   <213> Artificial Sequence
<220>
   <223> synthetic nucleotide probe
<400> 106
   attttgagct gccggttata cacca 25
<210> 107
   <211> 25
   <212> DNA
   <213> Artificial Sequence
<220>
   <223> synthetic nucleotide probe
<400> 107
   tgttctgttc agtacctagc tctgc 25
<210> 108
   <211> 25
   <212> DNA
   <213> Artificial Sequence
<220>
   <223> synthetic nucleotide probe
<400> 108
   gtaaatgcca aactaccgac ttgat 25
<210> 109
   <211> 25
   <212> DNA
   <213> Artificial Sequence
<220>
   <223> synthetic nucleotide probe
<400> 109
   tacgcttaga atcagtttta ctcca 25
<210> 110
   <211> 25
   <212> DNA
   <213> Artificial Sequence
<220>
   <223> synthetic nucleotide probe
<400> 110
   gttcagaaac tcataggcac cctta 25
<210> 111
   <211> 25
   <212> DNA
   <213> Artificial Sequence
<220>
   <223> synthetic nucleotide probe
<400> 111
   tgagcagtgg tgtccatcac atata 25
<210> 112
   <211> 25
   <212> DNA
   <213> Artificial Sequence
<220>
   <223> synthetic nucleotide probe
<400> 112
   atgtacaact cagatgtttc tcatt 25
<210> 113
   <211> 25
   <212> DNA
   <213> Artificial Sequence
<220>
   <223> synthetic nucleotide probe
<400> 113
   gctctgctct tttatattgc tttaa 25
<210> 114
   <211> 25
   <212> DNA
   <213> Artificial Sequence
<220>
   <223> synthetic nucleotide probe
<400> 114
   aatttcttac tatacttttc ataat 25
<210> 115
   <211> 25
   <212> DNA
   <213> Artificial Sequence
<220>
   <223> synthetic nucleotide probe
<400> 115
   atttcaccta ccaaagctgt gctgt 25
<210> 116
   <211> 25
   <212> DNA
   <213> Artificial Sequence
<220>
   <223> synthetic nucleotide probe
<400> 116
   actagctcat tatttccatc tttgg 25
<210> 117
   <211> 25
   <212> DNA
   <213> Artificial Sequence
<220>
   <223> synthetic nucleotide probe
<400> 117
   aaatgatttt tcacaactaa cttgt 25
<210> 118
   <211> 25
   <212> DNA
   <213> Artificial Sequence
<220>
   <223> synthetic nucleotide probe
<400> 118
   ttgcagtctg caccttatgg atcac 25
<210> 119
   <211> 25
   <212> DNA
   <213> Artificial Sequence
<220>
   <223> synthetic nucleotide probe
<400> 119
   tgatacatct gtgatttagg tcatt 25
<210> 120
   <211> 25
   <212> DNA
   <213> Artificial Sequence
<220>
   <223> synthetic nucleotide probe
<400> 120
   ggagacgcca atagcaatat ctagg 25
<210> 121
   <211> 25
   <212> DNA
   <213> Artificial Sequence
<220>
   <223> synthetic nucleotide probe
<400> 121
   ctgatgccac atagtcttgc ataaa 25
<210> 122
   <211> 25
   <212> DNA
   <213> Artificial Sequence
<220>
   <223> synthetic nucleotide probe
<400> 122
   agctgtgctg ttaatgccgt gaaag 25
<210> 123
   <211> 25
   <212> DNA
   <213> Artificial Sequence
<220>
   <223> synthetic nucleotide probe
<400> 123
   gaagtgcgcc aatgttgtct tttct 25
<210> 124
   <211> 25
   <212> DNA
   <213> Artificial Sequence
<220>
   <223> synthetic nucleotide probe
<400> 124
   gtgaaacctt catggatagt cttta 25
<210> 125
   <211> 25
   <212> DNA
   <213> Artificial Sequence
<220>
   <223> synthetic nucleotide probe
<400> 125
   tttactaaag tcctcctgcc aggta 25
<210> 126
   <211> 25
   <212> DNA
   <213> Artificial Sequence
<220>
   <223> synthetic nucleotide probe
<400> 126
   ggacatcaac cacagacaat ttaaa 25
<210> 127
   <211> 25
   <212> DNA
   <213> Artificial Sequence
<220>
   <223> synthetic nucleotide probe
<400> 127
   tgttgcatgc atatttcacc tacca 25
<210> 128
   <211> 25
   <212> DNA
   <213> Artificial Sequence
<220>
   <223> synthetic nucleotide probe
<400> 128
   ataaacctta gacatatcac accta 25
<210> 129
   <211> 25
   <212> DNA
   <213> Artificial Sequence
<220>
   <223> synthetic nucleotide probe
<400> 129
   tagtctttaa tctctgatct ttttg 25
<210> 130
   <211> 25
   <212> DNA
   <213> Artificial Sequence
<220>
   <223> synthetic nucleotide probe
<400> 130
   gagacagcgt gatacttaca actca 25
<210> 131
   <211> 25
   <212> DNA
   <213> Artificial Sequence
<220>
   <223> synthetic nucleotide probe
<400> 131
   gaccattgta ttttccacta gcagt 25
<210> 132
   <211> 25
   <212> DNA
   <213> Artificial Sequence
<220>
   <223> synthetic nucleotide probe
<400> 132
   ctgcagcagc aggttacata gcaaa 25
<210> 133
   <211> 25
   <212> DNA
   <213> Artificial Sequence
<220>
   <223> synthetic nucleotide probe
<400> 133
   gccgtgaaag tttaacgttt gcgat 25
<210> 134
   <211> 25
   <212> DNA
   <213> Artificial Sequence
<220>
   <223> synthetic nucleotide probe
<400> 134
   aactgccgta attttgatac atctg 25
<210> 135
   <211> 25
   <212> DNA
   <213> Artificial Sequence
<220>
   <223> synthetic nucleotide probe
<400> 135
   tatttaccat cacatgctgc agctg 25
<210> 136
   <211> 25
   <212> DNA
   <213> Artificial Sequence
<220>
   <223> synthetic nucleotide probe
<400> 136
   aacgtttgcg ataaactgcc gtaat 25
<210> 137
   <211> 25
   <212> DNA
   <213> Artificial Sequence
<220>
   <223> synthetic nucleotide probe
<400> 137
   ggaaatttgc attgagacca ttgta 25
<210> 138
   <211> 25
   <212> DNA
   <213> Artificial Sequence
<220>
   <223> synthetic nucleotide probe
<400> 138
   gcaccttatg gatcacaatt acctt 25
<210> 139
   <211> 25
   <212> DNA
   <213> Artificial Sequence
<220>
   <223> synthetic nucleotide probe
<400> 139
   agaagcaaaa cacagcacct ttacc 25
<210> 140
   <211> 25
   <212> DNA
   <213> Artificial Sequence
<220>
   <223> synthetic nucleotide probe
<400> 140
   cccttagtct cctcacataa atttc 25
<210> 141
   <211> 25
   <212> DNA
   <213> Artificial Sequence
<220>
   <223> synthetic nucleotide probe
<400> 141
   tgtgtaaggt gatgttcccg gtcgc 25
<210> 142
   <211> 25
   <212> DNA
   <213> Artificial Sequence
<220>
   <223> synthetic nucleotide probe
<400> 142
   ctgccaggta gttcccactg atgga 25
<210> 143
   <211> 25
   <212> DNA
   <213> Artificial Sequence
<220>
   <223> synthetic nucleotide probe
<400> 143
   gccttccttt accttgtagt acaag 25
<210> 144
   <211> 25
   <212> DNA
   <213> Artificial Sequence
<220>
   <223> synthetic nucleotide probe
<400> 144
   tttttcctct tgcaacaatg acggt 25
<210> 145
   <211> 25
   <212> DNA
   <213> Artificial Sequence
<220>
   <223> synthetic nucleotide probe
<400> 145
   gtcaatttac aaggccaggg ataga 25
<210> 146
   <211> 25
   <212> DNA
   <213> Artificial Sequence
<220>
   <223> synthetic nucleotide probe
<400> 146
   ttccacttca ttttacatgc cacta 25
<210> 147
   <211> 25
   <212> DNA
   <213> Artificial Sequence
<220>
   <223> synthetic nucleotide probe
<400> 147
   gtgctagaca attactgttc ttttc 25
<210> 148
   <211> 25
   <212> DNA
   <213> Artificial Sequence
<220>
   <223> synthetic nucleotide probe
<400> 148
   aatatctata actgcatttt gtgct 25
<210> 149
   <211> 25
   <212> DNA
   <213> Artificial Sequence
<220>
   <223> synthetic nucleotide probe
<400> 149
   gatagaaaac actccataat tgctt 25
<210> 150
   <211> 25
   <212> DNA
   <213> Artificial Sequence
<220>
   <223> synthetic nucleotide probe
<400> 150
   cattgatttt attaagcctt ccttt 25
<210> 151
   <211> 25
   <212> DNA
   <213> Artificial Sequence
<220>
   <223> synthetic nucleotide probe
<400> 151
   tacatgccac tatattgact ttaat 25
<210> 152
   <211> 25
   <212> DNA
   <213> Artificial Sequence
<220>
   <223> synthetic nucleotide probe
<400> 152
   tctggtatga aaggctccat tgatt 25
<210> 153
   <211> 25
   <212> DNA
   <213> Artificial Sequence
<220>
   <223> synthetic nucleotide probe
<400> 153
   gctttccttg attttgctga ggatt 25
<210> 154
   <211> 25
   <212> DNA
   <213> Artificial Sequence
<220>
   <223> synthetic nucleotide probe
<400> 154
   ggatatcagc gtttctctgt gtgct 25
<210> 155
   <211> 25
   <212> DNA
   <213> Artificial Sequence
<220>
   <223> synthetic nucleotide probe
<400> 155
   gaaagctttg tctgccaatg ttgtg 25
<210> 156
   <211> 25
   <212> DNA
   <213> Artificial Sequence
<220>
   <223> synthetic nucleotide probe
<400> 156
   cagagagtcc tctctaatgc tccta 25
<210> 157
   <211> 25
   <212> DNA
   <213> Artificial Sequence
<220>
   <223> synthetic nucleotide probe
<400> 157
   gatattgcac agtactggtc agtat 25
<210> 158
   <211> 25
   <212> DNA
   <213> Artificial Sequence
<220>
   <223> synthetic nucleotide probe
<400> 158
   gaccagggac ttgacccatt agggt 25
<210> 159
   <211> 25
   <212> DNA
   <213> Artificial Sequence
<220>
   <223> synthetic nucleotide probe
<400> 159
   agatatggta tcctctgagc gccac 25
<210> 160
   <211> 25
   <212> DNA
   <213> Artificial Sequence
<220>
   <223> synthetic nucleotide probe
<400> 160
   aatgctccta gaccagggac ttgac 25
<210> 161
   <211> 25
   <212> DNA
   <213> Artificial Sequence
<220>
   <223> synthetic nucleotide probe
<400> 161
   atcgtgacac tttcaacatg taggg 25
<210> 162
   <211> 25
   <212> DNA
   <213> Artificial Sequence
<220>
   <223> synthetic nucleotide probe
<400> 162
   ttggatgccc taactgctga tgtga 25
<210> 163
   <211> 25
   <212> DNA
   <213> Artificial Sequence
<220>
   <223> synthetic nucleotide probe
<400> 163
   gtgttttctg cttcatacga tattg 25
<210> 164
   <211> 25
   <212> DNA
   <213> Artificial Sequence
<220>
   <223> synthetic nucleotide probe
<400> 164
   aagggtgaca tccaggaggg gcctc 25
<210> 165
   <211> 25
   <212> DNA
   <213> Artificial Sequence
<220>
   <223> synthetic nucleotide probe
<400> 165
   caggaggggc ctctgaaatt tccca 25
<210> 166
   <211> 25
   <212> DNA
   <213> Artificial Sequence
<220>
   <223> synthetic nucleotide probe
<400> 166
   gatgcgggag tggttttcag agaca 25
<210> 167
   <211> 25
   <212> DNA
   <213> Artificial Sequence
<220>
   <223> synthetic nucleotide probe
<400> 167
   cagcaaggat tcaggagtgc ccctc 25
<210> 168
   <211> 25
   <212> DNA
   <213> Artificial Sequence
<220>
   <223> synthetic nucleotide probe
<400> 168
   gtgaactttc tgccaagatg cggga 25
<210> 169
   <211> 25
   <212> DNA
   <213> Artificial Sequence
<220>
   <223> synthetic nucleotide probe
<400> 169
   caaggctcgg gaactcatca gccgc 25
<210> 170
   <211> 25
   <212> DNA
   <213> Artificial Sequence
<220>
   <223> synthetic nucleotide probe
<400> 170
   aacacactgg aggacaaggc tcggg 25
<210> 171
   <211> 25
   <212> DNA
   <213> Artificial Sequence
<220>
   <223> synthetic nucleotide probe
<400> 171
   gacgtctcca gtgccttgga taagc 25
<210> 172
   <211> 25
   <212> DNA
   <213> Artificial Sequence
<220>
   <223> synthetic nucleotide probe
<400> 172
   ccaagccctc cagcaaggat tcagg 25
<210> 173
   <211> 25
   <212> DNA
   <213> Artificial Sequence
<220>
   <223> synthetic nucleotide probe
<400> 173
   tcatcagccg catcaaacag agtga 25
<210> 174
   <211> 25
   <212> DNA
   <213> Artificial Sequence
<220>
   <223> synthetic nucleotide probe
<400> 174
   gttctgtcga tcgtcttgga aggcc 25
<210> 175
   <211> 25
   <212> DNA
   <213> Artificial Sequence
<220>
   <223> synthetic nucleotide probe
<400> 175
   atcgccacct ggtgctcatg aggtg 25
<210> 176
   <211> 25
   <212> DNA
   <213> Artificial Sequence
<220>
   <223> synthetic nucleotide probe
<400> 176
   actcgtcttg gtattgcact gttgt 25
<210> 177
   <211> 25
   <212> DNA
   <213> Artificial Sequence
<220>
   <223> synthetic nucleotide probe
<400> 177
   attctcttcc catttttgta cattt 25
<210> 178
   <211> 25
   <212> DNA
   <213> Artificial Sequence
<220>
   <223> synthetic nucleotide probe
<400> 178
   tgctccgtga aaggacatcg ccacc 25
<210> 179
   <211> 25
   <212> DNA
   <213> Artificial Sequence
<220>
   <223> synthetic nucleotide probe
<400> 179
   ggagacaaac ctcgtcagat gctca 25
<210> 180
   <211> 25
   <212> DNA
   <213> Artificial Sequence
<220>
   <223> synthetic nucleotide probe
<400> 180
   tgaagtcttt ggtgttgctc cgtga 25
<210> 181
   <211> 25
   <212> DNA
   <213> Artificial Sequence
<220>
   <223> synthetic nucleotide probe
<400> 181
   tgattgttgc catgtgagag tttta 25
<210> 182
   <211> 25
   <212> DNA
   <213> Artificial Sequence
<220>
   <223> synthetic nucleotide probe
<400> 182
   actcctaatg tttggtgcta tgttt 25
<210> 183
   <211> 25
   <212> DNA
   <213> Artificial Sequence
<220>
   <223> synthetic nucleotide probe
<400> 183
   gtatgggtca tttcaaagag ggctt 25
<210> 184
   <211> 25
   <212> DNA
   <213> Artificial Sequence
<220>
   <223> synthetic nucleotide probe
<400> 184
   tggtgctatg ttttcctgag gagat 25
<210> 185
   <211> 25
   <212> DNA
   <213> Artificial Sequence
<220>
   <223> synthetic nucleotide probe
<400> 185
   aagtttctct agtgttttct gtgga 25
<210> 186
   <211> 25
   <212> DNA
   <213> Artificial Sequence
<220>
   <223> synthetic nucleotide probe
<400> 186
   gtatttttgg ctcgaagttt ctcta 25
<210> 187
   <211> 25
   <212> DNA
   <213> Artificial Sequence
<220>
   <223> synthetic nucleotide probe
<400> 187
   gaagccatag cactcctaat gtttg 25
<210> 188
   <211> 25
   <212> DNA
   <213> Artificial Sequence
<220>
   <223> synthetic nucleotide probe
<400> 188
   aagagggctt atgaggctgt gaaac 25
<210> 189
   <211> 25
   <212> DNA
   <213> Artificial Sequence
<220>
   <223> synthetic nucleotide probe
<400> 189
   cccagagctc ttaacgctgt gacca 25
<210> 190
   <211> 25
   <212> DNA
   <213> Artificial Sequence
<220>
   <223> synthetic nucleotide probe
<400> 190
   gaggctgtga aacccagagc tctta 25
<210> 191
   <211> 25
   <212> DNA
   <213> Artificial Sequence
<220>
   <223> synthetic nucleotide probe
<400> 191
   atttctcttc ttcagggcaa acttg 25
<210> 192
   <211> 25
   <212> DNA
   <213> Artificial Sequence
<220>
   <223> synthetic nucleotide probe
<400> 192
   gctggggagc acttaattct tccca 25
<210> 193
   <211> 25
   <212> DNA
   <213> Artificial Sequence
<220>
   <223> synthetic nucleotide probe

<400> 193
   ggagctccga tgtgaggggc aaggc 25
<210> 194
   <211> 25
   <212> DNA
   <213> Artificial Sequence
<220>
   <223> synthetic nucleotide probe
<400> 194
   tctggctggg gtccgtgaag cccag 25
<210> 195
   <211> 25
   <212> DNA
   <213> Artificial Sequence
<220>
   <223> synthetic nucleotide probe
<400> 195
   gccaaaccag ctcatttcaa caaag 25
<210> 196
   <211> 25
   <212> DNA
   <213> Artificial Sequence
<220>
   <223> synthetic nucleotide probe
<400> 196
   atgtgaacac catcatgcct ttata 25
<210> 197
   <211> 25
   <212> DNA
   <213> Artificial Sequence
<220>
   <223> synthetic nucleotide probe
<400> 197
   tgccatcaca tccctgatac tgtgt 25
<210> 198
   <211> 25
   <212> DNA
   <213> Artificial Sequence
<220>
   <223> synthetic nucleotide probe
<400> 198
   tttggactac gttcttggct ccaga 25
<210> 199
   <211> 25
   <212> DNA
   <213> Artificial Sequence
<220>
   <223> synthetic nucleotide probe
<400> 199
   gcagccaaat cttgcctgtg ctggg 25
<210> 200
   <211> 25
   <212> DNA
   <213> Artificial Sequence
<220>
   <223> synthetic nucleotide probe
<400> 200
   gctttgaagc accttccctg agaag 25
<210> 201
   <211> 25
   <212> DNA
   <213> Artificial Sequence
<220>
   <223> synthetic nucleotide probe
<400> 201
   tctgctttca catctctgag ctata 25
<210> 202
   <211> 25
   <212> DNA
   <213> Artificial Sequence
<220>
   <223> synthetic nucleotide probe
<400> 202
   taatgttgcc tggggcttaa cccac 25
<210> 203
   <211> 25
   <212> DNA
   <213> Artificial Sequence
<220>
   <223> synthetic nucleotide probe
<400> 203
   gctgaagagg cggaagaaag ctcgg 25
<210> 204
   <211> 25
   <212> DNA
   <213> Artificial Sequence
<220>
   <223> synthetic nucleotide probe
<400> 204
   ctcctgccca cgggaggaca agcaa 25
<210> 205
   <211> 25
   <212> DNA
   <213> Artificial Sequence
<220>
   <223> synthetic nucleotide probe
<400> 205
   cctgcccacg ggaggacaag caata 25
<210> 206
   <211> 25
   <212> DNA
   <213> Artificial Sequence
<220>
   <223> synthetic nucleotide probe
<400> 206
   ggacaagcaa taactctcta cagga 25
<210> 207
   <211> 25
   <212> DNA
   <213> Artificial Sequence
<220>
   <223> synthetic nucleotide probe
<400> 207
   aactctctac aggaatatat ttttt 25
<210> 208
   <211> 25
   <212> DNA
   <213> Artificial Sequence
<220>
   <223> synthetic nucleotide probe
<400> 208
   gttgactacg agcagatcaa gataa 25
<210> 209
   <211> 25
   <212> DNA
   <213> Artificial Sequence
<220>
   <223> synthetic nucleotide probe
<400> 209
   aatgcgcgtt gactacgagc agatc 25
<210> 210
   <211> 25
   <212> DNA
   <213> Artificial Sequence
<220>
   <223> synthetic nucleotide probe
<400> 210
   cacaatgcgc gttgactacg agcag 25
<210> 211
   <211> 25
   <212> DNA
   <213> Artificial Sequence
<220>
   <223> synthetic nucleotide probe
<400> 211
   gcgcgttgac tacgagcaga tcaag 25
<210> 212
   <211> 25
   <212> DNA
   <213> Artificial Sequence
<220>
   <223> synthetic nucleotide probe
<400> 212
   aagcaataac tctctacagg aatat 25
<210> 213
   <211> 25
   <212> DNA
   <213> Artificial Sequence
<220>
   <223> synthetic nucleotide probe
<400> 213
   agacagaact gtccacatct gcctc 25
<210> 214
   <211> 25
   <212> DNA
   <213> Artificial Sequence
<220>
   <223> synthetic nucleotide probe
<400> 214
   aatctttgag acaagcatat gctac 25
<210> 215
   <211> 25
   <212> DNA
   <213> Artificial Sequence
<220>
   <223> synthetic nucleotide probe
<400> 215
   cggccgtgcg tacttagaca tgcat 25
<210> 216
   <211> 25
   <212> DNA
   <213> Artificial Sequence
<220>
   <223> synthetic nucleotide probe
<400> 216
   ccattcgcag tttcactgta ccggc 25
<210> 217
   <211> 25
   <212> DNA
   <213> Artificial Sequence
<220>
   <223> synthetic nucleotide probe
<400> 217
   gtgcgtactt agacatgcat ggctt 25
<210> 218
   <211> 25
   <212> DNA
   <213> Artificial Sequence
<220>
   <223> synthetic nucleotide probe
<400> 218
   ggagcgagcg accaaaggaa ccata 25
<210> 219
   <211> 25
   <212> DNA
   <213> Artificial Sequence
<220>
   <223> synthetic nucleotide probe
<400> 219
   gcatatgcta ctggcaggat caacc 25
<210> 220
   <211> 25
   <212> DNA
   <213> Artificial Sequence
<220>
   <223> synthetic nucleotide probe
<400> 220
   aaccataact gatttaatga gccat 25
<210> 221
   <211> 25
   <212> DNA
   <213> Artificial Sequence
<220>
   <223> synthetic nucleotide probe
<400> 221
   gacatgcatg gcttaatctt tgaga 25
<210> 222
   <211> 25
   <212> DNA
   <213> Artificial Sequence
<220>
   <223> synthetic nucleotide probe
<400> 222
   gagcgaccaa aggaaccata actga 25
<210> 223
   <211> 25
   <212> DNA
   <213> Artificial Sequence
<220>
   <223> synthetic nucleotide probe
<400> 223
   caagtaggag aggagcgagc gacca 25
<210> 224
   <211> 25
   <212> DNA
   <213> Artificial Sequence
<220>
   <223> synthetic nucleotide probe
<400> 224
   aatgagccat tcgcagtttc actgt 25
<210> 225
   <211> 25
   <212> DNA
   <213> Artificial Sequence
<220>
   <223> synthetic nucleotide probe
<400> 225
   tctctaatcc catcctgagg ttgcc 25
<210> 226
   <211> 25
   <212> DNA
   <213> Artificial Sequence
<220>
   <223> synthetic nucleotide probe
<400> 226
   ggaagcttca tctgaccaat gtggg 25
<210> 227
   <211> 25
   <212> DNA
   <213> Artificial Sequence
<220>
   <223> synthetic nucleotide probe
<400> 227
   aaatgcaagg gtcttaccct cctct 25
<210> 228
   <211> 25
   <212> DNA
   <213> Artificial Sequence
<220>
   <223> synthetic nucleotide probe
<400> 228
   ccacccaccc aggtgtctaa gatag 25
<210> 229
   <211> 25
   <212> DNA
   <213> Artificial Sequence
<220>
   <223> synthetic nucleotide probe
<400> 229
   gcaaagccaa tatgaccact actga 25
<210> 230
   <211> 25
   <212> DNA
   <213> Artificial Sequence
<220>
   <223> synthetic nucleotide probe
<400> 230
   atcccctgaa tgtgaattgc tatcc 25
<210> 231
   <211> 25
   <212> DNA
   <213> Artificial Sequence
<220>
   <223> synthetic nucleotide probe
<400> 231
   agataggaca tgctcctttc tttct 25
<210> 232
   <211> 25
   <212> DNA
   <213> Artificial Sequence
<220>
   <223> synthetic nucleotide probe
<400> 232
   ttgctatcct tattgcccta ttaaa 25
<210> 233
   <211> 25
   <212> DNA
   <213> Artificial Sequence
<220>
   <223> synthetic nucleotide probe
<400> 233
   tggtatggtg aaactaatcc cctga 25
<210> 234
   <211> 25
   <212> DNA
   <213> Artificial Sequence
<220>
   <223> synthetic nucleotide probe
<400> 234
   ttgccatccc ccaaatgtgt ggtat 25
<210> 235
   <211> 25
   <212> DNA
   <213> Artificial Sequence
<220>
   <223> synthetic nucleotide probe
<400> 235
   cttgtgaaat gtgtccctaa gcctc 25
<210> 236
   <211> 25
   <212> DNA
   <213> Artificial Sequence
<220>
   <223> synthetic nucleotide probe
<400> 236
   taccctccaa ttcagactca gctga 25
<210> 237
   <211> 25
   <212> DNA
   <213> Artificial Sequence
<220>
   <223> synthetic nucleotide probe
<400> 237
   cagaacttca aatacttcct accct 25
<210> 238
   <211> 25
   <212> DNA
   <213> Artificial Sequence
<220>
   <223> synthetic nucleotide probe
<400> 238
   cctgccccaa ggaatcgtgc gggag 25
<210> 239
   <211> 25
   <212> DNA
   <213> Artificial Sequence
<220>
   <223> synthetic nucleotide probe
<400> 239
   gacagctggg tctccaagtg gcagc 25
<210> 240
   <211> 25
   <212> DNA
   <213> Artificial Sequence
<220>
   <223> synthetic nucleotide probe
<400> 240
   atcttctttg gactacaggt ggggt 25
<210> 241
   <211> 25
   <212> DNA
   <213> Artificial Sequence
<220>
   <223> synthetic nucleotide probe
<400> 241
   taggatgctg attttcctac ccgtg 25
<210> 242
   <211> 25
   <212> DNA
   <213> Artificial Sequence
<220>
   <223> synthetic nucleotide probe
<400> 242
   gtatatgact gcactagctc ttcct 25
<210> 243
   <211> 25
   <212> DNA
   <213> Artificial Sequence
<220>
   <223> synthetic nucleotide probe
<400> 243
   gagagcagca catcatttta tcatt 25
<210> 244
   <211> 25
   <212> DNA
   <213> Artificial Sequence
<220>
   <223> synthetic nucleotide probe
<400> 244
   gtcgaggagt ggcctacaaa tccag 25
<210> 245
   <211> 25
   <212> DNA
   <213> Artificial Sequence
<220>
   <223> synthetic nucleotide probe
<400> 245
   tgcaaggctg ccagcatctt tgctc 25
<210> 246
   <211> 25
   <212> DNA
   <213> Artificial Sequence
<220>
   <223> synthetic nucleotide probe
<400> 246
   atatgcggtg tcagtcactg gtcgc 25
<210> 247
   <211> 25
   <212> DNA
   <213> Artificial Sequence
<220>
   <223> synthetic nucleotide probe
<400> 247
   aagactgtta tgatcgcttt gattt 25
<210> 248
   <211> 25
   <212> DNA
   <213> Artificial Sequence
<220>
   <223> synthetic nucleotide probe
<400> 248
   gcccatctgt cattatgtta ctgtc 25
<210> 249
   <211> 25
   <212> DNA
   <213> Artificial Sequence
<220>
   <223> synthetic nucleotide probe
<400> 249
   agggcgatgc ctgggtttac aaaag 25
<210> 250
   <211> 25
   <212> DNA
   <213> Artificial Sequence
<220>
   <223> synthetic nucleotide probe
<400> 250
   agctcttaac tatgtctctt tgtaa 25
<210> 251
   <211> 25
   <212> DNA
   <213> Artificial Sequence
<220>
   <223> synthetic nucleotide probe
<400> 251
   gattcttcca tcctgccgga tgagt 25
<210> 252
   <211> 25
   <212> DNA
   <213> Artificial Sequence
<220>
   <223> synthetic nucleotide probe
<400> 252
   gaatctaaat caagcccatc tgtca 25
<210> 253
   <211> 25
   <212> DNA
   <213> Artificial Sequence
<220>
   <223> synthetic nucleotide probe
<400> 253
   gagctatctt aagaccaata tctct 25
<210> 254
   <211> 25
   <212> DNA
   <213> Artificial Sequence
<220>
   <223> synthetic nucleotide probe
<400> 254
   ggaagacatc ctatctagct gcaag 25
<210> 255
   <211> 25
   <212> DNA
   <213> Artificial Sequence
<220>
   <223> synthetic nucleotide probe
<400> 255
   gtgtgggtgt gatacagcct acata 25
<210> 256
   <211> 25
   <212> DNA
   <213> Artificial Sequence
<220>
   <223> synthetic nucleotide probe
<400> 256
   atgtggtggg tatcaggagg cagcg 25
<210> 257
   <211> 25
   <212> DNA
   <213> Artificial Sequence
<220>
   <223> synthetic nucleotide probe
<400> 257
   ggaggcagcg gcttaagggc gatgc 25
<210> 258
   <211> 25
   <212> DNA
   <213> Artificial Sequence
<220>
   <223> synthetic nucleotide probe
<400> 258
   ttgtgcttct cacctttggg tggga 25
<210> 259
   <211> 25
   <212> DNA
   <213> Artificial Sequence
<220>
   <223> synthetic nucleotide probe
<400> 259
   ggatgcctgc gtgtggttta ggtgt 25
<210> 260
   <211> 25
   <212> DNA
   <213> Artificial Sequence
<220>
   <223> synthetic nucleotide probe
<400> 260
   tagcaggatg tctggctgca cctgg 25
<210> 261
   <211> 25
   <212> DNA
   <213> Artificial Sequence
<220>
   <223> synthetic nucleotide probe
<400> 261
   tctccactca gtaccttgga tcaga 25
<210> 262
   <211> 25
   <212> DNA
   <213> Artificial Sequence
<220>
   <223> synthetic nucleotide probe
<400> 262
   gagtcatgcg gattatccac tcgcc 25
<210> 263
   <211> 25
   <212> DNA
   <213> Artificial Sequence
<220>
   <223> synthetic nucleotide probe
<400> 263
   ctggcatgac tgtttgtttc tccaa 25
<210> 264
   <211> 25
   <212> DNA
   <213> Artificial Sequence
<220>
   <223> synthetic nucleotide probe
<400> 264
   ccccactctc ttatttgtgc attcg 25
<210> 265
   <211> 25
   <212> DNA
   <213> Artificial Sequence
<220>
   <223> synthetic nucleotide probe
<400> 265
   agcacttgat gaactcgggg tacta 25
<210> 266
   <211> 25
   <212> DNA
   <213> Artificial Sequence
<220>
   <223> synthetic nucleotide probe
<400> 266
   gccagtgtgt cttacttggt tgctg 25
<210> 267
   <211> 25
   <212> DNA
   <213> Artificial Sequence
<220>
   <223> synthetic nucleotide probe
<400> 267
   ccctcttggc gtgagttgcg tattc 25
<210> 268
   <211> 25
   <212> DNA
   <213> Artificial Sequence
<220>
   <223> synthetic nucleotide probe
<400> 268
   ttggttgctg aacatcttgc cacct 25
<210> 269
   <211> 25
   <212> DNA
   <213> Artificial Sequence
<220>
   <223> synthetic nucleotide probe
<400> 269
   gaaggaccga ggtctttcca ttcct 25
<210> 270
   <211> 25
   <212> DNA
   <213> Artificial Sequence
<220>
   <223> synthetic nucleotide probe
<400> 270
   gagtactaat cttttgccca gaggc 25
<210> 271
   <211> 25
   <212> DNA
   <213> Artificial Sequence
<220>
   <223> synthetic nucleotide probe
<400> 271
   agtgaaagtg acatcctggc cacct 25
<210> 272
   <211> 25
   <212> DNA
   <213> Artificial Sequence
<220>
   <223> synthetic nucleotide probe
<400> 272
   acagtggcat agactccttc acaca 25
<210> 273
   <211> 25
   <212> DNA
   <213> Artificial Sequence
<220>
   <223> synthetic nucleotide probe
<400> 273
   acagggacag tcttcattta cttgt 25
<210> 274
   <211> 25
   <212> DNA
   <213> Artificial Sequence
<220>
   <223> synthetic nucleotide probe
<400> 274
   tccattcctc gagcagatac tcttg 25
<210> 275
   <211> 25
   <212> DNA
   <213> Artificial Sequence
<220>
   <223> synthetic nucleotide probe
<400> 275
   gcaccagcaa ctacttcttt atatt 25
<210> 276
   <211> 25
   <212> DNA
   <213> Artificial Sequence
<220>
   <223> synthetic nucleotide probe
<400> 276
   aaaaggagag tgacacaccc ctcca 25
<210> 277
   <211> 25
   <212> DNA
   <213> Artificial Sequence
<220>
   <223> synthetic nucleotide probe
<400> 277
   cacctcacgc atttgatcac agact 25
<210> 278
   <211> 25
   <212> DNA
   <213> Artificial Sequence
<220>
   <223> synthetic nucleotide probe
<400> 278
   ccttcacaca tcactgtggc accag 25
<210> 279
   <211> 25
   <212> DNA
   <213> Artificial Sequence
<220>
   <223> synthetic nucleotide probe
<400> 279
   ccttcacctc agtggagctt ctgag 25
<210> 280
   <211> 25
   <212> DNA
   <213> Artificial Sequence
<220>
   <223> synthetic nucleotide probe
<400> 280
   ggctttgctt agtatcatgt ccatg 25
<210> 281
   <211> 25
   <212> DNA
   <213> Artificial Sequence
<220>
   <223> synthetic nucleotide probe
<400> 281
   tgtaccttcg ttcaaatatc ctcat 25
<210> 282
   <211> 25
   <212> DNA
   <213> Artificial Sequence
<220>
   <223> synthetic nucleotide probe
<400> 282
   catctgtcac tcactatatt cacaa 25
<210> 283
   <211> 25
   <212> DNA
   <213> Artificial Sequence
<220>
   <223> synthetic nucleotide probe
<400> 283
   gttttatact gctcaagatc gtcat 25
<210> 284
   <211> 25
   <212> DNA
   <213> Artificial Sequence
<220>
   <223> synthetic nucleotide probe
<400> 284
   gggatagaaa ggccacctct tcact 25
<210> 285
   <211> 25
   <212> DNA
   <213> Artificial Sequence
<220>
   <223> synthetic nucleotide probe
<400> 285
   aactgcagtc ttctgctagc caata 25
<210> 286
   <211> 25
   <212> DNA
   <213> Artificial Sequence
<220>
   <223> synthetic nucleotide probe
<400> 286
   actcattcta acattgctta cttaa 25
<210> 287
   <211> 25
   <212> DNA
   <213> Artificial Sequence
<220>
   <223> synthetic nucleotide probe
<400> 287
   cacctcttca ctctctatag aatat 25
<210> 288
   <211> 25
   <212> DNA
   <213> Artificial Sequence
<220>
   <223> synthetic nucleotide probe
<400> 288
   gctacatagc cctatcgaaa tgcga 25
<210> 289
   <211> 25
   <212> DNA
   <213> Artificial Sequence
<220>
   <223> synthetic nucleotide probe
<400> 289
   tcctcatgta attgccatct gtcac 25
<210> 290
   <211> 25
   <212> DNA
   <213> Artificial Sequence
<220>
   <223> synthetic nucleotide probe
<400> 290
   ttgcacatgt cactactggg gaggt 25
<210> 291
   <211> 25
   <212> DNA
   <213> Artificial Sequence
<220>
   <223> synthetic nucleotide probe
<400> 291
   cctctcaatc actactcttc ttgaa 25
<210> 292
   <211> 25
   <212> DNA
   <213> Artificial Sequence
<220>
   <223> synthetic nucleotide probe
<400> 292
   gttctcaaaa gcttaccagt gtgga 25
<210> 293
   <211> 25
   <212> DNA
   <213> Artificial Sequence
<220>
   <223> synthetic nucleotide probe
<400> 293
   gtgttcagtt ctgttacaca gtgca 25
<210> 294
   <211> 25
   <212> DNA
   <213> Artificial Sequence
<220>
   <223> synthetic nucleotide probe
<400> 294
   gagctgtctt tgtcgtggag atctg 25
<210> 295
   <211> 25
   <212> DNA
   <213> Artificial Sequence
<220>
   <223> synthetic nucleotide probe
<400> 295
   acacagtgca ttgccctttg ttggg 25
<210> 296
   <211> 25
   <212> DNA
   <213> Artificial Sequence
<220>
   <223> synthetic nucleotide probe
<400> 296
   acacatgctt gtcggaacgc tttct 25
<210> 297
   <211> 25
   <212> DNA
   <213> Artificial Sequence
<220>
   <223> synthetic nucleotide probe
<400> 297
   acttggtgtt agccacgctg tttac 25
<210> 298
   <211> 25
   <212> DNA
   <213> Artificial Sequence
<220>
   <223> synthetic nucleotide probe
<400> 298
   gtgtccgaag ttgagatggc ctgcc 25
<210> 299
   <211> 25
   <212> DNA
   <213> Artificial Sequence
<220>
   <223> synthetic nucleotide probe
<400> 299
   gggagtctgt ttgttccaat gggtt 25
<210> 300
   <211> 25
   <212> DNA
   <213> Artificial Sequence
<220>
   <223> synthetic nucleotide probe
<400> 300
   ggagatctgg aactttgcac atgtc 25
<210> 301
   <211> 25
   <212> DNA
   <213> Artificial Sequence
<220>
   <223> synthetic nucleotide probe
<400> 301
   gatgagcagt cacactgtta ctcca 25
<210> 302
   <211> 25
   <212> DNA
   <213> Artificial Sequence
<220>
   <223> synthetic nucleotide probe
<400> 302
   gcactctcta aatcttcctt gtgag 25
<210> 303
   <211> 25
   <212> DNA
   <213> Artificial Sequence
<220>
   <223> synthetic nucleotide probe
<400> 303
   ggattatggg tcctgcaatt ctaca 25
<210> 304
   <211> 25
   <212> DNA
   <213> Artificial Sequence
<220>
   <223> synthetic nucleotide probe
<400> 304
   gaaaggatgt tttgtcccat ttcct 25
<210> 305
   <211> 25
   <212> DNA
   <213> Artificial Sequence
<220>
   <223> synthetic nucleotide probe
<400> 305
   aattgccaag gcagcgggat ctcgt 25
<210> 306
   <211> 25
   <212> DNA
   <213> Artificial Sequence
<220>
   <223> synthetic nucleotide probe
<400> 306
   tcctctctga gactgctaag taggg 25
<210> 307
   <211> 25
   <212> DNA
   <213> Artificial Sequence
<220>
   <223> synthetic nucleotide probe
<400> 307
   tgctcaacca ctgtggcgtt ctgct 25
<210> 308
   <211> 25
   <212> DNA
   <213> Artificial Sequence
<220>
   <223> synthetic nucleotide probe
<400> 308
   tgattgacac acagcactct ctaaa 25
<210> 309
   <211> 25
   <212> DNA
   <213> Artificial Sequence
<220>
   <223> synthetic nucleotide probe
<400> 309
   ctggacacta gggtcaggcg gggtg 25
<210> 310
   <211> 25
   <212> DNA
   <213> Artificial Sequence
<220>
   <223> synthetic nucleotide probe
<400> 310
   agaggcccag gaatcggagc gaagc 25
<210> 311
   <211> 25
   <212> DNA
   <213> Artificial Sequence
<220>
   <223> synthetic nucleotide probe
<400> 311
   ggggcagtga tggttgccag gacga 25
<210> 312
   <211> 25
   <212> DNA
   <213> Artificial Sequence
<220>
   <223> synthetic nucleotide probe
<400> 312
   tcatgcagca actcagctcg tcaat 25
<210> 313
   <211> 25
   <212> DNA
   <213> Artificial Sequence
<220>
   <223> synthetic nucleotide probe
<400> 313
   atgttcaaaa ttccgcttca agcct 25
<210> 314
   <211> 25
   <212> DNA
   <213> Artificial Sequence
<220>
   <223> synthetic nucleotide probe
<400> 314
   aaagcgctac tcgattaaca ggtgg 25
<210> 315
   <211> 25
   <212> DNA
   <213> Artificial Sequence
<220>
   <223> synthetic nucleotide probe
<400> 315
   actcatcagc ctttgtgcaa ctgtg 25
<210> 316
   <211> 25
   <212> DNA
   <213> Artificial Sequence
<220>
   <223> synthetic nucleotide probe
<400> 316
   gaacatttgc aactcatcag ccttt 25
<210> 317
   <211> 25
   <212> DNA
   <213> Artificial Sequence
<220>
   <223> synthetic nucleotide probe
<400> 317
   tcaacagcag cgattactag acatt 25
<210> 318
   <211> 25
   <212> DNA
   <213> Artificial Sequence
<220>
   <223> synthetic nucleotide probe
<400> 318
   acccctgacc tctagatgtt caaaa 25
<210> 319
   <211> 25
   <212> DNA
   <213> Artificial Sequence
<220>
   <223> synthetic nucleotide probe
<400> 319
   gtgatgcagc agttatctca gaatt 25
<210> 320
   <211> 25
   <212> DNA
   <213> Artificial Sequence
<220>
   <223> synthetic nucleotide probe
<400> 320
   gatggagtat ttgctgcctg tcaga 25
<210> 321
   <211> 25
   <212> DNA
   <213> Artificial Sequence
<220>
   <223> synthetic nucleotide probe
<400> 321
   aagccattac atttcttcaa agcgc 25
<210> 322
   <211> 25
   <212> DNA
   <213> Artificial Sequence
<220>
   <223> synthetic nucleotide probe
<400> 322
   tcagctcgtc aatcaactcc atgat 25
<210> 323
   <211> 25
   <212> DNA
   <213> Artificial Sequence
<220>
   <223> synthetic nucleotide probe
<400> 323
   gtgacaatca ttttgctgac agaat 25
<210> 324
   <211> 25
   <212> DNA
   <213> Artificial Sequence
<220>
   <223> synthetic nucleotide probe
<400> 324
   aagggcattt tctttgattc tcaaa 25
<210> 325
   <211> 25
   <212> DNA
   <213> Artificial Sequence
<220>
   <223> synthetic nucleotide probe
<400> 325
   ggagccatca tatgtcacag tgttc 25
<210> 326
   <211> 25
   <212> DNA
   <213> Artificial Sequence
<220>
   <223> synthetic nucleotide probe
<400> 326
   agagaaacgt gtgccctata cttcc 25
<210> 327
   <211> 25
   <212> DNA
   <213> Artificial Sequence
<220>
   <223> synthetic nucleotide probe
<400> 327
   gaaatccatc tatctacagc ctaag 25
<210> 328
   <211> 25
   <212> DNA
   <213> Artificial Sequence
<220>
   <223> synthetic nucleotide probe
<400> 328
   tagctcactg tcactcactg aatag 25
<210> 329
   <211> 25
   <212> DNA
   <213> Artificial Sequence
<220>
   <223> synthetic nucleotide probe
<400> 329
   gagacaccac ttttcaaagg acttc 25
<210> 330
   <211> 25
   <212> DNA
   <213> Artificial Sequence
<220>
   <223> synthetic nucleotide probe
<400> 330
   agttctttcc agtgttttgt agctc 25
<210> 331
   <211> 25
   <212> DNA
   <213> Artificial Sequence
<220>
   <223> synthetic nucleotide probe
<400> 331
   ggacttcttg gtttcagcat aacct 25
<210> 332
   <211> 25
   <212> DNA
   <213> Artificial Sequence
<220>
   <223> synthetic nucleotide probe
<400> 332
   gagaagccta tacatttagc tgaca 25
<210> 333
   <211> 25
   <212> DNA
   <213> Artificial Sequence
<220>
   <223> synthetic nucleotide probe
<400> 333
   tgccctatac ttcctgtgac aatca 25
<210> 334
   <211> 25
   <212> DNA
   <213> Artificial Sequence
<220>
   <223> synthetic nucleotide probe
<400> 334
   cttcccccac taaggtttga gacag 25
<210> 335
   <211> 25
   <212> DNA
   <213> Artificial Sequence
<220>
   <223> synthetic nucleotide probe
<400> 335
   gacgcaggac gagctcagtt gtaga 25
<210> 336
   <211> 25
   <212> DNA
   <213> Artificial Sequence
<220>
   <223> synthetic nucleotide probe

<400> 336
   gacgtatcca gcaagcggaa cctct 25
<210> 337
   <211> 25
   <212> DNA
   <213> Artificial Sequence
<220>
   <223> synthetic nucleotide probe
<400> 337
   ttcaatatcc cagtaaaccc atgta 25
<210> 338
   <211> 25
   <212> DNA
   <213> Artificial Sequence
<220>
   <223> synthetic nucleotide probe
<400> 338
   agcagtgata ccaaccacat ctgaa 25
<210> 339
   <211> 25
   <212> DNA
   <213> Artificial Sequence
<220>
   <223> synthetic nucleotide probe
<400> 339
   cttgagacca ggagacgtat ccagc 25
<210> 340
   <211> 25
   <212> DNA
   <213> Artificial Sequence
<220>
   <223> synthetic nucleotide probe
<400> 340
   actgatcatc ataactctgt atctg 25
<210> 341
   <211> 25
   <212> DNA
   <213> Artificial Sequence
<220>
   <223> synthetic nucleotide probe
<400> 341
   gaacccaagc tcaagacgca ggacg 25
<210> 342
   <211> 25
   <212> DNA
   <213> Artificial Sequence
<220>
   <223> synthetic nucleotide probe
<400> 342
   gcaagcggaa cctctgggaa aagca 25
<210> 343
   <211> 25
   <212> DNA
   <213> Artificial Sequence
<220>
   <223> synthetic nucleotide probe
<400> 343
   gcggaatgtg tgcagtatct agaaa 25
<210> 344
   <211> 25
   <212> DNA
   <213> Artificial Sequence
<220>
   <223> synthetic nucleotide probe
<400> 344
   tctgtggata aggtcacttc cccca 25
<210> 345
   <211> 25
   <212> DNA
   <213> Artificial Sequence
<220>
   <223> synthetic nucleotide probe
<400> 345
   ggttggtgca gcagtcatta aaagt 25
<210> 346
   <211> 25
   <212> DNA
   <213> Artificial Sequence
<220>
   <223> synthetic nucleotide probe
<400> 346
   gagtcaaggc cagactagat cagcc 25
<210> 347
   <211> 25
   <212> DNA
   <213> Artificial Sequence
<220>
   <223> synthetic nucleotide probe
<400> 347
   ttctcatgga gcttcctttc tagag 25
<210> 348
   <211> 25
   <212> DNA
   <213> Artificial Sequence
<220>
   <223> synthetic nucleotide probe
<400> 348
   caaaggggcg tgtcatgtgc ctcat 25
<210> 349
   <211> 25
   <212> DNA
   <213> Artificial Sequence
<220>
   <223> synthetic nucleotide probe
<400> 349
   caaggccaga ctagatcagc ctaag 25
<210> 350
   <211> 25
   <212> DNA
   <213> Artificial Sequence
<220>
   <223> synthetic nucleotide probe
<400> 350
   catggagctt cctttctaga gggga 25
<210> 351
   <211> 25
   <212> DNA
   <213> Artificial Sequence
<220>
   <223> synthetic nucleotide probe
<400> 351
   ctctattctc atggagcttc ctttc 25
<210> 352
   <211> 25
   <212> DNA
   <213> Artificial Sequence
<220>
   <223> synthetic nucleotide probe
<400> 352
   atttgagtag atttggcctc tattc 25
<210> 353
   <211> 25
   <212> DNA
   <213> Artificial Sequence
<220>
   <223> synthetic nucleotide probe
<400> 353
   gactttcaaa ggggcgtgtc atgtg 25
<210> 354
   <211> 25
   <212> DNA
   <213> Artificial Sequence
<220>
   <223> synthetic nucleotide probe
<400> 354
   ttggcctcta ttctcatgga gcttc 25
<210> 355
   <211> 25
   <212> DNA
   <213> Artificial Sequence
<220>
   <223> synthetic nucleotide probe
<400> 355
   gattctaata ggttggtgca gcagt 25
<210> 356
   <211> 25
   <212> DNA
   <213> Artificial Sequence
<220>
   <223> synthetic nucleotide probe
<400> 356
   gctacggcat catggggctg tacgt 25
<210> 357
   <211> 25
   <212> DNA
   <213> Artificial Sequence
<220>
   <223> synthetic nucleotide probe
<400> 357
   atcatggggc tgtacgtgtc catcg 25
<210> 358
   <211> 25
   <212> DNA
   <213> Artificial Sequence
<220>
   <223> synthetic nucleotide probe
<400> 358
   cattatgttc gaggagctgc cgtgc 25
<210> 359
   <211> 25
   <212> DNA
   <213> Artificial Sequence
<220>
   <223> synthetic nucleotide probe
<400> 359
   gctggcgccc gagagggaag gagcc 25
<210> 360
   <211> 25
   <212> DNA
   <213> Artificial Sequence
<220>
   <223> synthetic nucleotide probe
<400> 360
   gctggtcatc ggcaagttcg tgcgc 25
<210> 361
   <211> 25
   <212> DNA
   <213> Artificial Sequence
<220>
   <223> synthetic nucleotide probe
<400> 361
   gaggagttgt acgccaagct catct 25
<210> 362
   <211> 25
   <212> DNA
   <213> Artificial Sequence
<220>
   <223> synthetic nucleotide probe
<400> 362
   cgctcaccgg agaccatgat caagt 25
<210> 363
   <211> 25
   <212> DNA
   <213> Artificial Sequence
<220>
   <223> synthetic nucleotide probe
<400> 363
   gcggattctt cagcgagatc tcgca 25
<210> 364
   <211> 25
   <212> DNA
   <213> Artificial Sequence
<220>
   <223> synthetic nucleotide probe
<400> 364
   ttcgtgcgcg gattcttcag cgaga 25
<210> 365
   <211> 25
   <212> DNA
   <213> Artificial Sequence
<220>
   <223> synthetic nucleotide probe
<400> 365
   tcccccacgt gtactgtaga gtttt 25
<210> 366
   <211> 25
   <212> DNA
   <213> Artificial Sequence
<220>
   <223> synthetic nucleotide probe
<400> 366
   agatctcgca ctccattatg ttcga 25
<210> 367
   <211> 25
   <212> DNA
   <213> Artificial Sequence
<220>
   <223> synthetic nucleotide probe
<400> 367
   ggcccctggt ggaacagggc cgcgt 25
<210> 368
   <211> 25
   <212> DNA
   <213> Artificial Sequence
<220>
   <223> synthetic nucleotide probe
<400> 368
   tggtggaaga catgcagcgc cagtg 25
<210> 369
   <211> 25
   <212> DNA
   <213> Artificial Sequence
<220>
   <223> synthetic nucleotide probe
<400> 369
   gaagcgcctg gcagtgtacc aggcc 25
<210> 370
   <211> 25
   <212> DNA
   <213> Artificial Sequence
<220>
   <223> synthetic nucleotide probe
<400> 370
   agcaggccca gcagatacgc ctgca 25
<210> 371
   <211> 25
   <212> DNA
   <213> Artificial Sequence
<220>
   <223> synthetic nucleotide probe
<400> 371
   gtgcccagcg acaatcactg aacgc 25
<210> 372
   <211> 25
   <212> DNA
   <213> Artificial Sequence
<220>
   <223> synthetic nucleotide probe
<400> 372
   tggggcccct ggtggaacag ggccg 25
<210> 373
   <211> 25
   <212> DNA
   <213> Artificial Sequence
<220>
   <223> synthetic nucleotide probe
<400> 373
   aagcgcctgg cagtgtacca ggccg 25
<210> 374
   <211> 25
   <212> DNA
   <213> Artificial Sequence
<220>
   <223> synthetic nucleotide probe
<400> 374
   gcccagcgac aatcactgaa cgccg 25
<210> 375
   <211> 25
   <212> DNA
   <213> Artificial Sequence
<220>
   <223> synthetic nucleotide probe
<400> 375
   gcgcgcgcgg atggaggaga tgggc 25
<210> 376
   <211> 25
   <212> DNA
   <213> Artificial Sequence
<220>
   <223> synthetic nucleotide probe
<400> 376
   gcgacaatca ctgaacgccg aagcc 25
<210> 377
   <211> 25
   <212> DNA
   <213> Artificial Sequence
<220>
   <223> synthetic nucleotide probe
<400> 377
   ccctggtgga acagggccgc gtgcg 25
<210> 378
   <211> 25
   <212> DNA
   <213> Artificial Sequence
<220>
   <223> synthetic nucleotide probe
<400> 378
   cataagtcct ttcaattcca ccagg 25
<210> 379
   <211> 25
   <212> DNA
   <213> Artificial Sequence
<220>
   <223> synthetic nucleotide probe
<400> 379
   gctagacaat gatttggcca ggcct 25
<210> 380
   <211> 25
   <212> DNA
   <213> Artificial Sequence
<220>
   <223> synthetic nucleotide probe
<400> 380
   cagtgcatca catctgcaca ctctc 25
<210> 381
   <211> 25
   <212> DNA
   <213> Artificial Sequence
<220>
   <223> synthetic nucleotide probe
<400> 381
   ctgaccttgg aatcgtccct atatc 25
<210> 382
   <211> 25
   <212> DNA
   <213> Artificial Sequence
<220>
   <223> synthetic nucleotide probe
<400> 382
   tggaatcgtc cctatatcag ggcct 25
<210> 383
   <211> 25
   <212> DNA
   <213> Artificial Sequence
<220>
   <223> synthetic nucleotide probe
<400> 383
   gcagccccta agtgcaaaca cagca 25
<210> 384
   <211> 25
   <212> DNA
   <213> Artificial Sequence
<220>
   <223> synthetic nucleotide probe
<400> 384
   tctgcatata tgtctctttg gagtt 25
<210> 385
   <211> 25
   <212> DNA
   <213> Artificial Sequence
<220>
   <223> synthetic nucleotide probe
<400> 385
   gaaggctgga ttctatctac ataag 25
<210> 386
   <211> 25
   <212> DNA
   <213> Artificial Sequence
<220>
   <223> synthetic nucleotide probe
<400> 386
   gcccttaact atcaccagtg catca 25
<210> 387
   <211> 25
   <212> DNA
   <213> Artificial Sequence
<220>
   <223> synthetic nucleotide probe
<400> 387
   caccactgtg cacttagcca tgatg 25
<210> 388
   <211> 25
   <212> DNA
   <213> Artificial Sequence
<220>
   <223> synthetic nucleotide probe
<400> 388
   accacgaggc tgattcctct cattt 25
<210> 389
   <211> 25
   <212> DNA
   <213> Artificial Sequence
<220>
   <223> synthetic nucleotide probe
<400> 389
   tgcaaagtgg cagggaccgg cagag 25
<210> 390
   <211> 25
   <212> DNA
   <213> Artificial Sequence
<220>
   <223> synthetic nucleotide probe
<400> 390
   gcaaacacag catgggtcca gaaga 25
<210> 391
   <211> 25
   <212> DNA
   <213> Artificial Sequence
<220>
   <223> synthetic nucleotide probe
<400> 391
   gcatatatgt ctctttggag ttgga 25
<210> 392
   <211> 25
   <212> DNA
   <213> Artificial Sequence
<220>
   <223> synthetic nucleotide probe
<400> 392
   agacgtggtc tagaccaggg ctgct 25
<210> 393
   <211> 25
   <212> DNA
   <213> Artificial Sequence
<220>
   <223> synthetic nucleotide probe
<400> 393
   acttactgcc tgaccttgga atcgt 25
<210> 394
   <211> 25
   <212> DNA
   <213> Artificial Sequence
<220>
   <223> synthetic nucleotide probe
<400> 394
   ggcctagtaa ccaaggccct gtctc 25
<210> 395
   <211> 25
   <212> DNA
   <213> Artificial Sequence
<220>
   <223> synthetic nucleotide probe
<400> 395
   gcatgggtcc agaagacgtg gtcta 25
<210> 396
   <211> 25
   <212> DNA
   <213> Artificial Sequence
<220>
   <223> synthetic nucleotide probe
<400> 396
   gcatctgtct gctctgcata tatgt 25
<210> 397
   <211> 25
   <212> DNA
   <213> Artificial Sequence
<220>
   <223> synthetic nucleotide probe
<400> 397
   tctcagtttc tgcctgggca atggc 25
<210> 398
   <211> 25
   <212> DNA
   <213> Artificial Sequence
<220>
   <223> synthetic nucleotide probe
<400> 398
   ttactgcctg accttggaat cgtcc 25
<210> 399
   <211> 25
   <212> DNA
   <213> Artificial Sequence
<220>
   <223> synthetic nucleotide probe
<400> 399
   gcaggaactt ctagctcatt taaca 25
<210> 400
   <211> 25
   <212> DNA
   <213> Artificial Sequence
<220>
   <223> synthetic nucleotide probe
<400> 400
   actcctaatg tttggtgcta tgttt 25
<210> 401
   <211> 25
   <212> DNA
   <213> Artificial Sequence
<220>
   <223> synthetic nucleotide probe
<400> 401
   tggtgctatg ttttcctgag gagat 25
<210> 402
   <211> 25
   <212> DNA
   <213> Artificial Sequence
<220>
   <223> synthetic nucleotide probe
<400> 402
   gaagccatag cactcctaat gtttg 25
<210> 403
   <211> 25
   <212> DNA
   <213> Artificial Sequence
<220>
   <223> synthetic nucleotide probe
<400> 403
   aagagggctt atgaggctgt gaaac 25
<210> 404
   <211> 25
   <212> DNA
   <213> Artificial Sequence
<220>
   <223> synthetic nucleotide probe
<400> 404
   cccagagctc ttaacgctgt gacca 25
<210> 405
   <211> 25
   <212> DNA
   <213> Artificial Sequence
<220>
   <223> synthetic nucleotide probe
<400> 405
   gaggctgtga aacccagagc tctta 25
<210> 406
   <211> 25
   <212> DNA
   <213> Artificial Sequence
<220>
   <223> synthetic nucleotide probe
<400> 406
   tgctccattg gagtagtctc ccacc 25
<210> 407
   <211> 25
   <212> DNA
   <213> Artificial Sequence
<220>
   <223> synthetic nucleotide probe
<400> 407
   ggtagaggcc ttctaggtga gacac 25
<210> 408
   <211> 25
   <212> DNA
   <213> Artificial Sequence
<220>
   <223> synthetic nucleotide probe
<400> 408
   tacttatcta ctgtccgaag gcctt 25
<210> 409
   <211> 25
   <212> DNA
   <213> Artificial Sequence
<220>
   <223> synthetic nucleotide probe
<400> 409
   cctgcatttc ccattaatct acata 25
<210> 410
   <211> 25
   <212> DNA
   <213> Artificial Sequence
<220>
   <223> synthetic nucleotide probe
<400> 410
   aatgctgaga aatttgccac tggag 25
<210> 411
   <211> 25
   <212> DNA
   <213> Artificial Sequence
<220>
   <223> synthetic nucleotide probe
<400> 411
   tatacagttt aataagcctc ttgca 25
<210> 412
   <211> 25
   <212> DNA
   <213> Artificial Sequence
<220>
   <223> synthetic nucleotide probe
<400> 412
   atttaaaata ttgatccttc ccttg 25
<210> 413
   <211> 25
   <212> DNA
   <213> Artificial Sequence
<220>
   <223> synthetic nucleotide probe
<400> 413
   atctcacttg ttttagttct gatcc 25
<210> 414
   <211> 25
   <212> DNA
   <213> Artificial Sequence
<220>
   <223> synthetic nucleotide probe
<400> 414
   atttgggtcc aacttcaata atgta 25
<210> 415
   <211> 25
   <212> DNA
   <213> Artificial Sequence
<220>
   <223> synthetic nucleotide probe
<400> 415
   gactgtgggt caaatgtttc cattt 25
<210> 416
   <211> 25
   <212> DNA
   <213> Artificial Sequence
<220>
   <223> synthetic nucleotide probe
<400> 416
   aaatgaaact gttgctccat tggag 25
<210> 417
   <211> 25
   <212> DNA
   <213> Artificial Sequence
<220>
   <223> synthetic nucleotide probe
<400> 417
   gtatctgtaa ccacaatcac acata 25
<210> 418
   <211> 25
   <212> DNA
   <213> Artificial Sequence
<220>
   <223> synthetic nucleotide probe
<400> 418
   ggaccacctt catgttagtt gggta 25
<210> 419
   <211> 25
   <212> DNA
   <213> Artificial Sequence
<220>
   <223> synthetic nucleotide probe
<400> 419
   ttgcaagtta cttgttctct cacct 25
<210> 420
   <211> 25
   <212> DNA
   <213> Artificial Sequence
<220>
   <223> synthetic nucleotide probe
<400> 420
   ctttttgccc acactgcttt ggata 25
<210> 421
   <211> 25
   <212> DNA
   <213> Artificial Sequence
<220>
   <223> synthetic nucleotide probe
<400> 421
   agatcacccc tctaattatt tctga 25
<210> 422
   <211> 25
   <212> DNA
   <213> Artificial Sequence
<220>
   <223> synthetic nucleotide probe
<400> 422
   tatttcctcc ataataaccc tgcat 25
<210> 423
   <211> 25
   <212> DNA
   <213> Artificial Sequence
<220>
   <223> synthetic nucleotide probe
<400> 423
   gggatgttgc ttactctttt tgccc 25
<210> 424
   <211> 25
   <212> DNA
   <213> Artificial Sequence
<220>
   <223> synthetic nucleotide probe
<400> 424
   gtactatgtg actcatgctt ctgga 25
<210> 425
   <211> 25
   <212> DNA
   <213> Artificial Sequence
<220>
   <223> synthetic nucleotide probe
<400> 425
   gttctctcac ctgaggtatt ttttt 25
<210> 426
   <211> 25
   <212> DNA
   <213> Artificial Sequence
<220>
   <223> synthetic nucleotide probe
<400> 426
   gccactggag acaagcaatc tgaat 25
<210> 427
   <211> 25
   <212> DNA
   <213> Artificial Sequence
<220>
   <223> synthetic nucleotide probe
<400> 427
   tcatcctgtg agttatttcc tccat 25
<210> 428
   <211> 25
   <212> DNA
   <213> Artificial Sequence
<220>
   <223> synthetic nucleotide probe
<400> 428
   tgcaactagc aactcatctt cggaa 25
<210> 429
   <211> 25
   <212> DNA
   <213> Artificial Sequence
<220>
   <223> synthetic nucleotide probe
<400> 429
   ctgcccatag tcaccaaatt ctgtt 25
<210> 430
   <211> 25
   <212> DNA
   <213> Artificial Sequence
<220>
   <223> synthetic nucleotide probe
<400> 430
   tggaaaagga ttctctgcct cgctt 25
<210> 431
   <211> 25
   <212> DNA
   <213> Artificial Sequence
<220>
   <223> synthetic nucleotide probe
<400> 431
   gctaattgtc ctatgatgct attat 25
<210> 432
   <211> 25
   <212> DNA
   <213> Artificial Sequence
<220>
   <223> synthetic nucleotide probe
<400> 432
   ttcctcttct ccctttgcaa gagga 25
<210> 433
   <211> 25
   <212> DNA
   <213> Artificial Sequence
<220>
   <223> synthetic nucleotide probe
<400> 433
   atgacattta tcttcaaaac accaa 25
<210> 434
   <211> 25
   <212> DNA
   <213> Artificial Sequence
<220>
   <223> synthetic nucleotide probe
<400> 434
   gagtagtctc ccacctaaat atcaa 25
<210> 435
   <211> 25
   <212> DNA
   <213> Artificial Sequence
<220>
   <223> synthetic nucleotide probe
<400> 435
   ttcccacagc agctttgctc agtga 25
<210> 436
   <211> 25
   <212> DNA
   <213> Artificial Sequence
<220>
   <223> synthetic nucleotide probe
<400> 436
   ctcgctttgt gcgctctgag tttta 25
<210> 437
   <211> 25
   <212> DNA
   <213> Artificial Sequence
<220>
   <223> synthetic nucleotide probe
<400> 437
   atccatttgt aagcatttat cccat 25
<210> 438
   <211> 25
   <212> DNA
   <213> Artificial Sequence
<220>
   <223> synthetic nucleotide probe
<400> 438
   atgtatttat gctgctagac tgtgg 25
<210> 439
   <211> 25
   <212> DNA
   <213> Artificial Sequence
<220>
   <223> synthetic nucleotide probe
<400> 439
   tcttcaccac agacaccttc ttgtg 25
<210> 440
   <211> 25
   <212> DNA
   <213> Artificial Sequence
<220>
   <223> synthetic nucleotide probe
<400> 440
   gagcctaaca ctatcctgta attca 25
<210> 441
   <211> 25
   <212> DNA
   <213> Artificial Sequence
<220>
   <223> synthetic nucleotide probe
<400> 441
   gtccctgtct atacattctc tgtat 25
<210> 442
   <211> 25
   <212> DNA
   <213> Artificial Sequence
<220>
   <223> synthetic nucleotide probe
<400> 442
   taacctttgt aatgttcttc accac 25
<210> 443
   <211> 25
   <212> DNA
   <213> Artificial Sequence
<220>
   <223> synthetic nucleotide probe
<400> 443
   actctgctca gccctgtaac agggt 25
<210> 444
   <211> 25
   <212> DNA
   <213> Artificial Sequence
<220>
   <223> synthetic nucleotide probe
<400> 444
   ttttacttac ccatgtgagc ctaac 25
<210> 445
   <211> 25
   <212> DNA
   <213> Artificial Sequence
<220>
   <223> synthetic nucleotide probe
<400> 445
   ttcattgcct ttttcaccta agcat 25
<210> 446
   <211> 25
   <212> DNA
   <213> Artificial Sequence
<220>
   <223> synthetic nucleotide probe
<400> 446
   ttctctgtat cttttggggg taact 25
<210> 447
   <211> 25
   <212> DNA
   <213> Artificial Sequence
<220>
   <223> synthetic nucleotide probe
<400> 447
   aggaagagct ttgacttgtc cctgt 25
<210> 448
   <211> 25
   <212> DNA
   <213> Artificial Sequence
<220>
   <223> synthetic nucleotide probe
<400> 448
   gtttttcagt gttcagccat gtcag 25
<210> 449
   <211> 25
   <212> DNA
   <213> Artificial Sequence
<220>
   <223> synthetic nucleotide probe
<400> 449
   attatgatca tctaccacca actct 25
<210> 450
   <211> 25
   <212> DNA
   <213> Artificial Sequence
<220>
   <223> synthetic nucleotide probe
<400> 450
   gcaggggatg gcctgatcga gctgc 25
<210> 451
   <211> 25
   <212> DNA
   <213> Artificial Sequence
<220>
   <223> synthetic nucleotide probe
<400> 451
   tgagcgacga ccttacagag cgagc 25
<210> 452
   <211> 25
   <212> DNA
   <213> Artificial Sequence
<220>
   <223> synthetic nucleotide probe
<400> 452
   gaccttcggg aaggagttca cagaa 25
<210> 453
   <211> 25
   <212> DNA
   <213> Artificial Sequence
<220>
   <223> synthetic nucleotide probe
<400> 453
   gagttcacag aagcggtgga agcca 25
<210> 454
   <211> 25
   <212> DNA
   <213> Artificial Sequence
<220>
   <223> synthetic nucleotide probe
<400> 454
   cagccccgat gattcttaac acagc 25
<210> 455
   <211> 25
   <212> DNA
   <213> Artificial Sequence
<220>
   <223> synthetic nucleotide probe
<400> 455
   gcaggtgagc gacgacctta cagag 25
<210> 456
   <211> 25
   <212> DNA
   <213> Artificial Sequence
<220>
   <223> synthetic nucleotide probe
<400> 456
   caggggatgg cctgatcgag ctgcg 25
<210> 457
   <211> 25
   <212> DNA
   <213> Artificial Sequence
<220>
   <223> synthetic nucleotide probe
<400> 457
   gagcaacaga aaaaggcggc catca 25
<210> 458
   <211> 25
   <212> DNA
   <213> Artificial Sequence
<220>
   <223> synthetic nucleotide probe
<400> 458
   tcctggatga cgtgtccttg acaca 25
<210> 459
   <211> 25
   <212> DNA
   <213> Artificial Sequence
<220>
   <223> synthetic nucleotide probe
<400> 459
   tcgggaagga gttcacagaa gcggt 25
<210> 460
   <211> 25
   <212> DNA
   <213> Artificial Sequence
<220>
   <223> synthetic nucleotide probe
<400> 460
   tggatgacgt gtccttgaca catct 25
<210> 461
   <211> 25
   <212> DNA
   <213> Artificial Sequence
<220>
   <223> synthetic nucleotide probe
<400> 461
   tgttgaactt gtatccttca gcctt 25
<210> 462
   <211> 25
   <212> DNA
   <213> Artificial Sequence
<220>
   <223> synthetic nucleotide probe
<400> 462
   taatattgag tcttctggcc tataa 25
<210> 463
   <211> 25
   <212> DNA
   <213> Artificial Sequence
<220>
   <223> synthetic nucleotide probe
<400> 463
   ggtctgtctt cctaggtatt aatgt 25
<210> 464
   <211> 25
   <212> DNA
   <213> Artificial Sequence
<220>
   <223> synthetic nucleotide probe
<400> 464
   agttttcagt gtacaggtct accat 25
<210> 465
   <211> 25
   <212> DNA
   <213> Artificial Sequence
<220>
   <223> synthetic nucleotide probe
<400> 465
   gccttgctaa actgtgagtt ctcat 25
<210> 466
   <211> 25
   <212> DNA
   <213> Artificial Sequence
<220>
   <223> synthetic nucleotide probe
<400> 466
   ggcctataaa caaggtctgt cttcc 25
<210> 467
   <211> 25
   <212> DNA
   <213> Artificial Sequence
<220>
   <223> synthetic nucleotide probe
<400> 467
   gtagtcacag ttacacggca ggctg 25
<210> 468
   <211> 25
   <212> DNA
   <213> Artificial Sequence
<220>
   <223> synthetic nucleotide probe
<400> 468
   gttgggcacc ttgattgaga ttgca 25
<210> 469
   <211> 25
   <212> DNA
   <213> Artificial Sequence
<220>
   <223> synthetic nucleotide probe
<400> 469
   aattctaacc acttgttgct agtaa 25
<210> 470
   <211> 25
   <212> DNA
   <213> Artificial Sequence
<220>
   <223> synthetic nucleotide probe
<400> 470
   aggtctacca tgtcagcatt tcata 25
<210> 471
   <211> 25
   <212> DNA
   <213> Artificial Sequence
<220>
   <223> synthetic nucleotide probe
<400> 471
   aatggtgcca tattgtcact ccttc 25
<210> 472
   <211> 25
   <212> DNA
   <213> Artificial Sequence
<220>
   <223> synthetic nucleotide probe
<400> 472
   accagcccag gcaacatagc gtaaa 25
<210> 473
   <211> 25
   <212> DNA
   <213> Artificial Sequence
<220>
   <223> synthetic nucleotide probe
<400> 473
   gtgtttgttc caattagctt tgttg 25
<210> 474
   <211> 25
   <212> DNA
   <213> Artificial Sequence
<220>
   <223> synthetic nucleotide probe
<400> 474
   taggttgtcc cctaaagatt ctgaa 25
<210> 475
   <211> 25
   <212> DNA
   <213> Artificial Sequence
<220>
   <223> synthetic nucleotide probe
<400> 475
   tgcttagatt gttgtactgc tgcca 25
<210> 476
   <211> 25
   <212> DNA
   <213> Artificial Sequence
<220>
   <223> synthetic nucleotide probe
<400> 476
   ttaaacggtt gataatgcct ctaca 25
<210> 477
   <211> 25
   <212> DNA
   <213> Artificial Sequence
<220>
   <223> synthetic nucleotide probe
<400> 477
   tattctaccc tcttttttgg caagg 25
<210> 478
   <211> 25
   <212> DNA
   <213> Artificial Sequence
<220>
   <223> synthetic nucleotide probe
<400> 478
   caagtcattg cattgtgttc taatt 25
<210> 479
   <211> 25
   <212> DNA
   <213> Artificial Sequence
<220>
   <223> synthetic nucleotide probe
<400> 479
   catagcgtaa accctatctc taaaa 25

<210> 480
   <211> 25
   <212> DNA
   <213> Artificial Sequence
<220>
   <223> synthetic nucleotide probe
<400> 480
   aaccttggat ggatatcttc tcttt 25
<210> 481
   <211> 25
   <212> DNA
   <213> Artificial Sequence
<220>
   <223> synthetic nucleotide probe
<400> 481
   attgttgtac tgctgccatt tttat 25
<210> 482
   <211> 25
   <212> DNA
   <213> Artificial Sequence
<220>
   <223> synthetic nucleotide probe
<400> 482
   cacagtggct tctcaggagg ctgag 25
<210> 483
   <211> 25
   <212> DNA
   <213> Artificial Sequence
<220>
   <223> synthetic nucleotide probe
<400> 483
   ggatagaatc atggtgggca cagtg 25
<210> 484
   <211> 25
   <212> DNA
   <213> Artificial Sequence
<220>
   <223> synthetic nucleotide probe
<400> 484
   tctccttgtt taccctggta ttcta 25
<210> 485
   <211> 25
   <212> DNA
   <213> Artificial Sequence
<220>
   <223> synthetic nucleotide probe
<400> 485
   aagtgtctag ttcttgctaa aatca 25
<210> 486
   <211> 25
   <212> DNA
   <213> Artificial Sequence
<220>
   <223> synthetic nucleotide probe
<400> 486
   tggagaattc tttaggttgt cccct 25
<210> 487
   <211> 25
   <212> DNA
   <213> Artificial Sequence
<220>
   <223> synthetic nucleotide probe
<400> 487
   ggagggaggt ttgcttgagt ccagg 25
<210> 488
   <211> 25
   <212> DNA
   <213> Artificial Sequence
<220>
   <223> synthetic nucleotide probe
<400> 488
   tggcaaggag gacaaatacg caatg 25
<210> 489
   <211> 25
   <212> DNA
   <213> Artificial Sequence
<220>
   <223> synthetic nucleotide probe
<400> 489
   tcatctttga ataacgtctc cttgt 25
<210> 490
   <211> 25
   <212> DNA
   <213> Artificial Sequence
<220>
   <223> synthetic nucleotide probe
<400> 490
   gataatgcct ctacaacaac aagaa 25
<210> 491
   <211> 25
   <212> DNA
   <213> Artificial Sequence
<220>
   <223> synthetic nucleotide probe
<400> 491
   tgaacttgat acgtccgtgt gtccc 25
<210> 492
   <211> 25
   <212> DNA
   <213> Artificial Sequence
<220>
   <223> synthetic nucleotide probe
<400> 492
   gggcagtttt gaggcatgac taatg 25
<210> 493
   <211> 25
   <212> DNA
   <213> Artificial Sequence
<220>
   <223> synthetic nucleotide probe
<400> 493
   aaaagcgagg tggccatgtt atgct 25
<210> 494
   <211> 25
   <212> DNA
   <213> Artificial Sequence
<220>
   <223> synthetic nucleotide probe
<400> 494
   taactataag gtgcctcagt tttcc 25
<210> 495
   <211> 25
   <212> DNA
   <213> Artificial Sequence
<220>
   <223> synthetic nucleotide probe
<400> 495
   agatgctgtc attagtctat atggt 25
<210> 496
   <211> 25
   <212> DNA
   <213> Artificial Sequence
<220>
   <223> synthetic nucleotide probe
<400> 496
   ggaattctca agacctgagt atttt 25
<210> 497
   <211> 25
   <212> DNA
   <213> Artificial Sequence
<220>
   <223> synthetic nucleotide probe
<400> 497
   ctgacctacc tcaaagggca gtttt 25
<210> 498
   <211> 25
   <212> DNA
   <213> Artificial Sequence
<220>
   <223> synthetic nucleotide probe
<400> 498
   acaacgcatt gccacggaaa catac 25
<210> 499
   <211> 25
   <212> DNA
   <213> Artificial Sequence
<220>
   <223> synthetic nucleotide probe
<400> 499
   agcattttgg gatccttcag cacag 25
<210> 500
   <211> 25
   <212> DNA
   <213> Artificial Sequence
<220>
   <223> synthetic nucleotide probe
<400> 500
   gaagctaatt gtactaatct gagat 25
<210> 501
   <211> 25
   <212> DNA
   <213> Artificial Sequence
<220>
   <223> synthetic nucleotide probe
<400> 501
   atgtccacca tgaacttgat acgtc 25
<210> 502
   <211> 25
   <212> DNA
   <213> Artificial Sequence
<220>
   <223> synthetic nucleotide probe
<400> 502
   cattctgtcg accctggatg ttgaa 25
<210> 503
   <211> 25
   <212> DNA
   <213> Artificial Sequence
<220>
   <223> synthetic nucleotide probe
<400> 503
   ttgagagttt tgacctgcct gaaga 25
<210> 504
   <211> 25
   <212> DNA
   <213> Artificial Sequence
<220>
   <223> synthetic nucleotide probe
<400> 504
   aattgccacc tgtttgctgt gacat 25
<210> 505
   <211> 25
   <212> DNA
   <213> Artificial Sequence
<220>
   <223> synthetic nucleotide probe
<400> 505
   gtgcctctca tgatccttga cgagg 25
<210> 506
   <211> 25
   <212> DNA
   <213> Artificial Sequence
<220>
   <223> synthetic nucleotide probe
<400> 506
   tgcagtctcc ttcaagcatt ctgtc 25
<210> 507
   <211> 25
   <212> DNA
   <213> Artificial Sequence
<220>
   <223> synthetic nucleotide probe
<400> 507
   cctgcctcag atgatgccta tccag 25
<210> 508
   <211> 25
   <212> DNA
   <213> Artificial Sequence
<220>
   <223> synthetic nucleotide probe
<400> 508
   aaaacagcca agcttttctg ccaaa 25
<210> 509
   <211> 25
   <212> DNA
   <213> Artificial Sequence
<220>
   <223> synthetic nucleotide probe
<400> 509
   gggaatccaa tctgttgcag tctcc 25
<210> 510
   <211> 25
   <212> DNA
   <213> Artificial Sequence
<220>
   <223> synthetic nucleotide probe
<400> 510
   tgaagagcac cagattgcgc acctc 25
<210> 511
   <211> 25
   <212> DNA
   <213> Artificial Sequence
<220>
   <223> synthetic nucleotide probe
<400> 511
   aagcaaaaag ctctgttcct gcctc 25
<210> 512
   <211> 25
   <212> DNA
   <213> Artificial Sequence
<220>
   <223> synthetic nucleotide probe
<400> 512
   ttcccttcaa tcctctagac tttga 25
<210> 513
   <211> 25
   <212> DNA
   <213> Artificial Sequence
<220>
   <223> synthetic nucleotide probe
<400> 513
   ggtcaaagtt gctcagcgga gccca 25
<210> 514
   <211> 25
   <212> DNA
   <213> Artificial Sequence
<220>
   <223> synthetic nucleotide probe
<400> 514
   tgcacagaag ttagcgctat cccca 25
<210> 515
   <211> 25
   <212> DNA
   <213> Artificial Sequence
<220>
   <223> synthetic nucleotide probe
<400> 515
   tacccctggg aggtgccagt cattg 25
<210> 516
   <211> 25
   <212> DNA
   <213> Artificial Sequence
<220>
   <223> synthetic nucleotide probe
<400> 516
   gtttggaagc actgatcacc tgtta 25
<210> 517
   <211> 25
   <212> DNA
   <213> Artificial Sequence
<220>
   <223> synthetic nucleotide probe
<400> 517
   gaaggcactt tgtgtgtcag taccc 25
<210> 518
   <211> 25
   <212> DNA
   <213> Artificial Sequence
<220>
   <223> synthetic nucleotide probe
<400> 518
   gatcacctgt tagcattgcc attcc 25
<210> 519
   <211> 25
   <212> DNA
   <213> Artificial Sequence
<220>
   <223> synthetic nucleotide probe
<400> 519
   gagcccagta gattcaggca ccatc 25
<210> 520
   <211> 25
   <212> DNA
   <213> Artificial Sequence
<220>
   <223> synthetic nucleotide probe
<400> 520
   gtactcttta gatctcccat gtgta 25
<210> 521
   <211> 25
   <212> DNA
   <213> Artificial Sequence
<220>
   <223> synthetic nucleotide probe
<400> 521
   tgagggtcaa gcgaggccga cttgt 25
<210> 522
   <211> 25
   <212> DNA
   <213> Artificial Sequence
<220>
   <223> synthetic nucleotide probe
<400> 522
   ttgccattcc tctactgcaa tgtaa 25
<210> 523
   <211> 25
   <212> DNA
   <213> Artificial Sequence
<220>
   <223> synthetic nucleotide probe
<400> 523
   cgaaatccgt cccagtcaat aatct 25
<210> 524
   <211> 25
   <212> DNA
   <213> Artificial Sequence
<220>
   <223> synthetic nucleotide probe
<400> 524
   ggacagtgtt tcaacaagcc taggc 25
<210> 525
   <211> 25
   <212> DNA
   <213> Artificial Sequence
<220>
   <223> synthetic nucleotide probe
<400> 525
   gcatctaagg gactttgttg aactt 25
<210> 526
   <211> 25
   <212> DNA
   <213> Artificial Sequence
<220>
   <223> synthetic nucleotide probe
<400> 526
   gatggcctct gatttacact ggttc 25
<210> 527
   <211> 25
   <212> DNA
   <213> Artificial Sequence
<220>
   <223> synthetic nucleotide probe
<400> 527
   agaagtctgc cctagctgtt aaatt 25
<210> 528
   <211> 25
   <212> DNA
   <213> Artificial Sequence
<220>
   <223> synthetic nucleotide probe
<400> 528
   gagttaattg ttcctttctt cagtg 25
<210> 529
   <211> 25
   <212> DNA
   <213> Artificial Sequence
<220>
   <223> synthetic nucleotide probe
<400> 529
   tagacagctt ggatcctttc tctga 25
<210> 530
   <211> 25
   <212> DNA
   <213> Artificial Sequence
<220>
   <223> synthetic nucleotide probe
<400> 530
   ggtttaccag gatgtagtcc cactg 25
<210> 531
   <211> 25
   <212> DNA
   <213> Artificial Sequence
<220>
   <223> synthetic nucleotide probe
<400> 531
   gaaaacactt agttcattgg cttta 25
<210> 532
   <211> 25
   <212> DNA
   <213> Artificial Sequence
<220>
   <223> synthetic nucleotide probe
<400> 532
   gcgtattttt acactattgg ctcaa 25
<210> 533
   <211> 25
   <212> DNA
   <213> Artificial Sequence
<220>
   <223> synthetic nucleotide probe
<400> 533
   atttacacag ctagatttgg aagat 25
<210> 534
   <211> 25
   <212> DNA
   <213> Artificial Sequence
<220>
   <223> synthetic nucleotide probe
<400> 534
   ggatgagatt gatgcagccc ttgat 25
<210> 535
   <211> 25
   <212> DNA
   <213> Artificial Sequence
<220>
   <223> synthetic nucleotide probe
<400> 535
   attgatgcag cccttgattt taaaa 25
<210> 536
   <211> 25
   <212> DNA
   <213> Artificial Sequence
<220>
   <223> synthetic nucleotide probe
<400> 536
   agttcataat aatttctctt cgaaa 25
<210> 537
   <211> 25
   <212> DNA
   <213> Artificial Sequence
<220>
   <223> synthetic nucleotide probe
<400> 537
   ggaaggactt tcggtattgt attag 25
<210> 538
   <211> 25
   <212> DNA
   <213> Artificial Sequence
<220>
   <223> synthetic nucleotide probe
<400> 538
   cctttcttca gtgggccatt gtttt 25
<210> 539
   <211> 25
   <212> DNA
   <213> Artificial Sequence
<220>
   <223> synthetic nucleotide probe
<400> 539
   ttagtatttg ctcttcacca ctaca 25
<210> 540
   <211> 25
   <212> DNA
   <213> Artificial Sequence
<220>
   <223> synthetic nucleotide probe
<400> 540
   gataccttga gtaatgtttg cctat 25
<210> 541
   <211> 25
   <212> DNA
   <213> Artificial Sequence
<220>
   <223> synthetic nucleotide probe
<400> 541
   cactcccctt tacttcatgg atgag 25
<210> 542
   <211> 25
   <212> DNA
   <213> Artificial Sequence
<220>
   <223> synthetic nucleotide probe
<400> 542
   aagcctaggc tatctcgtaa gttga 25
<210> 543
   <211> 25
   <212> DNA
   <213> Artificial Sequence
<220>
   <223> synthetic nucleotide probe
<400> 543
   ggacgccgaa ctcgagcttg tagac 25
<210> 544
   <211> 25
   <212> DNA
   <213> Artificial Sequence
<220>
   <223> synthetic nucleotide probe
<400> 544
   aatatcccac tatagttgct tcatg 25
<210> 545
   <211> 25
   <212> DNA
   <213> Artificial Sequence
<220>
   <223> synthetic nucleotide probe
<400> 545
   cccaatttct caatgaagat ctaag 25
<210> 546
   <211> 25
   <212> DNA
   <213> Artificial Sequence
<220>
   <223> synthetic nucleotide probe
<400> 546
   gattatgtcc agttatttgc tttaa 25
<210> 547
   <211> 25
   <212> DNA
   <213> Artificial Sequence
<220>
   <223> synthetic nucleotide probe
<400> 547
   ggtggaatcc tttaagatta tgtcc 25
<210> 548
   <211> 25
   <212> DNA
   <213> Artificial Sequence
<220>
   <223> synthetic nucleotide probe
<400> 548
   aagacgatgg aggtggaatc cttta 25
<210> 549
   <211> 25
   <212> DNA
   <213> Artificial Sequence
<220>
   <223> synthetic nucleotide probe
<400> 549
   gagccaaaac cgcagcacta gaaaa 25
<210> 550
   <211> 25
   <212> DNA
   <213> Artificial Sequence
<220>
   <223> synthetic nucleotide probe
<400> 550
   gagactacca agacgagcca aaacc 25
<210> 551
   <211> 25
   <212> DNA
   <213> Artificial Sequence
<220>
   <223> synthetic nucleotide probe
<400> 551
   ttccagaacc agaatcagaa atgaa 25
<210> 552
   <211> 25
   <212> DNA
   <213> Artificial Sequence
<220>
   <223> synthetic nucleotide probe
<400> 552
   ccaagacgag ccaaaaccgc agcac 25
<210> 553
   <211> 25
   <212> DNA
   <213> Artificial Sequence
<220>
   <223> synthetic nucleotide probe
<400> 553
   ggacgaacag gaggtgtcag actgc 25
<210> 554
   <211> 25
   <212> DNA
   <213> Artificial Sequence
<220>
   <223> synthetic nucleotide probe
<400> 554
   gaagatgaga ctaccaagac gagcc 25
<210> 555
   <211> 25
   <212> DNA
   <213> Artificial Sequence
<220>
   <223> synthetic nucleotide probe
<400> 555
   gtgtcagact gctgaagccg actct 25
<210> 556
   <211> 25
   <212> DNA
   <213> Artificial Sequence
<220>
   <223> synthetic nucleotide probe
<400> 556
   cttgctttgt atgctcagtg tgttg 25
<210> 557
   <211> 25
   <212> DNA
   <213> Artificial Sequence
<220>
   <223> synthetic nucleotide probe
<400> 557
   gccctctttg gtactatatg ccatg 25
<210> 558
   <211> 25
   <212> DNA
   <213> Artificial Sequence
<220>
   <223> synthetic nucleotide probe
<400> 558
   acacctggca tgacacttgc tttgt 25
<210> 559
   <211> 25
   <212> DNA
   <213> Artificial Sequence
<220>
   <223> synthetic nucleotide probe
<400> 559
   gaatttccca tagaagctgg tgaca 25
<210> 560
   <211> 25
   <212> DNA
   <213> Artificial Sequence
<220>
   <223> synthetic nucleotide probe
<400> 560
   gaagctggtg acatgttcct ggaag 25
<210> 561
   <211> 25
   <212> DNA
   <213> Artificial Sequence
<220>
   <223> synthetic nucleotide probe
<400> 561
   caagaaggac aagcccctgt gtaag 25
<210> 562
   <211> 25
   <212> DNA
   <213> Artificial Sequence
<220>
   <223> synthetic nucleotide probe
<400> 562
   tgacatgttc ctggaagctc tgggc 25
<210> 563
   <211> 25
   <212> DNA
   <213> Artificial Sequence
<220>
   <223> synthetic nucleotide probe
<400> 563
   atatgccatg gatgtgaatt tccca 25
<210> 564
   <211> 25
   <212> DNA
   <213> Artificial Sequence
<220>
   <223> synthetic nucleotide probe
<400> 564
   gcccctgtgt aagaaacatg ctcat 25
<210> 565
   <211> 25
   <212> DNA
   <213> Artificial Sequence
<220>
   <223> synthetic nucleotide probe
<400> 565
   ggaaggtcag acctttttct ccaag 25
<210> 566
   <211> 25
   <212> DNA
   <213> Artificial Sequence
<220>
   <223> synthetic nucleotide probe
<400> 566
   ttattatgcc ctctttggta ctata 25
<210> 567
   <211> 25
   <212> DNA
   <213> Artificial Sequence
<220>
   <223> synthetic nucleotide probe
<400> 567
   gagaggacca accagaattc ccttt 25
<210> 568
   <211> 25
   <212> DNA
   <213> Artificial Sequence
<220>
   <223> synthetic nucleotide probe
<400> 568
   aagcatgtgt catccatatt tctct 25
<210> 569
   <211> 25
   <212> DNA
   <213> Artificial Sequence
<220>
   <223> synthetic nucleotide probe
<400> 569
   ctacctggag gggatcagcc cactg 25
<210> 570
   <211> 25
   <212> DNA
   <213> Artificial Sequence
<220>
   <223> synthetic nucleotide probe
<400> 570
   agttgaacag tgtgccctag ctttt 25
<210> 571
   <211> 25
   <212> DNA
   <213> Artificial Sequence
<220>
   <223> synthetic nucleotide probe
<400> 571
   ggagaatcgt gtgatcagtg tgcta 25
<210> 572
   <211> 25
   <212> DNA
   <213> Artificial Sequence
<220>
   <223> synthetic nucleotide probe
<400> 572
   gtagtgtatc actgagtcat ttgca 25
<210> 573
   <211> 25
   <212> DNA
   <213> Artificial Sequence
<220>
   <223> synthetic nucleotide probe
<400> 573
   tgggctgcct tatattgtgt gtgtg 25
<210> 574
   <211> 25
   <212> DNA
   <213> Artificial Sequence
<220>
   <223> synthetic nucleotide probe
<400> 574
   tgttttctgc cacagacctt tgggc 25
<210> 575
   <211> 25
   <212> DNA
   <213> Artificial Sequence
<220>
   <223> synthetic nucleotide probe
<400> 575
   tgttctctcc gtgaaactta ccttt 25
<210> 576
   <211> 25
   <212> DNA
   <213> Artificial Sequence
<220>
   <223> synthetic nucleotide probe
<400> 576
   aaatgctctt atttttccaa cagct 25
<210> 577
   <211> 25
   <212> DNA
   <213> Artificial Sequence
<220>
   <223> synthetic nucleotide probe
<400> 577
   cctagctttt cttgcaacca gagta 25
<210> 578
   <211> 25
   <212> DNA
   <213> Artificial Sequence
<220>
   <223> synthetic nucleotide probe
<400> 578
   gaattccctt tggacatttg tgttt 25
<210> 579
   <211> 25
   <212> DNA
   <213> Artificial Sequence
<220>
   <223> synthetic nucleotide probe
<400> 579
   gccaaccttg gctaaatgga gcagc 25
<210> 580
   <211> 25
   <212> DNA
   <213> Artificial Sequence
<220>
   <223> synthetic nucleotide probe
<400> 580
   attactgctg tggctagaga gtttg 25
<210> 581
   <211> 25
   <212> DNA
   <213> Artificial Sequence
<220>
   <223> synthetic nucleotide probe
<400> 581
   ttggagtgag ggaggctacc tggag 25
<210> 582
   <211> 25
   <212> DNA
   <213> Artificial Sequence
<220>
   <223> synthetic nucleotide probe
<400> 582
   atatggcatc cttcaatttc tgtat 25
<210> 583
   <211> 25
   <212> DNA
   <213> Artificial Sequence
<220>
   <223> synthetic nucleotide probe
<400> 583
   cagcccactg acagacctta atctt 25
<210> 584
   <211> 25
   <212> DNA
   <213> Artificial Sequence
<220>
   <223> synthetic nucleotide probe
<400> 584
   atcagtggcc aggccaacct tggct 25
<210> 585
   <211> 25
   <212> DNA
   <213> Artificial Sequence
<220>
   <223> synthetic nucleotide probe
<400> 585
   ttttccaaca gctaaactac tctta 25
<210> 586
   <211> 25
   <212> DNA
   <213> Artificial Sequence
<220>
   <223> synthetic nucleotide probe
<400> 586
   gcaactcgta cccaaatttc caaga 25
<210> 587
   <211> 25
   <212> DNA
   <213> Artificial Sequence
<220>
   <223> synthetic nucleotide probe
<400> 587
   acatgaccta tccaagcgca ttacc 25
<210> 588
   <211> 25
   <212> DNA
   <213> Artificial Sequence
<220>
   <223> synthetic nucleotide probe
<400> 588
   gtaaaagctt tggtttgtgt tcgtg 25
<210> 589
   <211> 25
   <212> DNA
   <213> Artificial Sequence
<220>
   <223> synthetic nucleotide probe
<400> 589
   tagttctctc ctcacttgta aactt 25
<210> 590
   <211> 25
   <212> DNA
   <213> Artificial Sequence
<220>
   <223> synthetic nucleotide probe
<400> 590
   gtatatgtat cttcctcaat ttccc 25
<210> 591
   <211> 25
   <212> DNA
   <213> Artificial Sequence
<220>
   <223> synthetic nucleotide probe
<400> 591
   ggaggcagtg aagggcttgc cctgc 25
<210> 592
   <211> 25
   <212> DNA
   <213> Artificial Sequence
<220>
   <223> synthetic nucleotide probe
<400> 592
   catcttcccc tgtgagtgac atgtc 25
<210> 593
   <211> 25
   <212> DNA
   <213> Artificial Sequence
<220>
   <223> synthetic nucleotide probe
<400> 593
   aggttggaag tgtgatgggt ggggg 25
<210> 594
   <211> 25
   <212> DNA
   <213> Artificial Sequence
<220>
   <223> synthetic nucleotide probe
<400> 594
   ggtatctttt tgtcacacca aaatc 25
<210> 595
   <211> 25
   <212> DNA
   <213> Artificial Sequence
<220>
   <223> synthetic nucleotide probe
<400> 595
   cccctcccat taaagatccg ggcag 25
<210> 596
   <211> 25
   <212> DNA
   <213> Artificial Sequence
<220>
   <223> synthetic nucleotide probe
<400> 596
   aaagtaacat caacactgtc ccatc 25
<210> 597
   <211> 25
   <212> DNA
   <213> Artificial Sequence
<220>
   <223> synthetic nucleotide probe
<400> 597
   gatcccctca gacattctca ggatt 25
<210> 598
   <211> 25
   <212> DNA
   <213> Artificial Sequence
<220>
   <223> synthetic nucleotide probe
<400> 598
   gactgtcaga gtggggaacc cctcc 25
<210> 599
   <211> 25
   <212> DNA
   <213> Artificial Sequence
<220>
   <223> synthetic nucleotide probe
<400> 599
   gggttggggt gcttgtatat gtatc 25
<210> 600
   <211> 25
   <212> DNA
   <213> Artificial Sequence
<220>
   <223> synthetic nucleotide probe
<400> 600
   tatgttctca ttctatggcc attgt 25
<210> 601
   <211> 25
   <212> DNA
   <213> Artificial Sequence
<220>
   <223> synthetic nucleotide probe
<400> 601
   gagtctcaga atgctcagga ccaag 25
<210> 602
   <211> 25
   <212> DNA
   <213> Artificial Sequence
<220>
   <223> synthetic nucleotide probe
<400> 602
   tgtggccaga cagatgacac ctttt 25
<210> 603
   <211> 25
   <212> DNA
   <213> Artificial Sequence
<220>
   <223> synthetic nucleotide probe
<400> 603
   gtctgctctg gtgtgatctg aaaag 25
<210> 604
   <211> 25
   <212> DNA
   <213> Artificial Sequence
<220>
   <223> synthetic nucleotide probe
<400> 604
   gctttatctc atgatgcttg cttgt 25
<210> 605
   <211> 25
   <212> DNA
   <213> Artificial Sequence
<220>
   <223> synthetic nucleotide probe
<400> 605
   ggggtagaaa ctggtgtctg ctctg 25
<210> 606
   <211> 25
   <212> DNA
   <213> Artificial Sequence
<220>
   <223> synthetic nucleotide probe
<400> 606
   caggagaccc agcgatctga gcata 25
<210> 607
   <211> 25
   <212> DNA
   <213> Artificial Sequence
<220>
   <223> synthetic nucleotide probe
<400> 607
   gaaaaggcgt cttcactgct ttatc 25
<210> 608
   <211> 25
   <212> DNA
   <213> Artificial Sequence
<220>
   <223> synthetic nucleotide probe
<400> 608
   tatggccatt gtgttgcctc tgtta 25
<210> 609
   <211> 25
   <212> DNA
   <213> Artificial Sequence
<220>
   <223> synthetic nucleotide probe
<400> 609
   atcactagtg catgctgtgg ccaga 25
<210> 610
   <211> 25
   <212> DNA
   <213> Artificial Sequence
<220>
   <223> synthetic nucleotide probe
<400> 610
   aacatcttca tgtgggctgg ggtag 25
<210> 611
   <211> 25
   <212> DNA
   <213> Artificial Sequence
<220>
   <223> synthetic nucleotide probe
<400> 611
   cccttcccgc atttattggt gtatt 25
<210> 612
   <211> 25
   <212> DNA
   <213> Artificial Sequence
<220>
   <223> synthetic nucleotide probe
<400> 612
   acctttgtag ctagcaccag tgcca 25
<210> 613
   <211> 25
   <212> DNA
   <213> Artificial Sequence
<220>
   <223> synthetic nucleotide probe
<400> 613
   ttcatctcag atttgttcat cacag 25
<210> 614
   <211> 25
   <212> DNA
   <213> Artificial Sequence
<220>
   <223> synthetic nucleotide probe
<400> 614
   gtcttcagta gacaagtcac ctttg 25
<210> 615
   <211> 25
   <212> DNA
   <213> Artificial Sequence
<220>
   <223> synthetic nucleotide probe
<400> 615
   ttaaggactc catgaacctg ggcta 25
<210> 616
   <211> 25
   <212> DNA
   <213> Artificial Sequence
<220>
   <223> synthetic nucleotide probe
<400> 616
   taatgttgct actcccatgg caaag 25
<210> 617
   <211> 25
   <212> DNA
   <213> Artificial Sequence
<220>
   <223> synthetic nucleotide probe
<400> 617
   gtttttgtcc taatgttgct actcc 25
<210> 618
   <211> 25
   <212> DNA
   <213> Artificial Sequence
<220>
   <223> synthetic nucleotide probe
<400> 618
   cagaggacat cttggggagg gggag 25
<210> 619
   <211> 25
   <212> DNA
   <213> Artificial Sequence
<220>
   <223> synthetic nucleotide probe
<400> 619
   caccttcttt agtcttgatt gccct 25
<210> 620
   <211> 25
   <212> DNA
   <213> Artificial Sequence
<220>
   <223> synthetic nucleotide probe
<400> 620
   ccattgcacc ttctttagtc ttgat 25
<210> 621
   <211> 25
   <212> DNA
   <213> Artificial Sequence
<220>
   <223> synthetic nucleotide probe
<400> 621
   gatgtggctt ttgtgatatt ctatc 25
<210> 622
   <211> 25
   <212> DNA
   <213> Artificial Sequence
<220>
   <223> synthetic nucleotide probe
<400> 622
   cacggtcagt tgtaactttg ccttc 25

<210> 623
   <211> 25
   <212> DNA
   <213> Artificial Sequence
<220>
   <223> synthetic nucleotide probe
<400> 623
   gactatccaa cttaacatga aactt 25
<210> 624
   <211> 25
   <212> DNA
   <213> Artificial Sequence
<220>
   <223> synthetic nucleotide probe
<400> 624
   gagatagcat tagctgccca ggatg 25
<210> 625
   <211> 25
   <212> DNA
   <213> Artificial Sequence
<220>
   <223> synthetic nucleotide probe
<400> 625
   aatggagcta tgtcttgttt taagt 25
<210> 626
   <211> 25
   <212> DNA
   <213> Artificial Sequence
<220>
   <223> synthetic nucleotide probe
<400> 626
   gagagggagg atgtcacggt cagtt 25
<210> 627
   <211> 25
   <212> DNA
   <213> Artificial Sequence
<220>
   <223> synthetic nucleotide probe
<400> 627
   agttggtctt ttgacgagag ggagg 25
<210> 628
   <211> 25
   <212> DNA
   <213> Artificial Sequence
<220>
   <223> synthetic nucleotide probe
<400> 628
   gtgcctcctt gacatttcgt tcaag 25
<210> 629
   <211> 25
   <212> DNA
   <213> Artificial Sequence
<220>
   <223> synthetic nucleotide probe
<400> 629
   gaaacttgtc accatgagat agcat 25
<210> 630
   <211> 25
   <212> DNA
   <213> Artificial Sequence
<220>
   <223> synthetic nucleotide probe
<400> 630
   aaagctacaa tctgttcaat gtttt 25
<210> 631
   <211> 25
   <212> DNA
   <213> Artificial Sequence
<220>
   <223> synthetic nucleotide probe
<400> 631
   ctgcccagga tgctgctata tatat 25
<210> 632
   <211> 25
   <212> DNA
   <213> Artificial Sequence
<220>
   <223> synthetic nucleotide probe
<400> 632
   aaaaactcat ttatacctgt gtatt 25
<210> 633
   <211> 25
   <212> DNA
   <213> Artificial Sequence
<220>
   <223> synthetic nucleotide probe
<400> 633
   tgaccgagca gcagagtgta acatc 25
<210> 634
   <211> 25
   <212> DNA
   <213> Artificial Sequence
<220>
   <223> synthetic nucleotide probe
<400> 634
   gtaacatcgt agtaactcag cccag 25
<210> 635
   <211> 25
   <212> DNA
   <213> Artificial Sequence
<220>
   <223> synthetic nucleotide probe
<400> 635
   atcagtgcgg tttctgtggc agagc 25
<210> 636
   <211> 25
   <212> DNA
   <213> Artificial Sequence
<220>
   <223> synthetic nucleotide probe
<400> 636
   gactttatcc agaatgaccg agcag 25
<210> 637
   <211> 25
   <212> DNA
   <213> Artificial Sequence
<220>
   <223> synthetic nucleotide probe
<400> 637
   tagaggggct actggatgtg ggaaa 25
<210> 638
   <211> 25
   <212> DNA
   <213> Artificial Sequence
<220>
   <223> synthetic nucleotide probe
<400> 638
   ctcagcccag aagaatcagt gcggt 25
<210> 639
   <211> 25
   <212> DNA
   <213> Artificial Sequence
<220>
   <223> synthetic nucleotide probe
<400> 639
   ggcttatcct gaagttcgca ttgtt 25
<210> 640
   <211> 25
   <212> DNA
   <213> Artificial Sequence
<220>
   <223> synthetic nucleotide probe
<400> 640
   tgggaaaacc acacaggttc cccag 25
<210> 641
   <211> 25
   <212> DNA
   <213> Artificial Sequence
<220>
   <223> synthetic nucleotide probe
<400> 641
   tgtactgtag gtgtgctcct gagaa 25
<210> 642
   <211> 25
   <212> DNA
   <213> Artificial Sequence
<220>
   <223> synthetic nucleotide probe
<400> 642
   gcgtgatgtt gttcaggctt atcct 25
<210> 643
   <211> 25
   <212> DNA
   <213> Artificial Sequence
<220>
   <223> synthetic nucleotide probe
<400> 643
   ggaggactta cccagttcaa gaata 25
<210> 644
   <211> 25
   <212> DNA
   <213> Artificial Sequence
<220>
   <223> synthetic nucleotide probe
<400> 644
   ggatggcttc taacaaaaac tacac 25
<210> 645
   <211> 25
   <212> DNA
   <213> Artificial Sequence
<220>
   <223> synthetic nucleotide probe
<400> 645
   gtgtgctatg gatggcttct aacaa 25
<210> 646
   <211> 25
   <212> DNA
   <213> Artificial Sequence
<220>
   <223> synthetic nucleotide probe
<400> 646
   tagttacaca tcttcaacag acccc 25
<210> 647
   <211> 25
   <212> DNA
   <213> Artificial Sequence
<220>
   <223> synthetic nucleotide probe
<400> 647
   agggtgaagc tatttatctg tagta 25
<210> 648
   <211> 25
   <212> DNA
   <213> Artificial Sequence
<220>
   <223> synthetic nucleotide probe
<400> 648
   ttagggccca attaataatc agcaa 25
<210> 649
   <211> 25
   <212> DNA
   <213> Artificial Sequence
<220>
   <223> synthetic nucleotide probe
<400> 649
   cttccatagc ctaatccctg ggcat 25
<210> 650
   <211> 25
   <212> DNA
   <213> Artificial Sequence
<220>
   <223> synthetic nucleotide probe
<400> 650
   cacatatgta ttcctgatcg ccaac 25
<210> 651
   <211> 25
   <212> DNA
   <213> Artificial Sequence
<220>
   <223> synthetic nucleotide probe
<400> 651
   cagaccccct ctagaaattt ttcag 25
<210> 652
   <211> 25
   <212> DNA
   <213> Artificial Sequence
<220>
   <223> synthetic nucleotide probe
<400> 652
   ttgaatgggt ccattttgcc cttcc 25
<210> 653
   <211> 25
   <212> DNA
   <213> Artificial Sequence
<220>
   <223> synthetic nucleotide probe
<400> 653
   cagggttaat agggcctggt ccctg 25
<210> 654
   <211> 25
   <212> DNA
   <213> Artificial Sequence
<220>
   <223> synthetic nucleotide probe
<400> 654
   ttaaaccctg gatcagtcct ttgat 25
<210> 655
   <211> 25
   <212> DNA
   <213> Artificial Sequence
<220>
   <223> synthetic nucleotide probe
<400> 655
   gtattcagta tttgaacgtc gtcct 25
<210> 656
   <211> 25
   <212> DNA
   <213> Artificial Sequence
<220>
   <223> synthetic nucleotide probe
<400> 656
   gtcttgcatt gtgaggtaca ggcgg 25
<210> 657
   <211> 25
   <212> DNA
   <213> Artificial Sequence
<220>
   <223> synthetic nucleotide probe
<400> 657
   ttttaccttg gatgctgact tctaa 25
<210> 658
   <211> 25
   <212> DNA
   <213> Artificial Sequence
<220>
   <223> synthetic nucleotide probe
<400> 658
   gtacaggcgg aagttggaat caggt 25
<210> 659
   <211> 25
   <212> DNA
   <213> Artificial Sequence
<220>
   <223> synthetic nucleotide probe
<400> 659
   gaccctttag tgagcttagc acagc 25
<210> 660
   <211> 25
   <212> DNA
   <213> Artificial Sequence
<220>
   <223> synthetic nucleotide probe
<400> 660
   cctggctggc tgtgacttac catag 25
<210> 661
   <211> 25
   <212> DNA
   <213> Artificial Sequence
<220>
   <223> synthetic nucleotide probe
<400> 661
   gaacgtcgtc ctgtttattg ttagt 25
<210> 662
   <211> 25
   <212> DNA
   <213> Artificial Sequence
<220>
   <223> synthetic nucleotide probe
<400> 662
   ctcacaacca gtcctgtctg tttat 25
<210> 663
   <211> 25
   <212> DNA
   <213> Artificial Sequence
<220>
   <223> synthetic nucleotide probe
<400> 663
   gaagtgttac caactagcca cacca 25
<210> 664
   <211> 25
   <212> DNA
   <213> Artificial Sequence
<220>
   <223> synthetic nucleotide probe
<400> 664
   atgtgaggat taacttctgc cagct 25
<210> 665
   <211> 25
   <212> DNA
   <213> Artificial Sequence
<220>
   <223> synthetic nucleotide probe
<400> 665
   ctagccacac catgaattgt ccgta 25
<210> 666
   <211> 25
   <212> DNA
   <213> Artificial Sequence
<220>
   <223> synthetic nucleotide probe
<400> 666
   cagcctcact gcttcaacgc agatt 25
<210> 667
   <211> 25
   <212> DNA
   <213> Artificial Sequence
<220>
   <223> synthetic nucleotide probe
<400> 667
   ttaggattct gtctctcatt agctg 25
<210> 668
   <211> 25
   <212> DNA
   <213> Artificial Sequence
<220>
   <223> synthetic nucleotide probe
<400> 668
   gtgctggtag tatcaccttt tgcca 25
<210> 669
   <211> 25
   <212> DNA
   <213> Artificial Sequence
<220>
   <223> synthetic nucleotide probe
<400> 669
   tatggtcctt atatgtgtac aacat 25
<210> 670
   <211> 25
   <212> DNA
   <213> Artificial Sequence
<220>
   <223> synthetic nucleotide probe
<400> 670
   gaagatgtgc ccttacttgg ctgat 25
<210> 671
   <211> 25
   <212> DNA
   <213> Artificial Sequence
<220>
   <223> synthetic nucleotide probe
<400> 671
   taaacagtcc tttaaccagc acagc 25
<210> 672
   <211> 25
   <212> DNA
   <213> Artificial Sequence
<220>
   <223> synthetic nucleotide probe
<400> 672
   tgaagtattt ttatgctctg aatgt 25
<210> 673
   <211> 25
   <212> DNA
   <213> Artificial Sequence
<220>
   <223> synthetic nucleotide probe
<400> 673
   caaagatcaa gggctgtccg caaca 25
<210> 674
   <211> 25
   <212> DNA
   <213> Artificial Sequence
<220>
   <223> synthetic nucleotide probe
<400> 674
   gatgaatatt tttctactgg tattt 25
<210> 675
   <211> 25
   <212> DNA
   <213> Artificial Sequence
<220>
   <223> synthetic nucleotide probe
<400> 675
   gacatagtgt ttattagcag ccatc 25
<210> 676
   <211> 25
   <212> DNA
   <213> Artificial Sequence
<220>
   <223> synthetic nucleotide probe
<400> 676
   gaaagctttt tgtctaagtg aattc 25
<210> 677
   <211> 25
   <212> DNA
   <213> Artificial Sequence
<220>
   <223> synthetic nucleotide probe
<400> 677
   gtgaattctt atgccttggt cagag 25
<210> 678
   <211> 25
   <212> DNA
   <213> Artificial Sequence
<220>
   <223> synthetic nucleotide probe
<400> 678
   tgttaactat acaacctggc taaag 25
<210> 679
   <211> 25
   <212> DNA
   <213> Artificial Sequence
<220>
   <223> synthetic nucleotide probe
<400> 679
   aaatgttctc atcagtttct tgcca 25
<210> 680
   <211> 25
   <212> DNA
   <213> Artificial Sequence
<220>
   <223> synthetic nucleotide probe
<400> 680
   tgctaagttc aagtttcgta atgct 25
<210> 681
   <211> 25
   <212> DNA
   <213> Artificial Sequence
<220>
   <223> synthetic nucleotide probe
<400> 681
   aagggctgtc cgcaacaggg aagaa 25
<210> 682
   <211> 25
   <212> DNA
   <213> Artificial Sequence
<220>
   <223> synthetic nucleotide probe
<400> 682
   cttatcttgg ctttcgagtc tgagt 25
<210> 683
   <211> 25
   <212> DNA
   <213> Artificial Sequence
<220>
   <223> synthetic nucleotide probe
<400> 683
   ggcctctaat atctttggga cacct 25
<210> 684
   <211> 25
   <212> DNA
   <213> Artificial Sequence
<220>
   <223> synthetic nucleotide probe
<400> 684
   gaaggaactc ctctgaagca agctc 25
<210> 685
   <211> 25
   <212> DNA
   <213> Artificial Sequence
<220>
   <223> synthetic nucleotide probe
<400> 685
   gacttggagt catctggact gcaga 25
<210> 686
   <211> 25
   <212> DNA
   <213> Artificial Sequence
<220>
   <223> synthetic nucleotide probe
<400> 686
   agagtgaaga gaagcccgtg cctgc 25
<210> 687
   <211> 25
   <212> DNA
   <213> Artificial Sequence
<220>
   <223> synthetic nucleotide probe
<400> 687
   gaccccaaca gcaggaatag ctccc 25
<210> 688
   <211> 25
   <212> DNA
   <213> Artificial Sequence
<220>
   <223> synthetic nucleotide probe
<400> 688
   gtggtggatc caatttttca ttagg 25
<210> 689
   <211> 25
   <212> DNA
   <213> Artificial Sequence
<220>
   <223> synthetic nucleotide probe
<400> 689
   catccagaag aaatccccct ggcgg 25
<210> 690
   <211> 25
   <212> DNA
   <213> Artificial Sequence
<220>
   <223> synthetic nucleotide probe
<400> 690
   acaaccacca ccttcaaggg agtcg 25
<210> 691
   <211> 25
   <212> DNA
   <213> Artificial Sequence
<220>
   <223> synthetic nucleotide probe
<400> 691
   gcaagctccg gagacttctt agatc 25
<210> 692
   <211> 25
   <212> DNA
   <213> Artificial Sequence
<220>
   <223> synthetic nucleotide probe
<400> 692
   gatgtacctt ttttgtcaac tctta 25
<210> 693
   <211> 25
   <212> DNA
   <213> Artificial Sequence
<220>
   <223> synthetic nucleotide probe
<400> 693
   tagtgatacc ttgatctttc ccact 25
<210> 694
   <211> 25
   <212> DNA
   <213> Artificial Sequence
<220>
   <223> synthetic nucleotide probe
<400> 694
   gtttcattga cctctagtga tacct 25
<210> 695
   <211> 25
   <212> DNA
   <213> Artificial Sequence
<220>
   <223> synthetic nucleotide probe
<400> 695
   gtacagccta gtgttaacat tcttg 25
<210> 696
   <211> 25
   <212> DNA
   <213> Artificial Sequence
<220>
   <223> synthetic nucleotide probe
<400> 696
   gattggcttt tgtcatccac tattg 25
<210> 697
   <211> 25
   <212> DNA
   <213> Artificial Sequence
<220>
   <223> synthetic nucleotide probe
<400> 697
   aacatttctc gatcactggt ttcag 25
<210> 698
   <211> 25
   <212> DNA
   <213> Artificial Sequence
<220>
   <223> synthetic nucleotide probe
<400> 698
   tttcccactt tctgttttcg gattg 25
<210> 699
   <211> 25
   <212> DNA
   <213> Artificial Sequence
<220>
   <223> synthetic nucleotide probe
<400> 699
   ggcctcctat gatgcagaca tggtg 25
<210> 700
   <211> 25
   <212> DNA
   <213> Artificial Sequence
<220>
   <223> synthetic nucleotide probe
<400> 700
   tcttggtatc tttttgtgcc ttatc 25
<210> 701
   <211> 25
   <212> DNA
   <213> Artificial Sequence
<220>
   <223> synthetic nucleotide probe
<400> 701
   aggagctggg actggtttga acaca 25
<210> 702
   <211> 25
   <212> DNA
   <213> Artificial Sequence
<220>
   <223> synthetic nucleotide probe
<400> 702
   cagatgggga gggggtactg gcctt 25
<210> 703
   <211> 25
   <212> DNA
   <213> Artificial Sequence
<220>
   <223> synthetic nucleotide probe
<400> 703
   atagagcctc tgatgtacga agtag 25
<210> 704
   <211> 25
   <212> DNA
   <213> Artificial Sequence
<220>
   <223> synthetic nucleotide probe
<400> 704
   cagtctctac aaacttacag ctcat 25
<210> 705
   <211> 25
   <212> DNA
   <213> Artificial Sequence
<220>
   <223> synthetic nucleotide probe
<400> 705
   aatcccctgc aagctattca aatgg 25
<210> 706
   <211> 25
   <212> DNA
   <213> Artificial Sequence
<220>
   <223> synthetic nucleotide probe
<400> 706
   gaagaaatca caaatcccct gcaag 25
<210> 707
   <211> 25
   <212> DNA
   <213> Artificial Sequence
<220>
   <223> synthetic nucleotide probe
<400> 707
   gtgatggata cgcttggcat tcctt 25
<210> 708
   <211> 25
   <212> DNA
   <213> Artificial Sequence
<220>
   <223> synthetic nucleotide probe
<400> 708
   gcattccttt tatcccagaa acacc 25
<210> 709
   <211> 25
   <212> DNA
   <213> Artificial Sequence
<220>
   <223> synthetic nucleotide probe
<400> 709
   gttgtttgtt ggctatttta ctgaa 25
<210> 710
   <211> 25
   <212> DNA
   <213> Artificial Sequence
<220>
   <223> synthetic nucleotide probe
<400> 710
   ggagcttttg cagatatacc gagaa 25
<210> 711
   <211> 25
   <212> DNA
   <213> Artificial Sequence
<220>
   <223> synthetic nucleotide probe
<400> 711
   tcagatatgc tgtgcaagtc ttgct 25
<210> 712
   <211> 25
   <212> DNA
   <213> Artificial Sequence
<220>
   <223> synthetic nucleotide probe
<400> 712
   gttgttgacc gtatttacag tctct 25
<210> 713
   <211> 25
   <212> DNA
   <213> Artificial Sequence
<220>
   <223> synthetic nucleotide probe
<400> 713
   gttgtaatcg cagtattcct tgtat 25
<210> 714
   <211> 25
   <212> DNA
   <213> Artificial Sequence
<220>
   <223> synthetic nucleotide probe
<400> 714
   agtagctctc tgcttgctga tagat 25
<210> 715
   <211> 25
   <212> DNA
   <213> Artificial Sequence
<220>
   <223> synthetic nucleotide probe
<400> 715
   attttagttt agcttcctga tttat 25
<210> 716
   <211> 25
   <212> DNA
   <213> Artificial Sequence
<220>
   <223> synthetic nucleotide probe
<400> 716
   gatggtttcc cagtgtgaga tttgt 25
<210> 717
   <211> 25
   <212> DNA
   <213> Artificial Sequence
<220>
   <223> synthetic nucleotide probe
<400> 717
   atggtttggt tggtcccagc aaagt 25
<210> 718
   <211> 25
   <212> DNA
   <213> Artificial Sequence
<220>
   <223> synthetic nucleotide probe
<400> 718
   gttctcggtg ttcaaactct tctaa 25
<210> 719
   <211> 25
   <212> DNA
   <213> Artificial Sequence
<220>
   <223> synthetic nucleotide probe
<400> 719
   tgcaaatatg ctgtgggttc tcggt 25
<210> 720
   <211> 25
   <212> DNA
   <213> Artificial Sequence
<220>
   <223> synthetic nucleotide probe
<400> 720
   aaggaattgc tgttactgta ctgca 25
<210> 721
   <211> 25
   <212> DNA
   <213> Artificial Sequence
<220>
   <223> synthetic nucleotide probe
<400> 721
   taaatctagt gtttctattt tagtt 25
<210> 722
   <211> 25
   <212> DNA
   <213> Artificial Sequence
<220>
   <223> synthetic nucleotide probe
<400> 722
   atataccatc ccatatatat gtggg 25
<210> 723
   <211> 25
   <212> DNA
   <213> Artificial Sequence
<220>
   <223> synthetic nucleotide probe
<400> 723
   ctgcttgctg atagatggtt tccca 25
<210> 724
   <211> 25
   <212> DNA
   <213> Artificial Sequence
<220>
   <223> synthetic nucleotide probe
<400> 724
   atgccagaga cataccaggc gcagc 25
<210> 725
   <211> 25
   <212> DNA
   <213> Artificial Sequence
<220>
   <223> synthetic nucleotide probe
<400> 725
   ggaagatcac atcctacttc ctcaa 25
<210> 726
   <211> 25
   <212> DNA
   <213> Artificial Sequence
<220>
   <223> synthetic nucleotide probe
<400> 726
   actgattatg gctcaggcta cccca 25
<210> 727
   <211> 25
   <212> DNA
   <213> Artificial Sequence
<220>
   <223> synthetic nucleotide probe
<400> 727
   tgcagcaaag ttttcccggg attga 25
<210> 728
   <211> 25
   <212> DNA
   <213> Artificial Sequence
<220>
   <223> synthetic nucleotide probe
<400> 728
   ctcaatgaag ggtcccaagc ccgtc 25
<210> 729
   <211> 25
   <212> DNA
   <213> Artificial Sequence
<220>
   <223> synthetic nucleotide probe
<400> 729
   tcgtggacac cgtagggatg ccaga 25
<210> 730
   <211> 25
   <212> DNA
   <213> Artificial Sequence
<220>
   <223> synthetic nucleotide probe
<400> 730
   gaggactcag catccgagaa tcagg 25
<210> 731
   <211> 25
   <212> DNA
   <213> Artificial Sequence
<220>
   <223> synthetic nucleotide probe
<400> 731
   tcttcccacc gatatttcct ggaac 25
<210> 732
   <211> 25
   <212> DNA
   <213> Artificial Sequence
<220>
   <223> synthetic nucleotide probe
<400> 732
   tggcctcaca ggtgcatcat agccg 25
<210> 733
   <211> 25
   <212> DNA
   <213> Artificial Sequence
<220>
   <223> synthetic nucleotide probe
<400> 733
   gggcaacgac tttgtgtcca ttgtc 25
<210> 734
   <211> 25
   <212> DNA
   <213> Artificial Sequence
<220>
   <223> synthetic nucleotide probe
<400> 734
   ccctgatgcc caccaaggac gtgga 25
<210> 735
   <211> 25
   <212> DNA
   <213> Artificial Sequence
<220>
   <223> synthetic nucleotide probe
<400> 735
   agcaccgctg cgacaagaag cgcca 25
<210> 736
   <211> 25
   <212> DNA
   <213> Artificial Sequence
<220>
   <223> synthetic nucleotide probe
<400> 736
   tcaactttgt ccacgtgatc gtcac 25
<210> 737
   <211> 25
   <212> DNA
   <213> Artificial Sequence
<220>
   <223> synthetic nucleotide probe
<400> 737
   gtgaggactt caagcttggc accat 25
<210> 738
   <211> 25
   <212> DNA
   <213> Artificial Sequence
<220>
   <223> synthetic nucleotide probe
<400> 738
   tggactacga gtgcaacctg gtgtc 25
<210> 739
   <211> 25
   <212> DNA
   <213> Artificial Sequence
<220>
   <223> synthetic nucleotide probe
<400> 739
   gtattaataa ggcattgccc cctgt 25
<210> 740
   <211> 25
   <212> DNA
   <213> Artificial Sequence
<220>
   <223> synthetic nucleotide probe
<400> 740
   tgtaaggctg cattgtgggt ttggg 25
<210> 741
   <211> 25
   <212> DNA
   <213> Artificial Sequence
<220>
   <223> synthetic nucleotide probe
<400> 741
   gttttgtaac actgtcctac tttat 25
<210> 742
   <211> 25
   <212> DNA
   <213> Artificial Sequence
<220>
   <223> synthetic nucleotide probe
<400> 742
   agtcgttgca gggtttggat cagct 25
<210> 743
   <211> 25
   <212> DNA
   <213> Artificial Sequence
<220>
   <223> synthetic nucleotide probe
<400> 743
   ggatcagctg taagttaggt atgcc 25
<210> 744
   <211> 25
   <212> DNA
   <213> Artificial Sequence
<220>
   <223> synthetic nucleotide probe
<400> 744
   aaggctgtta caatcaagtc gttgc 25
<210> 745
   <211> 25
   <212> DNA
   <213> Artificial Sequence
<220>
   <223> synthetic nucleotide probe
<400> 745
   agatgagtcc tatacgtggc aattt 25
<210> 746
   <211> 25
   <212> DNA
   <213> Artificial Sequence
<220>
   <223> synthetic nucleotide probe
<400> 746
   tctgaagcca gcattatctt ccaaa 25
<210> 747
   <211> 25
   <212> DNA
   <213> Artificial Sequence
<220>
   <223> synthetic nucleotide probe
<400> 747
   gccccctgtt tgcactcagg gttaa 25
<210> 748
   <211> 25
   <212> DNA
   <213> Artificial Sequence
<220>
   <223> synthetic nucleotide probe
<400> 748
   cgtggcaatt tttcaatgtc atctg 25
<210> 749
   <211> 25
   <212> DNA
   <213> Artificial Sequence
<220>
   <223> synthetic nucleotide probe
<400> 749
   tcagaactca tggccatttc ctgcc 25
<210> 750
   <211> 25
   <212> DNA
   <213> Artificial Sequence
<220>
   <223> synthetic nucleotide probe
<400> 750
   gagatacttg tcaagttgct cttaa 25
<210> 751
   <211> 25
   <212> DNA
   <213> Artificial Sequence
<220>
   <223> synthetic nucleotide probe
<400> 751
   tgggccgtcg acaaaggaaa tctga 25
<210> 752
   <211> 25
   <212> DNA
   <213> Artificial Sequence
<220>
   <223> synthetic nucleotide probe
<400> 752
   aatttcgaaa agcagttaca gacct 25
<210> 753
   <211> 25
   <212> DNA
   <213> Artificial Sequence
<220>
   <223> synthetic nucleotide probe
<400> 753
   aatctaccca tggctacagt tgata 25
<210> 754
   <211> 25
   <212> DNA
   <213> Artificial Sequence
<220>
   <223> synthetic nucleotide probe
<400> 754
   gtgggaacaa gagctataca ataac 25
<210> 755
   <211> 25
   <212> DNA
   <213> Artificial Sequence
<220>
   <223> synthetic nucleotide probe
<400> 755
   tagtcctaga ggatattttc atacc 25
<210> 756
   <211> 25
   <212> DNA
   <213> Artificial Sequence
<220>
   <223> synthetic nucleotide probe
<400> 756
   gatcccccaa atggtcctaa tctac 25
<210> 757
   <211> 25
   <212> DNA
   <213> Artificial Sequence
<220>
   <223> synthetic nucleotide probe
<400> 757
   agaaaagacg agatccccca aatgg 25
<210> 758
   <211> 25
   <212> DNA
   <213> Artificial Sequence
<220>
   <223> synthetic nucleotide probe
<400> 758
   tacccatggc tacagttgat ataaa 25
<210> 759
   <211> 25
   <212> DNA
   <213> Artificial Sequence
<220>
   <223> synthetic nucleotide probe
<400> 759
   ttcatacctt tgctggagat acttg 25
<210> 760
   <211> 25
   <212> DNA
   <213> Artificial Sequence
<220>
   <223> synthetic nucleotide probe
<400> 760
   atacctttgc tggagatact tgtca 25
<210> 761
   <211> 25
   <212> DNA
   <213> Artificial Sequence
<220>
   <223> synthetic nucleotide probe
<400> 761
   gcctgtgctg aactgatctc ttaaa 25
<210> 762
   <211> 25
   <212> DNA
   <213> Artificial Sequence
<220>
   <223> synthetic nucleotide probe
<400> 762
   aatactggtg ctcttgtcac aggta 25
<210> 763
   <211> 25
   <212> DNA
   <213> Artificial Sequence
<220>
   <223> synthetic nucleotide probe
<400> 763
   aaaatgctga tcttctctgg agtct 25
<210> 764
   <211> 25
   <212> DNA
   <213> Artificial Sequence
<220>
   <223> synthetic nucleotide probe
<400> 764
   tgctcttcca acagtgggtt ctagc 25
<210> 765
   <211> 25
   <212> DNA
   <213> Artificial Sequence
<220>
   <223> synthetic nucleotide probe
<400> 765
   acaactgaca agacaccagc ccata 25
<210> 766
   <211> 25
   <212> DNA
   <213> Artificial Sequence

<220>
   <223> synthetic nucleotide probe
<400> 766
   ttaggccttt tgtgcatacc attac 25
<210> 767
   <211> 25
   <212> DNA
   <213> Artificial Sequence
<220>
   <223> synthetic nucleotide probe
<400> 767
   ggtgcatgta caacagcatc caaca 25
<210> 768
   <211> 25
   <212> DNA
   <213> Artificial Sequence
<220>
   <223> synthetic nucleotide probe
<400> 768
   tctttttgtc ctcatcactc aatac 25
<210> 769
   <211> 25
   <212> DNA
   <213> Artificial Sequence
<220>
   <223> synthetic nucleotide probe
<400> 769
   gcaatcttgg aatcctcaac tgcag 25
<210> 770
   <211> 25
   <212> DNA
   <213> Artificial Sequence
<220>
   <223> synthetic nucleotide probe
<400> 770
   ggtagaacag cttgtttctt ttcca 25
<210> 771
   <211> 25
   <212> DNA
   <213> Artificial Sequence
<220>
   <223> synthetic nucleotide probe
<400> 771
   gcatccaaca tatctgtctt gttcc 25
<210> 772
   <211> 25
   <212> DNA
   <213> Artificial Sequence
<220>
   <223> synthetic nucleotide probe
<400> 772
   ttcagccctt taataatgga gcatc 25
<210> 773
   <211> 25
   <212> DNA
   <213> Artificial Sequence
<220>
   <223> synthetic nucleotide probe
<400> 773
   tttactatga tatccatttt ccaga 25
<210> 774
   <211> 25
   <212> DNA
   <213> Artificial Sequence
<220>
   <223> synthetic nucleotide probe
<400> 774
   gagactaact ctccacttgt atggg 25
<210> 775
   <211> 25
   <212> DNA
   <213> Artificial Sequence
<220>
   <223> synthetic nucleotide probe
<400> 775
   gggaactaca tttcactctt ggttt 25
<210> 776
   <211> 25
   <212> DNA
   <213> Artificial Sequence
<220>
   <223> synthetic nucleotide probe
<400> 776
   acctgtaacc ccaagcaaat ataga 25
<210> 777
   <211> 25
   <212> DNA
   <213> Artificial Sequence
<220>
   <223> synthetic nucleotide probe
<400> 777
   aactctccac ttgtatggga actac 25
<210> 778
   <211> 25
   <212> DNA
   <213> Artificial Sequence
<220>
   <223> synthetic nucleotide probe
<400> 778
   tcaggatata acagcacttc accga 25
<210> 779
   <211> 25
   <212> DNA
   <213> Artificial Sequence
<220>
   <223> synthetic nucleotide probe
<400> 779
   atttcactct tggttttcag gatat 25
<210> 780
   <211> 25
   <212> DNA
   <213> Artificial Sequence
<220>
   <223> synthetic nucleotide probe
<400> 780
   taacagcact tcaccgaaat attct 25
<210> 781
   <211> 25
   <212> DNA
   <213> Artificial Sequence
<220>
   <223> synthetic nucleotide probe
<400> 781
   tattagcaca caactatttt cagcc 25
<210> 782
   <211> 25
   <212> DNA
   <213> Artificial Sequence
<220>
   <223> synthetic nucleotide probe
<400> 782
   aagtttgttt atattcagaa gtctg 25
<210> 783
   <211> 25
   <212> DNA
   <213> Artificial Sequence
<220>
   <223> synthetic nucleotide probe
<400> 783
   gctgaaggca gatgtcgtcc caaag 25
<210> 784
   <211> 25
   <212> DNA
   <213> Artificial Sequence
<220>
   <223> synthetic nucleotide probe
<400> 784
   tggcaagcat gttgtgttcg gtcac 25
<210> 785
   <211> 25
   <212> DNA
   <213> Artificial Sequence
<220>
   <223> synthetic nucleotide probe
<400> 785
   gcctgaaacg atacgtgtgc ccact 25
<210> 786
   <211> 25
   <212> DNA
   <213> Artificial Sequence
<220>
   <223> synthetic nucleotide probe
<400> 786
   taatgctggt cctaacacca acggc 25
<210> 787
   <211> 25
   <212> DNA
   <213> Artificial Sequence
<220>
   <223> synthetic nucleotide probe
<400> 787
   ccgctttcct gacgagaact ttaca 25
<210> 788
   <211> 25
   <212> DNA
   <213> Artificial Sequence
<220>
   <223> synthetic nucleotide probe
<400> 788
   gtggccaggg tgctggcatg gtggc 25
<210> 789
   <211> 25
   <212> DNA
   <213> Artificial Sequence
<220>
   <223> synthetic nucleotide probe
<400> 789
   agaagggctt cggctacaaa ggctc 25
<210> 790
   <211> 25
   <212> DNA
   <213> Artificial Sequence
<220>
   <223> synthetic nucleotide probe
<400> 790
   aagtccatct acggaagccg ctttc 25
<210> 791
   <211> 25
   <212> DNA
   <213> Artificial Sequence
<220>
   <223> synthetic nucleotide probe
<400> 791
   tgtcctgtcc atggctaatg ctggt 25
<210> 792
   <211> 25
   <212> DNA
   <213> Artificial Sequence
<220>
   <223> synthetic nucleotide probe
<400> 792
   gttcggtcac gtcaaagagg gcatg 25
<210> 793
   <211> 25
   <212> DNA
   <213> Artificial Sequence
<220>
   <223> synthetic nucleotide probe
<400> 793
   gactgtggcc agttgagcta atctg 25
<210> 794
   <211> 25
   <212> DNA
   <213> Artificial Sequence
<220>
   <223> synthetic nucleotide probe
<400> 794
   tacagcagga gtggccatgt ggtcc 25
<210> 795
   <211> 25
   <212> DNA
   <213> Artificial Sequence
<220>
   <223> synthetic nucleotide probe
<400> 795
   gatgaggtac tcgtggttct ggagc 25
<210> 796
   <211> 25
   <212> DNA
   <213> Artificial Sequence
<220>
   <223> synthetic nucleotide probe
<400> 796
   ggcagatggt gcagccaaca atgct 25
<210> 797
   <211> 25
   <212> DNA
   <213> Artificial Sequence
<220>
   <223> synthetic nucleotide probe
<400> 797
   aacaatgctg accggtgctt atcct 25
<210> 798
   <211> 25
   <212> DNA
   <213> Artificial Sequence
<220>
   <223> synthetic nucleotide probe
<400> 798
   ggacattctt caattccaca tctgt 25
<210> 799
   <211> 25
   <212> DNA
   <213> Artificial Sequence
<220>
   <223> synthetic nucleotide probe
<400> 799
   gggctgaatt tagactctct cacag 25
<210> 800
   <211> 25
   <212> DNA
   <213> Artificial Sequence
<220>
   <223> synthetic nucleotide probe
<400> 800
   cagcctctgg agagtactca gtctc 25
<210> 801
   <211> 25
   <212> DNA
   <213> Artificial Sequence
<220>
   <223> synthetic nucleotide probe
<400> 801
   aaataagtca ttctccctag cagag 25
<210> 802
   <211> 25
   <212> DNA
   <213> Artificial Sequence
<220>
   <223> synthetic nucleotide probe
<400> 802
   ctctaagccc tgatccacaa taaaa 25
<210> 803
   <211> 25
   <212> DNA
   <213> Artificial Sequence
<220>
   <223> synthetic nucleotide probe
<400> 803
   ggagctctag aaacacttct gatgc 25
<210> 804
   <211> 25
   <212> DNA
   <213> Artificial Sequence
<220>
   <223> synthetic nucleotide probe
<400> 804
   tataagctct cgctagagtt cccca 25
<210> 805
   <211> 25
   <212> DNA
   <213> Artificial Sequence
<220>
   <223> synthetic nucleotide probe
<400> 805
   gagctctgga aaaaccccac agctt 25
<210> 806
   <211> 25
   <212> DNA
   <213> Artificial Sequence
<220>
   <223> synthetic nucleotide probe
<400> 806
   ggaaaagtgg tctgccctgt atgat 25
<210> 807
   <211> 25
   <212> DNA
   <213> Artificial Sequence
<220>
   <223> synthetic nucleotide probe
<400> 807
   gggtaacata tgcctggaca tcctg 25
<210> 808
   <211> 25
   <212> DNA
   <213> Artificial Sequence
<220>
   <223> synthetic nucleotide probe
<400> 808
   caatgcgccc acagtgaagt tcctc 25
<210> 809
   <211> 25
   <212> DNA
   <213> Artificial Sequence
<220>
   <223> synthetic nucleotide probe
<400> 809
   gggtagggac catccatgga gcagc 25
<210> 810
   <211> 25
   <212> DNA
   <213> Artificial Sequence
<220>
   <223> synthetic nucleotide probe
<400> 810
   gccttccctg aatcagacaa ccttt 25
<210> 811
   <211> 25
   <212> DNA
   <213> Artificial Sequence
<220>
   <223> synthetic nucleotide probe
<400> 811
   tccatccaga gccttctagg agaac 25
<210> 812
   <211> 25
   <212> DNA
   <213> Artificial Sequence
<220>
   <223> synthetic nucleotide probe
<400> 812
   atgatgtcag gaccattctg ctctc 25
<210> 813
   <211> 25
   <212> DNA
   <213> Artificial Sequence
<220>
   <223> synthetic nucleotide probe
<400> 813
   agatggtctg tcctttttgt gattt 25
<210> 814
   <211> 25
   <212> DNA
   <213> Artificial Sequence
<220>
   <223> synthetic nucleotide probe
<400> 814
   tgatagtccc ttgaacacac atgct 25
<210> 815
   <211> 25
   <212> DNA
   <213> Artificial Sequence
<220>
   <223> synthetic nucleotide probe
<400> 815
   gagggtatct gacaatgctg aggtt 25
<210> 816
   <211> 25
   <212> DNA
   <213> Artificial Sequence
<220>
   <223> synthetic nucleotide probe
<400> 816
   gcatttcaat cccacgtact tataa 25
<210> 817
   <211> 25
   <212> DNA
   <213> Artificial Sequence
<220>
   <223> synthetic nucleotide probe
<400> 817
   atctgtccgt gacctatcag ttatt 25
<210> 818
   <211> 25
   <212> DNA
   <213> Artificial Sequence
<220>
   <223> synthetic nucleotide probe
<400> 818
   gtacaatccg catccaacta ttaaa 25
<210> 819
   <211> 25
   <212> DNA
   <213> Artificial Sequence
<220>
   <223> synthetic nucleotide probe
<400> 819
   aaatggctgt ttagggtgct ttcaa 25
<210> 820
   <211> 25
   <212> DNA
   <213> Artificial Sequence
<220>
   <223> synthetic nucleotide probe
<400> 820
   tcacaagcta ttccctcaaa tctga 25
<210> 821
   <211> 25
   <212> DNA
   <213> Artificial Sequence
<220>
   <223> synthetic nucleotide probe
<400> 821
   aactgtgcca gttctttcca taata 25
<210> 822
   <211> 25
   <212> DNA
   <213> Artificial Sequence
<220>
   <223> synthetic nucleotide probe
<400> 822
   gagggagctg agtaacacca tcgat 25
<210> 823
   <211> 25
   <212> DNA
   <213> Artificial Sequence
<220>
   <223> synthetic nucleotide probe
<400> 823
   ggggttgggc tggatgccga ggtaa 25
<210> 824
   <211> 25
   <212> DNA
   <213> Artificial Sequence
<220>
   <223> synthetic nucleotide probe
<400> 824
   aaagctcagg attcttcgaa aagtt 25
<210> 825
   <211> 25
   <212> DNA
   <213> Artificial Sequence
<220>
   <223> synthetic nucleotide probe
<400> 825
   gaactaggtc aaaaatctgt ccgtg 25
<210> 826
   <211> 25
   <212> DNA
   <213> Artificial Sequence
<220>
   <223> synthetic nucleotide probe
<400> 826
   attaatacca tgacatcttg cttat 25
<210> 827
   <211> 25
   <212> DNA
   <213> Artificial Sequence
<220>
   <223> synthetic nucleotide probe
<400> 827
   gctgtagtgt tgaatacttg gcccc 25
<210> 828
   <211> 25
   <212> DNA
   <213> Artificial Sequence
<220>
   <223> synthetic nucleotide probe
<400> 828
   gccatgcctt tctgtatagt acaca 25
<210> 829
   <211> 25
   <212> DNA
   <213> Artificial Sequence
<220>
   <223> synthetic nucleotide probe
<400> 829
   tgagctgtct gtcatttacc tactt 25
<210> 830
   <211> 25
   <212> DNA
   <213> Artificial Sequence
<220>
   <223> synthetic nucleotide probe
<400> 830
   gtagcactca ctgaatagtt ttaaa 25
<210> 831
   <211> 25
   <212> DNA
   <213> Artificial Sequence
<220>
   <223> synthetic nucleotide probe
<400> 831
   ttggttgggc ttttaatcct gtgtg 25
<210> 832
   <211> 25
   <212> DNA
   <213> Artificial Sequence
<220>
   <223> synthetic nucleotide probe
<400> 832
   ctctgtagtt caaatctgtt agctt 25
<210> 833
   <211> 25
   <212> DNA
   <213> Artificial Sequence
<220>
   <223> synthetic nucleotide probe
<400> 833
   gatatttcgg aattggtttt actgt 25
<210> 834
   <211> 25
   <212> DNA
   <213> Artificial Sequence
<220>
   <223> synthetic nucleotide probe
<400> 834
   aaatattcca ttgctctgta gttca 25
<210> 835
   <211> 25
   <212> DNA
   <213> Artificial Sequence
<220>
   <223> synthetic nucleotide probe
<400> 835
   tgaatacttg gccccatgag ccatg 25
<210> 836
   <211> 25
   <212> DNA
   <213> Artificial Sequence
<220>
   <223> synthetic nucleotide probe
<400> 836
   ggtatgctta caattgtcat gtcta 25
<210> 837
   <211> 25
   <212> DNA
   <213> Artificial Sequence
<220>
   <223> synthetic nucleotide probe
<400> 837
   acctgtattt ctcagttgca gcact 25
<210> 838
   <211> 25
   <212> DNA
   <213> Artificial Sequence
<220>
   <223> synthetic nucleotide probe
<400> 838
   cccatgcatc tgcctggcat ttagg 25
<210> 839
   <211> 25
   <212> DNA
   <213> Artificial Sequence
<220>
   <223> synthetic nucleotide probe
<400> 839
   gcaattgagg cagttgacca tattc 25
<210> 840
   <211> 25
   <212> DNA
   <213> Artificial Sequence
<220>
   <223> synthetic nucleotide probe
<400> 840
   tcctcatctc atttggctgt gtaaa 25
<210> 841
   <211> 25
   <212> DNA
   <213> Artificial Sequence
<220>
   <223> synthetic nucleotide probe
<400> 841
   cctgccagca gttcttgaag cttct 25
<210> 842
   <211> 25
   <212> DNA
   <213> Artificial Sequence
<220>
   <223> synthetic nucleotide probe
<400> 842
   tccaagtcca ccagtctctg aaatt 25
<210> 843
   <211> 25
   <212> DNA
   <213> Artificial Sequence
<220>
   <223> synthetic nucleotide probe
<400> 843
   tggcatttag gcagcagagc ccctg 25
<210> 844
   <211> 25
   <212> DNA
   <213> Artificial Sequence
<220>
   <223> synthetic nucleotide probe
<400> 844
   ggagtggaat gttctatccc cacaa 25
<210> 845
   <211> 25
   <212> DNA
   <213> Artificial Sequence
<220>
   <223> synthetic nucleotide probe
<400> 845
   cagcccagga gtagtcttac ctctg 25
<210> 846
   <211> 25
   <212> DNA
   <213> Artificial Sequence
<220>
   <223> synthetic nucleotide probe
<400> 846
   cttacctctg aggaactttc tagat 25
<210> 847
   <211> 25
   <212> DNA
   <213> Artificial Sequence
<220>
   <223> synthetic nucleotide probe
<400> 847
   ggctgctaaa gccattgctg cactc 25
<210> 848
   <211> 25
   <212> DNA
   <213> Artificial Sequence
<220>
   <223> synthetic nucleotide probe
<400> 848
   gatgaggcat cgtgcctcac atccg 25
<210> 849
   <211> 25
   <212> DNA
   <213> Artificial Sequence
<220>
   <223> synthetic nucleotide probe
<400> 849
   taaagccatt gctgcactct gaggg 25
<210> 850
   <211> 25
   <212> DNA
   <213> Artificial Sequence
<220>
   <223> synthetic nucleotide probe
<400> 850
   gtgagggaaa tctaccttcg ttcat 25
<210> 851
   <211> 25
   <212> DNA
   <213> Artificial Sequence
<220>
   <223> synthetic nucleotide probe
<400> 851
   tggtgcaaga gcctctagcg gcttc 25
<210> 852
   <211> 25
   <212> DNA
   <213> Artificial Sequence
<220>
   <223> synthetic nucleotide probe
<400> 852
   ttcttctttc aaaatttcct ctcca 25
<210> 853
   <211> 25
   <212> DNA
   <213> Artificial Sequence
<220>
   <223> synthetic nucleotide probe
<400> 853
   aactggtgaa ggtgtcagcc atgtc 25
<210> 854
   <211> 25
   <212> DNA
   <213> Artificial Sequence
<220>
   <223> synthetic nucleotide probe
<400> 854
   catccgctcc acatggtgca agagc 25
<210> 855
   <211> 25
   <212> DNA
   <213> Artificial Sequence
<220>
   <223> synthetic nucleotide probe
<400> 855
   agatgacatg gttgttgccc cagtc 25
<210> 856
   <211> 25
   <212> DNA
   <213> Artificial Sequence
<220>
   <223> synthetic nucleotide probe
<400> 856
   aaatatgcag actcaccggg agcct 25
<210> 857
   <211> 25
   <212> DNA
   <213> Artificial Sequence
<220>
   <223> synthetic nucleotide probe
<400> 857
   catgttcctg gtgctgatat tctca 25
<210> 858
   <211> 25
   <212> DNA
   <213> Artificial Sequence
<220>
   <223> synthetic nucleotide probe
<400> 858
   ttatgccggt ctagcctgtg tggaa 25
<210> 859
   <211> 25
   <212> DNA
   <213> Artificial Sequence
<220>
   <223> synthetic nucleotide probe
<400> 859
   tcacccatgt gtccgtcaca ttaga 25
<210> 860
   <211> 25
   <212> DNA
   <213> Artificial Sequence
<220>
   <223> synthetic nucleotide probe
<400> 860
   gatattctca atagttatgc cggtc 25
<210> 861
   <211> 25
   <212> DNA
   <213> Artificial Sequence
<220>
   <223> synthetic nucleotide probe
<400> 861
   gatgaacgac agcttggtga tccag 25
<210> 862
   <211> 25
   <212> DNA
   <213> Artificial Sequence
<220>
   <223> synthetic nucleotide probe
<400> 862
   ttggtgatcc agctattttt cctgc 25
<210> 863
   <211> 25
   <212> DNA
   <213> Artificial Sequence
<220>
   <223> synthetic nucleotide probe
<400> 863
   ggcactcctc aatatggatt cccct 25
<210> 864
   <211> 25
   <212> DNA
   <213> Artificial Sequence
<220>
   <223> synthetic nucleotide probe
<400> 864
   gctgcctgat acgtgaatct tcttg 25
<210> 865
   <211> 25
   <212> DNA
   <213> Artificial Sequence
<220>
   <223> synthetic nucleotide probe
<400> 865
   gacatcaccc ttacagttga agctt 25
<210> 866
   <211> 25
   <212> DNA
   <213> Artificial Sequence
<220>
   <223> synthetic nucleotide probe
<400> 866
   gtgcatgttt gggaactggc attac 25
<210> 867
   <211> 25
   <212> DNA
   <213> Artificial Sequence
<220>
   <223> synthetic nucleotide probe
<400> 867
   ttctcagtac tccctcaaga ctgga 25
<210> 868
   <211> 25
   <212> DNA
   <213> Artificial Sequence
<220>
   <223> synthetic nucleotide probe
<400> 868
   actccagagc tgcagtgcca ctgga 25
<210> 869
   <211> 25
   <212> DNA
   <213> Artificial Sequence
<220>
   <223> synthetic nucleotide probe
<400> 869
   gtgccactgg aggagtcaga ctacc 25
<210> 870
   <211> 25
   <212> DNA
   <213> Artificial Sequence
<220>
   <223> synthetic nucleotide probe
<400> 870
   atagatgagc tctgagtttc tcagt 25
<210> 871
   <211> 25
   <212> DNA
   <213> Artificial Sequence
<220>
   <223> synthetic nucleotide probe
<400> 871
   gtcagactac catgacatcg taggg 25
<210> 872
   <211> 25
   <212> DNA
   <213> Artificial Sequence
<220>
   <223> synthetic nucleotide probe
<400> 872
   tcttctaacc tccttgggct atatt 25
<210> 873
   <211> 25
   <212> DNA
   <213> Artificial Sequence
<220>
   <223> synthetic nucleotide probe
<400> 873
   caggcctgag ggggaaccat ttttg 25
<210> 874
   <211> 25
   <212> DNA
   <213> Artificial Sequence
<220>
   <223> synthetic nucleotide probe
<400> 874
   ggacatcttg gtctttttct caggc 25
<210> 875
   <211> 25
   <212> DNA
   <213> Artificial Sequence
<220>
   <223> synthetic nucleotide probe
<400> 875
   tgctgagggt ggagtgtccc atcct 25
<210> 876
   <211> 25
   <212> DNA
   <213> Artificial Sequence
<220>
   <223> synthetic nucleotide probe
<400> 876
   gaggtatatt ggaactcttc taacc 25
<210> 877
   <211> 25
   <212> DNA
   <213> Artificial Sequence
<220>
   <223> synthetic nucleotide probe
<400> 877
   actgaccata catttttctt agccc 25
<210> 878
   <211> 25
   <212> DNA
   <213> Artificial Sequence
<220>
   <223> synthetic nucleotide probe
<400> 878
   gacattttta cataccgagc ctgag 25
<210> 879
   <211> 25
   <212> DNA
   <213> Artificial Sequence
<220>
   <223> synthetic nucleotide probe
<400> 879
   ttcatctgag cccccaaaag catta 25
<210> 880
   <211> 25
   <212> DNA
   <213> Artificial Sequence
<220>
   <223> synthetic nucleotide probe
<400> 880
   ttcttagccc ctcaagtaat atagc 25
<210> 881
   <211> 25
   <212> DNA
   <213> Artificial Sequence
<220>
   <223> synthetic nucleotide probe
<400> 881
   aaactggtca taaaggcact ctgtg 25
<210> 882
   <211> 25
   <212> DNA
   <213> Artificial Sequence
<220>
   <223> synthetic nucleotide probe
<400> 882
   ccttccagag tcctggctga ttggt 25
<210> 883
   <211> 25
   <212> DNA
   <213> Artificial Sequence
<220>
   <223> synthetic nucleotide probe
<400> 883
   gggactgaca tcttaagctc tcacc 25
<210> 884
   <211> 25
   <212> DNA
   <213> Artificial Sequence
<220>
   <223> synthetic nucleotide probe
<400> 884
   tacactactg tgtttcactg accat 25
<210> 885
   <211> 25
   <212> DNA
   <213> Artificial Sequence
<220>
   <223> synthetic nucleotide probe
<400> 885
   tgattggtgt tcgctgttca tctga 25
<210> 886
   <211> 25
   <212> DNA
   <213> Artificial Sequence
<220>
   <223> synthetic nucleotide probe
<400> 886
   ggacttctca tttttggagc tttcc 25
<210> 887
   <211> 25
   <212> DNA
   <213> Artificial Sequence
<220>
   <223> synthetic nucleotide probe
<400> 887
   gaatgacaat tcccctaacc attcc 25
<210> 888
   <211> 25
   <212> DNA
   <213> Artificial Sequence
<220>
   <223> synthetic nucleotide probe
<400> 888
   tttgcacttc cttcggagag catct 25
<210> 889
   <211> 25
   <212> DNA
   <213> Artificial Sequence
<220>
   <223> synthetic nucleotide probe
<400> 889
   cttccagtta tgcagcacct ggcta 25
<210> 890
   <211> 25
   <212> DNA
   <213> Artificial Sequence
<220>
   <223> synthetic nucleotide probe
<400> 890
   caacattacc tgtcatatac tgaag 25
<210> 891
   <211> 25
   <212> DNA
   <213> Artificial Sequence
<220>
   <223> synthetic nucleotide probe
<400> 891
   tggacaccaa ctctacaaca ttacc 25
<210> 892
   <211> 25
   <212> DNA
   <213> Artificial Sequence
<220>
   <223> synthetic nucleotide probe
<400> 892
   tggcaccatg tgccatctgt acata 25
<210> 893
   <211> 25
   <212> DNA
   <213> Artificial Sequence
<220>
   <223> synthetic nucleotide probe
<400> 893
   actatgacat ggtgcacttt cctcc 25
<210> 894
   <211> 25
   <212> DNA
   <213> Artificial Sequence
<220>
   <223> synthetic nucleotide probe
<400> 894
   ggaactaact atgttggact atgac 25
<210> 895
   <211> 25
   <212> DNA
   <213> Artificial Sequence
<220>
   <223> synthetic nucleotide probe
<400> 895
   gaatctcttc ttccagttat gcagc 25
<210> 896
   <211> 25
   <212> DNA
   <213> Artificial Sequence
<220>
   <223> synthetic nucleotide probe
<400> 896
   gcacctggct aagaatgtag tcatg 25
<210> 897
   <211> 25
   <212> DNA
   <213> Artificial Sequence
<220>
   <223> synthetic nucleotide probe
<400> 897
   tcctccttct caaattgcag cagga 25
<210> 898
   <211> 25
   <212> DNA
   <213> Artificial Sequence
<220>
   <223> synthetic nucleotide probe
<400> 898
   agattggaga ggttgatgtc gagca 25
<210> 899
   <211> 25
   <212> DNA
   <213> Artificial Sequence
<220>
   <223> synthetic nucleotide probe
<400> 899
   attccatcag ctttctctaa gtctt 25
<210> 900
   <211> 25
   <212> DNA
   <213> Artificial Sequence
<220>
   <223> synthetic nucleotide probe
<400> 900
   atagagggtc tcttcacgtt gatgc 25
<210> 901
   <211> 25
   <212> DNA
   <213> Artificial Sequence
<220>
   <223> synthetic nucleotide probe
<400> 901
   atccactgtg tgcatagagg gtctc 25
<210> 902
   <211> 25
   <212> DNA
   <213> Artificial Sequence
<220>
   <223> synthetic nucleotide probe
<400> 902
   tcagctttct ctaagtcttt gctca 25
<210> 903
   <211> 25
   <212> DNA
   <213> Artificial Sequence
<220>
   <223> synthetic nucleotide probe
<400> 903
   aagttcaacc ttaaaatgat gttag 25
<210> 904
   <211> 25
   <212> DNA
   <213> Artificial Sequence
<220>
   <223> synthetic nucleotide probe
<400> 904
   tctcttcacg ttgatgcttg gcatt 25
<210> 905
   <211> 25
   <212> DNA
   <213> Artificial Sequence
<220>
   <223> synthetic nucleotide probe
<400> 905
   gccaggcata acatatccac tgtgt 25
<210> 906
   <211> 25
   <212> DNA
   <213> Artificial Sequence
<220>
   <223> synthetic nucleotide probe
<400> 906
   tcacgttgat gcttggcatt ccatc 25
<210> 907
   <211> 25
   <212> DNA
   <213> Artificial Sequence
<220>
   <223> synthetic nucleotide probe
<400> 907
   gtgtgcatag agggtctctt cacgt 25
<210> 908
   <211> 25
   <212> DNA
   <213> Artificial Sequence
<220>
   <223> synthetic nucleotide probe
<400> 908
   tacagccagg cataacatat ccact 25
<210> 909
   <211> 25
   <212> DNA
   <213> Artificial Sequence

<220>
   <223> synthetic nucleotide probe
<400> 909
   ccatcagctt tctctaagtc tttgc 25
<210> 910
   <211> 25
   <212> DNA
   <213> Artificial Sequence
<220>
   <223> synthetic nucleotide probe
<400> 910
   aggccataat catcttttct ggtta 25
<210> 911
   <211> 25
   <212> DNA
   <213> Artificial Sequence
<220>
   <223> synthetic nucleotide probe
<400> 911
   aaatagcatc agtttgtcca atagt 25
<210> 912
   <211> 25
   <212> DNA
   <213> Artificial Sequence
<220>
   <223> synthetic nucleotide probe
<400> 912
   atgttgacac cttaatcggt cccag 25
<210> 913
   <211> 25
   <212> DNA
   <213> Artificial Sequence
<220>
   <223> synthetic nucleotide probe
<400> 913
   gaagctcctt gaccagggat gagaa 25
<210> 914
   <211> 25
   <212> DNA
   <213> Artificial Sequence
<220>
   <223> synthetic nucleotide probe
<400> 914
   caaccatcag ttagagaagc tcctt 25
<210> 915
   <211> 25
   <212> DNA
   <213> Artificial Sequence
<220>
   <223> synthetic nucleotide probe
<400> 915
   aagaacactt ctgggagccg aaagc 25
<210> 916
   <211> 25
   <212> DNA
   <213> Artificial Sequence
<220>
   <223> synthetic nucleotide probe
<400> 916
   gtgcctatag gaagactagt ctcat 25
<210> 917
   <211> 25
   <212> DNA
   <213> Artificial Sequence
<220>
   <223> synthetic nucleotide probe
<400> 917
   gccatctgcg aaatatcaac catca 25
<210> 918
   <211> 25
   <212> DNA
   <213> Artificial Sequence
<220>
   <223> synthetic nucleotide probe
<400> 918
   aaacgtatga ttcatccagc cttcc 25
<210> 919
   <211> 25
   <212> DNA
   <213> Artificial Sequence
<220>
   <223> synthetic nucleotide probe
<400> 919
   atcggtccca ggtatgagct ataat 25
<210> 920
   <211> 25
   <212> DNA
   <213> Artificial Sequence
<220>
   <223> synthetic nucleotide probe
<400> 920
   ggagccgaaa gccatctgcg aaata 25
<210> 921
   <211> 25
   <212> DNA
   <213> Artificial Sequence
<220>
   <223> synthetic nucleotide probe
<400> 921
   agactgtttg tacccttcat gaaat 25
<210> 922
   <211> 25
   <212> DNA
   <213> Artificial Sequence
<220>
   <223> synthetic nucleotide probe
<400> 922
   tcacaatagc ctttttatag tcagt 25
<210> 923
   <211> 25
   <212> DNA
   <213> Artificial Sequence
<220>
   <223> synthetic nucleotide probe
<400> 923
   gtaaatgact ctttgctaca tttta 25
<210> 924
   <211> 25
   <212> DNA
   <213> Artificial Sequence
<220>
   <223> synthetic nucleotide probe
<400> 924
   gcatgttcaa ctttttattg tggtc 25
<210> 925
   <211> 25
   <212> DNA
   <213> Artificial Sequence
<220>
   <223> synthetic nucleotide probe
<400> 925
   ggtaatttta tccactagca aatct 25
<210> 926
   <211> 25
   <212> DNA
   <213> Artificial Sequence
<220>
   <223> synthetic nucleotide probe
<400> 926
   gcatagatat gcgcatgttc aactt 25
<210> 927
   <211> 25
   <212> DNA
   <213> Artificial Sequence
<220>
   <223> synthetic nucleotide probe
<400> 927
   gaagttgcca tcagttttac taatc 25
<210> 928
   <211> 25
   <212> DNA
   <213> Artificial Sequence
<220>
   <223> synthetic nucleotide probe
<400> 928
   cagttttact aatcttctgt gaaat 25
<210> 929
   <211> 25
   <212> DNA
   <213> Artificial Sequence
<220>
   <223> synthetic nucleotide probe
<400> 929
   tagctgtttc agagagagta cggta 25
<210> 930
   <211> 25
   <212> DNA
   <213> Artificial Sequence
<220>
   <223> synthetic nucleotide probe
<400> 930
   ttcctatttc tttagggagt gctac 25
<210> 931
   <211> 25
   <212> DNA
   <213> Artificial Sequence
<220>
   <223> synthetic nucleotide probe
<400> 931
   gtatgtgcca cttctgagag tagta 25
<210> 932
   <211> 25
   <212> DNA
   <213> Artificial Sequence
<220>
   <223> synthetic nucleotide probe
<400> 932
   agccttgttg ggttgtcagc catgg 25
<210> 933
   <211> 25
   <212> DNA
   <213> Artificial Sequence
<220>
   <223> synthetic nucleotide probe
<400> 933
   aacagttaga tcagcccatc tcagc 25
<210> 934
   <211> 25
   <212> DNA
   <213> Artificial Sequence
<220>
   <223> synthetic nucleotide probe
<400> 934
   atggcaccct tgcaactgtg ggatc 25
<210> 935
   <211> 25
   <212> DNA
   <213> Artificial Sequence
<220>
   <223> synthetic nucleotide probe
<400> 935
   agaagtagtg gctggagact ctggc 25
<210> 936
   <211> 25
   <212> DNA
   <213> Artificial Sequence
<220>
   <223> synthetic nucleotide probe
<400> 936
   ctgcctcatc tctgtacgaa tttgg 25
<210> 937
   <211> 25
   <212> DNA
   <213> Artificial Sequence
<220>
   <223> synthetic nucleotide probe
<400> 937
   gatggtagat tcagcttctg ggaca 25
<210> 938
   <211> 25
   <212> DNA
   <213> Artificial Sequence
<220>
   <223> synthetic nucleotide probe
<400> 938
   ctcagcttgc tgtttcaatc acaat 25
<210> 939
   <211> 25
   <212> DNA
   <213> Artificial Sequence
<220>
   <223> synthetic nucleotide probe
<400> 939
   aatggaatcg cgttccatcc tgttc 25
<210> 940
   <211> 25
   <212> DNA
   <213> Artificial Sequence
<220>
   <223> synthetic nucleotide probe
<400> 940
   tggatttcaa cccctggaga aaacg 25
<210> 941
   <211> 25
   <212> DNA
   <213> Artificial Sequence
<220>
   <223> synthetic nucleotide probe
<400> 941
   acgaatttgg gtcccagcct tgttg 25
<210> 942
   <211> 25
   <212> DNA
   <213> Artificial Sequence
<220>
   <223> synthetic nucleotide probe
<400> 942
   catatttgca tacgcttcca gctac 25
<210> 943
   <211> 25
   <212> DNA
   <213> Artificial Sequence
<220>
   <223> synthetic nucleotide probe
<400> 943
   ggaaatcaca tagcagttac cccat 25
<210> 944
   <211> 25
   <212> DNA
   <213> Artificial Sequence
<220>
   <223> synthetic nucleotide probe
<400> 944
   gtggtgctga gagactacat ttata 25
<210> 945
   <211> 25
   <212> DNA
   <213> Artificial Sequence
<220>
   <223> synthetic nucleotide probe
<400> 945
   ataccgttac ttgggaaatc atctt 25
<210> 946
   <211> 25
   <212> DNA
   <213> Artificial Sequence
<220>
   <223> synthetic nucleotide probe
<400> 946
   caactgctgt atttaacatc tgcct 25
<210> 947
   <211> 25
   <212> DNA
   <213> Artificial Sequence
<220>
   <223> synthetic nucleotide probe
<400> 947
   gatatctata tccctacaac ctaat 25
<210> 948
   <211> 25
   <212> DNA
   <213> Artificial Sequence
<220>
   <223> synthetic nucleotide probe
<400> 948
   taactgtggt ttgcacccta acact 25
<210> 949
   <211> 25
   <212> DNA
   <213> Artificial Sequence
<220>
   <223> synthetic nucleotide probe
<400> 949
   gttaccccat ggcattgtga ctaat 25
<210> 950
   <211> 25
   <212> DNA
   <213> Artificial Sequence
<220>
   <223> synthetic nucleotide probe
<400> 950
   ccacctcata cacacacaat tcagt 25
<210> 951
   <211> 25
   <212> DNA
   <213> Artificial Sequence
<220>
   <223> synthetic nucleotide probe
<400> 951
   gtcatatagt aagcattttc cccca 25
<210> 952
   <211> 25
   <212> DNA
   <213> Artificial Sequence
<220>
   <223> synthetic nucleotide probe
<400> 952
   catatttcag gtttgttctc tttcc 25
<210> 953
   <211> 25
   <212> DNA
   <213> Artificial Sequence
<220>
   <223> synthetic nucleotide probe
<400> 953
   aaagtagtca ctatacaact cccct 25
<210> 954
   <211> 25
   <212> DNA
   <213> Artificial Sequence
<220>
   <223> synthetic nucleotide probe
<400> 954
   aagaccttgt tctcaaatct aggaa 25
<210> 955
   <211> 25
   <212> DNA
   <213> Artificial Sequence
<220>
   <223> synthetic nucleotide probe
<400> 955
   gaaggattca tttgttgcga gtgcc 25
<210> 956
   <211> 25
   <212> DNA
   <213> Artificial Sequence
<220>
   <223> synthetic nucleotide probe
<400> 956
   gttgcgagtg ccagtatact taaat 25
<210> 957
   <211> 25
   <212> DNA
   <213> Artificial Sequence
<220>
   <223> synthetic nucleotide probe
<400> 957
   ttttccaatc catttatccc tgggg 25
<210> 958
   <211> 25
   <212> DNA
   <213> Artificial Sequence
<220>
   <223> synthetic nucleotide probe
<400> 958
   cctgattttc acacaaatac tatat 25
<210> 959
   <211> 25
   <212> DNA
   <213> Artificial Sequence
<220>
   <223> synthetic nucleotide probe
<400> 959
   gtaaattggg ttcttcatgg aagtt 25
<210> 960
   <211> 25
   <212> DNA
   <213> Artificial Sequence
<220>
   <223> synthetic nucleotide probe
<400> 960
   ggaaatcatc tgtgacggaa gagta 25
<210> 961
   <211> 25
   <212> DNA
   <213> Artificial Sequence
<220>
   <223> synthetic nucleotide probe
<400> 961
   atgattttgt ttgtatccct accca 25
<210> 962
   <211> 25
   <212> DNA
   <213> Artificial Sequence
<220>
   <223> synthetic nucleotide probe
<400> 962
   ggaagccata gaattgctct ggtca 25
<210> 963
   <211> 25
   <212> DNA
   <213> Artificial Sequence
<220>
   <223> synthetic nucleotide probe
<400> 963
   agcacaccat agccttaact gaata 25
<210> 964
   <211> 25
   <212> DNA
   <213> Artificial Sequence
<220>
   <223> synthetic nucleotide probe
<400> 964
   tgggtgccaa aggtcaactg taatg 25
<210> 965
   <211> 25
   <212> DNA
   <213> Artificial Sequence
<220>
   <223> synthetic nucleotide probe
<400> 965
   gaccaatatc tgccagtaac gctgt 25
<210> 966
   <211> 25
   <212> DNA
   <213> Artificial Sequence
<220>
   <223> synthetic nucleotide probe
<400> 966
   aattgggata catttggctg tcaga 25
<210> 967
   <211> 25
   <212> DNA
   <213> Artificial Sequence
<220>
   <223> synthetic nucleotide probe
<400> 967
   taacgctgtt tatctcactt gcttt 25
<210> 968
   <211> 25
   <212> DNA
   <213> Artificial Sequence
<220>
   <223> synthetic nucleotide probe
<400> 968
   gacaactttt taattccttt gatct 25
<210> 969
   <211> 25
   <212> DNA
   <213> Artificial Sequence
<220>
   <223> synthetic nucleotide probe
<400> 969
   gtctactggg tataacatgt ctcac 25
<210> 970
   <211> 25
   <212> DNA
   <213> Artificial Sequence
<220>
   <223> synthetic nucleotide probe
<400> 970
   ggaaatctgc ctaatctgct tatat 25
<210> 971
   <211> 25
   <212> DNA
   <213> Artificial Sequence
<220>
   <223> synthetic nucleotide probe
<400> 971
   gctctggtca aaaccaagca cacca 25
<210> 972
   <211> 25
   <212> DNA
   <213> Artificial Sequence
<220>
   <223> synthetic nucleotide probe
<400> 972
   gattccctgc atcaactaag aaaag 25
<210> 973
   <211> 25
   <212> DNA
   <213> Artificial Sequence
<220>
   <223> synthetic nucleotide probe
<400> 973
   ttggtggcga atgtgttgcg ggtcc 25
<210> 974
   <211> 25
   <212> DNA
   <213> Artificial Sequence
<220>
   <223> synthetic nucleotide probe
<400> 974
   gcgtgttctg taatatttcc tctaa 25
<210> 975
   <211> 25
   <212> DNA
   <213> Artificial Sequence
<220>
   <223> synthetic nucleotide probe
<400> 975
   tctcctaacc ctctagtttt aattg 25
<210> 976
   <211> 25
   <212> DNA
   <213> Artificial Sequence
<220>
   <223> synthetic nucleotide probe
<400> 976
   tctcagtagt ggtgctgtat tgtac 25
<210> 977
   <211> 25
   <212> DNA
   <213> Artificial Sequence
<220>
   <223> synthetic nucleotide probe
<400> 977
   aattggacac ttctttgctg ttgca 25
<210> 978
   <211> 25
   <212> DNA
   <213> Artificial Sequence
<220>
   <223> synthetic nucleotide probe
<400> 978
   cttggattac aaaacctcga gccct 25
<210> 979
   <211> 25
   <212> DNA
   <213> Artificial Sequence
<220>
   <223> synthetic nucleotide probe
<400> 979
   ctgttgggct gctttctgta ttgtc 25
<210> 980
   <211> 25
   <212> DNA
   <213> Artificial Sequence
<220>
   <223> synthetic nucleotide probe
<400> 980
   atctatgccg tgtatttttg cttta 25
<210> 981
   <211> 25
   <212> DNA
   <213> Artificial Sequence
<220>
   <223> synthetic nucleotide probe
<400> 981
   ttgctgttgc aatctatgcc gtgta 25
<210> 982
   <211> 25
   <212> DNA
   <213> Artificial Sequence
<220>
   <223> synthetic nucleotide probe
<400> 982
   atagcatagt accataccac ttata 25
<210> 983
   <211> 25
   <212> DNA
   <213> Artificial Sequence
<220>
   <223> synthetic nucleotide probe
<400> 983
   gaacgcagag cgactcgagg tgtcc 25
<210> 984
   <211> 25
   <212> DNA
   <213> Artificial Sequence
<220>
   <223> synthetic nucleotide probe
<400> 984
   ggcaaagcat ttcatccaac ttatg 25
<210> 985
   <211> 25
   <212> DNA
   <213> Artificial Sequence
<220>
   <223> synthetic nucleotide probe
<400> 985
   gcctggagga gttgtacggc ctggc 25
<210> 986
   <211> 25
   <212> DNA
   <213> Artificial Sequence
<220>
   <223> synthetic nucleotide probe
<400> 986
   gcagcagccg gagatggcgg cggtg 25
<210> 987
   <211> 25
   <212> DNA
   <213> Artificial Sequence
<220>
   <223> synthetic nucleotide probe
<400> 987
   ttaaataggt gttgccatct ctttt 25
<210> 988
   <211> 25
   <212> DNA
   <213> Artificial Sequence
<220>
   <223> synthetic nucleotide probe
<400> 988
   gacacttgtc ctgaggttgg attct 25
<210> 989
   <211> 25
   <212> DNA
   <213> Artificial Sequence
<220>
   <223> synthetic nucleotide probe
<400> 989
   ggcagccctg ggaaacatgt ctaaa 25
<210> 990
   <211> 25
   <212> DNA
   <213> Artificial Sequence
<220>
   <223> synthetic nucleotide probe
<400> 990
   tcaacatatg ttgcgtccca caaaa 25
<210> 991
   <211> 25
   <212> DNA
   <213> Artificial Sequence
<220>
   <223> synthetic nucleotide probe
<400> 991
   tggcactgcg cttcttcaaa gaaaa 25
<210> 992
   <211> 25
   <212> DNA
   <213> Artificial Sequence
<220>
   <223> synthetic nucleotide probe
<400> 992
   taagcaagtt cttatgggcc catat 25
<210> 993
   <211> 25
   <212> DNA
   <213> Artificial Sequence
<220>
   <223> synthetic nucleotide probe
<400> 993
   ggcccatata atccagacac ttgtc 25
<210> 994
   <211> 25
   <212> DNA
   <213> Artificial Sequence
<220>
   <223> synthetic nucleotide probe
<400> 994
   ccctgaacat gcctgagctt gtcat 25
<210> 995
   <211> 25
   <212> DNA
   <213> Artificial Sequence
<220>
   <223> synthetic nucleotide probe
<400> 995
   tccctttctg aattagctgt acata 25
<210> 996
   <211> 25
   <212> DNA
   <213> Artificial Sequence
<220>
   <223> synthetic nucleotide probe
<400> 996
   gcatttatct atgtctttag gtgtc 25
<210> 997
   <211> 25
   <212> DNA
   <213> Artificial Sequence
<220>
   <223> synthetic nucleotide probe
<400> 997
   ggtaatttcc ttctaatatg ttggt 25
<210> 998
   <211> 25
   <212> DNA
   <213> Artificial Sequence
<220>
   <223> synthetic nucleotide probe
<400> 998
   taccattctt ccactgtgct gttat 25
<210> 999
   <211> 25
   <212> DNA
   <213> Artificial Sequence
<220>
   <223> synthetic nucleotide probe
<400> 999
   gtagatcaga acatcaggct ttcag 25
<210> 1000
   <211> 25
   <212> DNA
   <213> Artificial Sequence
<220>
   <223> synthetic nucleotide probe
<400> 1000
   atgcctgagc ttgtcataat atgtt 25
<210> 1001
   <211> 25
   <212> DNA
   <213> Artificial Sequence
<220>
   <223> synthetic nucleotide probe
<400> 1001
   atgttggtac tgtctatggc catac 25
<210> 1002
   <211> 25
   <212> DNA
   <213> Artificial Sequence
<220>
   <223> synthetic nucleotide probe
<400> 1002
   ttaggtgtca ttgttccctt tctga 25
<210> 1003
   <211> 25
   <212> DNA
   <213> Artificial Sequence
<220>
   <223> synthetic nucleotide probe
<400> 1003
   agagggactg tttaccattc ttcca 25
<210> 1004
   <211> 25
   <212> DNA
   <213> Artificial Sequence
<220>
   <223> synthetic nucleotide probe
<400> 1004
   gctgtacata taagccttcc tttgg 25
<210> 1005
   <211> 25
   <212> DNA
   <213> Artificial Sequence
<220>
   <223> synthetic nucleotide probe
<400> 1005
   cagaaagcac cgaatgaccc acagc 25
<210> 1006
   <211> 25
   <212> DNA
   <213> Artificial Sequence
<220>
   <223> synthetic nucleotide probe
<400> 1006
   ttccgtctgt actctcagaa agcac 25
<210> 1007
   <211> 25
   <212> DNA
   <213> Artificial Sequence
<220>
   <223> synthetic nucleotide probe
<400> 1007
   gaagtgctgt tatcggaaac catca 25
<210> 1008
   <211> 25
   <212> DNA
   <213> Artificial Sequence
<220>
   <223> synthetic nucleotide probe
<400> 1008
   gacttccagt ctttcaccag atgac 25
<210> 1009
   <211> 25
   <212> DNA
   <213> Artificial Sequence
<220>
   <223> synthetic nucleotide probe
<400> 1009
   gtatcatggt ccagcagtac tgttt 25
<210> 1010
   <211> 25
   <212> DNA
   <213> Artificial Sequence
<220>
   <223> synthetic nucleotide probe
<400> 1010
   tatcccgtgt taccaaatta ccatt 25
<210> 1011
   <211> 25
   <212> DNA
   <213> Artificial Sequence
<220>
   <223> synthetic nucleotide probe
<400> 1011
   gaaaccatca gacatttccg tctgt 25
<210> 1012
   <211> 25
   <212> DNA
   <213> Artificial Sequence
<220>
   <223> synthetic nucleotide probe
<400> 1012
   atgaaaaacc tgctcatcgt tcagc 25
<210> 1013
   <211> 25
   <212> DNA
   <213> Artificial Sequence
<220>
   <223> synthetic nucleotide probe
<400> 1013
   ttaaaactaa gtcatctccc agata 25
<210> 1014
   <211> 25
   <212> DNA
   <213> Artificial Sequence
<220>
   <223> synthetic nucleotide probe
<400> 1014
   gcaaaattct gtttatcccg tgtta 25
<210> 1015
   <211> 25
   <212> DNA
   <213> Artificial Sequence
<220>
   <223> synthetic nucleotide probe
<400> 1015
   ctcatcgttc agcttccaaa attct 25
<210> 1016
   <211> 25
   <212> DNA
   <213> Artificial Sequence
<220>
   <223> synthetic nucleotide probe
<400> 1016
   ccttttcagc ttgaccctgc aatat 25
<210> 1017
   <211> 25
   <212> DNA
   <213> Artificial Sequence
<220>
   <223> synthetic nucleotide probe
<400> 1017
   aatctgcact gatattacat ccaca 25
<210> 1018
   <211> 25
   <212> DNA
   <213> Artificial Sequence
<220>
   <223> synthetic nucleotide probe
<400> 1018
   acctcatagc tctcaagcca gttga 25
<210> 1019
   <211> 25
   <212> DNA
   <213> Artificial Sequence
<220>
   <223> synthetic nucleotide probe
<400> 1019
   aggagactgc ccagcacata atgaa 25
<210> 1020
   <211> 25
   <212> DNA
   <213> Artificial Sequence
<220>
   <223> synthetic nucleotide probe
<400> 1020
   gatattgttt gttcactcat ttagt 25
<210> 1021
   <211> 25
   <212> DNA
   <213> Artificial Sequence
<220>
   <223> synthetic nucleotide probe
<400> 1021
   acatgcacag gcctgcttgt gaatc 25
<210> 1022
   <211> 25
   <212> DNA
   <213> Artificial Sequence
<220>
   <223> synthetic nucleotide probe
<400> 1022
   gccagttgaa gaaaccttgc ctttt 25
<210> 1023
   <211> 25
   <212> DNA
   <213> Artificial Sequence
<220>
   <223> synthetic nucleotide probe
<400> 1023
   aagatttccc attcacttga tattg 25
<210> 1024
   <211> 25
   <212> DNA
   <213> Artificial Sequence
<220>
   <223> synthetic nucleotide probe
<400> 1024
   tacatccaca gtaccacagt attta 25
<210> 1025
   <211> 25
   <212> DNA
   <213> Artificial Sequence
<220>
   <223> synthetic nucleotide probe
<400> 1025
   gaagaaacag cctacctcat agctc 25
<210> 1026
   <211> 25
   <212> DNA
   <213> Artificial Sequence
<220>
   <223> synthetic nucleotide probe
<400> 1026
   gagcgcccat atgcatgcaa caaat 25
<210> 1027
   <211> 25
   <212> DNA
   <213> Artificial Sequence
<220>
   <223> synthetic nucleotide probe
<400> 1027
   tggatgtctt tgctgcagtc ttctc 25
<210> 1028
   <211> 25
   <212> DNA
   <213> Artificial Sequence
<220>
   <223> synthetic nucleotide probe
<400> 1028
   ggtcttgggt tcctgtatgg tggaa 25
<210> 1029
   <211> 25
   <212> DNA
   <213> Artificial Sequence
<220>
   <223> synthetic nucleotide probe
<400> 1029
   caaaggaact ttctggtccc ttcag 25
<210> 1030
   <211> 25
   <212> DNA
   <213> Artificial Sequence
<220>
   <223> synthetic nucleotide probe
<400> 1030
   ttggccacca gaccatgggc caaga 25
<210> 1031
   <211> 25
   <212> DNA
   <213> Artificial Sequence
<220>
   <223> synthetic nucleotide probe
<400> 1031
   caaagggcag tgggtggagg accgg 25
<210> 1032
   <211> 25
   <212> DNA
   <213> Artificial Sequence
<220>
   <223> synthetic nucleotide probe
<400> 1032
   tctcctagac ccgtgacctg agatg 25
<210> 1033
   <211> 25
   <212> DNA
   <213> Artificial Sequence
<220>
   <223> synthetic nucleotide probe
<400> 1033
   cgtggctata cctggggact tgatg 25
<210> 1034
   <211> 25
   <212> DNA
   <213> Artificial Sequence
<220>
   <223> synthetic nucleotide probe
<400> 1034
   cagccactca aaggaacttt ctggt 25
<210> 1035
   <211> 25
   <212> DNA
   <213> Artificial Sequence
<220>
   <223> synthetic nucleotide probe
<400> 1035
   ggtttggaaa ctgcaaatgt cccct 25
<210> 1036
   <211> 25
   <212> DNA
   <213> Artificial Sequence
<220>
   <223> synthetic nucleotide probe
<400> 1036
   aggtttgagt ccctccaaag ggcag 25
<210> 1037
   <211> 25
   <212> DNA
   <213> Artificial Sequence
<220>
   <223> synthetic nucleotide probe
<400> 1037
   ggcccaccgg aacgacatgg agacc 25
<210> 1038
   <211> 25
   <212> DNA
   <213> Artificial Sequence
<220>
   <223> synthetic nucleotide probe
<400> 1038
   tctctgggca cagtgggcct gtgtg 25
<210> 1039
   <211> 25
   <212> DNA
   <213> Artificial Sequence
<220>
   <223> synthetic nucleotide probe
<400> 1039
   ctctgggcac agtgggcctg tgtgt 25
<210> 1040
   <211> 25
   <212> DNA
   <213> Artificial Sequence
<220>
   <223> synthetic nucleotide probe
<400> 1040
   tttggatgtc tttgctgcag tcttc 25
<210> 1041
   <211> 25
   <212> DNA
   <213> Artificial Sequence
<220>
   <223> synthetic nucleotide probe
<400> 1041
   acctggggac ttgatgttcc ttcca 25
<210> 1042
   <211> 25
   <212> DNA
   <213> Artificial Sequence
<220>
   <223> synthetic nucleotide probe
<400> 1042
   ggctatacct ggggacttga tgttc 25
<210> 1043
   <211> 25
   <212> DNA
   <213> Artificial Sequence
<220>
   <223> synthetic nucleotide probe
<400> 1043
   ctcctagacc cgtgacctga gatgt 25
<210> 1044
   <211> 25
   <212> DNA
   <213> Artificial Sequence
<220>
   <223> synthetic nucleotide probe
<400> 1044
   tggaggaccg ggagctttgg gtgac 25
<210> 1045
   <211> 25
   <212> DNA
   <213> Artificial Sequence
<220>
   <223> synthetic nucleotide probe
<400> 1045
   caccagacca tgggccaaga gccgc 25
<210> 1046
   <211> 25
   <212> DNA
   <213> Artificial Sequence
<220>
   <223> synthetic nucleotide probe
<400> 1046
   gcagtgggtg gaggaccggg agctt 25
<210> 1047
   <211> 25
   <212> DNA
   <213> Artificial Sequence
<220>
   <223> synthetic nucleotide probe
<400> 1047
   tttctggtcc cttcagtatc ttcaa 25
<210> 1048
   <211> 25
   <212> DNA
   <213> Artificial Sequence
<220>
   <223> synthetic nucleotide probe
<400> 1048
   caccggaacg acatggagac catct 25
<210> 1049
   <211> 25
   <212> DNA
   <213> Artificial Sequence
<220>
   <223> synthetic nucleotide probe
<400> 1049
   ggctcggaaa gatggatttt tgcat 25
<210> 1050
   <211> 25
   <212> DNA
   <213> Artificial Sequence
<220>
   <223> synthetic nucleotide probe
<400> 1050
   gcagttttcg gctgtcaaat tatct 25
<210> 1051
   <211> 25
   <212> DNA
   <213> Artificial Sequence
<220>
   <223> synthetic nucleotide probe
<400> 1051
   gggcgttcag atgcaattgg accaa 25
<210> 1052
   <211> 25
   <212> DNA
   <213> Artificial Sequence
<220>
   <223> synthetic nucleotide probe

<400> 1052
   tagtcctcca gagcagtttt cggct 25
<210> 1053
   <211> 25
   <212> DNA
   <213> Artificial Sequence
<220>
   <223> synthetic nucleotide probe
<400> 1053
   attgccctga agtctggcta tggaa 25
<210> 1054
   <211> 25
   <212> DNA
   <213> Artificial Sequence
<220>
   <223> synthetic nucleotide probe
<400> 1054
   gcacggtggc cctgctgcag ttgat 25
<210> 1055
   <211> 25
   <212> DNA
   <213> Artificial Sequence
<220>
   <223> synthetic nucleotide probe
<400> 1055
   ggcgatcagc gaggttctcc aggac 25
<210> 1056
   <211> 25
   <212> DNA
   <213> Artificial Sequence
<220>
   <223> synthetic nucleotide probe
<400> 1056
   ggaccttagg tttgatgcgg aatct 25
<210> 1057
   <211> 25
   <212> DNA
   <213> Artificial Sequence
<220>
   <223> synthetic nucleotide probe
<400> 1057
   ggttctccag gaccttaggt ttgat 25
<210> 1058
   <211> 25
   <212> DNA
   <213> Artificial Sequence
<220>
   <223> synthetic nucleotide probe
<400> 1058
   cagcgaggtt ctccaggacc ttagg 25
<210> 1059
   <211> 25
   <212> DNA
   <213> Artificial Sequence
<220>
   <223> synthetic nucleotide probe
<400> 1059
   aattaaaacc atggaggcga tcagc 25
<210> 1060
   <211> 25
   <212> DNA
   <213> Artificial Sequence
<220>
   <223> synthetic nucleotide probe
<400> 1060
   ccttaggttt gatgcggaat ctgcc 25
<210> 1061
   <211> 25
   <212> DNA
   <213> Artificial Sequence
<220>
   <223> synthetic nucleotide probe
<400> 1061
   cgctgtacgg atgcagcagc tgaaa 25
<210> 1062
   <211> 25
   <212> DNA
   <213> Artificial Sequence
<220>
   <223> synthetic nucleotide probe
<400> 1062
   tctgccgagt gatggcggct cccca 25
<210> 1063
   <211> 25
   <212> DNA
   <213> Artificial Sequence
<220>
   <223> synthetic nucleotide probe
<400> 1063
   catggaggcg atcagcgagg ttctc 25
<210> 1064
   <211> 25
   <212> DNA
   <213> Artificial Sequence
<220>
   <223> synthetic nucleotide probe
<400> 1064
   gtttgatgcg gaatctgccg agtga 25
<210> 1065
   <211> 25
   <212> DNA
   <213> Artificial Sequence
<220>
   <223> synthetic nucleotide probe
<400> 1065
   actgcgctgc tgaccttcct gcagt 25
<210> 1066
   <211> 25
   <212> DNA
   <213> Artificial Sequence
<220>
   <223> synthetic nucleotide probe
<400> 1066
   attaaaacca tggaggcgat cagcg 25
<210> 1067
   <211> 25
   <212> DNA
   <213> Artificial Sequence
<220>
   <223> synthetic nucleotide probe
<400> 1067
   ccaggacctt aggtttgatg cggaa 25
<210> 1068
   <211> 25
   <212> DNA
   <213> Artificial Sequence
<220>
   <223> synthetic nucleotide probe
<400> 1068
   agtgcacggg gtgacccagg ccttc 25
<210> 1069
   <211> 25
   <212> DNA
   <213> Artificial Sequence
<220>
   <223> synthetic nucleotide probe
<400> 1069
   atctgccgag tgatggcggc tcccc 25
<210> 1070
   <211> 25
   <212> DNA
   <213> Artificial Sequence
<220>
   <223> synthetic nucleotide probe
<400> 1070
   tgtgtgtgag acaggacgca gcggg 25
<210> 1071
   <211> 25
   <212> DNA
   <213> Artificial Sequence
<220>
   <223> synthetic nucleotide probe
<400> 1071
   cagacccgtg ggcaggtggg gcatg 25
<210> 1072
   <211> 25
   <212> DNA
   <213> Artificial Sequence
<220>
   <223> synthetic nucleotide probe
<400> 1072
   gttgagttaa accccttgtg tgtga 25
<210> 1073
   <211> 25
   <212> DNA
   <213> Artificial Sequence
<220>
   <223> synthetic nucleotide probe
<400> 1073
   tcccttccgc aggtctgcag atgaa 25
<210> 1074
   <211> 25
   <212> DNA
   <213> Artificial Sequence
<220>
   <223> synthetic nucleotide probe
<400> 1074
   aatttcaggg ctcttggcgt gttgg 25
<210> 1075
   <211> 25
   <212> DNA
   <213> Artificial Sequence
<220>
   <223> synthetic nucleotide probe
<400> 1075
   tgaaatgcca ctgcgcattt tcaga 25
<210> 1076
   <211> 25
   <212> DNA
   <213> Artificial Sequence
<220>
   <223> synthetic nucleotide probe
<400> 1076
   tcttttctct tcgtggcgac ttaga 25
<210> 1077
   <211> 25
   <212> DNA
   <213> Artificial Sequence
<220>
   <223> synthetic nucleotide probe
<400> 1077
   gcctttggta gctaacagtc actga 25
<210> 1078
   <211> 25
   <212> DNA
   <213> Artificial Sequence
<220>
   <223> synthetic nucleotide probe
<400> 1078
   agaaatccta gtgcagcctt tggta 25
<210> 1079
   <211> 25
   <212> DNA
   <213> Artificial Sequence
<220>
   <223> synthetic nucleotide probe
<400> 1079
   tgaatggatg tttgttcctc ctgat 25
<210> 1080
   <211> 25
   <212> DNA
   <213> Artificial Sequence
<220>
   <223> synthetic nucleotide probe
<400> 1080
   gagttggcgg atattcggaa ctgtg 25
<210> 1081
   <211> 25
   <212> DNA
   <213> Artificial Sequence
<220>
   <223> synthetic nucleotide probe
<400> 1081
   tacctcggag ctgatgctgg gcgga 25
<210> 1082
   <211> 25
   <212> DNA
   <213> Artificial Sequence
<220>
   <223> synthetic nucleotide probe
<400> 1082
   accaatggct gcaccggtga tgcca 25
<210> 1083
   <211> 25
   <212> DNA
   <213> Artificial Sequence
<220>
   <223> synthetic nucleotide probe
<400> 1083
   caacacgtgt gaggactcgc tgctg 25
<210> 1084
   <211> 25
   <212> DNA
   <213> Artificial Sequence
<220>
   <223> synthetic nucleotide probe
<400> 1084
   gcctcaagcg agttggaccc gtggc 25
<210> 1085
   <211> 25
   <212> DNA
   <213> Artificial Sequence
<220>
   <223> synthetic nucleotide probe
<400> 1085
   gccacctacc ctatgttgaa caaga 25
<210> 1086
   <211> 25
   <212> DNA
   <213> Artificial Sequence
<220>
   <223> synthetic nucleotide probe
<400> 1086
   ggaaccaaca cactggtgct gcaca 25
<210> 1087
   <211> 25
   <212> DNA
   <213> Artificial Sequence
<220>
   <223> synthetic nucleotide probe
<400> 1087
   gtgagcttct gcactgacat ggacc 25
<210> 1088
   <211> 25
   <212> DNA
   <213> Artificial Sequence
<220>
   <223> synthetic nucleotide probe
<400> 1088
   aaccacatgc tcctggaaca caaaa 25
<210> 1089
   <211> 25
   <212> DNA
   <213> Artificial Sequence
<220>
   <223> synthetic nucleotide probe
<400> 1089
   gggcatctgc aagaggagcc cccaa 25
<210> 1090
   <211> 25
   <212> DNA
   <213> Artificial Sequence
<220>
   <223> synthetic nucleotide probe
<400> 1090
   caaaatggag cgcccagggc ccagc 25
<210> 1091
   <211> 25
   <212> DNA
   <213> Artificial Sequence
<220>
   <223> synthetic nucleotide probe
<400> 1091
   ccagcgcagc tgcatcgaga acatc 25
<210> 1092
   <211> 25
   <212> DNA
   <213> Artificial Sequence
<220>
   <223> synthetic nucleotide probe
<400> 1092
   aaattgatga cttgttcgta tgttc 25
<210> 1093
   <211> 25
   <212> DNA
   <213> Artificial Sequence
<220>
   <223> synthetic nucleotide probe
<400> 1093
   gaagtgcctt tatctgctta gacct 25
<210> 1094
   <211> 25
   <212> DNA
   <213> Artificial Sequence
<220>
   <223> synthetic nucleotide probe
<400> 1094
   tgccctcaaa catacagaac ttcca 25
<210> 1095
   <211> 25
   <212> DNA
   <213> Artificial Sequence
<220>
   <223> synthetic nucleotide probe
<400> 1095
   ctctgacatc ggatgccctc aaaca 25
<210> 1096
   <211> 25
   <212> DNA
   <213> Artificial Sequence
<220>
   <223> synthetic nucleotide probe
<400> 1096
   agctattttg ccaacatgtc agagt 25
<210> 1097
   <211> 25
   <212> DNA
   <213> Artificial Sequence
<220>
   <223> synthetic nucleotide probe
<400> 1097
   agaacttcca aactcaagtc cagcc 25
<210> 1098
   <211> 25
   <212> DNA
   <213> Artificial Sequence
<220>
   <223> synthetic nucleotide probe
<400> 1098
   aagtccagcc ataagctatt ttgcc 25
<210> 1099
   <211> 25
   <212> DNA
   <213> Artificial Sequence
<220>
   <223> synthetic nucleotide probe
<400> 1099
   agagctgggg ttaggatccg gttgg 25
<210> 1100
   <211> 25
   <212> DNA
   <213> Artificial Sequence
<220>
   <223> synthetic nucleotide probe
<400> 1100
   taggatccgg ttggactctg acatc 25
<210> 1101
   <211> 25
   <212> DNA
   <213> Artificial Sequence
<220>
   <223> synthetic nucleotide probe
<400> 1101
   aaagctaaca ccagtcattt atatt 25
<210> 1102
   <211> 25
   <212> DNA
   <213> Artificial Sequence
<220>
   <223> synthetic nucleotide probe
<400> 1102
   gatgatgctt caatttctta atagt 25
<210> 1103
   <211> 25
   <212> DNA
   <213> Artificial Sequence
<220>
   <223> synthetic nucleotide probe
<400> 1103
   aaacgttctc accaaatcca atgct 25
<210> 1104
   <211> 25
   <212> DNA
   <213> Artificial Sequence
<220>
   <223> synthetic nucleotide probe
<400> 1104
   atacgaggcg tcagctgctg gcctc 25
<210> 1105
   <211> 25
   <212> DNA
   <213> Artificial Sequence
<220>
   <223> synthetic nucleotide probe
<400> 1105
   aggagcttgg accttggtac tacct 25
<210> 1106
   <211> 25
   <212> DNA
   <213> Artificial Sequence
<220>
   <223> synthetic nucleotide probe
<400> 1106
   gatgcccgat tctggaaggg cccca 25
<210> 1107
   <211> 25
   <212> DNA
   <213> Artificial Sequence
<220>
   <223> synthetic nucleotide probe
<400> 1107
   cagaccaagg ctgcaagtgg ccctc 25
<210> 1108
   <211> 25
   <212> DNA
   <213> Artificial Sequence
<220>
   <223> synthetic nucleotide probe
<400> 1108
   gcttaccatc ctgtctacca gatga 25
<210> 1109
   <211> 25
   <212> DNA
   <213> Artificial Sequence
<220>
   <223> synthetic nucleotide probe
<400> 1109
   ctctgtgatc tcatttcatc tgcac 25
<210> 1110
   <211> 25
   <212> DNA
   <213> Artificial Sequence
<220>
   <223> synthetic nucleotide probe
<400> 1110
   gtggcacgtg acagctaggg ttcaa 25
<210> 1111
   <211> 25
   <212> DNA
   <213> Artificial Sequence
<220>
   <223> synthetic nucleotide probe
<400> 1111
   tgagcgtttc ccagaggatg gaccc 25
<210> 1112
   <211> 25
   <212> DNA
   <213> Artificial Sequence
<220>
   <223> synthetic nucleotide probe
<400> 1112
   tgagggtggt gacacaaccc cttcc 25
<210> 1113
   <211> 25
   <212> DNA
   <213> Artificial Sequence
<220>
   <223> synthetic nucleotide probe
<400> 1113
   tcatctgcac tgccatacgt ggagt 25
<210> 1114
   <211> 25
   <212> DNA
   <213> Artificial Sequence
<220>
   <223> synthetic nucleotide probe
<400> 1114
   cctgtgctcg ggaagtcccg cagaa 25
<210> 1115
   <211> 25
   <212> DNA
   <213> Artificial Sequence
<220>
   <223> synthetic nucleotide probe
<400> 1115
   ccttggccgc ttcgagcgga tgctg 25
<210> 1116
   <211> 25
   <212> DNA
   <213> Artificial Sequence
<220>
   <223> synthetic nucleotide probe
<400> 1116
   ccgcttcgag cggatgctgg cggcg 25
<210> 1117
   <211> 25
   <212> DNA
   <213> Artificial Sequence
<220>
   <223> synthetic nucleotide probe
<400> 1117
   tggcccgagc cctgtgctcg ggaag 25
<210> 1118
   <211> 25
   <212> DNA
   <213> Artificial Sequence
<220>
   <223> synthetic nucleotide probe
<400> 1118
   ccgagccctg tgctcgggaa gtccc 25
<210> 1119
   <211> 25
   <212> DNA
   <213> Artificial Sequence
<220>
   <223> synthetic nucleotide probe
<400> 1119
   tgctcgggaa gtcccgcaga acgcc 25
<210> 1120
   <211> 25
   <212> DNA
   <213> Artificial Sequence
<220>
   <223> synthetic nucleotide probe
<400> 1120
   ctgctggtcg acgtgggtca gaggc 25
<210> 1121
   <211> 25
   <212> DNA
   <213> Artificial Sequence
<220>
   <223> synthetic nucleotide probe
<400> 1121
   ctggtcgacg tgggtcagag gctgc 25
<210> 1122
   <211> 25
   <212> DNA
   <213> Artificial Sequence
<220>
   <223> synthetic nucleotide probe
<400> 1122
   gcccgccaga cttaagggac ctggt 25
<210> 1123
   <211> 25
   <212> DNA
   <213> Artificial Sequence
<220>
   <223> synthetic nucleotide probe
<400> 1123
   caggcccgcc agacttaagg gacct 25
<210> 1124
   <211> 25
   <212> DNA
   <213> Artificial Sequence
<220>
   <223> synthetic nucleotide probe
<400> 1124
   acttaaggga cctggtcacc acgct 25
<210> 1125
   <211> 25
   <212> DNA
   <213> Artificial Sequence
<220>
   <223> synthetic nucleotide probe
<400> 1125
   ggttgctgca gtctggtcag atccc 25
<210> 1126
   <211> 25
   <212> DNA
   <213> Artificial Sequence
<220>
   <223> synthetic nucleotide probe
<400> 1126
   agctgacggc ggagctgatc gagca 25
<210> 1127
   <211> 25
   <212> DNA
   <213> Artificial Sequence
<220>
   <223> synthetic nucleotide probe
<400> 1127
   gtgggccatc tccaggggat gtaga 25
<210> 1128
   <211> 25
   <212> DNA
   <213> Artificial Sequence
<220>
   <223> synthetic nucleotide probe
<400> 1128
   gtcagatccc tggcagagaa cgcag 25
<210> 1129
   <211> 25
   <212> DNA
   <213> Artificial Sequence
<220>
   <223> synthetic nucleotide probe
<400> 1129
   tagcaaatgc ttcaactctg gctga 25
<210> 1130
   <211> 25
   <212> DNA
   <213> Artificial Sequence
<220>
   <223> synthetic nucleotide probe
<400> 1130
   tgatcgagca ggcggcgcag tacac 25
<210> 1131
   <211> 25
   <212> DNA
   <213> Artificial Sequence
<220>
   <223> synthetic nucleotide probe
<400> 1131
   aaggttccgc aagtcagagt actgg 25
<210> 1132
   <211> 25
   <212> DNA
   <213> Artificial Sequence
<220>
   <223> synthetic nucleotide probe
<400> 1132
   tggcatctct caaatcgctg actta 25
<210> 1133
   <211> 25
   <212> DNA
   <213> Artificial Sequence
<220>
   <223> synthetic nucleotide probe
<400> 1133
   tccaggtgct ggtttgccaa ctgac 25
<210> 1134
   <211> 25
   <212> DNA
   <213> Artificial Sequence
<220>
   <223> synthetic nucleotide probe
<400> 1134
   tccgggggtg atctgaaccc tctgg 25
<210> 1135
   <211> 25
   <212> DNA
   <213> Artificial Sequence
<220>
   <223> synthetic nucleotide probe
<400> 1135
   aacgcagatc agggcccact gatga 25
<210> 1136
   <211> 25
   <212> DNA
   <213> Artificial Sequence
<220>
   <223> synthetic nucleotide probe
<400> 1136
   tctccagcga ggaggtctca gtccc 25
<210> 1137
   <211> 25
   <212> DNA
   <213> Artificial Sequence
<220>
   <223> synthetic nucleotide probe
<400> 1137
   gcgtgcacgg tgttgaactg ggaca 25
<210> 1138
   <211> 25
   <212> DNA
   <213> Artificial Sequence
<220>
   <223> synthetic nucleotide probe
<400> 1138
   ctatgctccg agtagagttc atctt 25
<210> 1139
   <211> 25
   <212> DNA
   <213> Artificial Sequence
<220>
   <223> synthetic nucleotide probe
<400> 1139
   ctccttggta gcgtgcacgg tgttg 25
<210> 1140
   <211> 25
   <212> DNA
   <213> Artificial Sequence
<220>
   <223> synthetic nucleotide probe
<400> 1140
   tgactgtgca gactctggct cgagc 25
<210> 1141
   <211> 25
   <212> DNA
   <213> Artificial Sequence
<220>
   <223> synthetic nucleotide probe
<400> 1141
   aggtgtaggc aggtgggctc tgctt 25
<210> 1142
   <211> 25
   <212> DNA
   <213> Artificial Sequence
<220>
   <223> synthetic nucleotide probe
<400> 1142
   gaaaggggct ttcatgtcgt ttcct 25
<210> 1143
   <211> 25
   <212> DNA
   <213> Artificial Sequence
<220>
   <223> synthetic nucleotide probe
<400> 1143
   tcttcctcat ccctagggtg ttgtg 25
<210> 1144
   <211> 25
   <212> DNA
   <213> Artificial Sequence
<220>
   <223> synthetic nucleotide probe
<400> 1144
   gaactctttt taaactctat gctcc 25
<210> 1145
   <211> 25
   <212> DNA
   <213> Artificial Sequence
<220>
   <223> synthetic nucleotide probe
<400> 1145
   gtctgcagac ctcagagagg tccca 25
<210> 1146
   <211> 25
   <212> DNA
   <213> Artificial Sequence
<220>
   <223> synthetic nucleotide probe
<400> 1146
   gaactgggac actggggaga aaggg 25
<210> 1147
   <211> 25
   <212> DNA
   <213> Artificial Sequence
<220>
   <223> synthetic nucleotide probe
<400> 1147
   aaagtaccag gcactaggtc ggcgc 25
<210> 1148
   <211> 25
   <212> DNA
   <213> Artificial Sequence
<220>
   <223> synthetic nucleotide probe
<400> 1148
   gcgctgccgg gagatcgagc aggtg 25
<210> 1149
   <211> 25
   <212> DNA
   <213> Artificial Sequence
<220>
   <223> synthetic nucleotide probe
<400> 1149
   tggccccggt gcagattaag gttga 25
<210> 1150
   <211> 25
   <212> DNA
   <213> Artificial Sequence
<220>
   <223> synthetic nucleotide probe
<400> 1150
   cagccagttc accattgtgc tggag 25
<210> 1151
   <211> 25
   <212> DNA
   <213> Artificial Sequence
<220>
   <223> synthetic nucleotide probe
<400> 1151
   gccgagcagg aaatgcgctg actcc 25
<210> 1152
   <211> 25
   <212> DNA
   <213> Artificial Sequence
<220>
   <223> synthetic nucleotide probe
<400> 1152
   cctggattcc agttgggttt ctcgg 25
<210> 1153
   <211> 25
   <212> DNA
   <213> Artificial Sequence
<220>
   <223> synthetic nucleotide probe
<400> 1153
   gctggactcc tacggggatg actac 25
<210> 1154
   <211> 25
   <212> DNA
   <213> Artificial Sequence
<220>
   <223> synthetic nucleotide probe
<400> 1154
   aacgagcggg tcctgaacag gctcc 25
<210> 1155
   <211> 25
   <212> DNA
   <213> Artificial Sequence
<220>
   <223> synthetic nucleotide probe
<400> 1155
   tcggggtcca gacaaactgc tgccc 25
<210> 1156
   <211> 25
   <212> DNA
   <213> Artificial Sequence
<220>
   <223> synthetic nucleotide probe
<400> 1156
   ggttcctcat gagagtgctg gactc 25
<210> 1157
   <211> 25
   <212> DNA
   <213> Artificial Sequence
<220>
   <223> synthetic nucleotide probe
<400> 1157
   ggcggcgcca gcgggaatta aatcg 25
<210> 1158
   <211> 25
   <212> DNA
   <213> Artificial Sequence
<220>
   <223> synthetic nucleotide probe
<400> 1158
   tgtgtctttc cagaaggtca cgtgg 25
<210> 1159
   <211> 25
   <212> DNA
   <213> Artificial Sequence
<220>
   <223> synthetic nucleotide probe
<400> 1159
   caaagatggg gtgccaagac ggtgc 25
<210> 1160
   <211> 25
   <212> DNA
   <213> Artificial Sequence
<220>
   <223> synthetic nucleotide probe
<400> 1160
   tcgcccagga tgacgcgggg gccga 25
<210> 1161
   <211> 25
   <212> DNA
   <213> Artificial Sequence
<220>
   <223> synthetic nucleotide probe
<400> 1161
   gtggaaatgt ctcgggactt gggtc 25
<210> 1162
   <211> 25
   <212> DNA
   <213> Artificial Sequence
<220>
   <223> synthetic nucleotide probe
<400> 1162
   cgtgaacagc cttttatctc caagc 25
<210> 1163
   <211> 25
   <212> DNA
   <213> Artificial Sequence
<220>
   <223> synthetic nucleotide probe
<400> 1163
   gaaactcatc tccttcctga ggagc 25
<210> 1164
   <211> 25
   <212> DNA
   <213> Artificial Sequence
<220>
   <223> synthetic nucleotide probe
<400> 1164
   gaatttcgtg aacagccttt tatct 25
<210> 1165
   <211> 25
   <212> DNA
   <213> Artificial Sequence
<220>
   <223> synthetic nucleotide probe
<400> 1165
   ccaggacacc gctgtcctgg cattt 25
<210> 1166
   <211> 25
   <212> DNA
   <213> Artificial Sequence
<220>
   <223> synthetic nucleotide probe
<400> 1166
   cagcctttta tctccaagcg gaaag 25
<210> 1167
   <211> 25
   <212> DNA
   <213> Artificial Sequence
<220>
   <223> synthetic nucleotide probe
<400> 1167
   ttcctcattg gattactgtg tttta 25
<210> 1168
   <211> 25
   <212> DNA
   <213> Artificial Sequence
<220>
   <223> synthetic nucleotide probe
<400> 1168
   gaaggtcacg tggaaatgtc tcggg 25
<210> 1169
   <211> 25
   <212> DNA
   <213> Artificial Sequence
<220>
   <223> synthetic nucleotide probe
<400> 1169
   ctgggagacc gaccctgatt ttgtg 25
<210> 1170
   <211> 25
   <212> DNA
   <213> Artificial Sequence
<220>
   <223> synthetic nucleotide probe
<400> 1170
   aatcagtccc caatgcctgg aaatt 25
<210> 1171
   <211> 25
   <212> DNA
   <213> Artificial Sequence
<220>
   <223> synthetic nucleotide probe
<400> 1171
   gcctggaaat tcctcattgg attac 25
<210> 1172
   <211> 25
   <212> DNA
   <213> Artificial Sequence
<220>
   <223> synthetic nucleotide probe
<400> 1172
   cctgaggtgc attttctcat catcc 25
<210> 1173
   <211> 25
   <212> DNA
   <213> Artificial Sequence
<220>
   <223> synthetic nucleotide probe
<400> 1173
   catccttgct ttaccacaat gagca 25
<210> 1174
   <211> 25
   <212> DNA
   <213> Artificial Sequence
<220>
   <223> synthetic nucleotide probe
<400> 1174
   atttgtggcc actcactttg tagga 25
<210> 1175
   <211> 25
   <212> DNA
   <213> Artificial Sequence
<220>
   <223> synthetic nucleotide probe
<400> 1175
   gcatcgcatg cagcgcaagg ttctc 25
<210> 1176
   <211> 25
   <212> DNA
   <213> Artificial Sequence
<220>
   <223> synthetic nucleotide probe
<400> 1176
   gaggaaggag ctgtagtgtc ctgct 25
<210> 1177
   <211> 25
   <212> DNA
   <213> Artificial Sequence
<220>
   <223> synthetic nucleotide probe
<400> 1177
   ctgggaagtg tgcttttggc atccg 25
<210> 1178
   <211> 25
   <212> DNA
   <213> Artificial Sequence
<220>
   <223> synthetic nucleotide probe
<400> 1178
   cggcgagatc cagcatcgca tgcag 25
<210> 1179
   <211> 25
   <212> DNA
   <213> Artificial Sequence
<220>
   <223> synthetic nucleotide probe
<400> 1179
   ccagcatcgc atgcagcgca aggtt 25
<210> 1180
   <211> 25
   <212> DNA
   <213> Artificial Sequence
<220>
   <223> synthetic nucleotide probe
<400> 1180
   ctgcctgcca ttgacaatgt tgctg 25
<210> 1181
   <211> 25
   <212> DNA
   <213> Artificial Sequence
<220>
   <223> synthetic nucleotide probe
<400> 1181
   gcgagatcca gcatcgcatg cagcg 25
<210> 1182
   <211> 25
   <212> DNA
   <213> Artificial Sequence
<220>
   <223> synthetic nucleotide probe
<400> 1182
   gttctgggaa gtgtgctttt ggcat 25
<210> 1183
   <211> 25
   <212> DNA
   <213> Artificial Sequence
<220>
   <223> synthetic nucleotide probe
<400> 1183
   gaaggagctg tagtgtcctg ctgcc 25
<210> 1184
   <211> 25
   <212> DNA
   <213> Artificial Sequence
<220>
   <223> synthetic nucleotide probe
<400> 1184
   tcgcatgcag cgcaaggttc tctac 25
<210> 1185
   <211> 25
   <212> DNA
   <213> Artificial Sequence
<220>
   <223> synthetic nucleotide probe
<400> 1185
   ttgacaatgt tgctgggacc tctgc 25
<210> 1186
   <211> 25
   <212> DNA
   <213> Artificial Sequence
<220>
   <223> synthetic nucleotide probe
<400> 1186
   gttacattgt cttactctct tttac 25
<210> 1187
   <211> 25
   <212> DNA
   <213> Artificial Sequence
<220>
   <223> synthetic nucleotide probe
<400> 1187
   gttacattgt cttactctct tttac 25
<210> 1188
   <211> 25
   <212> DNA
   <213> Artificial Sequence
<220>
   <223> synthetic nucleotide probe
<400> 1188
   gaggccccag ggccaatcaa tttca 25
<210> 1189
   <211> 25
   <212> DNA
   <213> Artificial Sequence
<220>
   <223> synthetic nucleotide probe
<400> 1189
   gtaccattca ggaagattac ctaag 25
<210> 1190
   <211> 25
   <212> DNA
   <213> Artificial Sequence
<220>
   <223> synthetic nucleotide probe
<400> 1190
   gaaacacagg ccatcaggga aaacg 25
<210> 1191
   <211> 25
   <212> DNA
   <213> Artificial Sequence
<220>
   <223> synthetic nucleotide probe
<400> 1191
   gaaaaatcca actctcatcc tgggc 25
<210> 1192
   <211> 25
   <212> DNA
   <213> Artificial Sequence
<220>
   <223> synthetic nucleotide probe
<400> 1192
   catcctgggc agaggttgcc tagtt 25
<210> 1193
   <211> 25
   <212> DNA
   <213> Artificial Sequence
<220>
   <223> synthetic nucleotide probe
<400> 1193
   gatacatggc tgttcgtgac attct 25
<210> 1194
   <211> 25
   <212> DNA
   <213> Artificial Sequence
<220>
   <223> synthetic nucleotide probe
<400> 1194
   aatgtcctgc cagtttaagg gtaca 25
<210> 1195
   <211> 25
   <212> DNA
   <213> Artificial Sequence
<220>
   <223> synthetic nucleotide probe
<400> 1195
   gggtacattg tagagccgaa ctttg 25

<210> 1196
   <211> 25
   <212> DNA
   <213> Artificial Sequence
<220>
   <223> synthetic nucleotide probe
<400> 1196
   gagccgaact ttgagttact gtgca 25
<210> 1197
   <211> 25
   <212> DNA
   <213> Artificial Sequence
<220>
   <223> synthetic nucleotide probe
<400> 1197
   tactttacaa tgttccctta agcaa 25
<210> 1198
   <211> 25
   <212> DNA
   <213> Artificial Sequence
<220>
   <223> synthetic nucleotide probe
<400> 1198
   gataataaac ctctaaacct gccca 25
<210> 1199
   <211> 25
   <212> DNA
   <213> Artificial Sequence
<220>
   <223> synthetic nucleotide probe
<400> 1199
   aacctgccca gcggaagtgt gtttt 25
<210> 1200
   <211> 25
   <212> DNA
   <213> Artificial Sequence
<220>
   <223> synthetic nucleotide probe
<400> 1200
   tacttttttt tccatagctg ggata 25
<210> 1201
   <211> 25
   <212> DNA
   <213> Artificial Sequence
<220>
   <223> synthetic nucleotide probe
<400> 1201
   caagagtagc ctcaacctgt gcttc 25
<210> 1202
   <211> 25
   <212> DNA
   <213> Artificial Sequence
<220>
   <223> synthetic nucleotide probe
<400> 1202
   cagggtggca gatgaagctt tctac 25
<210> 1203
   <211> 25
   <212> DNA
   <213> Artificial Sequence
<220>
   <223> synthetic nucleotide probe
<400> 1203
   acaacccttc gctgacgtga ttggt 25
<210> 1204
   <211> 25
   <212> DNA
   <213> Artificial Sequence
<220>
   <223> synthetic nucleotide probe
<400> 1204
   accatccttg ctacagccta gaaca 25
<210> 1205
   <211> 25
   <212> DNA
   <213> Artificial Sequence
<220>
   <223> synthetic nucleotide probe
<400> 1205
   tgacattgac gagtcgtccc caggc 25
<210> 1206
   <211> 25
   <212> DNA
   <213> Artificial Sequence
<220>
   <223> synthetic nucleotide probe
<400> 1206
   taaccccaag tctagcaagc aggcc 25
<210> 1207
   <211> 25
   <212> DNA
   <213> Artificial Sequence
<220>
   <223> synthetic nucleotide probe
<400> 1207
   gcaagcaggc caaagatgtc tcccg 25
<210> 1208
   <211> 25
   <212> DNA
   <213> Artificial Sequence
<220>
   <223> synthetic nucleotide probe
<400> 1208
   gccaccctgt ggaaacacta catct 25
<210> 1209
   <211> 25
   <212> DNA
   <213> Artificial Sequence
<220>
   <223> synthetic nucleotide probe
<400> 1209
   atctgcaata tcttaatcct actca 25
<210> 1210
   <211> 25
   <212> DNA
   <213> Artificial Sequence
<220>
   <223> synthetic nucleotide probe
<400> 1210
   gaagctcttc acagtcattg gatta 25
<210> 1211
   <211> 25
   <212> DNA
   <213> Artificial Sequence
<220>
   <223> synthetic nucleotide probe
<400> 1211
   tgtttgtgat tgcatgtttc cttcc 25
<210> 1212
   <211> 25
   <212> DNA
   <213> Artificial Sequence
<220>
   <223> synthetic nucleotide probe
<400> 1212
   tacttttctt tctaacatat caatg 25
<210> 1213
   <211> 25
   <212> DNA
   <213> Artificial Sequence
<220>
   <223> synthetic nucleotide probe
<400> 1213
   ataccatact tatatacctg caact 25
<210> 1214
   <211> 25
   <212> DNA
   <213> Artificial Sequence
<220>
   <223> synthetic nucleotide probe
<400> 1214
   atgctctgta actctgtact gctag 25
<210> 1215
   <211> 25
   <212> DNA
   <213> Artificial Sequence
<220>
   <223> synthetic nucleotide probe
<400> 1215
   aatacagcca gtgcttaatg cttat 25
<210> 1216
   <211> 25
   <212> DNA
   <213> Artificial Sequence
<220>
   <223> synthetic nucleotide probe
<400> 1216
   aatgtggatt tgtcggcttt tatgt 25
<210> 1217
   <211> 25
   <212> DNA
   <213> Artificial Sequence
<220>
   <223> synthetic nucleotide probe
<400> 1217
   gcaagtgaca atacattcca ccaca 25
<210> 1218
   <211> 25
   <212> DNA
   <213> Artificial Sequence
<220>
   <223> synthetic nucleotide probe
<400> 1218
   aatacactct tgttcttcta gcttt 25
<210> 1219
   <211> 25
   <212> DNA
   <213> Artificial Sequence
<220>
   <223> synthetic nucleotide probe
<400> 1219
   aaaccgggtg cttcaaagta catga 25
<210> 1220
   <211> 25
   <212> DNA
   <213> Artificial Sequence
<220>
   <223> synthetic nucleotide probe
<400> 1220
   ggaacactat acctgtcatg gatga 25
<210> 1221
   <211> 25
   <212> DNA
   <213> Artificial Sequence
<220>
   <223> synthetic nucleotide probe
<400> 1221
   ggatgaactg aagactttgc ctgtt 25
<210> 1222
   <211> 25
   <212> DNA
   <213> Artificial Sequence
<220>
   <223> synthetic nucleotide probe
<400> 1222
   ggaggcccaa tttcactcaa atgtt 25
<210> 1223
   <211> 25
   <212> DNA
   <213> Artificial Sequence
<220>
   <223> synthetic nucleotide probe
<400> 1223
   gtttttctca acactacttt tctga 25
<210> 1224
   <211> 25
   <212> DNA
   <213> Artificial Sequence
<220>
   <223> synthetic nucleotide probe
<400> 1224
   gctcagctgg gagcatcatt ctcct 25
<210> 1225
   <211> 25
   <212> DNA
   <213> Artificial Sequence
<220>
   <223> synthetic nucleotide probe
<400> 1225
   gctcagctgg gagcatcatt ctcct 25
<210> 1226
   <211> 25
   <212> DNA
   <213> Artificial Sequence
<220>
   <223> synthetic nucleotide probe
<400> 1226
   gagaatggct tatgggggcc caggt 25
<210> 1227
   <211> 25
   <212> DNA
   <213> Artificial Sequence
<220>
   <223> synthetic nucleotide probe
<400> 1227
   gtttaatggt gatgcctcgc gtaca 25
<210> 1228
   <211> 25
   <212> DNA
   <213> Artificial Sequence
<220>
   <223> synthetic nucleotide probe
<400> 1228
   tctctagtcc tacccagttt taaag 25
<210> 1229
   <211> 25
   <212> DNA
   <213> Artificial Sequence
<220>
   <223> synthetic nucleotide probe
<400> 1229
   gcctcgcgta caggatctgg ttacc 25
<210> 1230
   <211> 25
   <212> DNA
   <213> Artificial Sequence
<220>
   <223> synthetic nucleotide probe
<400> 1230
   gttgggcaga tcagtgtctc tagtc 25
<210> 1231
   <211> 25
   <212> DNA
   <213> Artificial Sequence
<220>
   <223> synthetic nucleotide probe
<400> 1231
   caccatcatg tctaggccta tgcta 25
<210> 1232
   <211> 25
   <212> DNA
   <213> Artificial Sequence
<220>
   <223> synthetic nucleotide probe
<400> 1232
   gacctcatct cccgcaaata aatgt 25
<210> 1233
   <211> 25
   <212> DNA
   <213> Artificial Sequence
<220>
   <223> synthetic nucleotide probe
<400> 1233
   aacgcccgca gcacaacgaa gaggc 25
<210> 1234
   <211> 25
   <212> DNA
   <213> Artificial Sequence
<220>
   <223> synthetic nucleotide probe
<400> 1234
   ccgcagcaca acgaagaggc caatg 25
<210> 1235
   <211> 25
   <212> DNA
   <213> Artificial Sequence
<220>
   <223> synthetic nucleotide probe
<400> 1235
   aagaggccaa tgggcactct tccag 25
<210> 1236
   <211> 25
   <212> DNA
   <213> Artificial Sequence
<220>
   <223> synthetic nucleotide probe
<400> 1236
   cactcttcca gaggctttgt ggtgc 25
<210> 1237
   <211> 25
   <212> DNA
   <213> Artificial Sequence
<220>
   <223> synthetic nucleotide probe
<400> 1237
   tccagaggct ttgtggtgcg ggacc 25
<210> 1238
   <211> 25
   <212> DNA
   <213> Artificial Sequence
<220>
   <223> synthetic nucleotide probe
<400> 1238
   acctgctcca ctgtttaaca ctaaa 25
<210> 1239
   <211> 25
   <212> DNA
   <213> Artificial Sequence
<220>
   <223> synthetic nucleotide probe
<400> 1239
   aaccaaggtc atgagcattc gtgct 25
<210> 1240
   <211> 25
   <212> DNA
   <213> Artificial Sequence
<220>
   <223> synthetic nucleotide probe
<400> 1240
   taagataaca gactccagct cctgg 25
<210> 1241
   <211> 25
   <212> DNA
   <213> Artificial Sequence
<220>
   <223> synthetic nucleotide probe
<400> 1241
   taggattcca cttcctgtgt catga 25
<210> 1242
   <211> 25
   <212> DNA
   <213> Artificial Sequence
<220>
   <223> synthetic nucleotide probe
<400> 1242
   ccacttcctg tgtcatgacc tcagg 25
<210> 1243
   <211> 25
   <212> DNA
   <213> Artificial Sequence
<220>
   <223> synthetic nucleotide probe
<400> 1243
   gacctcagga aataaacgtc cttga 25
<210> 1244
   <211> 25
   <212> DNA
   <213> Artificial Sequence
<220>
   <223> synthetic nucleotide probe
<400> 1244
   tgggatgttc tggcagctgt gtcat 25
<210> 1245
   <211> 25
   <212> DNA
   <213> Artificial Sequence
<220>
   <223> synthetic nucleotide probe
<400> 1245
   ttgttgccat cacgttccta caaaa 25
<210> 1246
   <211> 25
   <212> DNA
   <213> Artificial Sequence
<220>
   <223> synthetic nucleotide probe
<400> 1246
   gcctttctct ccgtaaacta tttag 25
<210> 1247
   <211> 25
   <212> DNA
   <213> Artificial Sequence
<220>
   <223> synthetic nucleotide probe
<400> 1247
   aatagtgaac ttgattcccc tgctt 25
<210> 1248
   <211> 25
   <212> DNA
   <213> Artificial Sequence
<220>
   <223> synthetic nucleotide probe
<400> 1248
   atgctgctgg gttcattcat tcatt 25
<210> 1249
   <211> 25
   <212> DNA
   <213> Artificial Sequence
<220>
   <223> synthetic nucleotide probe
<400> 1249
   ctgcttccac taaatccagt tgtga 25
<210> 1250
   <211> 25
   <212> DNA
   <213> Artificial Sequence
<220>
   <223> synthetic nucleotide probe
<400> 1250
   gcactccgta actcaacatg gcatg 25
<210> 1251
   <211> 25
   <212> DNA
   <213> Artificial Sequence
<220>
   <223> synthetic nucleotide probe
<400> 1251
   gagaatattg gctgctgatt gttgc 25
<210> 1252
   <211> 25
   <212> DNA
   <213> Artificial Sequence
<220>
   <223> synthetic nucleotide probe
<400> 1252
   gtttagggat ctttctgatg gtctt 25
<210> 1253
   <211> 25
   <212> DNA
   <213> Artificial Sequence
<220>
   <223> synthetic nucleotide probe
<400> 1253
   ttttcagtat ctctgtacct gttaa 25
<210> 1254
   <211> 25
   <212> DNA
   <213> Artificial Sequence
<220>
   <223> synthetic nucleotide probe
<400> 1254
   cttagttcta agtcattgtt cccat 25
<210> 1255
   <211> 25
   <212> DNA
   <213> Artificial Sequence
<220>
   <223> synthetic nucleotide probe
<400> 1255
   aaatcttgtc agaagatccc agaaa 25
<210> 1256
   <211> 25
   <212> DNA
   <213> Artificial Sequence
<220>
   <223> synthetic nucleotide probe
<400> 1256
   aaagttctaa ttttcattag caatt 25
<210> 1257
   <211> 25
   <212> DNA
   <213> Artificial Sequence
<220>
   <223> synthetic nucleotide probe
<400> 1257
   atttgtactt tttggcctgg gatat 25
<210> 1258
   <211> 25
   <212> DNA
   <213> Artificial Sequence
<220>
   <223> synthetic nucleotide probe
<400> 1258
   gcctgggata tgggttttaa atgga 25
<210> 1259
   <211> 25
   <212> DNA
   <213> Artificial Sequence
<220>
   <223> synthetic nucleotide probe
<400> 1259
   aatggacatt gtctgtacca gcttc 25
<210> 1260
   <211> 25
   <212> DNA
   <213> Artificial Sequence
<220>
   <223> synthetic nucleotide probe
<400> 1260
   cattgtctgt accagcttca ttaaa 25
<210> 1261
   <211> 25
   <212> DNA
   <213> Artificial Sequence
<220>
   <223> synthetic nucleotide probe
<400> 1261
   aatgaccttt ccttaacttg aagct 25
<210> 1262
   <211> 25
   <212> DNA
   <213> Artificial Sequence
<220>
   <223> synthetic nucleotide probe
<400> 1262
   gacctttcct taacttgaag ctact 25
<210> 1263
   <211> 25
   <212> DNA
   <213> Artificial Sequence
<220>
   <223> synthetic nucleotide probe
<400> 1263
   gagggtctgg accaaaagaa gagga 25
<210> 1264
   <211> 25
   <212> DNA
   <213> Artificial Sequence
<220>
   <223> synthetic nucleotide probe
<400> 1264
   aggtgagagt aatgactaac tccaa 25
<210> 1265
   <211> 25
   <212> DNA
   <213> Artificial Sequence
<220>
   <223> synthetic nucleotide probe
<400> 1265
   ctaactccaa agatggcttc actga 25
<210> 1266
   <211> 25
   <212> DNA
   <213> Artificial Sequence
<220>
   <223> synthetic nucleotide probe
<400> 1266
   aagttcgagt tgtgctcttc acgtt 25
<210> 1267
   <211> 25
   <212> DNA
   <213> Artificial Sequence
<220>
   <223> synthetic nucleotide probe
<400> 1267
   tgtgctcttc acgttggttc gataa 25
<210> 1268
   <211> 25
   <212> DNA
   <213> Artificial Sequence
<220>
   <223> synthetic nucleotide probe
<400> 1268
   cacgttggtt cgataatggc cttta 25
<210> 1269
   <211> 25
   <212> DNA
   <213> Artificial Sequence
<220>
   <223> synthetic nucleotide probe
<400> 1269
   gtaaatattc cattctgatt tcata 25
<210> 1270
   <211> 25
   <212> DNA
   <213> Artificial Sequence
<220>
   <223> synthetic nucleotide probe
<400> 1270
   attaaacatt tatgcctccc ttttg 25
<210> 1271
   <211> 25
   <212> DNA
   <213> Artificial Sequence
<220>
   <223> synthetic nucleotide probe
<400> 1271
   cctccctttt gtgttgacac tgtag 25
<210> 1272
   <211> 25
   <212> DNA
   <213> Artificial Sequence
<220>
   <223> synthetic nucleotide probe
<400> 1272
   gttgacactg tagctcatac tggaa 25
<210> 1273
   <211> 25
   <212> DNA
   <213> Artificial Sequence
<220>
   <223> synthetic nucleotide probe
<400> 1273
   gtgattatcg accatggtat gcatg 25
<210> 1274
   <211> 25
   <212> DNA
   <213> Artificial Sequence
<220>
   <223> synthetic nucleotide probe
<400> 1274
   ggtatgcatg atcgttgtaa ttgtt 25
<210> 1275
   <211> 25
   <212> DNA
   <213> Artificial Sequence
<220>
   <223> synthetic nucleotide probe
<400> 1275
   ttttttgttt cagtaccaga ggcac 25
<210> 1276
   <211> 25
   <212> DNA
   <213> Artificial Sequence
<220>
   <223> synthetic nucleotide probe
<400> 1276
   gtaccagagg cactgacttc aataa 25
<210> 1277
   <211> 25
   <212> DNA
   <213> Artificial Sequence
<220>
   <223> synthetic nucleotide probe
<400> 1277
   aataatctct tgttatgcag ggagt 25
<210> 1278
   <211> 25
   <212> DNA
   <213> Artificial Sequence
<220>
   <223> synthetic nucleotide probe
<400> 1278
   tatgcaggga gtacagttct tttca 25
<210> 1279
   <211> 25
   <212> DNA
   <213> Artificial Sequence
<220>
   <223> synthetic nucleotide probe
<400> 1279
   tcttttcatt catacataag ttcag 25
<210> 1280
   <211> 25
   <212> DNA
   <213> Artificial Sequence
<220>
   <223> synthetic nucleotide probe
<400> 1280
   taagttcagt agttgcttcc ctaac 25
<210> 1281
   <211> 25
   <212> DNA
   <213> Artificial Sequence
<220>
   <223> synthetic nucleotide probe
<400> 1281
   gttgcttccc taactgcaaa ggcaa 25
<210> 1282
   <211> 25
   <212> DNA
   <213> Artificial Sequence
<220>
   <223> synthetic nucleotide probe
<400> 1282
   actgcaaagg caatctcatt tagtt 25
<210> 1283
   <211> 25
   <212> DNA
   <213> Artificial Sequence
<220>
   <223> synthetic nucleotide probe
<400> 1283
   gagtagctct tgaaagcagc tttga 25
<210> 1284
   <211> 25
   <212> DNA
   <213> Artificial Sequence
<220>
   <223> synthetic nucleotide probe
<400> 1284
   agaagtatgt gtgttacacc ctcac 25
<210> 1285
   <211> 25
   <212> DNA
   <213> Artificial Sequence
<220>
   <223> synthetic nucleotide probe
<400> 1285
   tgctgtgtgg ggcagttcaa cacaa 25
<210> 1286
   <211> 25
   <212> DNA
   <213> Artificial Sequence
<220>
   <223> synthetic nucleotide probe
<400> 1286
   gttggcatgt caaatgcatc ctcta 25
<210> 1287
   <211> 25
   <212> DNA
   <213> Artificial Sequence
<220>
   <223> synthetic nucleotide probe
<400> 1287
   acagcctgat gtttgggacc ttttt 25
<210> 1288
   <211> 25
   <212> DNA
   <213> Artificial Sequence
<220>
   <223> synthetic nucleotide probe
<400> 1288
   gttgggattt gcttcattgt ttgac 25
<210> 1289
   <211> 25
   <212> DNA
   <213> Artificial Sequence
<220>
   <223> synthetic nucleotide probe
<400> 1289
   tgcacagtct gttacaggtt gacac 25
<210> 1290
   <211> 25
   <212> DNA
   <213> Artificial Sequence
<220>
   <223> synthetic nucleotide probe
<400> 1290
   ttgacacatt gcttgacctg attta 25
<210> 1291
   <211> 25
   <212> DNA
   <213> Artificial Sequence
<220>
   <223> synthetic nucleotide probe
<400> 1291
   tagtgtagct ttaatgtgct gcaca 25
<210> 1292
   <211> 25
   <212> DNA
   <213> Artificial Sequence
<220>
   <223> synthetic nucleotide probe
<400> 1292
   gtgctgcaca tgatactggc agccc 25
<210> 1293
   <211> 25
   <212> DNA
   <213> Artificial Sequence
<220>
   <223> synthetic nucleotide probe
<400> 1293
   actggcagcc ctagagttca tagat 25
<210> 1294
   <211> 25
   <212> DNA
   <213> Artificial Sequence
<220>
   <223> synthetic nucleotide probe
<400> 1294
   gttcatagat ggacttttgg gaccc 25
<210> 1295
   <211> 25
   <212> DNA
   <213> Artificial Sequence
<220>
   <223> synthetic nucleotide probe
<400> 1295
   gggacccagc agttttgaaa tgtgt 25
<210> 1296
   <211> 25
   <212> DNA
   <213> Artificial Sequence
<220>
   <223> synthetic nucleotide probe
<400> 1296
   gcagcccctg tctaactgaa atttc 25
<210> 1297
   <211> 25
   <212> DNA
   <213> Artificial Sequence
<220>
   <223> synthetic nucleotide probe
<400> 1297
   ctaactgaaa tttctcttca ccttg 25
<210> 1298
   <211> 25
   <212> DNA
   <213> Artificial Sequence
<220>
   <223> synthetic nucleotide probe
<400> 1298
   cttcaccttg tacacttgac agctg 25
<210> 1299
   <211> 25
   <212> DNA
   <213> Artificial Sequence
<220>
   <223> synthetic nucleotide probe
<400> 1299
   agagtgtctg atgcggccac tcatt 25
<210> 1300
   <211> 25
   <212> DNA
   <213> Artificial Sequence
<220>
   <223> synthetic nucleotide probe
<400> 1300
   tgaagccgcc ctaaggattt tcctt 25
<210> 1301
   <211> 25
   <212> DNA
   <213> Artificial Sequence
<220>
   <223> synthetic nucleotide probe
<400> 1301
   ggggaacttc ttcatgggtg gttca 25
<210> 1302
   <211> 25
   <212> DNA
   <213> Artificial Sequence
<220>
   <223> synthetic nucleotide probe
<400> 1302
   ttgtgtgttc tgtacatgtg atgtt 25
<210> 1303
   <211> 25
   <212> DNA
   <213> Artificial Sequence
<220>
   <223> synthetic nucleotide probe
<400> 1303
   ctcccaatgc tgctcagctt gcagt 25
<210> 1304
   <211> 25
   <212> DNA
   <213> Artificial Sequence
<220>
   <223> synthetic nucleotide probe
<400> 1304
   gaggaggatg catttcaaaa gcttg 25
<210> 1305
   <211> 25
   <212> DNA
   <213> Artificial Sequence
<220>
   <223> synthetic nucleotide probe
<400> 1305
   gatgtcgtgc aaactgtact gtgaa 25
<210> 1306
   <211> 25
   <212> DNA
   <213> Artificial Sequence
<220>
   <223> synthetic nucleotide probe
<400> 1306
   ataggttgtc tctgcataca cgaac 25
<210> 1307
   <211> 25
   <212> DNA
   <213> Artificial Sequence
<220>
   <223> synthetic nucleotide probe
<400> 1307
   gattctttac ttagcttgtt tttag 25
<210> 1308
   <211> 25
   <212> DNA
   <213> Artificial Sequence
<220>
   <223> synthetic nucleotide probe
<400> 1308
   atttatatcc catctagaat tcagc 25
<210> 1309
   <211> 25
   <212> DNA
   <213> Artificial Sequence
<220>
   <223> synthetic nucleotide probe
<400> 1309
   tgcagtcatg cagggagcca acgtc 25
<210> 1310
   <211> 25
   <212> DNA
   <213> Artificial Sequence
<220>
   <223> synthetic nucleotide probe
<400> 1310
   gtggtgcact taacttgtgg aattt 25
<210> 1311
   <211> 25
   <212> DNA
   <213> Artificial Sequence
<220>
   <223> synthetic nucleotide probe
<400> 1311
   gtttgactgt accattgact gttat 25
<210> 1312
   <211> 25
   <212> DNA
   <213> Artificial Sequence
<220>
   <223> synthetic nucleotide probe
<400> 1312
   gatgaagttg cattacacct cactg 25
<210> 1313
   <211> 25
   <212> DNA
   <213> Artificial Sequence
<220>
   <223> synthetic nucleotide probe
<400> 1313
   aagttcagcg ttgtatgtct ctctc 25
<210> 1314
   <211> 25
   <212> DNA
   <213> Artificial Sequence
<220>
   <223> synthetic nucleotide probe
<400> 1314
   aatactgtac catactggtc tttgc 25
<210> 1315
   <211> 25
   <212> DNA
   <213> Artificial Sequence
<220>
   <223> synthetic nucleotide probe
<400> 1315
   tctttctggt gcccaaactt tcagg 25
<210> 1316
   <211> 25
   <212> DNA
   <213> Artificial Sequence
<220>
   <223> synthetic nucleotide probe
<400> 1316
   tacacgaacc taacccaaat ttgct 25
<210> 1317
   <211> 25
   <212> DNA
   <213> Artificial Sequence
<220>
   <223> synthetic nucleotide probe
<400> 1317
   gaattcagct aggtgctgct gctgc 25
<210> 1318
   <211> 25
   <212> DNA
   <213> Artificial Sequence
<220>
   <223> synthetic nucleotide probe
<400> 1318
   acgtcctcgt aactcagcgg aaggg 25
<210> 1319
   <211> 25
   <212> DNA
   <213> Artificial Sequence
<220>
   <223> synthetic nucleotide probe
<400> 1319
   ctctctctac actgtggtgc actta 25
<210> 1320
   <211> 25
   <212> DNA
   <213> Artificial Sequence
<220>
   <223> synthetic nucleotide probe
<400> 1320
   aatgacttga gtccagtgaa atctc 25
<210> 1321
   <211> 25
   <212> DNA
   <213> Artificial Sequence
<220>
   <223> synthetic nucleotide probe
<400> 1321
   tagcagtacc tccctaaagc atttt 25
<210> 1322
   <211> 25
   <212> DNA
   <213> Artificial Sequence
<220>
   <223> synthetic nucleotide probe
<400> 1322
   acacctcact gcaaggattc tttac 25
<210> 1323
   <211> 25
   <212> DNA
   <213> Artificial Sequence
<220>
   <223> synthetic nucleotide probe
<400> 1323
   ggctccccag aattcctaga ctggg 25
<210> 1324
   <211> 25
   <212> DNA
   <213> Artificial Sequence
<220>
   <223> synthetic nucleotide probe
<400> 1324
   ctctgtttcc tttgatgacg ctttg 25
<210> 1325
   <211> 25
   <212> DNA
   <213> Artificial Sequence
<220>
   <223> synthetic nucleotide probe
<400> 1325
   gaagtcacag gccctgtcat tgcgc 25
<210> 1326
   <211> 25
   <212> DNA
   <213> Artificial Sequence
<220>
   <223> synthetic nucleotide probe
<400> 1326
   gatgccaagt ccaatagcct catct 25
<210> 1327
   <211> 25
   <212> DNA
   <213> Artificial Sequence
<220>
   <223> synthetic nucleotide probe
<400> 1327
   cccacaacta cactctgtac ttcat 25
<210> 1328
   <211> 25
   <212> DNA
   <213> Artificial Sequence
<220>
   <223> synthetic nucleotide probe
<400> 1328
   ggagtacaaa ttcagcgtgg atgtg 25
<210> 1329
   <211> 25
   <212> DNA
   <213> Artificial Sequence
<220>
   <223> synthetic nucleotide probe
<400> 1329
   gattcagatt gagtcctgag gcatt 25
<210> 1330
   <211> 25
   <212> DNA
   <213> Artificial Sequence
<220>
   <223> synthetic nucleotide probe
<400> 1330
   gtaggaatcc tggttgtggg gacca 25
<210> 1331
   <211> 25
   <212> DNA
   <213> Artificial Sequence
<220>
   <223> synthetic nucleotide probe
<400> 1331
   actctggatc cagtgacagc aggtg 25
<210> 1332
   <211> 25
   <212> DNA
   <213> Artificial Sequence
<220>
   <223> synthetic nucleotide probe
<400> 1332
   acagcaggtg tcatgggtca agcat 25
<210> 1333
   <211> 25
   <212> DNA
   <213> Artificial Sequence
<220>
   <223> synthetic nucleotide probe
<400> 1333
   aatcatatat agcattttca ggcat 25
<210> 1334
   <211> 25
   <212> DNA
   <213> Artificial Sequence
<220>
   <223> synthetic nucleotide probe
<400> 1334
   ggcatgttcc tggtagttct tttga 25
<210> 1335
   <211> 25
   <212> DNA
   <213> Artificial Sequence
<220>
   <223> synthetic nucleotide probe
<400> 1335
   ctggtagttc ttttgagtct gacat 25
<210> 1336
   <211> 25
   <212> DNA
   <213> Artificial Sequence
<220>
   <223> synthetic nucleotide probe
<400> 1336
   gtatgatggt ttgactgtat ggcag 25
<210> 1337
   <211> 25
   <212> DNA
   <213> Artificial Sequence
<220>
   <223> synthetic nucleotide probe
<400> 1337
   ggatcttgat tgataactgc catga 25
<210> 1338
   <211> 25
   <212> DNA
   <213> Artificial Sequence
<220>
   <223> synthetic nucleotide probe
<400> 1338
   gtgtgttcat cctagagtta ttttt 25

<210> 1339
   <211> 25
   <212> DNA
   <213> Artificial Sequence
<220>
   <223> synthetic nucleotide probe
<400> 1339
   ccttcccctc caaattgtat acatt 25
<210> 1340
   <211> 25
   <212> DNA
   <213> Artificial Sequence
<220>
   <223> synthetic nucleotide probe
<400> 1340
   tgttgtagca gcctcttgtt ttttt 25
<210> 1341
   <211> 25
   <212> DNA
   <213> Artificial Sequence
<220>
   <223> synthetic nucleotide probe
<400> 1341
   gcgtggcagc ggaagacgat tccca 25
<210> 1342
   <211> 25
   <212> DNA
   <213> Artificial Sequence
<220>
   <223> synthetic nucleotide probe
<400> 1342
   aattcctatg ttcagtagcg tggtt 25
<210> 1343
   <211> 25
   <212> DNA
   <213> Artificial Sequence
<220>
   <223> synthetic nucleotide probe
<400> 1343
   aactgccatg atattttgct ttgat 25
<210> 1344
   <211> 25
   <212> DNA
   <213> Artificial Sequence
<220>
   <223> synthetic nucleotide probe
<400> 1344
   acatgtagtt gcacacggtt cagta 25
<210> 1345
   <211> 25
   <212> DNA
   <213> Artificial Sequence
<220>
   <223> synthetic nucleotide probe
<400> 1345
   tttcctcagt cttcaatgac gagag 25
<210> 1346
   <211> 25
   <212> DNA
   <213> Artificial Sequence
<220>
   <223> synthetic nucleotide probe
<400> 1346
   atgttcacaa cttgcgtgcg tggca 25
<210> 1347
   <211> 25
   <212> DNA
   <213> Artificial Sequence
<220>
   <223> synthetic nucleotide probe
<400> 1347
   agtccttgaa gctttacagt taatt 25
<210> 1348
   <211> 25
   <212> DNA
   <213> Artificial Sequence
<220>
   <223> synthetic nucleotide probe
<400> 1348
   acctttatgc tcgttccata tttgg 25
<210> 1349
   <211> 25
   <212> DNA
   <213> Artificial Sequence
<220>
   <223> synthetic nucleotide probe
<400> 1349
   tatggcagcc aacctttatg ctcgt 25
<210> 1350
   <211> 25
   <212> DNA
   <213> Artificial Sequence
<220>
   <223> synthetic nucleotide probe
<400> 1350
   gtttcatgta ccaagaccct tttca 25
<210> 1351
   <211> 25
   <212> DNA
   <213> Artificial Sequence
<220>
   <223> synthetic nucleotide probe
<400> 1351
   gtttgtcttt tgtcttaaca gttct 25
<210> 1352
   <211> 25
   <212> DNA
   <213> Artificial Sequence
<220>
   <223> synthetic nucleotide probe
<400> 1352
   gaatgctgtc ctcaaagtat ataat 25
<210> 1353
   <211> 25
   <212> DNA
   <213> Artificial Sequence
<220>
   <223> synthetic nucleotide probe
<400> 1353
   tgctgtcctc aaagtatata atgtt 25
<210> 1354
   <211> 25
   <212> DNA
   <213> Artificial Sequence
<220>
   <223> synthetic nucleotide probe
<400> 1354
   gatgcacttg caaatgtcag cattg 25
<210> 1355
   <211> 25
   <212> DNA
   <213> Artificial Sequence
<220>
   <223> synthetic nucleotide probe
<400> 1355
   accaagaccc ttttcacagt acaat 25
<210> 1356
   <211> 25
   <212> DNA
   <213> Artificial Sequence
<220>
   <223> synthetic nucleotide probe
<400> 1356
   gaatactttt cagccaataa tttat 25
<210> 1357
   <211> 25
   <212> DNA
   <213> Artificial Sequence
<220>
   <223> synthetic nucleotide probe
<400> 1357
   gacccttttc acagtacaat aaaca 25
<210> 1358
   <211> 25
   <212> DNA
   <213> Artificial Sequence
<220>
   <223> synthetic nucleotide probe
<400> 1358
   taatgtttca tgtaccaaga ccctt 25
<210> 1359
   <211> 25
   <212> DNA
   <213> Artificial Sequence
<220>
   <223> synthetic nucleotide probe
<400> 1359
   ctgctgttac cggccatata agaat 25
<210> 1360
   <211> 25
   <212> DNA
   <213> Artificial Sequence
<220>
   <223> synthetic nucleotide probe
<400> 1360
   catgtaccaa gacccttttc acagt 25
<210> 1361
   <211> 25
   <212> DNA
   <213> Artificial Sequence
<220>
   <223> synthetic nucleotide probe
<400> 1361
   aaatgactac ttacagcaca tatta 25
<210> 1362
   <211> 25
   <212> DNA
   <213> Artificial Sequence
<220>
   <223> synthetic nucleotide probe
<400> 1362
   ggtatgggct tactggactc caaca 25
<210> 1363
   <211> 25
   <212> DNA
   <213> Artificial Sequence
<220>
   <223> synthetic nucleotide probe
<400> 1363
   taacagttct gaatgctgtc ctcaa 25
<210> 1364
   <211> 25
   <212> DNA
   <213> Artificial Sequence
<220>
   <223> synthetic nucleotide probe
<400> 1364
   gtcttttgtc ttaacagttc tgaat 25
<210> 1365
   <211> 25
   <212> DNA
   <213> Artificial Sequence
<220>
   <223> synthetic nucleotide probe
<400> 1365
   gaagccaatt caccagggac cagat 25
<210> 1366
   <211> 25
   <212> DNA
   <213> Artificial Sequence
<220>
   <223> synthetic nucleotide probe
<400> 1366
   actccaacat cttttgtact ctttc 25
<210> 1367
   <211> 25
   <212> DNA
   <213> Artificial Sequence
<220>
   <223> synthetic nucleotide probe
<400> 1367
   agttctgaat gctgtcctca aagta 25
<210> 1368
   <211> 25
   <212> DNA
   <213> Artificial Sequence
<220>
   <223> synthetic nucleotide probe
<400> 1368
   gccctttctg gttactgtgg cttta 25
<210> 1369
   <211> 25
   <212> DNA
   <213> Artificial Sequence
<220>
   <223> synthetic nucleotide probe
<400> 1369
   gagatctgag gaggcttcgt gggct 25
<210> 1370
   <211> 25
   <212> DNA
   <213> Artificial Sequence
<220>
   <223> synthetic nucleotide probe
<400> 1370
   gcactggcac ctagacatgt acaac 25
<210> 1371
   <211> 25
   <212> DNA
   <213> Artificial Sequence
<220>
   <223> synthetic nucleotide probe
<400> 1371
   cgggtggttc actatgagat ctgag 25
<210> 1372
   <211> 25
   <212> DNA
   <213> Artificial Sequence
<220>
   <223> synthetic nucleotide probe
<400> 1372
   tggtatagct gggaccagcc gggcc 25
<210> 1373
   <211> 25
   <212> DNA
   <213> Artificial Sequence
<220>
   <223> synthetic nucleotide probe
<400> 1373
   tgggtcctct aactaggact ccctc 25
<210> 1374
   <211> 25
   <212> DNA
   <213> Artificial Sequence
<220>
   <223> synthetic nucleotide probe
<400> 1374
   ccctgaccgc tcacagcatg agaga 25
<210> 1375
   <211> 25
   <212> DNA
   <213> Artificial Sequence
<220>
   <223> synthetic nucleotide probe
<400> 1375
   gccgggcctg aggttgaact ggggt 25
<210> 1376
   <211> 25
   <212> DNA
   <213> Artificial Sequence
<220>
   <223> synthetic nucleotide probe
<400> 1376
   cggtgatccc tccaaagaac cagag 25
<210> 1377
   <211> 25
   <212> DNA
   <213> Artificial Sequence
<220>
   <223> synthetic nucleotide probe
<400> 1377
   tacgaacctt acccggatga tggca 25
<210> 1378
   <211> 25
   <212> DNA
   <213> Artificial Sequence
<220>
   <223> synthetic nucleotide probe
<400> 1378
   acacctgttt cttggcatgt catgt 25
<210> 1379
   <211> 25
   <212> DNA
   <213> Artificial Sequence
<220>
   <223> synthetic nucleotide probe
<400> 1379
   aaccgtgtgg atacatcccc cacac 25
<210> 1380
   <211> 25
   <212> DNA
   <213> Artificial Sequence
<220>
   <223> synthetic nucleotide probe
<400> 1380
   tcatgtgctg ggtgggggac gtgta 25
<210> 1381
   <211> 25
   <212> DNA
   <213> Artificial Sequence
<220>
   <223> synthetic nucleotide probe
<400> 1381
   tctaactagg actccctcat tccta 25
<210> 1382
   <211> 25
   <212> DNA
   <213> Artificial Sequence
<220>
   <223> synthetic nucleotide probe
<400> 1382
   ggactccctc attcctagaa attta 25
<210> 1383
   <211> 25
   <212> DNA
   <213> Artificial Sequence
<220>
   <223> synthetic nucleotide probe
<400> 1383
   catgaccaag gacatgttcc cgggg 25
<210> 1384
   <211> 25
   <212> DNA
   <213> Artificial Sequence
<220>
   <223> synthetic nucleotide probe
<400> 1384
   gatccctcca aagaaccaga gcggg 25
<210> 1385
   <211> 25
   <212> DNA
   <213> Artificial Sequence
<220>
   <223> synthetic nucleotide probe
<400> 1385
   ccagagcggg tggttcacta tgaga 25
<210> 1386
   <211> 25
   <212> DNA
   <213> Artificial Sequence
<220>
   <223> synthetic nucleotide probe
<400> 1386
   attggccttt tacctggata taaat 25
<210> 1387
   <211> 25
   <212> DNA
   <213> Artificial Sequence
<220>
   <223> synthetic nucleotide probe
<400> 1387
   accatccaac agacctggtg ctcta 25
<210> 1388
   <211> 25
   <212> DNA
   <213> Artificial Sequence
<220>
   <223> synthetic nucleotide probe
<400> 1388
   ccatccaaca gacctggtgc tctaa 25
<210> 1389
   <211> 25
   <212> DNA
   <213> Artificial Sequence
<220>
   <223> synthetic nucleotide probe
<400> 1389
   tgctctaatg ccaagttata cacgg 25
<210> 1390
   <211> 25
   <212> DNA
   <213> Artificial Sequence
<220>
   <223> synthetic nucleotide probe
<400> 1390
   ataggctctc agtaagaagt ctgat 25
<210> 1391
   <211> 25
   <212> DNA
   <213> Artificial Sequence
<220>
   <223> synthetic nucleotide probe
<400> 1391
   ggtataaagc tctcaaatgt gacca 25
<210> 1392
   <211> 25
   <212> DNA
   <213> Artificial Sequence
<220>
   <223> synthetic nucleotide probe
<400> 1392
   aagctctcaa atgtgaccat gtgaa 25
<210> 1393
   <211> 25
   <212> DNA
   <213> Artificial Sequence
<220>
   <223> synthetic nucleotide probe
<400> 1393
   taatggactc agctctgtct gctca 25
<210> 1394
   <211> 25
   <212> DNA
   <213> Artificial Sequence
<220>
   <223> synthetic nucleotide probe
<400> 1394
   aatgccattg tgcagagaag caccc 25
<210> 1395
   <211> 25
   <212> DNA
   <213> Artificial Sequence
<220>
   <223> synthetic nucleotide probe
<400> 1395
   gaagcaccct aatgcataag ctttt 25
<210> 1396
   <211> 25
   <212> DNA
   <213> Artificial Sequence
<220>
   <223> synthetic nucleotide probe
<400> 1396
   ctaatgcata agctttttaa tgctg 25
<210> 1397
   <211> 25
   <212> DNA
   <213> Artificial Sequence
<220>
   <223> synthetic nucleotide probe
<400> 1397
   aattaaatgc cactttttca gaggt 25
<210> 1398
   <211> 25
   <212> DNA
   <213> Artificial Sequence
<220>
   <223> synthetic nucleotide probe
<400> 1398
   ccactttttc agaggtgaat taatg 25
<210> 1399
   <211> 25
   <212> DNA
   <213> Artificial Sequence
<220>
   <223> synthetic nucleotide probe
<400> 1399
   taaatggaac tattccatca atagg 25
<210> 1400
   <211> 25
   <212> DNA
   <213> Artificial Sequence
<220>
   <223> synthetic nucleotide probe
<400> 1400
   cactgtatac cgatcaggaa tcttg 25
<210> 1401
   <211> 25
   <212> DNA
   <213> Artificial Sequence
<220>
   <223> synthetic nucleotide probe
<400> 1401
   ataccgatca ggaatcttgc tccaa 25
<210> 1402
   <211> 25
   <212> DNA
   <213> Artificial Sequence
<220>
   <223> synthetic nucleotide probe
<400> 1402
   ttgccactat gaccaaacgc acagt 25
<210> 1403
   <211> 25
   <212> DNA
   <213> Artificial Sequence
<220>
   <223> synthetic nucleotide probe
<400> 1403
   aaacgcacag tctgttctgc agcaa 25
<210> 1404
   <211> 25
   <212> DNA
   <213> Artificial Sequence
<220>
   <223> synthetic nucleotide probe
<400> 1404
   ctgcagcaac aacgggattc aatca 25
<210> 1405
   <211> 25
   <212> DNA
   <213> Artificial Sequence
<220>
   <223> synthetic nucleotide probe
<400> 1405
   tcaactcagt cgtgattcag ccgta 25
<210> 1406
   <211> 25
   <212> DNA
   <213> Artificial Sequence
<220>
   <223> synthetic nucleotide probe
<400> 1406
   tcagccgtag aaatgctttt ccttt 25
<210> 1407
   <211> 25
   <212> DNA
   <213> Artificial Sequence
<220>
   <223> synthetic nucleotide probe
<400> 1407
   ttatcttgtt tgagcttttc ctttc 25
<210> 1408
   <211> 25
   <212> DNA
   <213> Artificial Sequence
<220>
   <223> synthetic nucleotide probe
<400> 1408
   gaacttgtgt tgtactctgt agaaa 25
<210> 1409
   <211> 25
   <212> DNA
   <213> Artificial Sequence
<220>
   <223> synthetic nucleotide probe
<400> 1409
   gtcccaatgg ggaacctaaa tctgt 25
<210> 1410
   <211> 25
   <212> DNA
   <213> Artificial Sequence
<220>
   <223> synthetic nucleotide probe
<400> 1410
   gttttaattg cacagacaca tggac 25
<210> 1411
   <211> 25
   <212> DNA
   <213> Artificial Sequence
<220>
   <223> synthetic nucleotide probe
<400> 1411
   aaagtcattt tgtatctgcc aagtg 25
<210> 1412
   <211> 25
   <212> DNA
   <213> Artificial Sequence
<220>
   <223> synthetic nucleotide probe
<400> 1412
   atctgccaag tgtggtacct tcctt 25
<210> 1413
   <211> 25
   <212> DNA
   <213> Artificial Sequence
<220>
   <223> synthetic nucleotide probe
<400> 1413
   tagggagcat tttccttcta gtcta 25
<210> 1414
   <211> 25
   <212> DNA
   <213> Artificial Sequence
<220>
   <223> synthetic nucleotide probe
<400> 1414
   gcattgtctg aagttagcac ctctt 25
<210> 1415
   <211> 25
   <212> DNA
   <213> Artificial Sequence
<220>
   <223> synthetic nucleotide probe
<400> 1415
   gcacctcttg gactgaatcg tttgt 25
<210> 1416
   <211> 25
   <212> DNA
   <213> Artificial Sequence
<220>
   <223> synthetic nucleotide probe
<400> 1416
   gaatcgtttg tctagactac atgta 25
<210> 1417
   <211> 25
   <212> DNA
   <213> Artificial Sequence
<220>
   <223> synthetic nucleotide probe
<400> 1417
   gatcatgtgc atatcatccc attgt 25
<210> 1418
   <211> 25
   <212> DNA
   <213> Artificial Sequence
<220>
   <223> synthetic nucleotide probe
<400> 1418
   atcatcccat tgtaaagcga cttca 25
<210> 1419
   <211> 25
   <212> DNA
   <213> Artificial Sequence
<220>
   <223> synthetic nucleotide probe
<400> 1419
   gtgtgtgctg tcgcttgtcg acaac 25
<210> 1420
   <211> 25
   <212> DNA
   <213> Artificial Sequence
<220>
   <223> synthetic nucleotide probe
<400> 1420
   gtcgcttgtc gacaacagct ttttg 25
<210> 1421
   <211> 25
   <212> DNA
   <213> Artificial Sequence
<220>
   <223> synthetic nucleotide probe
<400> 1421
   atttgtgagc cttcattaac tcgaa 25
<210> 1422
   <211> 25
   <212> DNA
   <213> Artificial Sequence
<220>
   <223> synthetic nucleotide probe
<400> 1422
   gttagaatag gctgcatctt tttaa 25
<210> 1423
   <211> 25
   <212> DNA
   <213> Artificial Sequence
<220>
   <223> synthetic nucleotide probe
<400> 1423
   acaactctgg cttttgagat gactt 25
<210> 1424
   <211> 25
   <212> DNA
   <213> Artificial Sequence
<220>
   <223> synthetic nucleotide probe
<400> 1424
   ttcacctgca gtcgcctaga aaact 25
<210> 1425
   <211> 25
   <212> DNA
   <213> Artificial Sequence
<220>
   <223> synthetic nucleotide probe
<400> 1425
   aaacttgctc tcaaacttca gggtt 25
<210> 1426
   <211> 25
   <212> DNA
   <213> Artificial Sequence
<220>
   <223> synthetic nucleotide probe
<400> 1426
   aagtctcatt tctgtgtttt gcctg 25
<210> 1427
   <211> 25
   <212> DNA
   <213> Artificial Sequence
<220>
   <223> synthetic nucleotide probe
<400> 1427
   cctctgatgc tgggacccgg aaggc 25
<210> 1428
   <211> 25
   <212> DNA
   <213> Artificial Sequence
<220>
   <223> synthetic nucleotide probe
<400> 1428
   atacctgttg tgagacccga ggggc 25
<210> 1429
   <211> 25
   <212> DNA
   <213> Artificial Sequence
<220>
   <223> synthetic nucleotide probe
<400> 1429
   cggcgcggtt ttttatggtg acaca 25
<210> 1430
   <211> 25
   <212> DNA
   <213> Artificial Sequence
<220>
   <223> synthetic nucleotide probe
<400> 1430
   tccaggctca gtattgtgac cgcgg 25
<210> 1431
   <211> 25
   <212> DNA
   <213> Artificial Sequence
<220>
   <223> synthetic nucleotide probe
<400> 1431
   tgccttaccc gatggcttgt gacgc 25
<210> 1432
   <211> 25
   <212> DNA
   <213> Artificial Sequence
<220>
   <223> synthetic nucleotide probe
<400> 1432
   tgttcgctgt ggacgctgta gaggc 25
<210> 1433
   <211> 25
   <212> DNA
   <213> Artificial Sequence
<220>
   <223> synthetic nucleotide probe
<400> 1433
   gttggccagt ctgtacctgg acttc 25
<210> 1434
   <211> 25
   <212> DNA
   <213> Artificial Sequence
<220>
   <223> synthetic nucleotide probe
<400> 1434
   gaataaatct tctgtatcct caaaa 25
<210> 1435
   <211> 25
   <212> DNA
   <213> Artificial Sequence
<220>
   <223> synthetic nucleotide probe
<400> 1435
   gtttacaggg tctgtttcac ccagc 25
<210> 1436
   <211> 25
   <212> DNA
   <213> Artificial Sequence
<220>
   <223> synthetic nucleotide probe
<400> 1436
   ttcacccagc ccggaaagtc agaga 25
<210> 1437
   <211> 25
   <212> DNA
   <213> Artificial Sequence
<220>
   <223> synthetic nucleotide probe
<400> 1437
   caactccatc tacattggtt cccag 25
<210> 1438
   <211> 25
   <212> DNA
   <213> Artificial Sequence
<220>
   <223> synthetic nucleotide probe
<400> 1438
   ctcatagcac attacccaag aatct 25
<210> 1439
   <211> 25
   <212> DNA
   <213> Artificial Sequence
<220>
   <223> synthetic nucleotide probe
<400> 1439
   gaacacctat attcgttatg aactt 25
<210> 1440
   <211> 25
   <212> DNA
   <213> Artificial Sequence
<220>
   <223> synthetic nucleotide probe
<400> 1440
   ttaatgcaca gctacttcac acctt 25
<210> 1441
   <211> 25
   <212> DNA
   <213> Artificial Sequence
<220>
   <223> synthetic nucleotide probe
<400> 1441
   cacaccttaa acttgctttg atttg 25
<210> 1442
   <211> 25
   <212> DNA
   <213> Artificial Sequence
<220>
   <223> synthetic nucleotide probe
<400> 1442
   aataacctgt ctttgttttt gatgt 25
<210> 1443
   <211> 25
   <212> DNA
   <213> Artificial Sequence
<220>
   <223> synthetic nucleotide probe
<400> 1443
   gtaaatgcca gtagtgacca agaac 25
<210> 1444
   <211> 25
   <212> DNA
   <213> Artificial Sequence
<220>
   <223> synthetic nucleotide probe
<400> 1444
   tacactatac tggagggatt tcatt 25
<210> 1445
   <211> 25
   <212> DNA
   <213> Artificial Sequence
<220>
   <223> synthetic nucleotide probe
<400> 1445
   gatttagaac tcattccttg tgttt 25
<210> 1446
   <211> 25
   <212> DNA
   <213> Artificial Sequence
<220>
   <223> synthetic nucleotide probe
<400> 1446
   actggataat cgtagctttt aatgt 25
<210> 1447
   <211> 25
   <212> DNA
   <213> Artificial Sequence
<220>
   <223> synthetic nucleotide probe
<400> 1447
   gtagctttta atgttgcgcc tcttc 25
<210> 1448
   <211> 25
   <212> DNA
   <213> Artificial Sequence
<220>
   <223> synthetic nucleotide probe
<400> 1448
   gtgacaacat tggctacatt acctt 25
<210> 1449
   <211> 25
   <212> DNA
   <213> Artificial Sequence
<220>
   <223> synthetic nucleotide probe
<400> 1449
   tgcgcctctt caggttctta aggga 25
<210> 1450
   <211> 25
   <212> DNA
   <213> Artificial Sequence
<220>
   <223> synthetic nucleotide probe
<400> 1450
   gctgtgcttg caaagacttc atagt 25
<210> 1451
   <211> 25
   <212> DNA
   <213> Artificial Sequence
<220>
   <223> synthetic nucleotide probe
<400> 1451
   atcttccggc atccaaggat tccat 25
<210> 1452
   <211> 25
   <212> DNA
   <213> Artificial Sequence
<220>
   <223> synthetic nucleotide probe
<400> 1452
   gagctgaaag acacagacgc cgctg 25
<210> 1453
   <211> 25
   <212> DNA
   <213> Artificial Sequence
<220>
   <223> synthetic nucleotide probe
<400> 1453
   aaagaaggac gcagagccag ccaca 25
<210> 1454
   <211> 25
   <212> DNA
   <213> Artificial Sequence
<220>
   <223> synthetic nucleotide probe
<400> 1454
   atctgcagaa acgagctgtg cttgc 25
<210> 1455
   <211> 25
   <212> DNA
   <213> Artificial Sequence
<220>
   <223> synthetic nucleotide probe
<400> 1455
   gtctctttgc tatatgacct tgaaa 25
<210> 1456
   <211> 25
   <212> DNA
   <213> Artificial Sequence
<220>
   <223> synthetic nucleotide probe
<400> 1456
   gaggaagcgg ctggcaactg aaggc 25
<210> 1457
   <211> 25
   <212> DNA
   <213> Artificial Sequence
<220>
   <223> synthetic nucleotide probe
<400> 1457
   ctcttttcca agctgtttcg ctttg 25
<210> 1458
   <211> 25
   <212> DNA
   <213> Artificial Sequence
<220>
   <223> synthetic nucleotide probe
<400> 1458
   cgttttcatc ccgctaatct tggga 25
<210> 1459
   <211> 25
   <212> DNA
   <213> Artificial Sequence
<220>
   <223> synthetic nucleotide probe
<400> 1459
   gtttcgcttt gcaatatatt actgg 25
<210> 1460
   <211> 25
   <212> DNA
   <213> Artificial Sequence
<220>
   <223> synthetic nucleotide probe
<400> 1460
   ggaacacttg ctactggata atcgt 25
<210> 1461
   <211> 25
   <212> DNA
   <213> Artificial Sequence
<220>
   <223> synthetic nucleotide probe
<400> 1461
   gaagcgaaat tgttttgcct ctgtc 25
<210> 1462
   <211> 25
   <212> DNA
   <213> Artificial Sequence
<220>
   <223> synthetic nucleotide probe
<400> 1462
   gagtcacagt agtcttcagc acagt 25
<210> 1463
   <211> 25
   <212> DNA
   <213> Artificial Sequence
<220>
   <223> synthetic nucleotide probe
<400> 1463
   ggaagcggct ggcaactgaa ggctg 25
<210> 1464
   <211> 25
   <212> DNA
   <213> Artificial Sequence
<220>
   <223> synthetic nucleotide probe
<400> 1464
   tgcagtcata acttgttttc tccta 25
<210> 1465
   <211> 25
   <212> DNA
   <213> Artificial Sequence
<220>
   <223> synthetic nucleotide probe
<400> 1465
   tcctctttag ccacagggaa cctcc 25
<210> 1466
   <211> 25
   <212> DNA
   <213> Artificial Sequence
<220>
   <223> synthetic nucleotide probe
<400> 1466
   gaccttgaaa atcttccggc atcca 25
<210> 1467
   <211> 25
   <212> DNA
   <213> Artificial Sequence
<220>
   <223> synthetic nucleotide probe
<400> 1467
   ggattccatt gtgcatcaag ctggc 25
<210> 1468
   <211> 25
   <212> DNA
   <213> Artificial Sequence
<220>
   <223> synthetic nucleotide probe
<400> 1468
   ttcatcccgc taatcttggg aataa 25
<210> 1469
   <211> 25
   <212> DNA
   <213> Artificial Sequence
<220>
   <223> synthetic nucleotide probe
<400> 1469
   gagactcaac atcaggatcc acagc 25
<210> 1470
   <211> 25
   <212> DNA
   <213> Artificial Sequence
<220>
   <223> synthetic nucleotide probe
<400> 1470
   acagacttta tcgctctgtg gctca 25
<210> 1471
   <211> 25
   <212> DNA
   <213> Artificial Sequence
<220>
   <223> synthetic nucleotide probe
<400> 1471
   aagcagcaac agctgaggcg cacca 25
<210> 1472
   <211> 25
   <212> DNA
   <213> Artificial Sequence
<220>
   <223> synthetic nucleotide probe
<400> 1472
   gcttccattt ctttaacgtc tgttc 25
<210> 1473
   <211> 25
   <212> DNA
   <213> Artificial Sequence
<220>
   <223> synthetic nucleotide probe
<400> 1473
   tctgttccct taacatcgct gaaat 25
<210> 1474
   <211> 25
   <212> DNA
   <213> Artificial Sequence
<220>
   <223> synthetic nucleotide probe
<400> 1474
   gaagagatgc cttgcggtgt ggcca 25
<210> 1475
   <211> 25
   <212> DNA
   <213> Artificial Sequence
<220>
   <223> synthetic nucleotide probe
<400> 1475
   gactcagaaa ccttggtact cgccc 25
<210> 1476
   <211> 25
   <212> DNA
   <213> Artificial Sequence
<220>
   <223> synthetic nucleotide probe
<400> 1476
   actggctcct gcattaaccc agaaa 25
<210> 1477
   <211> 25
   <212> DNA
   <213> Artificial Sequence
<220>
   <223> synthetic nucleotide probe
<400> 1477
   taacccagaa atacctcgct tctat 25
<210> 1478
   <211> 25
   <212> DNA
   <213> Artificial Sequence
<220>
   <223> synthetic nucleotide probe
<400> 1478
   ctcgcttcta tctgtgcact tagct 25
<210> 1479
   <211> 25
   <212> DNA
   <213> Artificial Sequence
<220>
   <223> synthetic nucleotide probe
<400> 1479
   gggaacttac ccactgtaat cacct 25
<210> 1480
   <211> 25
   <212> DNA
   <213> Artificial Sequence
<220>
   <223> synthetic nucleotide probe
<400> 1480
   ttgtgccaag gaagtagctg cccca 25
<210> 1481
   <211> 25
   <212> DNA
   <213> Artificial Sequence
<220>
   <223> synthetic nucleotide probe
<400> 1481
   ccttctccgc gtcattgttg gaaga 25
<210> 1482
   <211> 25
   <212> DNA
   <213> Artificial Sequence

<220>
   <223> synthetic nucleotide probe
<400> 1482
   aggagagatg catcgagcag tccca 25
<210> 1483
   <211> 25
   <212> DNA
   <213> Artificial Sequence
<220>
   <223> synthetic nucleotide probe
<400> 1483
   gctgcttttc atttattact tcttc 25
<210> 1484
   <211> 25
   <212> DNA
   <213> Artificial Sequence
<220>
   <223> synthetic nucleotide probe
<400> 1484
   cttcttcttt ccaggacctg acaga 25
<210> 1485
   <211> 25
   <212> DNA
   <213> Artificial Sequence
<220>
   <223> synthetic nucleotide probe
<400> 1485
   ttatgtccaa acttagcacc tgcaa 25
<210> 1486
   <211> 25
   <212> DNA
   <213> Artificial Sequence
<220>
   <223> synthetic nucleotide probe
<400> 1486
   tgtgcgtctg cgagcgcaca cacat 25
<210> 1487
   <211> 25
   <212> DNA
   <213> Artificial Sequence
<220>
   <223> synthetic nucleotide probe
<400> 1487
   aggagttgcg gttgctccat gttct 25
<210> 1488
   <211> 25
   <212> DNA
   <213> Artificial Sequence
<220>
   <223> synthetic nucleotide probe
<400> 1488
   gctccatgtt ctgacttagg gcaat 25
<210> 1489
   <211> 25
   <212> DNA
   <213> Artificial Sequence
<220>
   <223> synthetic nucleotide probe
<400> 1489
   ctgcacttgg ggtctgtctg tacag 25
<210> 1490
   <211> 25
   <212> DNA
   <213> Artificial Sequence
<220>
   <223> synthetic nucleotide probe
<400> 1490
   gtctgtacag ttactcatgt cattg 25
<210> 1491
   <211> 25
   <212> DNA
   <213> Artificial Sequence
<220>
   <223> synthetic nucleotide probe
<400> 1491
   ttcagtgaga cctctgcttt catgc 25
<210> 1492
   <211> 25
   <212> DNA
   <213> Artificial Sequence
<220>
   <223> synthetic nucleotide probe
<400> 1492
   agcatgtttg catagcaacc agtca 25
<210> 1493
   <211> 25
   <212> DNA
   <213> Artificial Sequence
<220>
   <223> synthetic nucleotide probe
<400> 1493
   gttgccagtg atgattttcc tattc 25
<210> 1494
   <211> 25
   <212> DNA
   <213> Artificial Sequence
<220>
   <223> synthetic nucleotide probe
<400> 1494
   atttctgctg atatactcac cttag 25
<210> 1495
   <211> 25
   <212> DNA
   <213> Artificial Sequence
<220>
   <223> synthetic nucleotide probe
<400> 1495
   gctgcctttc ttcagcaaca gaccc 25
<210> 1496
   <211> 25
   <212> DNA
   <213> Artificial Sequence
<220>
   <223> synthetic nucleotide probe
<400> 1496
   agcaaccagt caagagcatt tacac 25
<210> 1497
   <211> 25
   <212> DNA
   <213> Artificial Sequence
<220>
   <223> synthetic nucleotide probe
<400> 1497
   tgaaggtgcc ccaggggctc ctatt 25
<210> 1498
   <211> 25
   <212> DNA
   <213> Artificial Sequence
<220>
   <223> synthetic nucleotide probe
<400> 1498
   agtatggttt cctgaagtat tctga 25
<210> 1499
   <211> 25
   <212> DNA
   <213> Artificial Sequence
<220>
   <223> synthetic nucleotide probe
<400> 1499
   taaaactaaa tttctttcat gtcct 25
<210> 1500
   <211> 25
   <212> DNA
   <213> Artificial Sequence
<220>
   <223> synthetic nucleotide probe
<400> 1500
   tgctcagaac cctggtgctt tattt 25
<210> 1501
   <211> 25
   <212> DNA
   <213> Artificial Sequence
<220>
   <223> synthetic nucleotide probe
<400> 1501
   gcgtacagca acctcttggt caaac 25
<210> 1502
   <211> 25
   <212> DNA
   <213> Artificial Sequence
<220>
   <223> synthetic nucleotide probe
<400> 1502
   ttctcttcca ctcaatattg ccatt 25
<210> 1503
   <211> 25
   <212> DNA
   <213> Artificial Sequence
<220>
   <223> synthetic nucleotide probe
<400> 1503
   ggactagtcc tcattagcat gtttg 25
<210> 1504
   <211> 25
   <212> DNA
   <213> Artificial Sequence
<220>
   <223> synthetic nucleotide probe
<400> 1504
   catacccaca tagttagcac cagca 25
<210> 1505
   <211> 25
   <212> DNA
   <213> Artificial Sequence
<220>
   <223> synthetic nucleotide probe
<400> 1505
   gagcatttac actatttctg ctgat 25
<210> 1506
   <211> 25
   <212> DNA
   <213> Artificial Sequence
<220>
   <223> synthetic nucleotide probe
<400> 1506
   agaaggattg accatgcata cccac 25
<210> 1507
   <211> 25
   <212> DNA
   <213> Artificial Sequence
<220>
   <223> synthetic nucleotide probe
<400> 1507
   gcaccagcaa cttcagtgag acctc 25
<210> 1508
   <211> 25
   <212> DNA
   <213> Artificial Sequence
<220>
   <223> synthetic nucleotide probe
<400> 1508
   ttgaggatct tattcagcgc tgcct 25
<210> 1509
   <211> 25
   <212> DNA
   <213> Artificial Sequence
<220>
   <223> synthetic nucleotide probe
<400> 1509
   gccagttctc aaagttgttc tcacc 25
<210> 1510
   <211> 25
   <212> DNA
   <213> Artificial Sequence
<220>
   <223> synthetic nucleotide probe
<400> 1510
   tactcacctt agaactgctc agaac 25
<210> 1511
   <211> 25
   <212> DNA
   <213> Artificial Sequence
<220>
   <223> synthetic nucleotide probe
<400> 1511
   gtatttcctg gattacacat agtat 25
<210> 1512
   <211> 25
   <212> DNA
   <213> Artificial Sequence
<220>
   <223> synthetic nucleotide probe
<400> 1512
   gtctatgagc gtctgacctg gacca 25
<210> 1513
   <211> 25
   <212> DNA
   <213> Artificial Sequence
<220>
   <223> synthetic nucleotide probe
<400> 1513
   gttactcctg tatcattgct cataa 25
<210> 1514
   <211> 25
   <212> DNA
   <213> Artificial Sequence
<220>
   <223> synthetic nucleotide probe
<400> 1514
   agcctctgtg cactgtttgg tggcc 25
<210> 1515
   <211> 25
   <212> DNA
   <213> Artificial Sequence
<220>
   <223> synthetic nucleotide probe
<400> 1515
   tgtatttaaa gccctcagtc tgtcc 25
<210> 1516
   <211> 25
   <212> DNA
   <213> Artificial Sequence
<220>
   <223> synthetic nucleotide probe
<400> 1516
   gcctgaatgg acaggggcca cttca 25
<210> 1517
   <211> 25
   <212> DNA
   <213> Artificial Sequence
<220>
   <223> synthetic nucleotide probe
<400> 1517
   atcactgtca cacaattcca atgga 25
<210> 1518
   <211> 25
   <212> DNA
   <213> Artificial Sequence
<220>
   <223> synthetic nucleotide probe
<400> 1518
   gacctttgct catctgtgtc agcaa 25
<210> 1519
   <211> 25
   <212> DNA
   <213> Artificial Sequence
<220>
   <223> synthetic nucleotide probe
<400> 1519
   cccggcgtgt gccgggcctg aatgg 25
<210> 1520
   <211> 25
   <212> DNA
   <213> Artificial Sequence
<220>
   <223> synthetic nucleotide probe
<400> 1520
   gctggagcgc aagacgtgct gacac 25
<210> 1521
   <211> 25
   <212> DNA
   <213> Artificial Sequence
<220>
   <223> synthetic nucleotide probe
<400> 1521
   aggtgatgtc ctgttcacat acctg 25
<210> 1522
   <211> 25
   <212> DNA
   <213> Artificial Sequence
<220>
   <223> synthetic nucleotide probe
<400> 1522
   gccttcaagc gccagtttgt ggagc 25
<210> 1523
   <211> 25
   <212> DNA
   <213> Artificial Sequence
<220>
   <223> synthetic nucleotide probe
<400> 1523
   cctccatggg cattcttgtt gttgg 25
<210> 1524
   <211> 25
   <212> DNA
   <213> Artificial Sequence
<220>
   <223> synthetic nucleotide probe
<400> 1524
   tcatggtttc catggttacc ggcct 25
<210> 1525
   <211> 25
   <212> DNA
   <213> Artificial Sequence
<220>
   <223> synthetic nucleotide probe
<400> 1525
   cccagccatc actcatcttt gagga 25
<210> 1526
   <211> 25
   <212> DNA
   <213> Artificial Sequence
<220>
   <223> synthetic nucleotide probe
<400> 1526
   gcgaagtgat ggactctgcc aaggtg 25
<210> 1527
   <211> 25
   <212> DNA
   <213> Artificial Sequence
<220>
   <223> synthetic nucleotide probe
<400> 1527
   gccacttcac agcatgtcag ggaaa 25
<210> 1528
   <211> 25
   <212> DNA
   <213> Artificial Sequence
<220>
   <223> synthetic nucleotide probe
<400> 1528
   gtcctgttgt gtggggcgaa gtgat 25
<210> 1529
   <211> 25
   <212> DNA
   <213> Artificial Sequence
<220>
   <223> synthetic nucleotide probe
<400> 1529
   gtgctgacac agtgagtttt ctctg 25
<210> 1530
   <211> 25
   <212> DNA
   <213> Artificial Sequence
<220>
   <223> synthetic nucleotide probe
<400> 1530
   gcagcttgtc atcagctaca ctggc 25
<210> 1531
   <211> 25
   <212> DNA
   <213> Artificial Sequence
<220>
   <223> synthetic nucleotide probe
<400> 1531
   gtgtcagcaa gttgacgtca cccgg 25
<210> 1532
   <211> 25
   <212> DNA
   <213> Artificial Sequence
<220>
   <223> synthetic nucleotide probe
<400> 1532
   ctgccaggtg gacatgctgt gggtg 25
<210> 1533
   <211> 25
   <212> DNA
   <213> Artificial Sequence
<220>
   <223> synthetic nucleotide probe
<400> 1533
   caatgagatc ttggactctg cctct 25
<210> 1534
   <211> 25
   <212> DNA
   <213> Artificial Sequence
<220>
   <223> synthetic nucleotide probe
<400> 1534
   agatcccgcg gttgttggtg ggtgc 25
<210> 1535
   <211> 25
   <212> DNA
   <213> Artificial Sequence
<220>
   <223> synthetic nucleotide probe
<400> 1535
   tctacttcac tcttcctgtt gaaaa 25
<210> 1536
   <211> 25
   <212> DNA
   <213> Artificial Sequence
<220>
   <223> synthetic nucleotide probe
<400> 1536
   cagactctgc attccaaacc aaggc 25
<210> 1537
   <211> 25
   <212> DNA
   <213> Artificial Sequence
<220>
   <223> synthetic nucleotide probe
<400> 1537
   gactgactga acttgacctg tgacc 25
<210> 1538
   <211> 25
   <212> DNA
   <213> Artificial Sequence
<220>
   <223> synthetic nucleotide probe
<400> 1538
   agcaacagag agtaggcggc tgggc 25
<210> 1539
   <211> 25
   <212> DNA
   <213> Artificial Sequence
<220>
   <223> synthetic nucleotide probe
<400> 1539
   tgcctggact cgctggagca aagga 25
<210> 1540
   <211> 25
   <212> DNA
   <213> Artificial Sequence
<220>
   <223> synthetic nucleotide probe
<400> 1540
   ccgcccatga ttcttcggac tgact 25
<210> 1541
   <211> 25
   <212> DNA
   <213> Artificial Sequence
<220>
   <223> synthetic nucleotide probe
<400> 1541
   gccccttagt cactcagaca tacgg 25
<210> 1542
   <211> 25
   <212> DNA
   <213> Artificial Sequence
<220>
   <223> synthetic nucleotide probe
<400> 1542
   cgccgacggg tacctgtaca tgtac 25
<210> 1543
   <211> 25
   <212> DNA
   <213> Artificial Sequence
<220>
   <223> synthetic nucleotide probe
<400> 1543
   gtcatgtgcc tttctatttt catct 25
<210> 1544
   <211> 25
   <212> DNA
   <213> Artificial Sequence
<220>
   <223> synthetic nucleotide probe
<400> 1544
   taggggagct agaagccact ttcca 25
<210> 1545
   <211> 25
   <212> DNA
   <213> Artificial Sequence
<220>
   <223> synthetic nucleotide probe
<400> 1545
   gggcttccta cctgtgtgag aggtc 25
<210> 1546
   <211> 25
   <212> DNA
   <213> Artificial Sequence
<220>
   <223> synthetic nucleotide probe
<400> 1546
   tcgcttccct tttcatattt acaga 25
<210> 1547
   <211> 25
   <212> DNA
   <213> Artificial Sequence
<220>
   <223> synthetic nucleotide probe
<400> 1547
   acttgaaagg ttgcctggac tcgct 25
<210> 1548
   <211> 25
   <212> DNA
   <213> Artificial Sequence
<220>
   <223> synthetic nucleotide probe
<400> 1548
   gcatgaacgt gccaagccag catag 25
<210> 1549
   <211> 25
   <212> DNA
   <213> Artificial Sequence
<220>
   <223> synthetic nucleotide probe
<400> 1549
   ccctgcgcct ggatgaggac agcga 25
<210> 1550
   <211> 25
   <212> DNA
   <213> Artificial Sequence
<220>
   <223> synthetic nucleotide probe
<400> 1550
   cagaactcaa gtgtggtggc cgtct 25
<210> 1551
   <211> 25
   <212> DNA
   <213> Artificial Sequence
<220>
   <223> synthetic nucleotide probe
<400> 1551
   aattggatcg ctctgggatt tcttc 25
<210> 1552
   <211> 25
   <212> DNA
   <213> Artificial Sequence
<220>
   <223> synthetic nucleotide probe
<400> 1552
   ggacagcgag gttctttctg atact 25
<210> 1553
   <211> 25
   <212> DNA
   <213> Artificial Sequence
<220>
   <223> synthetic nucleotide probe
<400> 1553
   cccaccaggt gtgctgggca gactt 25
<210> 1554
   <211> 25
   <212> DNA
   <213> Artificial Sequence
<220>
   <223> synthetic nucleotide probe
<400> 1554
   aaatgatctg ttcttctact tcact 25
<210> 1555
   <211> 25
   <212> DNA
   <213> Artificial Sequence
<220>
   <223> synthetic nucleotide probe
<400> 1555
   aaacaacctc aagtacctca gactc 25
<210> 1556
   <211> 25
   <212> DNA
   <213> Artificial Sequence
<220>
   <223> synthetic nucleotide probe
<400> 1556
   gggcagactt cagctgggac agaag 25
<210> 1557
   <211> 25
   <212> DNA
   <213> Artificial Sequence
<220>
   <223> synthetic nucleotide probe
<400> 1557
   ttcgggaaag tgctcatggc ctcca 25
<210> 1558
   <211> 25
   <212> DNA
   <213> Artificial Sequence
<220>
   <223> synthetic nucleotide probe
<400> 1558
   caagcttcag tatttgcctc gcttc 25
<210> 1559
   <211> 25
   <212> DNA
   <213> Artificial Sequence
<220>
   <223> synthetic nucleotide probe
<400> 1559
   gcgtaaggaa accgtggcgt cgcgc 25
<210> 1560
   <211> 25
   <212> DNA
   <213> Artificial Sequence
<220>
   <223> synthetic nucleotide probe
<400> 1560
   ccacaaaaac atctgctcgc tagcc 25
<210> 1561
   <211> 25
   <212> DNA
   <213> Artificial Sequence
<220>
   <223> synthetic nucleotide probe
<400> 1561
   tctgcttgtc aaggccagtt ctgca 25
<210> 1562
   <211> 25
   <212> DNA
   <213> Artificial Sequence
<220>
   <223> synthetic nucleotide probe
<400> 1562
   gcgagtgtgc cctgatgaag cagca 25
<210> 1563
   <211> 25
   <212> DNA
   <213> Artificial Sequence
<220>
   <223> synthetic nucleotide probe
<400> 1563
   gaggtcgtag cgggagacag caaca 25
<210> 1564
   <211> 25
   <212> DNA
   <213> Artificial Sequence
<220>
   <223> synthetic nucleotide probe
<400> 1564
   tgggacagaa gtccgatctc cctag 25
<210> 1565
   <211> 25
   <212> DNA
   <213> Artificial Sequence
<220>
   <223> synthetic nucleotide probe
<400> 1565
   ggcagtctgc ctaaagattc ctttc 25
<210> 1566
   <211> 25
   <212> DNA
   <213> Artificial Sequence
<220>
   <223> synthetic nucleotide probe
<400> 1566
   gccttctccc atacattcca aaagg 25
<210> 1567
   <211> 25
   <212> DNA
   <213> Artificial Sequence
<220>
   <223> synthetic nucleotide probe
<400> 1567
   gttcaacagt aagcagcacc tccaa 25
<210> 1568
   <211> 25
   <212> DNA
   <213> Artificial Sequence
<220>
   <223> synthetic nucleotide probe
<400> 1568
   tctcctttcg gccagtatca taaga 25
<210> 1569
   <211> 25
   <212> DNA
   <213> Artificial Sequence
<220>
   <223> synthetic nucleotide probe
<400> 1569
   tggacgccat aatcctgagg ctcct 25
<210> 1570
   <211> 25
   <212> DNA
   <213> Artificial Sequence
<220>
   <223> synthetic nucleotide probe
<400> 1570
   ggctcctaga ggctgagggg gcaac 25
<210> 1571
   <211> 25
   <212> DNA
   <213> Artificial Sequence
<220>
   <223> synthetic nucleotide probe
<400> 1571
   tgagggggca acggtgtgat ccagc 25
<210> 1572
   <211> 25
   <212> DNA
   <213> Artificial Sequence
<220>
   <223> synthetic nucleotide probe
<400> 1572
   gcaagccagt tgtcaaacac agcca 25
<210> 1573
   <211> 25
   <212> DNA
   <213> Artificial Sequence
<220>
   <223> synthetic nucleotide probe
<400> 1573
   gtgagagagg cagtggccgt cctcc 25
<210> 1574
   <211> 25
   <212> DNA
   <213> Artificial Sequence
<220>
   <223> synthetic nucleotide probe
<400> 1574
   ttcctgtacc tttgactaac gctca 25
<210> 1575
   <211> 25
   <212> DNA
   <213> Artificial Sequence
<220>
   <223> synthetic nucleotide probe
<400> 1575
   cttccgggcc tgcatgcagt agaca 25
<210> 1576
   <211> 25
   <212> DNA
   <213> Artificial Sequence
<220>
   <223> synthetic nucleotide probe
<400> 1576
   atcagccatt ttatcgaggc acgtg 25
<210> 1577
   <211> 25
   <212> DNA
   <213> Artificial Sequence
<220>
   <223> synthetic nucleotide probe
<400> 1577
   tagctaatgt gctgagcttg tgcct 25
<210> 1578
   <211> 25
   <212> DNA
   <213> Artificial Sequence
<220>
   <223> synthetic nucleotide probe
<400> 1578
   tagaccacgt aaccttcaag tatgt 25
<210> 1579
   <211> 25
   <212> DNA
   <213> Artificial Sequence
<220>
   <223> synthetic nucleotide probe
<400> 1579
   gaactactct cccaaggaaa atatt 25
<210> 1580
   <211> 25
   <212> DNA
   <213> Artificial Sequence
<220>
   <223> synthetic nucleotide probe
<400> 1580
   taaggaagcg cgggtcccgc atgag 25
<210> 1581
   <211> 25
   <212> DNA
   <213> Artificial Sequence
<220>
   <223> synthetic nucleotide probe
<400> 1581
   gccatcatct tgttgaatca gccat 25
<210> 1582
   <211> 25
   <212> DNA
   <213> Artificial Sequence
<220>
   <223> synthetic nucleotide probe
<400> 1582
   tacaacgggc acagacagag cacct 25
<210> 1583
   <211> 25
   <212> DNA
   <213> Artificial Sequence
<220>
   <223> synthetic nucleotide probe
<400> 1583
   tttacttccg gaatactcaa gcaaa 25
<210> 1584
   <211> 25
   <212> DNA
   <213> Artificial Sequence
<220>
   <223> synthetic nucleotide probe
<400> 1584
   tatggtgacc aatgaatctc cctta 25
<210> 1585
   <211> 25
   <212> DNA
   <213> Artificial Sequence
<220>
   <223> synthetic nucleotide probe
<400> 1585
   gattgtttta actgattact gtaga 25
<210> 1586
   <211> 25
   <212> DNA
   <213> Artificial Sequence
<220>
   <223> synthetic nucleotide probe
<400> 1586
   ttcctagtct tctgtgtatg tgatg 25
<210> 1587
   <211> 25
   <212> DNA
   <213> Artificial Sequence
<220>
   <223> synthetic nucleotide probe
<400> 1587
   aggatgtctt tcaggattat tttaa 25
<210> 1588
   <211> 25
   <212> DNA
   <213> Artificial Sequence
<220>
   <223> synthetic nucleotide probe
<400> 1588
   taattactta cttattacct agatt 25
<210> 1589
   <211> 25
   <212> DNA
   <213> Artificial Sequence
<220>
   <223> synthetic nucleotide probe
<400> 1589
   gactacagga gacgacgggg ccttt 25
<210> 1590
   <211> 25
   <212> DNA
   <213> Artificial Sequence
<220>
   <223> synthetic nucleotide probe
<400> 1590
   gacagaactg tttgttatgt accat 25
<210> 1591
   <211> 25
   <212> DNA
   <213> Artificial Sequence
<220>
   <223> synthetic nucleotide probe
<400> 1591
   actgtgcctt gtgttactta atcat 25
<210> 1592
   <211> 25
   <212> DNA
   <213> Artificial Sequence
<220>
   <223> synthetic nucleotide probe
<400> 1592
   gcgacgaacg ccgggatttc ggcgg 25
<210> 1593
   <211> 25
   <212> DNA
   <213> Artificial Sequence
<220>
   <223> synthetic nucleotide probe
<400> 1593
   gtatagcccc acagattaaa tttaa 25
<210> 1594
   <211> 25
   <212> DNA
   <213> Artificial Sequence
<220>
   <223> synthetic nucleotide probe
<400> 1594
   ttgccaccat ttgtagacca cgtaa 25
<210> 1595
   <211> 25
   <212> DNA
   <213> Artificial Sequence
<220>
   <223> synthetic nucleotide probe
<400> 1595
   gaagacaatg ctttccatat tgtga 25
<210> 1596
   <211> 25
   <212> DNA
   <213> Artificial Sequence
<220>
   <223> synthetic nucleotide probe
<400> 1596
   gctttaatgt gcttttactt ccgga 25
<210> 1597
   <211> 25
   <212> DNA
   <213> Artificial Sequence
<220>
   <223> synthetic nucleotide probe
<400> 1597
   atttttttgc gtgaaagtgt tacat 25
<210> 1598
   <211> 25
   <212> DNA
   <213> Artificial Sequence
<220>
   <223> synthetic nucleotide probe
<400> 1598
   cggataagga agcgcgggtc ccgca 25
<210> 1599
   <211> 25
   <212> DNA
   <213> Artificial Sequence
<220>
   <223> synthetic nucleotide probe
<400> 1599
   ctgggctcgg ggtgactaca ggaga 25
<210> 1600
   <211> 25
   <212> DNA
   <213> Artificial Sequence
<220>
   <223> synthetic nucleotide probe
<400> 1600
   aaagatggct actgtgcctt gtgtt 25
<210> 1601
   <211> 25
   <212> DNA
   <213> Artificial Sequence
<220>
   <223> synthetic nucleotide probe
<400> 1601
   aaggccatca cgtatgccaa aggat 25
<210> 1602
   <211> 25
   <212> DNA
   <213> Artificial Sequence
<220>
   <223> synthetic nucleotide probe
<400> 1602
   gactcttctt gcagtttaca acggg 25
<210> 1603
   <211> 25
   <212> DNA
   <213> Artificial Sequence
<220>
   <223> synthetic nucleotide probe
<400> 1603
   gagacattga tatatccttt tgcta 25
<210> 1604
   <211> 25
   <212> DNA
   <213> Artificial Sequence
<220>
   <223> synthetic nucleotide probe
<400> 1604
   gattactgta gatcaacctg atgat 25
<210> 1605
   <211> 25
   <212> DNA
   <213> Artificial Sequence
<220>
   <223> synthetic nucleotide probe
<400> 1605
   ggctcggggt gactacagga gacga 25
<210> 1606
   <211> 25
   <212> DNA
   <213> Artificial Sequence
<220>
   <223> synthetic nucleotide probe
<400> 1606
   gcctcgtttt ccggataagg aagcg 25
<210> 1607
   <211> 25
   <212> DNA
   <213> Artificial Sequence
<220>
   <223> synthetic nucleotide probe
<400> 1607
   ccatttgtag accacgtaac cttca 25
<210> 1608
   <211> 25
   <212> DNA
   <213> Artificial Sequence
<220>
   <223> synthetic nucleotide probe
<400> 1608
   ttcgtgttgc catcatcttg ttgaa 25
<210> 1609
   <211> 25
   <212> DNA
   <213> Artificial Sequence
<220>
   <223> synthetic nucleotide probe
<400> 1609
   ttacctacat ctcatacttg cttta 25
<210> 1610
   <211> 25
   <212> DNA
   <213> Artificial Sequence
<220>
   <223> synthetic nucleotide probe
<400> 1610
   gagcttgtgc cttggtgatt gattg 25
<210> 1611
   <211> 25
   <212> DNA
   <213> Artificial Sequence
<220>
   <223> synthetic nucleotide probe
<400> 1611
   caaggctttt cggagaggtt ttcat 25
<210> 1612
   <211> 25
   <212> DNA
   <213> Artificial Sequence
<220>
   <223> synthetic nucleotide probe
<400> 1612
   gggtgactac aggagacgac ggggc 25
<210> 1613
   <211> 25
   <212> DNA
   <213> Artificial Sequence
<220>
   <223> synthetic nucleotide probe
<400> 1613
   tgttacatat tctttcactt gtatg 25
<210> 1614
   <211> 25
   <212> DNA
   <213> Artificial Sequence
<220>
   <223> synthetic nucleotide probe
<400> 1614
   gataaggtaa catggggttt ttctg 25
<210> 1615
   <211> 25
   <212> DNA
   <213> Artificial Sequence
<220>
   <223> synthetic nucleotide probe
<400> 1615
   gaattacatt gtatagcccc acaga 25
<210> 1616
   <211> 25
   <212> DNA
   <213> Artificial Sequence
<220>
   <223> synthetic nucleotide probe
<400> 1616
   gatatatcct tttgctacaa gctat 25
<210> 1617
   <211> 25
   <212> DNA
   <213> Artificial Sequence
<220>
   <223> synthetic nucleotide probe
<400> 1617
   tgacggtggc tataccaggg actct 25
<210> 1618
   <211> 25
   <212> DNA
   <213> Artificial Sequence
<220>
   <223> synthetic nucleotide probe
<400> 1618
   gaaactatta ctcctaagaa ttaca 25
<210> 1619
   <211> 25
   <212> DNA
   <213> Artificial Sequence
<220>
   <223> synthetic nucleotide probe
<400> 1619
   gctggcgacg aacgccggga tttcg 25
<210> 1620
   <211> 25
   <212> DNA
   <213> Artificial Sequence
<220>
   <223> synthetic nucleotide probe
<400> 1620
   atgcagcttt caaatcattg ggggg 25
<210> 1621
   <211> 25
   <212> DNA
   <213> Artificial Sequence
<220>
   <223> synthetic nucleotide probe
<400> 1621
   gaggcacgtg atcagtgttg caaca 25
<210> 1622
   <211> 25
   <212> DNA
   <213> Artificial Sequence
<220>
   <223> synthetic nucleotide probe
<400> 1622
   gggcaggagc ctcgcaatat gtggc 25
<210> 1623
   <211> 25
   <212> DNA
   <213> Artificial Sequence
<220>
   <223> synthetic nucleotide probe
<400> 1623
   ggctcctcag cctaagacta tggct 25
<210> 1624
   <211> 25
   <212> DNA
   <213> Artificial Sequence
<220>
   <223> synthetic nucleotide probe
<400> 1624
   agaaacactc aggcctgacc taggc 25
<210> 1625
   <211> 25
   <212> DNA
   <213> Artificial Sequence

<220>
   <223> synthetic nucleotide probe
<400> 1625
   taaccatcat gtatgcccac gaggg 25
<210> 1626
   <211> 25
   <212> DNA
   <213> Artificial Sequence
<220>
   <223> synthetic nucleotide probe
<400> 1626
   taaccatcat gtatgcccac gaggg 25
<210> 1627
   <211> 25
   <212> DNA
   <213> Artificial Sequence
<220>
   <223> synthetic nucleotide probe
<400> 1627
   gacccctctg agtgtggtca gtgcc 25
<210> 1628
   <211> 25
   <212> DNA
   <213> Artificial Sequence
<220>
   <223> synthetic nucleotide probe
<400> 1628
   tccctgtgat tgccctgtta agtat 25
<210> 1629
   <211> 25
   <212> DNA
   <213> Artificial Sequence
<220>
   <223> synthetic nucleotide probe
<400> 1629
   tccctgtgat tgccctgtta agtat 25
<210> 1630
   <211> 25
   <212> DNA
   <213> Artificial Sequence
<220>
   <223> synthetic nucleotide probe
<400> 1630
   tgctcctgct tacgaagtat tccca 25
<210> 1631
   <211> 25
   <212> DNA
   <213> Artificial Sequence
<220>
   <223> synthetic nucleotide probe
<400> 1631
   tgctcctgct tacgaagtat tccca 25
<210> 1632
   <211> 25
   <212> DNA
   <213> Artificial Sequence
<220>
   <223> synthetic nucleotide probe
<400> 1632
   gattgcttgt gacaacggct gcatc 25
<210> 1633
   <211> 25
   <212> DNA
   <213> Artificial Sequence
<220>
   <223> synthetic nucleotide probe
<400> 1633
   ggctcctgtc tgactattcc aggat 25
<210> 1634
   <211> 25
   <212> DNA
   <213> Artificial Sequence
<220>
   <223> synthetic nucleotide probe
<400> 1634
   ccctaccgat agaacagtgg ctcag 25
<210> 1635
   <211> 25
   <212> DNA
   <213> Artificial Sequence
<220>
   <223> synthetic nucleotide probe
<400> 1635
   gcatgaaggc tcctgtctga ctatt 25
<210> 1636
   <211> 25
   <212> DNA
   <213> Artificial Sequence
<220>
   <223> synthetic nucleotide probe
<400> 1636
   tgcatctggg acctcaagtt ctgcc 25
<210> 1637
   <211> 25
   <212> DNA
   <213> Artificial Sequence
<220>
   <223> synthetic nucleotide probe
<400> 1637
   ccaccaacac ctagctgctg gatat 25
<210> 1638
   <211> 25
   <212> DNA
   <213> Artificial Sequence
<220>
   <223> synthetic nucleotide probe
<400> 1638
   cacagacacc ctaccgatag aacag 25
<210> 1639
   <211> 25
   <212> DNA
   <213> Artificial Sequence
<220>
   <223> synthetic nucleotide probe
<400> 1639
   tggcctgctc agacgggaaa gtact 25
<210> 1640
   <211> 25
   <212> DNA
   <213> Artificial Sequence
<220>
   <223> synthetic nucleotide probe
<400> 1640
   gtatctgcat gaaggctcct gtctg 25
<210> 1641
   <211> 25
   <212> DNA
   <213> Artificial Sequence
<220>
   <223> synthetic nucleotide probe
<400> 1641
   caaggaatac cacagacacc ctacc 25
<210> 1642
   <211> 25
   <212> DNA
   <213> Artificial Sequence
<220>
   <223> synthetic nucleotide probe
<400> 1642
   aaccatagct atcatgtgtt tccca 25
<210> 1643
   <211> 25
   <212> DNA
   <213> Artificial Sequence
<220>
   <223> synthetic nucleotide probe
<400> 1643
   atagctatca tgtgtttccc aaatc 25
<210> 1644
   <211> 25
   <212> DNA
   <213> Artificial Sequence
<220>
   <223> synthetic nucleotide probe
<400> 1644
   acagggccca ctttgtctat gggat 25
<210> 1645
   <211> 25
   <212> DNA
   <213> Artificial Sequence
<220>
   <223> synthetic nucleotide probe
<400> 1645
   ttcccaatca ctggtcatct gaccc 25
<210> 1646
   <211> 25
   <212> DNA
   <213> Artificial Sequence
<220>
   <223> synthetic nucleotide probe
<400> 1646
   ttcccaatca ctggtcatct gaccc 25
<210> 1647
   <211> 25
   <212> DNA
   <213> Artificial Sequence
<220>
   <223> synthetic nucleotide probe
<400> 1647
   ggaaatctag tcatcttccc tgtga 25
<210> 1648
   <211> 25
   <212> DNA
   <213> Artificial Sequence
<220>
   <223> synthetic nucleotide probe
<400> 1648
   ggaaatctag tcatcttccc tgtga 25
<210> 1649
   <211> 25
   <212> DNA
   <213> Artificial Sequence
<220>
   <223> synthetic nucleotide probe
<400> 1649
   gacatgaatg agacacaccc actga 25
<210> 1650
   <211> 25
   <212> DNA
   <213> Artificial Sequence
<220>
   <223> synthetic nucleotide probe
<400> 1650
   gcaactctgc aggtggggtc tatgc 25
<210> 1651
   <211> 25
   <212> DNA
   <213> Artificial Sequence
<220>
   <223> synthetic nucleotide probe
<400> 1651
   aagtactgct attcagtcta cccca 25
<210> 1652
   <211> 25
   <212> DNA
   <213> Artificial Sequence
<220>
   <223> synthetic nucleotide probe
<400> 1652
   tattactgcc ttctgaaact tcctc 25
<210> 1653
   <211> 25
   <212> DNA
   <213> Artificial Sequence
<220>
   <223> synthetic nucleotide probe
<400> 1653
   tattactgcc ttctgaaact tcctc 25
<210> 1654
   <211> 25
   <212> DNA
   <213> Artificial Sequence
<220>
   <223> synthetic nucleotide probe
<400> 1654
   gttactgatt tcttgtatct cccag 25
<210> 1655
   <211> 25
   <212> DNA
   <213> Artificial Sequence
<220>
   <223> synthetic nucleotide probe
<400> 1655
   gctacatgtg taagtctgcc taaat 25
<210> 1656
   <211> 25
   <212> DNA
   <213> Artificial Sequence
<220>
   <223> synthetic nucleotide probe
<400> 1656
   aatctagccc cagacatact gtgtt 25
<210> 1657
   <211> 25
   <212> DNA
   <213> Artificial Sequence
<220>
   <223> synthetic nucleotide probe
<400> 1657
   cctcccattt tgttctcgga agatt 25
<210> 1658
   <211> 25
   <212> DNA
   <213> Artificial Sequence
<220>
   <223> synthetic nucleotide probe
<400> 1658
   gtccatttga gattctgcac tccat 25
<210> 1659
   <211> 25
   <212> DNA
   <213> Artificial Sequence
<220>
   <223> synthetic nucleotide probe
<400> 1659
   cccctcctgc taggccagtg aaggg 25
<210> 1660
   <211> 25
   <212> DNA
   <213> Artificial Sequence
<220>
   <223> synthetic nucleotide probe
<400> 1660
   taattggatt tgtaccgtcc tccca 25
<210> 1661
   <211> 25
   <212> DNA
   <213> Artificial Sequence
<220>
   <223> synthetic nucleotide probe
<400> 1661
   gtcaagtatg tctcaacact agcat 25
<210> 1662
   <211> 25
   <212> DNA
   <213> Artificial Sequence
<220>
   <223> synthetic nucleotide probe
<400> 1662
   gaaaagttca cttggacgct ggggc 25
<210> 1663
   <211> 25
   <212> DNA
   <213> Artificial Sequence
<220>
   <223> synthetic nucleotide probe
<400> 1663
   ttgtcaatat atcgaactgt tccca 25
<210> 1664
   <211> 25
   <212> DNA
   <213> Artificial Sequence
<220>
   <223> synthetic nucleotide probe
<400> 1664
   taactctgca ttctggcttc tgtat 25
<210> 1665
   <211> 25
   <212> DNA
   <213> Artificial Sequence
<220>
   <223> synthetic nucleotide probe
<400> 1665
   tgtctaagtg tttttcttcg tggtc 25
<210> 1666
   <211> 25
   <212> DNA
   <213> Artificial Sequence
<220>
   <223> synthetic nucleotide probe
<400> 1666
   aggcctcctg aattgagttt gatgc 25
<210> 1667
   <211> 25
   <212> DNA
   <213> Artificial Sequence
<220>
   <223> synthetic nucleotide probe
<400> 1667
   gactggaatg ggaccaggcc gtcgc 25
<210> 1668
   <211> 25
   <212> DNA
   <213> Artificial Sequence
<220>
   <223> synthetic nucleotide probe
<400> 1668
   gatgcagagc tttttagcca tgaag 25
<210> 1669
   <211> 25
   <212> DNA
   <213> Artificial Sequence
<220>
   <223> synthetic nucleotide probe
<400> 1669
   atacagagag cacccatatg gacgt 25
<210> 1670
   <211> 25
   <212> DNA
   <213> Artificial Sequence
<220>
   <223> synthetic nucleotide probe
<400> 1670
   gtagatgctt ttttctttgt tgttt 25
<210> 1671
   <211> 25
   <212> DNA
   <213> Artificial Sequence
<220>
   <223> synthetic nucleotide probe
<400> 1671
   tgactttttc attacgtggg ttttg 25
<210> 1672
   <211> 25
   <212> DNA
   <213> Artificial Sequence
<220>
   <223> synthetic nucleotide probe
<400> 1672
   gtatctccca ggattcctgt tgctt 25
<210> 1673
   <211> 25
   <212> DNA
   <213> Artificial Sequence
<220>
   <223> synthetic nucleotide probe
<400> 1673
   ttgctttacc cacaacagac aagta 25
<210> 1674
   <211> 25
   <212> DNA
   <213> Artificial Sequence
<220>
   <223> synthetic nucleotide probe
<400> 1674
   ccttattcca ttcttaactc tgcat 25
<210> 1675
   <211> 25
   <212> DNA
   <213> Artificial Sequence
<220>
   <223> synthetic nucleotide probe
<400> 1675
   gttgaatgac tacatcttct ccttt 25
<210> 1676
   <211> 25
   <212> DNA
   <213> Artificial Sequence
<220>
   <223> synthetic nucleotide probe
<400> 1676
   agctgcttac ttgttgtatg ccttc 25
<210> 1677
   <211> 25
   <212> DNA
   <213> Artificial Sequence
<220>
   <223> synthetic nucleotide probe
<400> 1677
   gtatgccttc actagaatca ggtct 25
<210> 1678
   <211> 25
   <212> DNA
   <213> Artificial Sequence
<220>
   <223> synthetic nucleotide probe
<400> 1678
   aatcaggtct attgcacgtc tggcc 25
<210> 1679
   <211> 25
   <212> DNA
   <213> Artificial Sequence
<220>
   <223> synthetic nucleotide probe
<400> 1679
   ggaaactagg ccggtgcatt ttacg 25
<210> 1680
   <211> 25
   <212> DNA
   <213> Artificial Sequence
<220>
   <223> synthetic nucleotide probe
<400> 1680
   gagctggcaa ctgctttcac agagt 25
<210> 1681
   <211> 25
   <212> DNA
   <213> Artificial Sequence
<220>
   <223> synthetic nucleotide probe
<400> 1681
   gaacatgtgg cgtatcttgt gtgaa 25
<210> 1682
   <211> 25
   <212> DNA
   <213> Artificial Sequence
<220>
   <223> synthetic nucleotide probe
<400> 1682
   ctggcccaag ggtgtaatcc ctcac 25
<210> 1683
   <211> 25
   <212> DNA
   <213> Artificial Sequence
<220>
   <223> synthetic nucleotide probe
<400> 1683
   aatccctcac aggtttgaac cctgt 25
<210> 1684
   <211> 25
   <212> DNA
   <213> Artificial Sequence
<220>
   <223> synthetic nucleotide probe
<400> 1684
   ttttcccaag tggccattgg ccctg 25
<210> 1685
   <211> 25
   <212> DNA
   <213> Artificial Sequence
<220>
   <223> synthetic nucleotide probe
<400> 1685
   gctttttttc aatcttgtgg gcaca 25
<210> 1686
   <211> 25
   <212> DNA
   <213> Artificial Sequence
<220>
   <223> synthetic nucleotide probe
<400> 1686
   gcaactggca ccatacagga agatt 25
<210> 1687
   <211> 25
   <212> DNA
   <213> Artificial Sequence
<220>
   <223> synthetic nucleotide probe
<400> 1687
   caaattccag ccaacgtcct tgttg 25
<210> 1688
   <211> 25
   <212> DNA
   <213> Artificial Sequence
<220>
   <223> synthetic nucleotide probe
<400> 1688
   aacgtccttg ttgcactttg ggtat 25
<210> 1689
   <211> 25
   <212> DNA
   <213> Artificial Sequence
<220>
   <223> synthetic nucleotide probe
<400> 1689
   gcactttggg tattctgaga ttttc 25
<210> 1690
   <211> 25
   <212> DNA
   <213> Artificial Sequence
<220>
   <223> synthetic nucleotide probe
<400> 1690
   tcttgccatt cccttaggct ttagc 25
<210> 1691
   <211> 25
   <212> DNA
   <213> Artificial Sequence
<220>
   <223> synthetic nucleotide probe
<400> 1691
   ggctttagca gctttgcatt tcctg 25
<210> 1692
   <211> 25
   <212> DNA
   <213> Artificial Sequence
<220>
   <223> synthetic nucleotide probe
<400> 1692
   ttgcatttcc tgttgtattt attct 25
<210> 1693
   <211> 25
   <212> DNA
   <213> Artificial Sequence
<220>
   <223> synthetic nucleotide probe
<400> 1693
   tattctcagc cattttgggc atatg 25
<210> 1694
   <211> 25
   <212> DNA
   <213> Artificial Sequence
<220>
   <223> synthetic nucleotide probe
<400> 1694
   cagactggaa acgggacttt ctatt 25
<210> 1695
   <211> 25
   <212> DNA
   <213> Artificial Sequence
<220>
   <223> synthetic nucleotide probe
<400> 1695
   cttctccccc aataactgtg ggtct 25
<210> 1696
   <211> 25
   <212> DNA
   <213> Artificial Sequence
<220>
   <223> synthetic nucleotide probe
<400> 1696
   tcagagaaag ttagttcggc tcgat 25
<210> 1697
   <211> 25
   <212> DNA
   <213> Artificial Sequence
<220>
   <223> synthetic nucleotide probe
<400> 1697
   gatagttaat gttttatgct tccat 25
<210> 1698
   <211> 25
   <212> DNA
   <213> Artificial Sequence
<220>
   <223> synthetic nucleotide probe
<400> 1698
   tattccattt gcaacttatc cccaa 25
<210> 1699
   <211> 25
   <212> DNA
   <213> Artificial Sequence
<220>
   <223> synthetic nucleotide probe
<400> 1699
   gcaaaagtac ccctttgcac agata 25
<210> 1700
   <211> 25
   <212> DNA
   <213> Artificial Sequence
<220>
   <223> synthetic nucleotide probe
<400> 1700
   gaaatgcggc tagttcagag agatt 25
<210> 1701
   <211> 25
   <212> DNA
   <213> Artificial Sequence
<220>
   <223> synthetic nucleotide probe
<400> 1701
   aattttttgt atcaagtccc tgaat 25
<210> 1702
   <211> 25
   <212> DNA
   <213> Artificial Sequence
<220>
   <223> synthetic nucleotide probe
<400> 1702
   gacaggcttg ccgaaattga ggaca 25
<210> 1703
   <211> 25
   <212> DNA
   <213> Artificial Sequence
<220>
   <223> synthetic nucleotide probe
<400> 1703
   aacagccaag gttacggtgg ttatg 25
<210> 1704
   <211> 25
   <212> DNA
   <213> Artificial Sequence
<220>
   <223> synthetic nucleotide probe
<400> 1704
   gtgtcctccc tgtccaaatt gggaa 25
<210> 1705
   <211> 25
   <212> DNA
   <213> Artificial Sequence
<220>
   <223> synthetic nucleotide probe
<400> 1705
   gattcatttg aaggtggctc ctgcc 25
<210> 1706
   <211> 25
   <212> DNA
   <213> Artificial Sequence
<220>
   <223> synthetic nucleotide probe
<400> 1706
   ataatacttc cttatgtagc catta 25
<210> 1707
   <211> 25
   <212> DNA
   <213> Artificial Sequence
<220>
   <223> synthetic nucleotide probe
<400> 1707
   aatgtcaatt tgtttgttgg ttgtt 25
<210> 1708
   <211> 25
   <212> DNA
   <213> Artificial Sequence
<220>
   <223> synthetic nucleotide probe
<400> 1708
   gagtggttat gggaaggtat ccagg 25
<210> 1709
   <211> 25
   <212> DNA
   <213> Artificial Sequence
<220>
   <223> synthetic nucleotide probe
<400> 1709
   gactacactg gttacaacaa ctact 25
<210> 1710
   <211> 25
   <212> DNA
   <213> Artificial Sequence
<220>
   <223> synthetic nucleotide probe
<400> 1710
   ggtggtcatc aaaatagcta caaac 25
<210> 1711
   <211> 25
   <212> DNA
   <213> Artificial Sequence
<220>
   <223> synthetic nucleotide probe
<400> 1711
   aagtttggaa gacaggcttg ccgaa 25
<210> 1712
   <211> 25
   <212> DNA
   <213> Artificial Sequence
<220>
   <223> synthetic nucleotide probe
<400> 1712
   ggaaccaggg atatagtaac tattg 25
<210> 1713
   <211> 25
   <212> DNA
   <213> Artificial Sequence
<220>
   <223> synthetic nucleotide probe
<400> 1713
   gagctgtggt ggacttcata gatga 25
<210> 1714
   <211> 25
   <212> DNA
   <213> Artificial Sequence
<220>
   <223> synthetic nucleotide probe
<400> 1714
   agtccctgaa tggaagtatg acgtt 25
<210> 1715
   <211> 25
   <212> DNA
   <213> Artificial Sequence
<220>
   <223> synthetic nucleotide probe
<400> 1715
   aaaagcccag tgtgacagtg tcatg 25
<210> 1716
   <211> 25
   <212> DNA
   <213> Artificial Sequence
<220>
   <223> synthetic nucleotide probe
<400> 1716
   gaagtttaat tctgagttct catta 25
<210> 1717
   <211> 25
   <212> DNA
   <213> Artificial Sequence
<220>
   <223> synthetic nucleotide probe
<400> 1717
   ggaggatatg actacactgg ttaca 25
<210> 1718
   <211> 25
   <212> DNA
   <213> Artificial Sequence
<220>
   <223> synthetic nucleotide probe
<400> 1718
   gagagatttt tagagctgtg gtgga 25
<210> 1719
   <211> 25
   <212> DNA
   <213> Artificial Sequence
<220>
   <223> synthetic nucleotide probe
<400> 1719
   ttattccatt tgcaacttat cccca 25
<210> 1720
   <211> 25
   <212> DNA
   <213> Artificial Sequence
<220>
   <223> synthetic nucleotide probe
<400> 1720
   aggttacggt ggttatggag gatat 25
<210> 1721
   <211> 25
   <212> DNA
   <213> Artificial Sequence
<220>
   <223> synthetic nucleotide probe
<400> 1721
   atttgctttc attgttttat ttctt 25
<210> 1722
   <211> 25
   <212> DNA
   <213> Artificial Sequence
<220>
   <223> synthetic nucleotide probe
<400> 1722
   agaaatttgc tttcattgtt ttatt 25
<210> 1723
   <211> 25
   <212> DNA
   <213> Artificial Sequence
<220>
   <223> synthetic nucleotide probe
<400> 1723
   cctttccccc agtattgtag agcaa 25
<210> 1724
   <211> 25
   <212> DNA
   <213> Artificial Sequence
<220>
   <223> synthetic nucleotide probe
<400> 1724
   ggtatccagg cgaggtggtc atcaa 25
<210> 1725
   <211> 25
   <212> DNA
   <213> Artificial Sequence
<220>
   <223> synthetic nucleotide probe
<400> 1725
   gtatgacgtt gggtccctct gaagt 25
<210> 1726
   <211> 25
   <212> DNA
   <213> Artificial Sequence
<220>
   <223> synthetic nucleotide probe
<400> 1726
   gtatgacgtt gggtccctct gaagt 25
<210> 1727
   <211> 25
   <212> DNA
   <213> Artificial Sequence
<220>
   <223> synthetic nucleotide probe
<400> 1727
   aacaactact atggatatgg tgatt 25
<210> 1728
   <211> 25
   <212> DNA
   <213> Artificial Sequence
<220>
   <223> synthetic nucleotide probe
<400> 1728
   tgtgcttttt agaacaaatc tggat 25
<210> 1729
   <211> 25
   <212> DNA
   <213> Artificial Sequence
<220>
   <223> synthetic nucleotide probe
<400> 1729
   gagagcgcgt gcagagactt ggtgg 25
<210> 1730
   <211> 25
   <212> DNA
   <213> Artificial Sequence
<220>
   <223> synthetic nucleotide probe
<400> 1730
   tggcgagatg tgtctctcaa tcctg 25
<210> 1731
   <211> 25
   <212> DNA
   <213> Artificial Sequence
<220>
   <223> synthetic nucleotide probe
<400> 1731
   tctctcaatc ctgtggcttt ggtga 25
<210> 1732
   <211> 25
   <212> DNA
   <213> Artificial Sequence
<220>
   <223> synthetic nucleotide probe
<400> 1732
   gtttttgttc ttagataacc cacat 25
<210> 1733
   <211> 25
   <212> DNA
   <213> Artificial Sequence
<220>
   <223> synthetic nucleotide probe
<400> 1733
   tgaaatgata cttgtactcc cccta 25
<210> 1734
   <211> 25
   <212> DNA
   <213> Artificial Sequence
<220>
   <223> synthetic nucleotide probe
<400> 1734
   ctttgtcaac tgctgtgaat gctgt 25
<210> 1735
   <211> 25
   <212> DNA
   <213> Artificial Sequence
<220>
   <223> synthetic nucleotide probe
<400> 1735
   gaatgctgta tggtgtgtgt tctct 25
<210> 1736
   <211> 25
   <212> DNA
   <213> Artificial Sequence
<220>
   <223> synthetic nucleotide probe
<400> 1736
   ggactgagct tgtggtgtgc tttgc 25
<210> 1737
   <211> 25
   <212> DNA
   <213> Artificial Sequence
<220>
   <223> synthetic nucleotide probe
<400> 1737
   gcagagttca ccagtgagct caggt 25
<210> 1738
   <211> 25
   <212> DNA
   <213> Artificial Sequence
<220>
   <223> synthetic nucleotide probe
<400> 1738
   gttctaatgt ctgttagcta cccat 25
<210> 1739
   <211> 25
   <212> DNA
   <213> Artificial Sequence
<220>
   <223> synthetic nucleotide probe
<400> 1739
   aagaatgctg tttgctgcag ttctg 25
<210> 1740
   <211> 25
   <212> DNA
   <213> Artificial Sequence
<220>
   <223> synthetic nucleotide probe
<400> 1740
   aagctagtgc tttgtcttag tagtt 25
<210> 1741
   <211> 25
   <212> DNA
   <213> Artificial Sequence
<220>
   <223> synthetic nucleotide probe
<400> 1741
   ggggcaatcg tgggggcaat gtagg 25
<210> 1742
   <211> 25
   <212> DNA
   <213> Artificial Sequence
<220>
   <223> synthetic nucleotide probe
<400> 1742
   tcgtttcagg cttcatttta gcttc 25
<210> 1743
   <211> 25
   <212> DNA
   <213> Artificial Sequence
<220>
   <223> synthetic nucleotide probe
<400> 1743
   tcacaccttt ttgaaatctg cccta 25
<210> 1744
   <211> 25
   <212> DNA
   <213> Artificial Sequence
<220>
   <223> synthetic nucleotide probe
<400> 1744
   accctccaga ttactacggc tatga 25
<210> 1745
   <211> 25
   <212> DNA
   <213> Artificial Sequence
<220>
   <223> synthetic nucleotide probe
<400> 1745
   attgttataa cttcacacct ttttg 25
<210> 1746
   <211> 25
   <212> DNA
   <213> Artificial Sequence
<220>
   <223> synthetic nucleotide probe
<400> 1746
   tggatatggc taccctccag attac 25
<210> 1747
   <211> 25
   <212> DNA
   <213> Artificial Sequence
<220>
   <223> synthetic nucleotide probe
<400> 1747
   aaacaagctg ggcacactgt taaat 25
<210> 1748
   <211> 25
   <212> DNA
   <213> Artificial Sequence
<220>
   <223> synthetic nucleotide probe
<400> 1748
   gctcttggac attattgggc ttgca 25
<210> 1749
   <211> 25
   <212> DNA
   <213> Artificial Sequence
<220>
   <223> synthetic nucleotide probe
<400> 1749
   catgattttg cagaaccttt ggttt 25
<210> 1750
   <211> 25
   <212> DNA
   <213> Artificial Sequence
<220>
   <223> synthetic nucleotide probe
<400> 1750
   caatgctttt atcgtttcag gcttc 25
<210> 1751
   <211> 25
   <212> DNA
   <213> Artificial Sequence
<220>
   <223> synthetic nucleotide probe
<400> 1751
   gttcccgtgg atctcggggc aatcg 25
<210> 1752
   <211> 25
   <212> DNA
   <213> Artificial Sequence
<220>
   <223> synthetic nucleotide probe
<400> 1752
   gattccaagc gtcgtcagac caaca 25
<210> 1753
   <211> 25
   <212> DNA
   <213> Artificial Sequence
<220>
   <223> synthetic nucleotide probe
<400> 1753
   tcaacagcag agaggccgtg gttcc 25
<210> 1754
   <211> 25
   <212> DNA
   <213> Artificial Sequence
<220>
   <223> synthetic nucleotide probe
<400> 1754
   ggctatgaag atccctacta cggct 25
<210> 1755
   <211> 25
   <212> DNA
   <213> Artificial Sequence
<220>
   <223> synthetic nucleotide probe
<400> 1755
   aaagccgtga caatttgttc tttga 25
<210> 1756
   <211> 25
   <212> DNA
   <213> Artificial Sequence
<220>
   <223> synthetic nucleotide probe
<400> 1756
   tcacagaggg gggcaccttt gggac 25
<210> 1757
   <211> 25
   <212> DNA
   <213> Artificial Sequence
<220>
   <223> synthetic nucleotide probe
<400> 1757
   acctttggga ccaccaagag gctct 25
<210> 1758
   <211> 25
   <212> DNA
   <213> Artificial Sequence
<220>
   <223> synthetic nucleotide probe
<400> 1758
   gtatttccaa tttcttgttc atgta 25
<210> 1759
   <211> 25
   <212> DNA
   <213> Artificial Sequence
<220>
   <223> synthetic nucleotide probe
<400> 1759
   gtcgtcagac caacaaccaa cagaa 25
<210> 1760
   <211> 25
   <212> DNA
   <213> Artificial Sequence
<220>
   <223> synthetic nucleotide probe
<400> 1760
   tgggcttgca gagttccctt attct 25

## Description

### BACKGROUND OF THE INVENTION

Large randomized trials have shown that chemotherapy administered in the perioperative setting (*e.g*., adjuvant chemotherapy) can cure patients otherwise destined to recur with systemic, incurable cancer (1). Once this recurrence has happened, the same chemotherapy is not curative. Therefore, the adjuvant window is a privileged period of time, when the decision to administer additional therapy or not, as well as the type, duration and intensity of such therapy takes center stage. Node-negative, estrogen receptor (ER)-positive, HER2-negative patients generally show a favorable prognosis when treated with adjuvant hormonal therapy only. However, because an unknown subset of these patients develops recurrences, most are currently treated not only with hormonal therapy but also cytotoxic chemotherapy, even though it is probably unnecessary for most. Our goal was to stratify these patients into those that are most or least likely to develop a recurrence within 10 years after surgery. Our approach was to develop a multi-gene transcription-level-based classifier of 10-year-relapse (disease recurrence within 10 years) using a large database of existing, publicly available microarray datasets. The probability of relapse and relapse risk score group reported by our method can be used to assign systemic chemotherapy to only those patients most likely to benefit from it.

Filipits et al (2011) Clin Cancer Res, 17, 6012-6020 states that it discloses a multigene risk score providing additional prognostic information to the risk of distant recurrence of breast cancer patients, independent from clinicopathologic parameters.

### BRIEF SUMMARY OF THE INVENTION

The present invention is based, in part, on the identification of a panel of gene expression markers for node-negative, ER-positive, HER2-negative breast cancer patients. The probability of relapse and relapse risk score group using the panel of gene expression markers of the invention can be used to assign systemic chemotherapy to only those patients most likely to benefit from it.

The invention can be used on tissue from LN-, ER+, HER2- breast cancer patients by any assay where transcript levels (or their expression products) of primary genes (or their alternate genes) in the Random Forest Relapse Score (RFRS) signature are measured. These measurements can be used to assign an RFRS value and to determine the likelihood of breast cancer relapse. Those breast cancer patients with tumors at high risk of relapse can be treated more aggressively whereas those at low risk of relapse can more safely avoid the risks and side effects of systemic chemotherapy. Thus, this method can provide rapid and useful information for clinical decision making. The scope of the present invention is defined in the appended claims.

Thus, in one embodiment, the invention relates to a method of evaluating the likelihood of a relapse for a patient that has a lymph node-negative, estrogen receptor-positive, HER2-negative breast cancer, the method comprising:
(a) detecting in a breast tumor sample from the patient the levels of expression of the 17 genes identified in Table 1 as primary predictors, wherein at least one alternate gene listed in Table 1 can be detected in place of the corresponding primary gene listed in Table 1; or (b) detecting in a breast tumor sample from the patient the levels of expression of the 8 genes identified in Table 2 as primary predictors, wherein at least one alternate gene listed in Table 2 can be detected in place of the corresponding primary gene listed in Table 2; and correlating the levels of expression with the likelihood of a relapse. In some embodiments, the method further comprises detecting the level of expression of one or more reference genes, *e.g.,* one or more reference genes selected from the genes identified in Table 3, or one or more reference genes selected from the genes ARPC2, ATF4, ATP5B, B2M, CDH4, CELF1, CLTA, CLTC, COPB1, CTBP1, CYC1, CYFIP1, DAZAP2, DHX15, DIMT1, EEF1A1, FLOT2, GADPH, GUSB, HADHA, HDLBP, HMBS, HNRNPC, HPRT1, HSP90AB1, MTCH1, MYL12B, NACA, NDUFB8, PGK1, PPIA, PPIB, PTBP1, RPL13A, RPLPO, RPS13, RPS23, RPS3, S100A6, SDHA, SEC31A, SET, SF3B1, SFRS3, SNRNP200, STARD7, SUMO1, TBP, TFRC, TMBIM6, TPT1, TRA2B, TUBA1C, UBB, UBC, UBE2D2, UBE2D3, VAMP3, XPO1, YTHDC1, YWHAZ, and 18S rRNA. In some embodiments, the step of determining the levels of expression of the gene comprises detecting the level of expression of RNA. In some embodiments, the determining step comprises detecting the level of expression of protein. The RNA may be detected using any known methods, *e.g.,* a method comprising a quantitative PCR reaction. In some embodiments, detecting the level of expression of the RNA comprises hybridizing a nucleic acid obtained from the sample to an array that comprises probes to all of the 17 genes set forth in Table 1 as primary predictors, and/or to one or more corresponding alternates of said genes as listed in Table 1; or hybridizing a nucleic acid obtained from the sample to an array that comprises probes to all of the 8 genes set forth in Table 2 as primary predictors, and/or to one or more corresponding alternates of said genes as listed in Table 2.

In a further aspect, the disclosure provides a kit for detecting RNA expression comprising primers and/or probes for detecting the level of expression of the 17 genes set forth in Table 1, and/or one or more corresponding alternates thereof; or for detecting the level of expression of the 8 genes set forth in Table 2, and/or one or more alternates thereof. In some aspects of the disclosure, the kit further comprises primers and/or probes for detecting the level of RNA expression of one or more reference genes, *e.g.,* one or more reference genes selected from the genes identified in Table 3, or one or more reference genes selected from the genes ARPC2, ATF4, ATP5B, B2M, CDH4, CELF1, CLTA, CLTC, COPB1, CTBP1, CYC1, CYFIP1, DAZAP2, DHX15, DIMT1, EEF1A1, FLOT2, GADPH, GUSB, HADHA, HDLBP, HMBS, HNRNPC, HPRT1, HSP90AB1, MTCH1, MYL12B, NACA, NDUFB8, PGK1, PPIA, PPIB, PTBP1, RPL13A, RPLPO, RPS13, RPS23, RPS3, S100A6, SDHA, SEC31A, SET, SF3B1, SFRS3, SNRNP200, STARD7, SUMO1, TBP, TFRC, TMBIM6, TPT1, TRA2B, TUBA1C, UBB, UBC, UBE2D2, UBE2D3, VAMP3, XPO1, YTHDC1, YWHAZ, and 18S rRNA.

In a further aspect, the disclosure relates to a microarray comprising probes for detecting the level of expression of the 17 genes set forth in Table 1, and/or one or more corresponding alternates thereof; or for detecting the level of expression of the 8 genes set forth in Table 2, and/or one or more alternates thereof. In some aspects of the disclosure, the microarray further comprises probes for detecting the level of expression of one or more reference genes, *e.g.,* one or more reference genes selected from the genes identified in Table 3, or one or more reference genes selected from the genes ARPC2, ATF4, ATP5B, B2M, CDH4, CELF1, CLTA, CLTC, COPB1, CTBP1, CYC1, CYFIP1, DAZAP2, DHX15, DIMT1, EEF1A1, FLOT2, GADPH, GUSB, HADHA, HDLBP, HMBS, HNRNPC, HPRT1, HSP90AB1, MTCH1, MYL12B, NACA, NDUFB8, PGK1, PPIA, PPIB, PTBP1, RPL13A, RPLPO, RPS13, RPS23, RPS3, S100A6, SDHA, SEC31A, SET, SF3B1, SFRS3, SNRNP200, STARD7, SUMO1, TBP, TFRC, TMBIM6, TPT1, TRA2B, TUBA1C, UBB, UBC, UBE2D2, UBE2D3, VAMP3, XPO1, YTHDC1, YWHAZ, and 18S rRNA.

In an additional aspect, the invention relates to a computer-implemented method for evaluating the likelihood of a relapse for a patient that has a lymph node-negative, estrogen receptor-positive, HER2-negative breast cancer, the method comprising:
receiving, at one or more computer systems, information describing the level of expression of the 17 genes set forth in Table 1 as primary predictors, or one or more alternate genes listed in Table 1 in place of the corresponding primary predictor listed in Table 1; or information describing the level of expression of the 8 genes set forth in Table 2 as primary predictors, or one or more alternate genes listed in Table 2 in place of the primary predictor listed in Table 2; in a breast tumor tissue sample obtained from the patient;
performing, with one or more processors associated with the computer system, a random forest analysis in which the level of expression of each gene in the analysis is assigned to a terminal leaf of each decision tree, representing a vote for either "relapse" or no "relapse";
generating, with the one or more processors associated with the one or more computer systems, a random forest relapse score (RFRS), wherein, if the RFRS is greater than or equal to 0.606 the patient is assigned to a high risk group, if greater than or equal to 0.333 and less than 0.606 the patient is assigned to an intermediate risk group and if less than 0.333 the patient is assigned to low risk group.

In some embodiments, the computer-implemented method further comprises generating, with the one or more processors associated with the one or more computer systems, a likelihood of relapse by comparison of the RFRS score for the patient to a loess fit of RFRS versus likelihood of relapse for a training dataset.

In another aspect, the invention relates to a non-transitory computer-readable medium storing program code for evaluating the likelihood of a relapse for a patient that has a lymph node-negative, estrogen receptor-positive, HER2-negative breast cancer, the computer-readable medium comprising:
code for receiving information describing the level of expression of the 17 genes identified in Table 1 as primary predictors, or one or more alternate genes listed in Table 1 in place of the corresponding primary predictor listed in Table 1; or information describing the level of expression of the 8 genes identified in Table 2 as primary predictors, or one or more alternate genes listed in Table 2 in place of the corresponding primary predictor listed in Table 2; in a breast tumor tissue sample obtained from the patient;
code for performing a random forest analysis in which the level of expression of each gene in the analysis is assigned to a terminal leaf of each decision tree, representing a vote for either "relapse" or no "relapse"; and
code for generating a random forest relapse score (RFRS), wherein if the RFRS is greater than or equal to 0.606 the patient is assigned to a high risk group, if greater than or equal to 0.333 and less than 0.606 the patient is assigned to an intermediate risk group and if less than 0.333 the patient is assigned to low risk group. In some embodiments, the non-transitory computer-readable medium storing program code further comprises code for generating a likelihood of relapse by comparison of the RFRS score for the patient to a loess fit of RFRS versus likelihood of relapse for a training dataset.

### BRIEF DESCRIPTION OF THE DRAWINGS

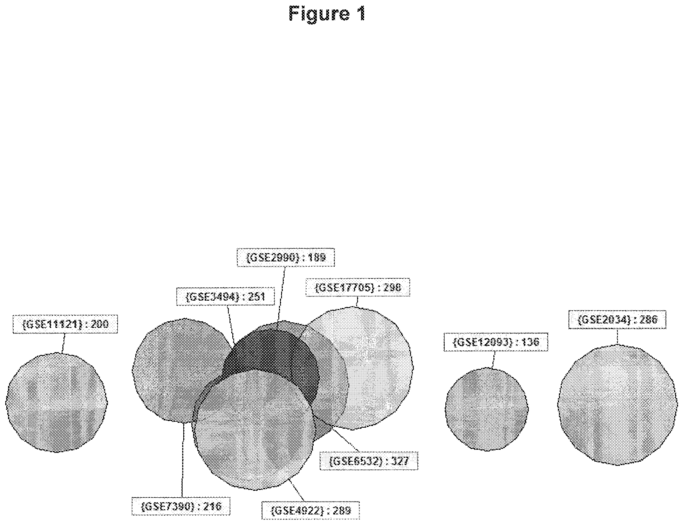

Figure 1 shows an analysis of the studies employed in Example 1 to identify duplicates. The diagram shows the approximate overlap between GEO datasets used. Three studies show zero overlap while the other six show significant overlap.

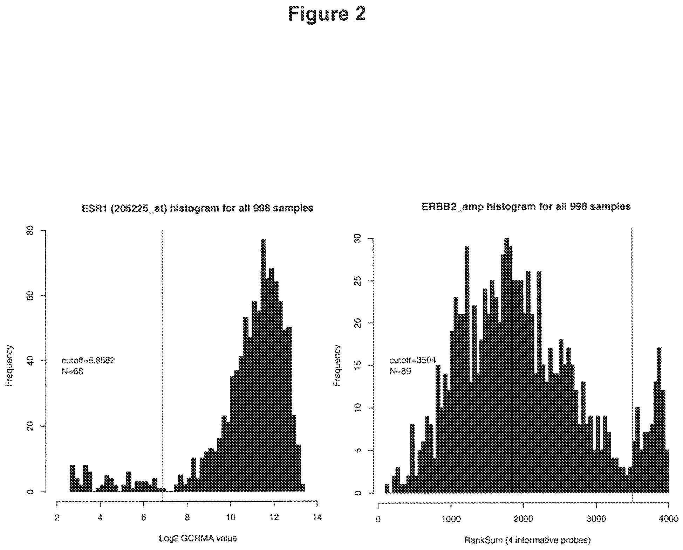

Figure 2 shows estrogen receptor and HER2 status for 998 samples employed in Example 1. Expression status was determined using the "205225_at" probe set for ER and the rank sum of the 216835_s_at (ERBB2), 210761_s_at (GRB7), 202991_at (STARD3) and 55616_at (PGAP3) probe sets for HER2. Threshold values were chosen by mixed model clustering. A total of 68 samples were determined to be ER-negative and 89 samples were determined to be HER2-positive. In total, 140 samples were either HER2-positive or ER-negative (17 were both) and were filtered from further analysis.

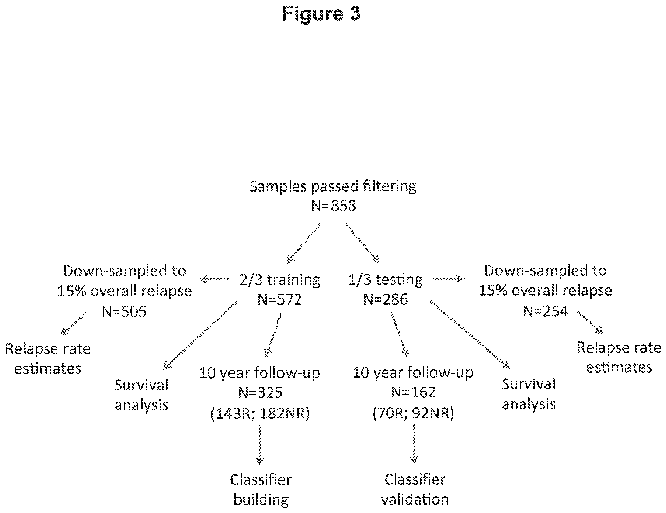

Figure 3 illustrates the breakdown of samples for analysis. A total of 858 samples passed all filtering steps including 487 samples with 10-year follow-up data (213 relapse; 274 no relapse). The remaining 371 samples had insufficient follow-up for 10-year classification analysis but were retained for use in survival analysis. The 858 samples were broken into two-thirds training and one-third testing sets resulting in: a training set of 572 samples for use in survival analysis and 325 samples with 10yr follow-up (143 relapse; 182 no relapse) for classification analysis; and a testing set of 286 samples for use in survival analysis and 162 samples with 10-year follow-up (70 relapse; 92 no relapse) for classification analysis

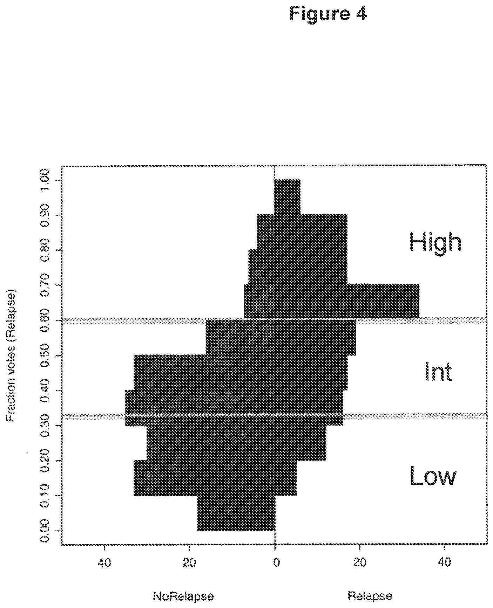

Figure 4 illustrates risk group threshold determination. The distribution of RFRS scores was determined for patients in the training dataset (N=325) comparing those with a known relapse (right side) versus those with no known relapse (left side). As expected, patients without a known relapse tend to have a higher predicted likelihood of relapse (by RFRS) and vice versa. Mixed model clustering was used to identify thresholds (0.333 and 0.606) for defining low, intermediate, and high-risk groups as indicated.

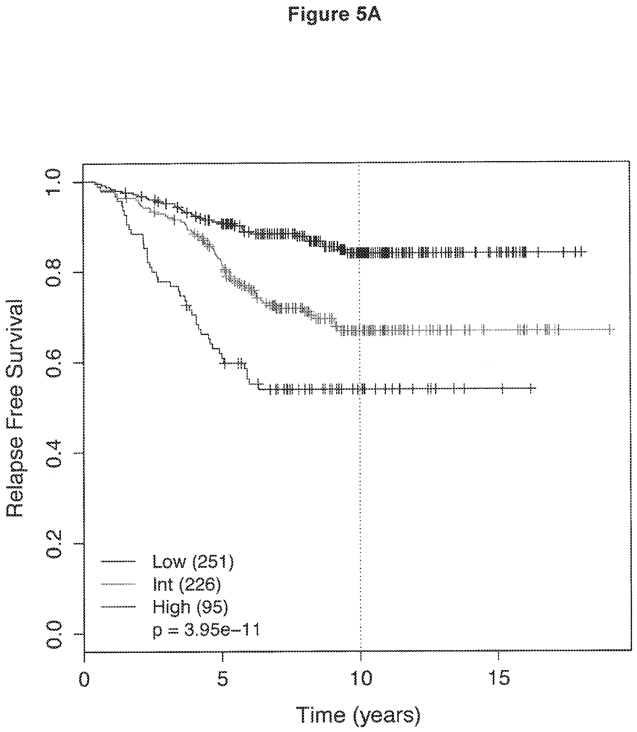

Figure 5A-C provide data illustrating likelihood of relapse according to RFRS group. The survival plot shows relapse-free survival comparing (from top to bottom) low-risk, intermediate-risk, and high-risk groups as determined by RFRS for: (A) the full-gene-set model on training data; (B) the 8-gene-set model on independent test data; (C) the 8-gene-set model on the independent NKI data set. Significance between risk groups was determined by Kaplan-Meier logrank test (with test for linear trend).

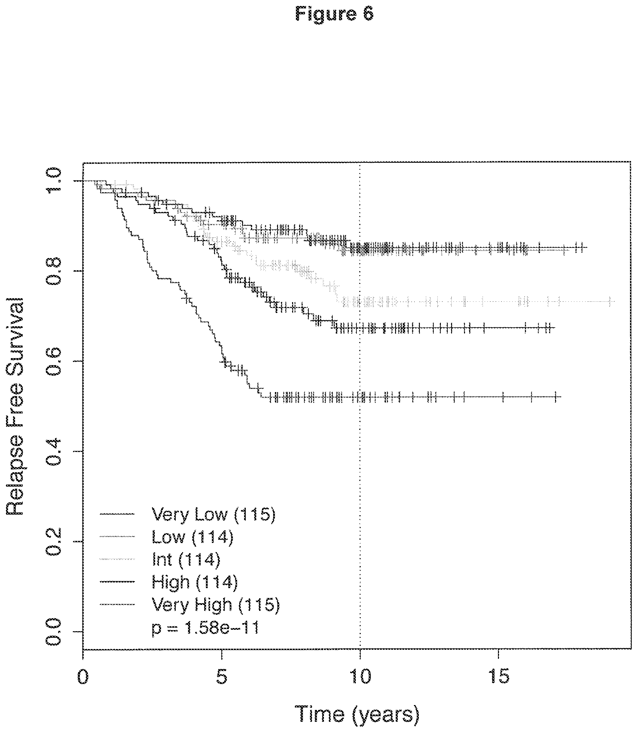

Figure 6 illustrates likelihood of relapse according to RFRS group with breakdown into additional risk groups. The survival plot shows relapse-free survival comparing (from top to bottom) very-low-risk, low-risk, intermediate-risk, high-risk, and very-high-risk groups as determined by RFRS. Significance between risk groups was determined by Kaplan-Meier logrank test (with test for linear trend).

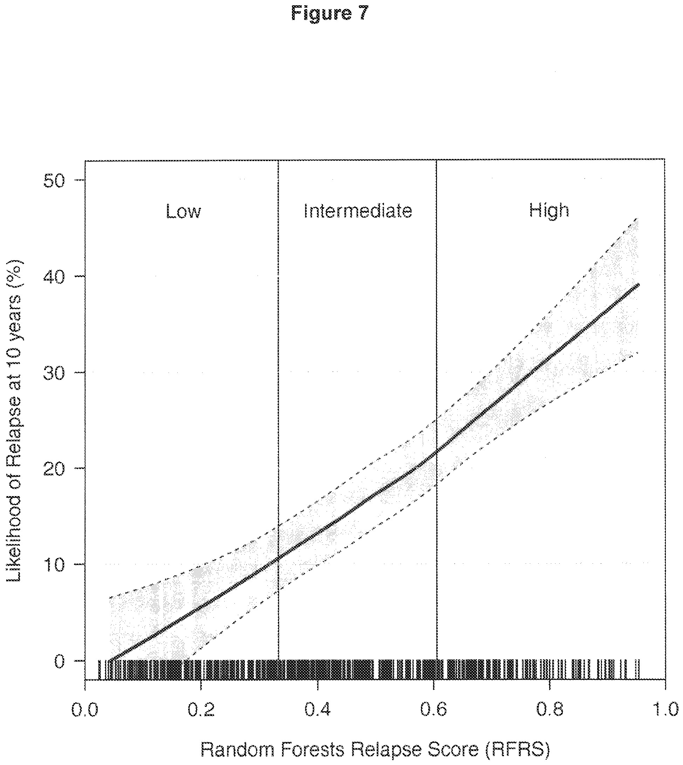

Figure 7 illustrates estimated likelihood of relapse at 10 years for any RFRS value. The likelihood of relapse was calculated in the training data set (N=505) for 50 RFRS intervals (from 0 to 1). A smooth curve was fitted using a loess function and 95% confidence intervals plotted to represent the error in the fit. Short vertical marks just above the x-axis, one for each patient, represent the distribution of RFRS values observed in the training data. Thresholds for risk groups are indicated. The plot shows a linear relationship between RFRS and likelihood of relapse at 10 years with the likelihood ranging from approximately 0 to 40%.

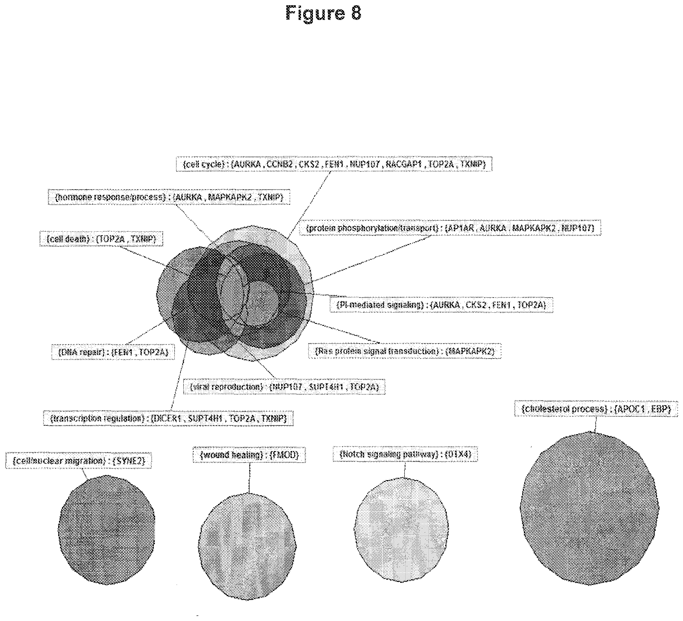

Figure 8 shows a gene ontology analysis of the genes identified for the 17-gene signature panel. A Gene Ontology (GO) analysis was performed using DAVID to identify the associated GO biological processes for the 17-gene model. The diagram represents the approximate overlap between GO terms. To simplify, redundant terms were grouped together. Genes in the 17-gene list are involved in a wide range of biological processes known to be involved in breast cancer biology including cell cycle, hormone response, cell death, DNA repair, transcription regulation, wound healing and others. Since the 8-gene set is entirely contained in the 17-gene set it would be involved in many of the same processes.

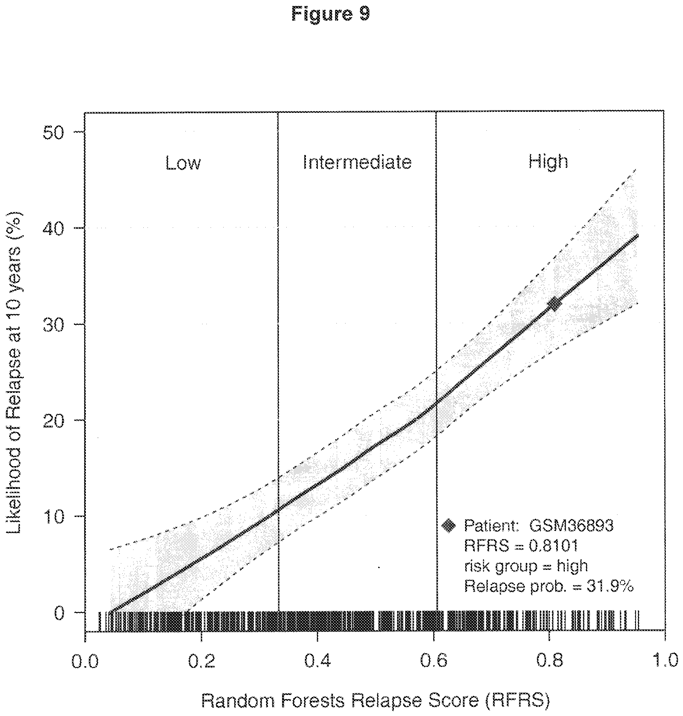

Figure 9 provides a sample patient report of risk of relapse generated in accordance with the invention. Using the RFRS algorithm, a patient would be assigned an RFRS value. If RFRS is greater than or equal to 0.606 the patient is assigned to the "high-risk" group, if greater than or equal to 0.333 and less than 0.606 the patient is assigned to "intermediate-risk" group and if less than 0.333 the patient is assigned to "low-risk" group. The patient's RFRS value is also used to determine a likelihood of relapse by comparison to a precalculated loess fit of RFRS versus likelihood of relapse for the training dataset. The patient's estimated likelihood of relapse is determined, added to the summary plot, and output as a new report.

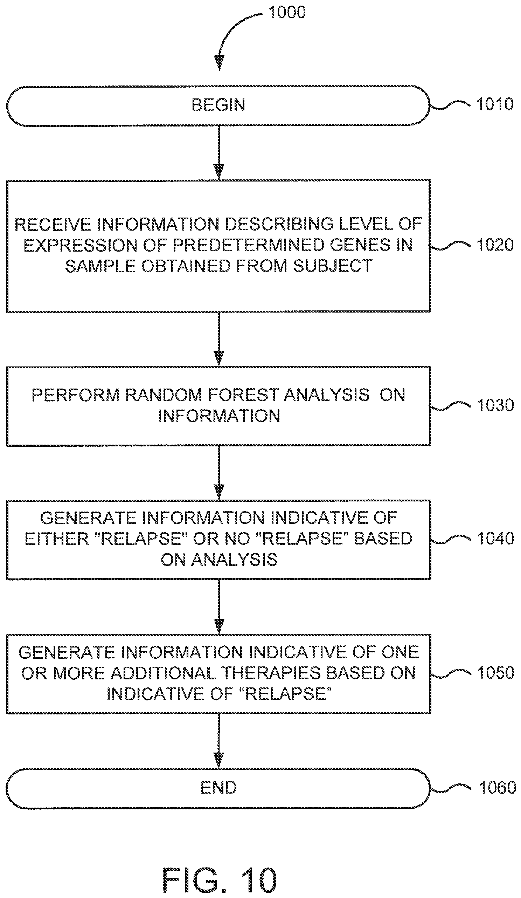

Figure 10 (FIG. 10) is a flowchart of a method for identifying LN⁻ER⁺HER2⁻ breast cancer patients that are candidates for additional treatment in one embodiment.

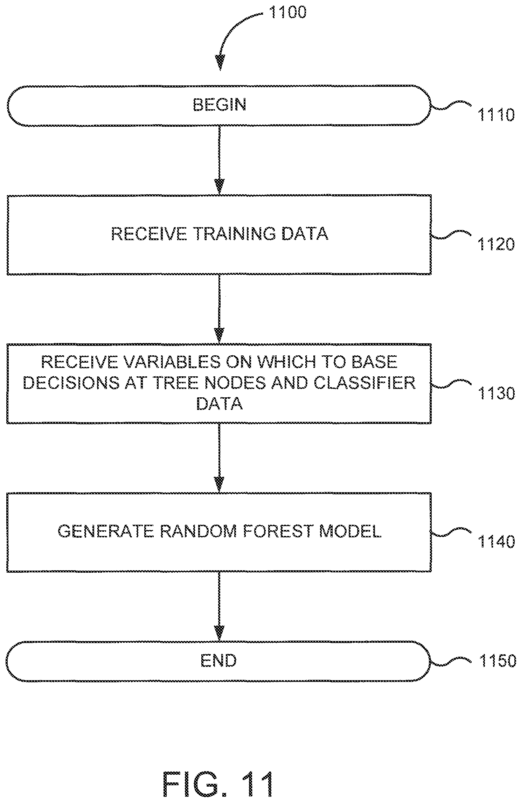

Figure 11 (FIG.11) is a flowchart of a method for generating an RF model for identifying LN⁻ER⁺HER2⁻ breast cancer patients that are candidates for additional treatment in one embodiment.

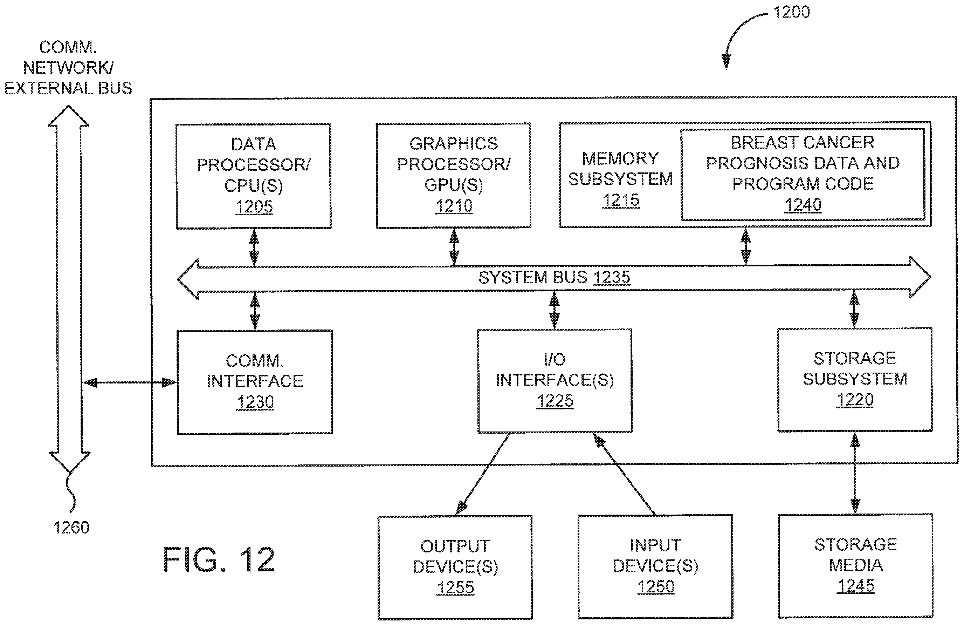

Figure 12 (FIG. 12) is a block diagram of computer system 1200 that may incorporate an embodiment, be incorporated into an embodiment, or be used to practice any of the innovations, embodiments, and/or examples found within this disclosure.

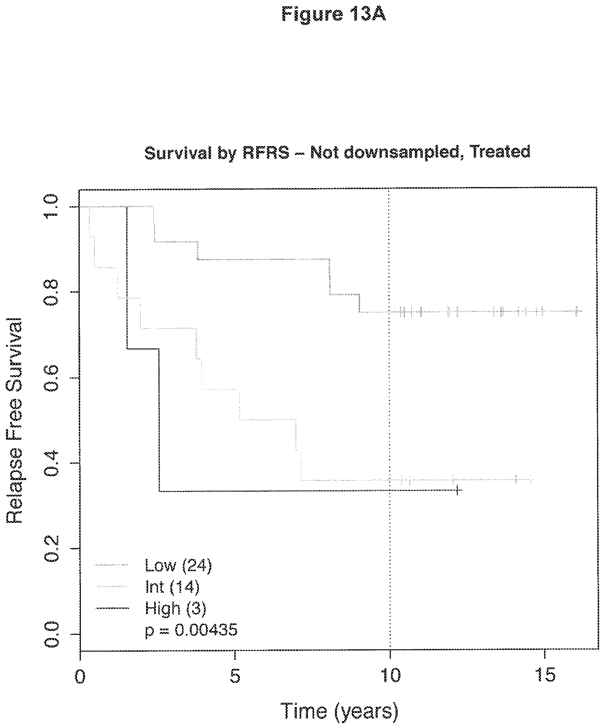

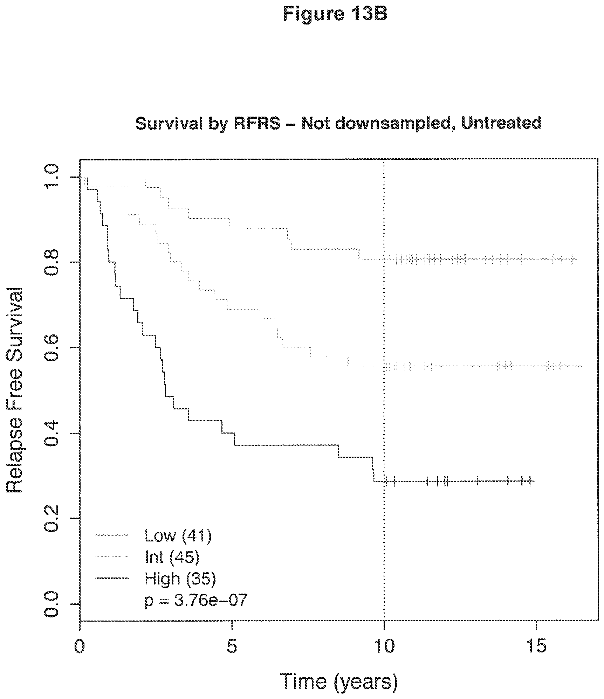

Figure 13A and B illustrate likelihood of relapse according to RFRS group stratified by treatment status. The survival plot shows relapse-free survival comparing (from top to bottom) low-risk, intermediate-risk, and high-risk groups as determined by RFRS for: (A) hormone-therapy-treated and (B) untreated. Significance between risk groups was determined by Kaplan-Meier logrank test (with test for linear trend).

### DETAILED DESCRIPTION OF THE INVENTION

An "estrogen receptor positive, lymph node-negative, HER2-negative" or "ER⁺LN⁻HER2⁻" patient as used herein refers to a patient that has no discernible breast cancer in the lymph nodes; and has breast tumor cells that express estrogen receptor and do not show evidence of HER2 genomic (DNA) amplification or HER2 over-expression. LN- status is typically determined when the sentinel node is surgically removed and examined by microscopy for cytological evidence of disease. Patients are considered LN- (N0) if zero positive nodes were observed. Patients are considered LN+ if one or more lymph nodes were considered positive for disease (1-2 positive = N1; 3-6 positive = N2, etc). ER⁺ status is typically assessed by immunohistochemistry (IHC) where a positive determination is made when greater than a small percentage (typically greater than 3%, 5% or 10%) of cells stain positive. ER status can also be tested by quantitative PCR or biochemical assays. HER2⁻status is generally determined by either IHC, fluorescence in situ hybridization (FISH) or some combination of the two methods. Typically, a patient is first tested by IHC and scored on a scale from 0 to 3 where a "3+" score indicates strong complete membrane staining on >5-10% of tumor cells and is considered positive. No staining (score of "0") or a "1+" score, indicating faint partial membrane staining in greater than 5-10% of cells, is considered negative. An intermediate score of "2+", indicating weak to moderate complete membrane staining in more than 5-10% of cells, may prompt further testing by FISH. A typical HER2 FISH scheme would consider a patient HER2⁺ if the ratio of a HER2 probe to a centromeric (reference) probe is more than 4:1 in ∼5% or more of cells after examining 20 or more metaphase spreads. Otherwise the patient is considered HER2⁻. Quantitative PCR, array-based hybridization, and other methods may also be used to determine HER2 status. The specific methods and cutoff points for determining LN, ER and HER2 status may vary from hospital to hospital. For the purpose of this invention, a patient will be considered "ER⁺LN⁻ HER2⁻" if reported as such by their health care provider or if determined by any accepted and approved methods, including but not limited to those detailed above.

In the current invention, a "gene set forth in" a table or a "gene identified in" a table are used interchangeably to refer to the gene that is listed in that table. For example, a gene "identified in" Table 4 refers to the gene that corresponds to the gene listed in Table 4. As understood in the art, there are naturally occurring polymorphisms for many gene sequences. Genes that are naturally occurring allelic variations for the purposes of this invention are those genes encoded by the same genetic locus. The proteins encoded by allelic variations of a gene set forth in Table 4 (or in any of Tables 1-3 or Table 4) typically have at least 95% amino acid sequence identity to one another, *i.e*., an allelic variant of a gene indicated in Table 4 typically encodes a protein product that has at least 95% identity, often at least 96%, at least 97%, at least 98%, or at least 99%, or greater, identity to the amino acid sequence encoded by the nucleotide sequence denoted by the Entrez Gene ID number (April 1, 2012) shown in Table 4 for that gene. For example, an allelic variant of a gene encoding CCNB2 (gene: cyclin B2) typically has at least 95% identity, often at least 96%, at least 97%, at least 98%, or at least 99%, or greater, to the CCNB2 protein sequence encoded by the nucleic acid sequence available under Entrez Gene ID no. 9133). A "gene identified in" a table, such as Table 4, also refers to a gene that can be unambiguously mapped to the same genetic locus as that of a gene assigned to a genetic locus using the probes for the gene that are listed in Appendix 3. Similarly, a "gene identified in Table 1" or a "gene identified in Table 2" refers to a gene that can be unambiguously mapped to a genetic locus using the probes for that gene that are listed in Appendix 4 (panel of 17 genes from Table 1, which includes the genes for the 8 gene panel identified in Table 2); and a "gene identified in Table 3" refers to a gene that can be unambiguously mapped to a genetic locus using the probes for that gene that are listed in Appendix 5.

The terms "identical" or "100% identity," in the context of two or more nucleic acids or proteins refer to two or more sequences or subsequences that are the same sequences. Two sequences are "substantially identical" or a certain percent identity if two sequences have a specified percentage of amino acid residues or nucleotides that are the same (i.e., 70% identity, optionally 75%, 80%, 85%, 90%, or 95% identity, over a specified region, or, when not specified, over the entire sequence), when compared and aligned for maximum correspondence over a comparison window, or designated region as measured using known sequence comparison algorithms, e.g., BLAST using the default parameters, or by manual alignment and visual inspection.

A "gene product" or "gene expression product" in the context of this invention refers to an RNA or protein encoded by the gene.

The term "evaluating a biomarker" in an LN⁻ER⁺HER2⁻ patient refers to determining the level of expression of a gene product encoded by a gene, or allelic variant of the gene, listed in Table 4. Preferably, the gene is listed in Table 1 or Table 2 as either a primary or alternate gene. Typically, the RNA expression level is determined.

### Introduction

The invention is based, in part, on the identification of a panel of at least eight genes whose gene expression level correlates with breast cancer prognosis. In some aspects, the panel of at least eight genes comprises at least eight genes, or at least 9, 10, 11, 12, 13, 14, 15, 16, 17, 18, 19, 20, 25, 30, 35, 40, 45, or 50, or more genes, identified in Table 4 with the proviso that the gene is one of those also listed in Table 5. In some aspects, the panel of genes comprises at least 8 primary genes, or at least 9, 10, 11, 12, 13, 14, 15, 16, or all 17 primary genes identified in Table 1; or the 8 primary genes set forth in Table 2. Table 1 also shows alternate genes for each of the seventeen that can replace the specific primary gene in the analysis. At least one alternate gene can be evaluated in place of the corresponding primary gene listed in Table 1, or can be evaluated in addition to the corresponding primary gene listed in Table 1. Similarly, Table 2 shows alternate genes for each of the eight that can replace, or be assayed in addition to, the specific primary gene in the analysis. The results of the expression analysis are then evaluated using an algorithm to determine breast cancer patients that are likely to have a recurrence, and accordingly, are candidates for treatment with more aggressive therapy, such as chemotherapy.

The invention therefore relates to measurement of expression levels of a biomarker panel, *e.g.,* a 17-gene expression panel, or an 8-gene expression panel, in a breast cancer patient prior to the patient undergoing chemotherapy. In some embodiments, probes to detect such transcripts may be applied in the form of a diagnostic device to predict which LN⁻ER⁺HER2⁻ breast cancer patients have a greater risk for relapse.

The methods of the invention comprise determining the expression levels of all seventeen primary genes, and/or at least one corresponding alternate gene shown in Table 1. However, in some aspects of the disclosure, the expression level of fewer genes, *e.g.*, 5, 6, 7, 8, 9, 10, 11, 12, 13, 14, 15, or 16 genes, may be evaluated. In some embodiments, the methods of the invention comprise determining the expression level of all eight gene and/or at least one corresponding alternate gene shown in Table 2. Gene expression levels may be measured using any number of methods known in the art. In typical embodiments, the method involves measuring the level of RNA. RNA expression can be quantified using any method, *e.g*., employing a quantitative amplification method such as qPCR. In other embodiments, the methods employ array-based assays. In still other embodiments, protein products may be detected. The gene expression patterns are determined using a sample obtained from breast tumor.

In the context of this invention, an "alternate gene" refers to a gene that can be evaluated for expression levels instead of, or in addition to, the gene for which the "alternate gene" is the designated alternate in Table 1. For example, one of the genes in Table 1 is CCNB2. MELK and GINS1 are both alternatives that can be evaluated for expression instead of CCNB2 or in addition to CCNB2, when evaluating the gene expression levels of the 17 genes set forth in Table 1. With respect to Table 2, an "alternate gene" refers to a gene that can be evaluated for expression levels instead of, or in addition to, the gene for which the "alternate gene" is the designated alternate in Table 2. For example, one of the genes in Table 2 is CCNB2. MELK and TOP2A are both alternatives that can be evaluated for expression instead of CCNB2 or in addition to CCNB2 when evaluating the gene expression levels of the 8 genes set forth in Table 2.

### Methods for Quantifying RNA

The quantity of RNA encoded by a gene set forth in Table 1 or Table 2 and optionally, a gene set forth in Table 3 or an alternative reference gene, can be readily determined according to any method known in the art for quantifying RNA. Various methods involving amplification reactions and/or reactions in which probes are linked to a solid support and used to quantify RNA may be used. Alternatively, the RNA may be linked to a solid support and quantified using a probe to the sequence of interest.

An "RNA nucleic acid sample" analyzed in the invention is obtained from a breast tumor sample obtained from the patient. An "RNA nucleic acid sample" comprises RNA, but need not be purely RNA, *e.g.,* DNA may also be present in the sample. Techniques for obtaining an RNA sample from tumors are well known in the art.

In some embodiments, the target RNA is first reverse transcribed and the resulting cDNA is quantified. In some embodiments, RT-PCR or other quantitative amplification techniques are used to quantify the target RNA. Amplification of cDNA using PCR is well known (see U.S. Patents 4,683,195 and 4,683,202; PCR PROTOCOLS: A GUIDE TO METHODS AND APPLICATIONS (Innis et al., eds, 1990)). Methods of quantitative amplification are disclosed in, e.g., U.S. Patent Nos. 6,180,349; 6,033,854; and 5,972,602, as well as in, e.g., Gibson et al., Genome Research 6:995-1001 (1996); DeGraves, et al., Biotechniques 34(1):106-10, 112-5 (2003); Deiman B, et al., Mol Biotechnol. 20(2):163-79 (2002). Alternative methods for determining the level of a mRNA of interest in a sample may involve other nucleic acid amplification methods such as ligase chain reaction (Barany (1991) Proc. Natl. Acad. Sci. USA 88:189-193), self-sustained sequence replication (Guatelli et al. (1990) Proc. Natl. Acad. Sci. USA 87:1874-1878), transcriptional amplification system (Kwoh et al. (1989) Proc. Natl. Acad. Sci. USA 86:1173-1177), Q-Beta Replicase (Lizardi et al. (1988) Bio/Technology 6:1197), rolling circle replication (U.S. Patent No. 5,854,033) or any other nucleic acid amplification method, followed by the detection of the amplified molecules using techniques well known to those of skill in the art.

In general, quantitative amplification is based on the monitoring of the signal (e.g., fluorescence of a probe) representing copies of the template in cycles of an amplification (e.g., PCR) reaction. One method for detection of amplification products is the 5'-3' exonuclease "hydrolysis" PCR assay (also referred to as the TaqMan™ assay) (U.S. Pat. Nos. 5,210,015 and 5,487,972; Holland et al., PNAS USA 88: 7276-7280 (1991); Lee et al., Nucleic Acids Res. 21: 3761-3766 (1993)). This assay detects the accumulation of a specific PCR product by hybridization and cleavage of a doubly labeled fluorogenic probe (the "TaqMan™" probe) during the amplification reaction. The fluorogenic probe consists of an oligonucleotide labeled with both a fluorescent reporter dye and a quencher dye. During PCR, this probe is cleaved by the 5'-exonuclease activity of DNA polymerase if, and only if, it hybridizes to the segment being amplified. Cleavage of the probe generates an increase in the fluorescence intensity of the reporter dye.

Another method of detecting amplification products that relies on the use of energy transfer is the "beacon probe" method described by Tyagi and Kramer, Nature Biotech. 14:303-309 (1996), which is also the subject of U.S. Patent Nos. 5,119,801 and 5,312,728. This method employs oligonucleotide hybridization probes that can form hairpin structures. On one end of the hybridization probe (either the 5' or 3' end), there is a donor fluorophore, and on the other end, an acceptor moiety. In the case of the Tyagi and Kramer method, this acceptor moiety is a quencher, that is, the acceptor absorbs energy released by the donor, but then does not itself fluoresce. Thus, when the beacon is in the open conformation, the fluorescence of the donor fluorophore is detectable, whereas when the beacon is in hairpin (closed) conformation, the fluorescence of the donor fluorophore is quenched. When employed in PCR, the molecular beacon probe, which hybridizes to one of the strands of the PCR product, is in "open conformation," and fluorescence is detected, while those that remain unhybridized will not fluoresce (Tyagi and Kramer, Nature Biotechnol. 14: 303-306 (1996)). As a result, the amount of fluorescence will increase as the amount of PCR product increases, and thus may be used as a measure of the progress of the PCR. Those of skill in the art will recognize that other methods of quantitative amplification are also available.

Various other techniques for performing quantitative amplification of nucleic acids are also known. For example, some methodologies employ one or more probe oligonucleotides that are structured such that a change in fluorescence is generated when the oligonucleotide(s) is hybridized to a target nucleic acid. For example, one such method involves a dual fluorophore approach that exploits fluorescence resonance energy transfer (FRET), e.g., LightCycler™ hybridization probes, where two oligo probes anneal to the amplicon. The oligonucleotides are designed to hybridize in a head-to-tail orientation with the fluorophores separated at a distance that is compatible with efficient energy transfer. Other examples of labeled oligonucleotides that are structured to emit a signal when bound to a nucleic acid or incorporated into an extension product include: Scorpions™ probes (e.g., Whitcombe et al., Nature Biotechnology 17:804-807, 1999, and U.S. Pat. No. 6,326,145), Sunrise™ (or Amplifluor™) probes (e.g., Nazarenko et al., Nuc. Acids Res. 25:2516-2521, 1997, and U.S. Pat. No. 6,117,635), and probes that form a secondary structure that results in reduced signal without a quencher and that emits increased signal when hybridized to a target (e.g., Lux probes™).

In other embodiments, intercalating agents that produce a signal when intercalated in double stranded DNA may be used. Exemplary agents include SYBR GREEN™ and SYBR GOLD™. Since these agents are not template-specific, it is assumed that the signal is generated based on template-specific amplification. This can be confirmed by monitoring signal as a function of temperature because melting point of template sequences will generally be much higher than, for example, primer-dimers, etc.

In other embodiments, the mRNA is immobilized on a solid surface and contacted with a probe, *e.g.,* in a dot blot or Northern format. In an alternative embodiment, the probe(s) are immobilized on a solid surface and the mRNA is contacted with the probe(s), for example, in a gene chip array. A skilled artisan can readily adapt known mRNA detection methods for use in detecting the level of mRNA encoding the biomarkers or other proteins of interest.

In some embodiments, microarrays, e.g., are employed. DNA microarrays provide one method for the simultaneous measurement of the expression levels of large numbers of genes. Each array consists of a reproducible pattern of capture probes attached to a solid support. Labeled RNA or DNA is hybridized to complementary probes on the array and then detected by laser scanning. Hybridization intensities for each probe on the array are determined and converted to a quantitative value representing relative gene expression levels. See, U.S. Patent Nos. 6,040,138, 5,800,992 and 6,020,135, 6,033,860, and 6,344,316. High-density oligonucleotide arrays are particularly useful for determining the gene expression profile for a large number of RNA's in a sample.

Techniques for the synthesis of these arrays using mechanical synthesis methods are described in, e.g., U.S. Patent No. 5,384,261. Although a planar array surface is often employed the array may be fabricated on a surface of virtually any shape or even a multiplicity of surfaces. Arrays may be peptides or nucleic acids on beads, gels, polymeric surfaces, fibers such as fiber optics, glass or any other appropriate substrate, see U.S. Patent Nos. 5,770,358, 5,789,162, 5,708,153, 6,040,193 and 5,800,992. Arrays may be packaged in such a manner as to allow for diagnostics or other manipulation of an all-inclusive device.

Primer and probes for use in amplifying and detecting the target sequence of interest can be selected using well-known techniques.

In some embodiments, the methods of the invention further comprise detecting level of expression of one or more reference genes that can be used as controls to determine expression levels. Such genes are typically expressed constitutively at a high level and can act as a reference for determining accurate gene expression level estimates. Examples of control genes are provided in Table 3 and the following list: ARPC2, ATF4, ATP5B, B2M, CDH4, CELF1, CLTA, CLTC, COPB1, CTBP1, CYC1, CYFIP1, DAZAP2, DHX15, DIMT1, EEF1A1, FLOT2, GADPH, GUSB, HADHA, HDLBP, HMBS, HNRNPC, HPRT1, HSP90AB1, MTCH1, MYL12B, NACA, NDUFB8, PGK1, PPIA, PPIB, PTBP1, RPL13A, RPLP0, RPS13, RPS23, RPS3, S100A6, SDHA, SEC31A, SET, SF3B1, SFRS3, SNRNP200, STARD7, SUMO1, TBP, TFRC, TMBIM6, TPT1, TRA2B, TUBA1C, UBB, UBC, UBE2D2, UBE2D3, VAMP3, XPO1, YTHDC1, YWHAZ, and 18S rRNA genes. Accordingly, a determination of RNA expression levels of the genes of interest, *e.g*., the gene expression levels of the panel of genes identified in Table 1, and/or an alternate; or the gene expression levels of the panel of genes identified in Table 2, and/or an alternate; may also comprise determining expression levels of one or more reference genes set forth in Table 3 or one or more reference genes selected from the genes ARPC2, ATF4, ATP5B, B2M, CDH4, CELF1, CLTA, CLTC, COPB1, CTBP1, CYC1, CYFIP1, DAZAP2, DHX15, DIMT1, EEF1A1, FLOT2, GADPH, GUSB, HADHA, HDLBP, HMBS, HNRNPC, HPRT1, HSP90AB1, MTCH1, MYL12B, NACA, NDUFB8, PGK1, PPIA, PPIB, PTBP1, RPL13A, RPLPO, RPS13, RPS23, RPS3, S100A6, SDHA, SEC31A, SET, SF3B1, SFRS3, SNRNP200, STARD7, SUMO1, TBP, TFRC, TMBIM6, TPT1, TRA2B, TUBA1C, UBB, UBC, UBE2D2, UBE2D3, VAMP3, XPO1, YTHDC1, YWHAZ, and 18S rRNA.

In the context of this invention, "determining the levels of expression" of an RNA of interest encompasses any method known in the art for quantifying an RNA of interest.

### Detection of protein levels

In some embodiments, *e.g*., where the expression level of a protein encoded by a biomarker gene set forth in Table 1 or Table 2 is measured. Often, such measurements may be performed using immunoassays. Protein expression level is determined using a breast tumor sample obtained from the patient.

A general overview of the applicable technology can be found in Harlow & Lane, Antibodies: A Laboratory Manual (1988) and Harlow & Lane, Using Antibodies (1999). Methods of producing polyclonal and monoclonal antibodies that react specifically with an allelic variant are known to those of skill in the art (*see, e.g.,* Coligan, Current Protocols in Immunology (1991); Harlow & Lane, *supra;* Goding, Monoclonal Antibodies: Principles and Practice (2d ed. 1986); and Kohler & Milstein, Nature 256:495-497 (1975)). Such techniques include antibody preparation by selection of antibodies from libraries of recombinant antibodies in phage or similar vectors, as well as preparation of polyclonal and monoclonal antibodies by immunizing rabbits or mice (*see, e.g*., Huse et al., Science 246:1275-1281 (1989); Ward et al., Nature 341:544-546 (1989)).

Polymorphic alleles can be detected by a variety of immunoassay methods. For a review of immunological and immunoassay procedures, see Basic and Clinical Immunology (Stites & Terr eds., 7th ed. 1991). Moreover, the immunoassays can be performed in any of several configurations, which are reviewed extensively in Enzyme Immunoassay (Maggio, ed., 1980); and Harlow & Lane, *supra*. For a review of the general immunoassays, see also Methods in Cell Biology: Antibodies in Cell Biology, volume 37 (Asai, ed. 1993); Basic and Clinical Immunology (Stites & Terr, eds., 7th ed. 1991).

Commonly used assays include noncompetitive assays, *e.g*., sandwich assays, and competitive assays. Typically, an assay such as an ELISA assay can be used. The amount of the polypeptide variant can be determined by performing quantitative analyses.

Other detection techniques, *e.g*., MALDI, may be used to directly detect the presence of proteins correlated with treatment outcomes.

As indicated above, evaluation of protein expression levels may additionally include determining the levels of protein expression of control genes, *e.g.,* of one or more genes identified in Table 3.

### Devices and Kits

In a further aspect, the disclosure provides diagnostic devices and kits for identifying gene expression products of a panel of genes that is associated with prognosis for a LN⁻ER⁺HER2⁻ breast cancer patient.

In some aspects, a diagnostic device comprises probes to detect at least 8, 9, 10, 11, 12, 13, 14, 15, 16, or all 17 gene expression products set forth in Table 1, and/or alternates. In some aspects, a diagnostic device comprises probes to detect the expression products of the 8 genes set forth in Table 2, and/or alternates. In some aspects, the present disclosure provides oligonucleotide probes attached to a solid support, such as an array slide or chip, *e.g.,* as described in DNA Microarrays: A Molecular Cloning Manual, 2003, Eds. Bowtell and Sambrook, Cold Spring Harbor Laboratory Press. Construction of such devices are well known in the art, for example as described in US Patents and Patent Publications U.S. Patent No. 5,837,832; PCT application WO95/11995; U.S. Patent No. 5,807,522; US Patent Nos. 7,157,229, 7,083,975, 6,444,175, 6,375,903, 6,315,958, 6,295,153, and 5,143,854, 2007/0037274, 2007/0140906, 2004/0126757, 2004/0110212, 2004/0110211, 2003/0143550, 2003/0003032, and 2002/0041420. Nucleic acid arrays are also reviewed in the following references: Biotechnol Annu Rev 8:85-101 (2002); Sosnowski et al, Psychiatr Genet 12(4):181-92 (Dec. 2002); Heller, Annu Rev Biomed Eng 4: 129-53 (2002); Kolchinsky et al, Hum. Mutat 19(4):343-60 (Apr. 2002); and McGail et al, Adv Biochem Eng Biotechnol 77:21-42 (2002).

An array can be composed of a large number of unique, single-stranded polynucleotides, usually either synthetic antisense polynucleotides or fragments of cDNAs, fixed to a solid support. Typical polynucleotides are preferably about 6-60 nucleotides in length, more preferably about 15-30 nucleotides in length, and most preferably about 18-25 nucleotides in length. For certain types of arrays or other detection kits/systems, it may be preferable to use oligonucleotides that are only about 7-20 nucleotides in length. In other types of arrays, such as arrays used in conjunction with chemiluminescent detection technology, preferred probe lengths can be, for example, about 15-80 nucleotides in length, preferably about 50-70 nucleotides in length, more preferably about 55-65 nucleotides in length, and most preferably about 60 nucleotides in length.

A person skilled in the art will recognize that, based on the known sequence information, detection reagents can be developed and used to assay any gene expression product set forth in Table 1 or Table 2 (or in some embodiments Table 3 or another reference gene described herein) and that such detection reagents can be incorporated into a kit. The term "kit" as used herein in the context of detection reagents, are intended to refer to such things as combinations of multiple gene expression detection reagents, or one or more gene expression detection reagents in combination with one or more other types of elements or components (e.g., other types of biochemical reagents, containers, packages such as packaging intended for commercial sale, substrates to which gene expression detection reagents are attached, electronic hardware components, etc.). Accordingly, the present disclosure further provides gene expression detection kits and systems, including but not limited to, packaged probe and primer sets (e.g., TaqMan probe/primer sets), arrays/microarrays of nucleic acid molecules where the arrays/microarrays comprise probes to detect the level of RNA transcript, and beads that contain one or more probes, primers, or other detection reagents for detecting one or more RNA transcripts encoded by a gene in a gene expression panel of the present invention. The kits can optionally include various electronic hardware components; for example, arrays ("DNA chips") and microfluidic systems ("lab-on-a-chip" systems) provided by various manufacturers typically comprise hardware components. Other kits (e.g., probe/primer sets) may not include electronic hardware components, but may be comprised of, for example, one or more biomarker detection reagents (along with, optionally, other biochemical reagents) packaged in one or more containers.

In some aspects, a detection kit typically contains one or more detection reagents and other components (e.g. a buffer, enzymes such as DNA polymerases) necessary to carry out an assay or reaction, such as amplification for detecting the level of transcript. A kit may further contain means for determining the amount of a target nucleic acid, and means for comparing the amount with a standard, and can comprise instructions for using the kit to detect the nucleic acid molecule of interest. In one aspect of the present disclosure, kits are provided which contain the necessary reagents to carry out one or more assays to detect one or more RNA transcripts of a gene disclosed herein. In one aspect of the present disclosure, biomarker detection kits/systems are in the form of nucleic acid arrays, or compartmentalized kits, including microfluidic/lab-on-a-chip systems.

Detection kits/systems for detecting expression of a panel of genes may contain, for example, one or more probes, or pairs or sets of probes, that hybridize to a nucleic acid molecule encoded by a gene set forth in Table 1 or Table 2. In some embodiments, the presence of more than one biomarker can be simultaneously evaluated in an assay. For example, in some embodiments probes or probe sets to different biomarkers are immobilized as arrays or on beads. For example, the same substrate can comprise probes for detecting expression of at least 8, 9, 10, 11, 12, 13, 14, 15, 16, or 17 or more of the genes set forth in Table 1, and/or alternates to the genes. In some embodiments, the same substrate can comprise probes for detecting expression of 8 or more genes set forth in Table 2, and/or alternates to the genes.

Using such arrays or other kits/systems, the present disclosure provides methods of identifying the levels of expression of a gene described herein in a test sample. Such methods typically involve incubating a test sample of nucleic acids obtained from a breast tumor from a LN⁻ER⁺HER2⁻patient with an array comprising one or more probes that selectively hybridizes to a nucleic acid encoded by a gene identified in Table 1 or Table 2. Such an array may additionally comprise probes to one or more reference genes identified in Table 3, or one or more reference genes selected from the genes ARPC2, ATF4, ATP5B, B2M, CDH4, CELF1, CLTA, CLTC, COPB1, CTBP1, CYC1, CYFIP1, DAZAP2, DHX15, DIMT1, EEF1A1, FLOT2, GADPH, GUSB, HADHA, HDLBP, HMBS, HNRNPC, HPRT1, HSP90AB1, MTCH1, MYL12B, NACA, NDUFB8, PGK1, PPIA, PPIB, PTBP1, RPL13A, RPLP0, RPS13, RPS23, RPS3, S100A6, SDHA, SEC31A, SET, SF3B1, SFRS3, SNRNP200, STARD7, SUMO1, TBP, TFRC, TMBIM6, TPT1, TRA2B, TUBA1C, UBB, UBC, UBE2D2, UBE2D3, VAMP3, XPO1, YTHDC1, YWHAZ, and 18S rRNA. The array comprises probes to all 17 genes identified in Table 1, and/or alternates; or all 8 genes identified in Table 2, and/or alternates. Conditions for incubating a gene detection reagent (or a kit/system that employs one or more such biomarker detection reagents) with a test sample vary. Incubation conditions depend on such factors as the format employed in the assay, the detection methods employed, and the type and nature of the detection reagents used in the assay. One skilled in the art will recognize that any one of the commonly available hybridization, amplification and array assay formats can readily be adapted to detect a gene set forth in Table 1 or Table 2.

A gene expression detection kit of the present disclosure may include components that are used to prepare nucleic acids from a test sample for the subsequent amplification and/or detection of a gene transcript.

In some aspects, a gene expression kit comprises one or more reagents, *e.g*., antibodies, for detecting protein products of a gene identified in Table 1 or Table 2 and optionally Table 3.

### Correlating Gene Expression Levels with Prognostic Outcomes

The present invention provides methods of determining the levels of a gene expression product to evaluate the likelihood that a LN-ER+HER2- breast cancer patient will have a relapse. Accordingly, the method provides a way of identifying LN⁻ER⁺HER2⁻ breast cancer patients that are candidates for additional treatment, *e.g*., chemotherapy.

FIG. 10 is a flowchart of a method for identifying LN⁻ER⁺HER2⁻ breast cancer patients that are candidates for additional treatment in one embodiment. Implementations of or processing in method 1000 depicted in FIG. 10 may be performed by software (e.g., instructions or code modules) when executed by a central processing unit (CPU or processor) of a logic machine, such as a computer system or information processing device, by hardware components of an electronic device or application-specific integrated circuits, or by combinations of software and hardware elements. Method 1000 depicted in FIG. 10 begins in step 1010.

In step 1020, information is received describing one or more levels of expression of one or more predetermined genes in a sample obtained from a subject. For example, the level of a gene expression product associated with a prognostic outcome for a LN⁻ER⁺HER2⁻breast cancer patient may be recorded. In one embodiment, input data includes a text file (e.g., a tab-delimited text file) of normalized expression values for 17 transcripts from primary genes (or an indicated alternative) from Table 1. In one embodiment, input data includes a text file (e.g., a tab-delimited text file) of normalized expression values for 8 transcripts from the primary genes (or an indicated alternative) from Table 2. For example, the text file may have the gene expression values for the 17 transcripts/genes as columns and patient(s) as rows. An illustrative patient data file (patient_data.txt) is presented in Appendix 1.

In step 1030, a random forest analysis is performed on the information describing the one or more levels of expression of the one or more predetermined genes in the sample obtained from the subject. A Random Forest (RF) algorithm is used to determine a Relapse Score (RS) when applied to independent patient data. A sample R program for running the RF algorithm is presented in Appendix 2. A Random Forest Relapse Score (RFRS) algorithm as used herein typically consists of a predetermined number of decision trees suitably adapted to ensure at least a fully deterministic model. Each node (branch) in each tree represents a binary decision based on transcript levels for transcripts described herein. Based on these decisions, the subject is assigned to a terminal leaf of each decision tree, representing a vote for either "relapse" or no "relapse". The fraction of votes for "relapse" to votes for "no relapse" represents the RFRS - a measure of the probability of relapse. In some embodiments, if a subject's RFRS is greater than or equal to 0.606, the subject is assigned to one or more "high risk" groups. If an RFRS is greater than or equal to 0.333 and less than 0.606, the subject is assigned to one or more "intermediate risk" group . If an RFRS is less than 0.333, the subject is assigned to one or more "low risk" groups. In further embodiments, a subject's RFRS value is also used to determine a likelihood of relapse by comparison to a loess fit of RFRS versus likelihood of relapse for a training dataset. A subject's estimated likelihood of relapse is determined, added to a summary plot, and output as a new report.

In step 1040, information indicative of either "relapse" or no "relapse" is generated based on the random forest analysis. In some embodiments, information indicative of either "relapse" or no "relapse" is generated to include one or more summary statistics. For example, information indicative of either "relapse" or no "relapse" may be representative of how assignments to a terminal leaf of each decision tree, representing a vote for either "relapse" or no "relapse", are made. In further embodiment, information indicative of either "relapse" or no "relapse" is generated for the fraction of votes for "relapse" to votes for "no relapse" as discussed above to represent the RFRS.

In step 1050, information indicative of one or more additional therapies is generated based on indicative of "relapse". For example, if an RFRS is greater than or equal to 0.606, the subject is assigned to a "high risk" group from which the one or more additional therapies may be selected. If an RFRS score is greater than or equal to 0.333 and less than 0.606, the subject is assigned to an "intermediate risk" group from which all or none of the one or more additional therapies may be selected. If an RFRS is less than 0.333, the subject is assigned to a "low risk" group. In various embodiments, a subject's RFRS value is also used to determine a likelihood of relapse by comparison to a loess fit of RFRS versus likelihood of relapse for a training dataset described in FIG. 11 and in the Examples section. FIG. 10 ends in step 1060.

FIG. 11 is a flowchart of a method for generating an RF model for identifying LN⁻ER⁺HER2⁻ breast cancer patients that are candidates for additional treatment in one embodiment. Implementations of or processing in method 1100 depicted in FIG. 11 may be performed by software (e.g., instructions or code modules) when executed by a central processing unit (CPU or processor) of a logic machine, such as a computer system or information processing device, by hardware components of an electronic device or application-specific integrated circuits, or by combinations of software and hardware elements. Method 1100 depicted in FIG. 11 begins in step 1110.

In step 1120, training data is received. For example, training data was generated as discussed below in the Examples section. In step 1130, variables on which to base decisions at tree nodes and classifier data are received. In one embodiment, classification was performed on training samples with either a relapse or no relapse after 10yr follow-up. In one example, a binary classification (e.g., relapse versus no relapse) is specified. However, additional classifier data may be included, such as a probability (proportion of "votes") for relapse which is termed the Random Forests Relapse Score (RFRS). Risk group thresholds can be determined from the distribution of relapse probabilities using mixed model clustering to set cutoffs for low, intermediate and high risk groups.

In step 1140, a random forest model is generated. For example, a random forest model may be generated with at least 100,001 trees (i.e., using an odd number to ensure a substantially fully deterministic model). FIG. 11 ends in step 1150.

### Hardware Description

The disclosure thus includes a computer system to implement the algorithm. Such a computer system can comprise code for interpreting the results of an expression analysis evaluating the level of expression of the 17 genes, or a designated alternate gene) identified in Table 1; or code for interpreting the results of an expression analysis evaluating the level of expression of the 8 genes, or a designated alternate gene, identified in Table 2. Thus in an exemplary embodiment, the expression analysis results are provided to a computer where a central processor executes a computer program for determining the propensity for relapse for a LN⁻ER⁺HER2⁻ breast cancer patient.

The disclosure also provides the use of a computer system, such as that described above, which comprises: (1) a computer; (2) a stored bit pattern encoding the expression results obtained by the methods of the invention, which may be stored in the computer; and, optionally, (3) a program for determining the likelihood of relapse.

The disclosure further provides methods of generating a report based on the detection of gene expression products for a LN⁻ER⁺HER2⁻ breast cancer patient. Such a report is based on the detection of gene expression products encoded by the 17 genes, or one of the designated alternates, set forth in Table 1; or detection of gene expression products encoded by the 8 genes, or one of the designated alternates, set forth in Table 2.

FIG. 12 is a block diagram of a computer system 1200 that may incorporate an embodiment, be incorporated into an embodiment, or be used to practice any of the innovations, embodiments, and/or examples found within this disclosure. FIG. 12 is merely illustrative of a computing device, general-purpose computer system programmed according to one or more disclosed techniques, or specific information processing device for an embodiment incorporating an invention whose teachings may be presented herein. One of ordinary skill in the art would recognize other variations, modifications, and alternatives.

Computer system 1200 can include hardware and/or software elements configured for performing logic operations and calculations, input/output operations, machine communications, or the like. Computer system 1200 may include familiar computer components, such as one or more one or more data processors or central processing units (CPUs) 1205, one or more graphics processors or graphical processing units (GPUs) 1210, memory subsystem 1215, storage subsystem 1220, one or more input/output (I/O) interfaces 1225, communications interface 1230, or the like. Computer system 1200 can include system bus 1235 interconnecting the above components and providing functionality, such connectivity and inter-device communication. Computer system 1200 may be embodied as a computing device, such as a personal computer (PC), a workstation, a mini-computer, a mainframe, a cluster or farm of computing devices, a laptop, a notebook, a netbook, a PDA, a smartphone, a consumer electronic device, a gaming console, or the like.

The one or more data processors or central processing units (CPUs) 1205 can include hardware and/or software elements configured for executing logic or program code or for providing application-specific functionality. Some examples of CPU(s) 1205 can include one or more microprocessors (e.g., single core and multi-core) or micro-controllers. CPUs 1205 may include 4-bit, 8-bit, 12-bit, 16-bit, 32-bit, 64-bit, or the like architectures with similar or divergent internal and external instruction and data designs. CPUs 1205 may further include a single core or multiple cores. Commercially available processors may include those provided by Intel of Santa Clara, California (e.g., x86, x86_64, PENTIUM, CELERON, CORE, CORE 2, CORE ix, ITANIUM, XEON, etc.) or by Advanced Micro Devices of Sunnyvale, California (e.g., x86, AMC_64, ATHLON, DURON, TURION, ATHLON XP/64, OPTERON, PHENOM, etc). Commercially available processors may further include those conforming to the Advanced RISC Machine (ARM) architecture (e.g., ARMv7-9), POWER and POWERPC architecture, CELL architecture, and or the like. CPU(s) 1205 may also include one or more field-gate programmable arrays (FPGAs), application-specific integrated circuits (ASICs), or other microcontrollers. The one or more data processors or central processing units (CPUs) 1205 may include any number of registers, logic units, arithmetic units, caches, memory interfaces, or the like. The one or more data processors or central processing units (CPUs) 1205 may further be integrated, irremovably or moveably, into one or more motherboards or daughter boards.

The one or more graphics processor or graphical processing units (GPUs) 1210 can include hardware and/or software elements configured for executing logic or program code associated with graphics or for providing graphics-specific functionality. GPUs 1210 may include any conventional graphics processing unit, such as those provided by conventional video cards. Some examples of GPUs are commercially available from NVIDIA, ATI, and other vendors. In various embodiments, GPUs 1210 may include one or more vector or parallel processing units. These GPUs may be user programmable, and include hardware elements for encoding/decoding specific types of data (e.g., video data) or for accelerating 2D or 3D drawing operations, texturing operations, shading operations, or the like. The one or more graphics processors or graphical processing units (GPUs) 1210 may include any number of registers, logic units, arithmetic units, caches, memory interfaces, or the like. The one or more data processors or central processing units (CPUs) 1205 may further be integrated, irremovably or moveably, into one or more motherboards or daughter boards that include dedicated video memories, frame buffers, or the like.

Memory subsystem 1215 can include hardware and/or software elements configured for storing information. Memory subsystem 1215 may store information using machine-readable articles, information storage devices, or computer-readable storage media. Some examples of these articles used by memory subsystem 1270 can include random access memories (RAM), read-only-memories (ROMS), volatile memories, non-volatile memories, and other semiconductor memories. In various embodiments, memory subsystem 1215 can include data and program code 1240.

Storage subsystem 1220 can include hardware and/or software elements configured for storing information. Storage subsystem 1220 may store information using machine-readable articles, information storage devices, or computer-readable storage media. Storage subsystem 1220 may store information using storage media 1245. Some examples of storage media 1245 used by storage subsystem 1220 can include floppy disks, hard disks, optical storage media such as CD-ROMS, DVDs and bar codes, removable storage devices, networked storage devices, or the like. In some embodiments, all or part of breast cancer prognosis data and program code 1240 may be stored using storage subsystem 1220.

In various embodiments, computer system 1200 may include one or more hypervisors or operating systems, such as WINDOWS, WINDOWS NT, WINDOWS XP, VISTA, WINDOWS 7 or the like from Microsoft of Redmond, Washington, Mac OS or Mac OS X from Apple Inc. of Cupertino, California, SOLARIS from Sun Microsystems, LINUX, UNIX, and other UNIX-based or UNIX-like operating systems. Computer system 1200 may also include one or more applications configured to execute, perform, or otherwise implement techniques disclosed herein. These applications may be embodied as breast cancer prognosis data and program code 1240. Additionally, computer programs, executable computer code, human-readable source code, shader code, rendering engines, or the like, and data, such as image files, models including geometrical descriptions of objects, ordered geometric descriptions of objects, procedural descriptions of models, scene descriptor files, or the like, may be stored in memory subsystem 1215 and/or storage subsystem 1220.

The one or more input/output (I/O) interfaces 1225 can include hardware and/or software elements configured for performing I/O operations. One or more input devices 1250 and/or one or more output devices 1255 may be communicatively coupled to the one or more I/O interfaces 1225.

The one or more input devices 1250 can include hardware and/or software elements configured for receiving information from one or more sources for computer system 1200. Some examples of the one or more input devices 1250 may include a computer mouse, a trackball, a track pad, a joystick, a wireless remote, a drawing tablet, a voice command system, an eye tracking system, external storage systems, a monitor appropriately configured as a touch screen, a communications interface appropriately configured as a transceiver, or the like. In various embodiments, the one or more input devices 1250 may allow a user of computer system 1200 to interact with one or more non-graphical or graphical user interfaces to enter a comment, select objects, icons, text, user interface widgets, or other user interface elements that appear on a monitor/display device via a command, a click of a button, or the like.

The one or more output devices 1255 can include hardware and/or software elements configured for outputting information to one or more destinations for computer system 1200. Some examples of the one or more output devices 1255 can include a printer, a fax, a feedback device for a mouse or joystick, external storage systems, a monitor or other display device, a communications interface appropriately configured as a transceiver, or the like. The one or more output devices 1255 may allow a user of computer system 1200 to view objects, icons, text, user interface widgets, or other user interface elements.

A display device or monitor may be used with computer system 1200 and can include hardware and/or software elements configured for displaying information. Some examples include familiar display devices, such as a television monitor, a cathode ray tube (CRT), a liquid crystal display (LCD), or the like.

Communications interface 1230 can include hardware and/or software elements configured for performing communications operations, including sending and receiving data. Some examples of communications interface 1230 may include a network communications interface, an external bus interface, an Ethernet card, a modem (telephone, satellite, cable, ISDN), (asynchronous) digital subscriber line (DSL) unit, FireWire interface, USB interface, or the like. For example, communications interface 1230 may be coupled to communications network/external bus 1280, such as a computer network, to a FireWire bus, a USB hub, or the like. In other embodiments, communications interface 1230 may be physically integrated as hardware on a motherboard or daughter board of computer system 1200, may be implemented as a software program, or the like, or may be implemented as a combination thereof.

In various embodiments, computer system 1200 may include software that enables communications over a network, such as a local area network or the Internet, using one or more communications protocols, such as the HTTP, TCP/IP, RTP/RTSP protocols, or the like. In some embodiments, other communications software and/or transfer protocols may also be used, for example IPX, UDP or the like, for communicating with hosts over the network or with a device directly connected to computer system 1200.

As suggested, FIG. 12 is merely representative of a general-purpose computer system appropriately configured or specific data processing device capable of implementing or incorporating various embodiments of an invention presented within this disclosure. Many other hardware and/or software configurations may be apparent to the skilled artisan which are suitable for use in implementing an invention presented within this disclosure or with various embodiments of an invention presented within this disclosure. For example, a computer system or data processing device may include desktop, portable, rack-mounted, or tablet configurations. Additionally, a computer system or information processing device may include a series of networked computers or clusters/grids of parallel processing devices. In still other embodiments, a computer system or information processing device may perform techniques described above as implemented upon a chip or an auxiliary processing board.

Many hardware and/or software configurations of a computer system may be apparent to the skilled artisan, which are suitable for use in implementing a RFRS algorithm as desribed herein. For example, a computer system or data processing device may include desktop, portable, rack-mounted, or tablet configurations. Additionally, a computer system or information processing device may include a series of networked computers or clusters/grids of parallel processing devices. In still other embodiments, a computer system or information processing device may use techniques described above as implemented upon a chip or an auxiliary processing board.

Various embodiments of an algorithm as described herein can be implemented in the form of logic in software, firmware, hardware, or a combination thereof. The logic may be stored in or on a machine-accessible memory, a machine-readable article, a tangible computer-readable medium, a computer-readable storage medium, or other computer/machine-readable media as a set of instructions adapted to direct a central processing unit (CPU or processor) of a logic machine to perform a set of steps that may be disclosed in various embodiments of an invention presented within this disclosure. The logic may form part of a software program or computer program product as code modules become operational with a processor of a computer system or an information-processing device when executed to perform a method or process in various embodiments of an invention presented within this disclosure. Based on this disclosure and the teachings provided herein, a person of ordinary skill in the art will appreciate other ways, variations, modifications, alternatives, and/or methods for implementing in software, firmware, hardware, or combinations thereof any of the disclosed operations or functionalities of various embodiments of one or more of the presented inventions.

### EXAMPLES

The experiments outlined in the initial examples that identified markers for prognosis stratified node-negative, ER-positive, HER2-negative breast cancer patients into those that are most or least likely to develop a recurrence within 10 years after surgery. A multi-gene transcription-level-based classifier of 10-year-relapse (disease recurrence within 10 years) was developed using a large database of existing, publicly available microarray datasets. The probability of relapse and relapse risk score group using the panel of gene expression markers of the invention can be used to assign systemic chemotherapy to only those patients most likely to benefit from it.

### Methods:

**Literature search and curation:** Studies were collected which provided gene expression data for ER+, LN-, HER2- patients with no systemic chemotherapy (hormonal-therapy allowed). Each study was required to have a sample size of at least 100, report LN status, and include time and events for either recurrence free survival (RFS) or distant metastasis free survival (DMFS). The latter were grouped together for survival analysis where all events represent either a local or distant relapse. If ER or HER2 status was not reported, it was determined by array, but preference was given to studies with clinical determination first. A minimum of 10 years follow up was required for training the classifier. However, patients with shorter follow-up were included in survival analyses. Patients with immediately postoperative events (time = 0) were excluded. Nine studies¹⁻⁹ meeting the above criteria were identified by searching Pubmed and the Gene Expression Omnibus (GEO) database¹⁰. To allow combination of the largest number of samples, only the common Affymetrix U133A gene expression platform was used. 2175 breast cancer samples were identified. After filtering for only those samples which were ER+, node-negative, and had not received systemic chemotherapy, 1403 samples remained. Duplicate analysis removed a further 405 samples due to the significant amount of redundancy between studies (Figure 1). Filtering for ER+ and HER2- status using array determinations eliminated another 140 samples (Figure 2). Some ER- samples were from the Schmidt et al. Cancer Res 68, 5405-5413 (2008)⁵ dataset (31/201) which did not provide clinical ER status and thus for that study we relied solely on arrays for determination of ER status. However, there were also a small number (37/760) from the remaining studies, which represent discrepancies between array status and clinical determination. In such cases, both the clinical and array-based determinations were required to be positive for inclusion in further analysis. A total of 858 samples passed all filtering steps including 487 samples with 10 year follow-up data (213 relapse; 274 no relapse). The remaining 371 samples had insufficient follow-up for 10-year classification analysis, but were retained for use in the survival analysis. None of the 858 samples were treated with systemic chemotherapy but 302 (35.2%) were treated with adjuvant hormonal therapy of which 95.4% were listed as tamoxifen. The 858 samples were broken into two-thirds training and one-third testing sets resulting in: (A) a training set of 572 samples for use in survival analysis and 325 samples with 10yr follow-up (143 relapse; 182 no relapse) for classification analysis; and (B) a testing set of 286 samples for use in survival analysis and 162 samples with 10 year follow-up (70 relapse; 92 no relapse) for classification analysis. Table 6 outlines the datasets used in the analysis and Figure 3 illustrates the breakdown of samples for analysis.

**Pre-processing:** All data processing and analyses were completed with open source R/Bioconductor packages. Raw data (Cel files) were downloaded from GEO. Duplicate samples were identified and removed if they had the same database identifier (e.g., GSM accession), same sample/patient id, or showed a high correlation (r > 0.99) compared to any other sample in the dataset. Raw data were normalized and summarized using, the 'affy' and 'gcrma' libraries. Probes were mapped to Entrez gene symbols using both standard and custom annotation files¹¹. ER and HER2 expression status was determined using standard probes. For the Affymetrix U133A array we and others have found the probe "205225_at" to be most effective for determining ER status¹². Similarly a rank sum of the best probes for ERBB2 (216835_s_at), GRB7 (210761_s_at), STARD3 (202991_at) and PGAP3 (55616_at) was used to determine HER2 amplicon status. Cutoff values for ER and HER2 status were chosen by mixed model clustering ('mclust' library). Unsupervised clustering was performed to assess the extent of batch effects. Once all pre-filtering was complete, data were randomly split into training (2/3) and test (1/3) data sets while balancing for study of origin and number of relapses with 10 year follow-up. The test data set was put aside, left untouched, and only used for final validation, once each for the full-gene, 17-gene and 8-gene classifiers. Probes sets were then filtered for a minimum of 20% samples with expression above background threshold (raw value > 100) and coefficient of variation between 0.7 and 10. A total of 3048 probesets/genes passed this filtering and formed the basis for the 'full-gene set' model described below.

**Classification:** Classification was performed on only training samples with either a relapse or no relapse after 10yr follow-up using the 'randomForest' library. Forests were created with at least 100,001 trees (odd number ensures fully deterministic model) and otherwise default settings. Performance was assessed by area under the curve (AUC) of a receiver operating characteristic (ROC) curve, calculated with the 'ROCR' package, from Random Forests internal out-of-bag (OOB) testing results. By default, RF performs a binary classification (e.g., relapse versus no relapse). However it also reports a probability (proportion of "votes") for relapse which we term Random Forests Relapse Score (RFRS). Risk group thresholds were determined from the distribution of relapse probabilities using mixed model clustering to set cutoffs for low, intermediate and high risk groups (Figure 4).

**Determination of optimal 17-gene and 8-gene sets:** Initially an optimal set of 20 genes was selected by removing redundant probe sets and extracting the top 100 genes (by reported Gini variable importance), k-means clustering (k=20) these genes and selecting the best gene from each cluster (again by variable importance). Additional genes in each cluster serve as robust alternates in case of failure to migrate primary genes to an assay platform. A gene might fail to migrate due to problems with prober/primer design or differences in the sensitivity of a specific assay for that gene. The top 100 genes/probesets were also manually checked for sequence correctness by alignment to the reference genome. Seven genes/probesets with ambiguous or erroneous alignments were marked for exclusion. Three genes/probesets were also excluded because of their status as hypothetical proteins (KIAA0101, KIAA0776, KIAA1467). After these removals, a set of 17 primary genes and 73 alternate genes remained. All but two primary genes have two or more alternates (TXNIP is without alternate, and APOC1 has a single alternate). Table 1 lists the final gene set, their top two alternate genes (where available) and their variable importance values (See Table 4 for complete list). The above procedure was repeated to produce an optimal set of 8 genes, this time starting from the top 90 non-redundant probe-sets (excluding the 10 genes with problems identified above), k-means clustering (k=8) these genes and selecting the best gene from each cluster. All 8 genes were also included in the 17-gene set and have at least two alternates (Table 2, Table 5). Using the final optimized 17-gene and 8-gene sets as input, new RF models were built on training data.

Validation (testing and survival analysis): Survival analysis on all training data, now also including those patients with less than 10 years of follow-up, was performed with risk group as a factor, for the full-gene, 17-gene, and 8-gene models, using the 'survival' package. Note, the risk scores and groups for samples used in training were assigned from internal OOB cross-validation. Only those patients not used in initial training (without 10 year follow-up) were assigned a risk score and group by de novo classification. Significance between risk groups was determined by Kaplan-Meier logrank test (with test for linear trend). However, to directly compare relapse rates per risk group to that reported by Paik et al., N Engl J Med 351: 2817-2826 (2004)¹³, the overall relapse rates in our patient cohort were randomly down-sampled to the same rate (15%) as in their cohort¹³ and results averaged over 1000 iterations. To illustrate, the training data set includes 572 samples with 143 relapse events (I.e., 25.0% relapse rate). Samples with relapse events were randomly eliminated from the cohort until only 15% of remaining samples had relapse events (76/505 = 15%). This "down-sampled" dataset was then classified using the RFRS model to assign each sample to a risk group and the rates of relapse determined for each group. The entire down-sampling procedure was then repeated 1000 times to obtain average estimated rates of relapse for each risk group given the overall rate of relapse of 15%. Setting the overall relapse rate to 15% is also useful because this more closely mirrors the general population rate of relapse. Without this down-sampling, expected relapse rates in each risk group would appear unrealistically high. See Figure 2 for explanation of the break-down of samples into training and test sets used for classifier building and survival analysis.

Next, the full-gene, 17-gene and 8-gene RF models along with risk group cutoffs were applied to the independent test data. The same performance metrics, survival analysis and estimates of 10 year relapse rates were performed as above. The 17-gene model was also tested on the independent test data, stratified by treatment (untreated vs hormone therapy treated), to evaluate whether performance of the signature was biased towards one patient subpopulation or the other. These independent test data were not used in any way during the training phase. However, these samples represent a random subset of the same patient populations that were used in training. Therefore, they are not as fully independent as recommended by the Institute of Medicine (IOM) 'committee on the review of omics-based tests for predicting patient outcomes in clinical trials'¹⁸. Therefore, an additional independent validation was performed against the NKI dataset¹⁹ obtained from the http address bioinformatics.nki.nl/data.php. These data represent a set of 295 consecutive patients with primary stage I or II breast carcinomas. The dataset was filtered down to the 89 patients who were node-negative, ER-positive, HER2-negative and not treated by systemic chemotherapy¹⁹. Relapse times and events were defined by any of distant metastasis, regional recurrence or local recurrence. Expression values from the NKI Agilent array data were re-scaled to the same distribution as that used in training using the 'preprocessCore' package. Values for the 8-gene and 17-gene-set RFRS models were extracted for further analysis. If more than one Agilent probe set could be mapped to an RFRS gene then the probe set with greatest variance was used. The full-gene-set model was not applied to NKI data because only 2530/3048 Affymetrix-defined genes (probe sets) in the full-gene-set could be mapped to Agilent genes (probe sets) in the NKI dataset. However, the 17-gene and 8-gene RFRS models were applied to NKI data to calculate predicted probabilities of relapse. Patients were divided into low, intermediate, and high risk groups by ranking according to probability of relapse and then dividing so that the proportions in each risk group were identical to that observed in training. ROC AUC, survival p-values and estimated rates of relapse were then calculated as above. It should be noted that while the NKI clinical data described here (N=89) had an average follow-up time of 9.55 years (excluding relapse events), 34 patients had a follow-up time less than 10 years (range 1.78-9.83 years). These patients would not have met our criteria for inclusion in the training dataset and likely represent some events which have not occurred yet. If anything, this is likely to reduce the AUC estimate and underestimate p-value significance in survival analysis.

**Selection of control genes:** While not necessary for Affymetrix, migration to other assay technologies (e.g., RT-PCR approaches) may employ highly expressed and invariant genes to act as a reference for determining accurate gene expression level estimates. To this end, we developed two sets of reference genes. The first was chosen by the following criteria: (1) filtered if not expressed above background threshold (raw value > 100) in 99% of samples; (2) filtered if not in top 5th percentile (overall) for mean expression; (3) Filtered if not in top 10th percentile (remaining genes) for standard deviation; (4) ranked by coefficient of variation. The top 30 control genes from set #1 are listed in Table 3. Control genes underwent the same manual checks for sequence correctness by alignment to the reference genome as above and five genes were marked for exclusion. The second set of control genes were chosen to represent three ranges of mean expression levels encompassed by genes in the 17-gene signature (low: 0-400; medium: 500-900; high: 1200-1600). For each mean expression range, genes were (1) filtered if not expressed above background threshold (raw value > 100) in 99% of samples; (2) ranked by coefficient of variation. The top 5 genes from each range in set #2 are listed in Table 3 along with previously reported reference genes (Paik *et al*., *supra*)¹³

### Results:

Internal OOB cross-validation for the initial (full-gene-set) model on training data reported an ROC AUC of 0.704. This was comparable or better than reported by Johannes et al (2010) who tested a number of different classifiers on a smaller subset of the same data and found AUCs of 0.559 to 0.671¹⁴. It also compares favorably to the AUC value of 0.688 when the OncotypeDX algorithm was applied to this same training dataset. Mixed model clustering analysis identified three risk groups with probabilities for low risk < 0.333; 0.333 ≤ intermediate risk < 0.606; and high risk ≥ 0.606 (Figure 4). Survival analysis determined a highly significant difference in relapse rate between risk groups (p = 3.95E-11) (Figure 5A). After down-sampling to a 15% overall rate of relapse, approximately 46.7% (n=235) of patients were placed in the low-risk group and were found to have a 10yr risk of relapse of only 8.0%. Similarly, 38.6% (n=195) and 14.9% (n=75) of patients were placed in the intermediate and high risk groups with rates of relapse of 17.6% and 30.3% respectively. These results are very similar to those for which Paik *et al., supra* reported as 51% of patients in the low-risk category with a rate of distant recurrence at 10 years of 6.8% (95% CI: 4.0-9.6); 22% in intermediate-risk category with recurrence rate of 14.3% (95% CI: 8.3-20.3); and 27% in high-risk category with recurrence rate of 30.5% (95%CI: 23.6-37.4)¹³. The linear relationship between risk group and rate of relapse continues if groups are broken down further. For example, if "very low-risk" and "very high-risk" groups are defined these have even lower (7.1%) and higher (32.8%) rates of relapse (Figure 6). This observation is consistent with the idea that the random forests relapse score (RFRS) is a quantitative, linear measure directly related to probability of relapse. Figure 7 shows the likelihood of relapse at 10 years, calculated for 50 RFRS intervals (from 0 to 1), with a smooth curve fitted, using a loess function and 95% confidence intervals representing error in the fit. The distribution of RFRS values observed in the training data is represented by short vertical marks just above the x axis, one for each patient.

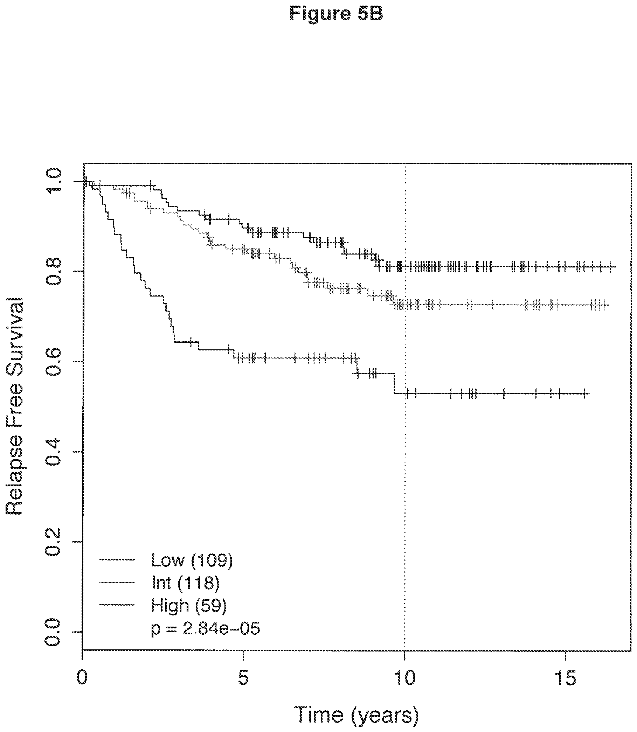

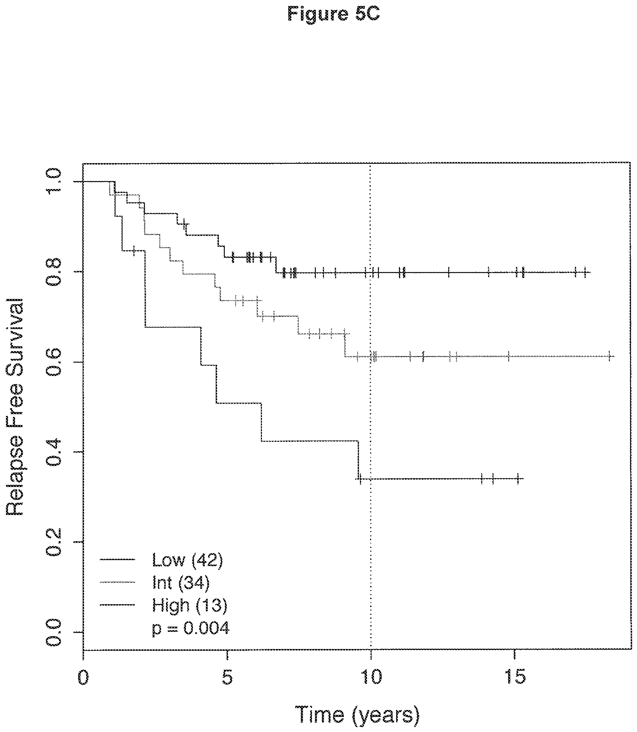

Validation of the models against the independent test dataset also showed very similar results to training estimates. The full-gene-set model had an AUC of 0.730 and the 17-gene and 8-gene optimized models had minimal reduction in performance with AUC of 0.715 and 0.690 respectively. Again, this compared favorably to the AUC value of 0.712 when the OncotypeDX algorithm was applied to the same test dataset. Survival analysis again found very significant differences between the risk groups for the full-gene (p = 6.54E-06), 17-gene (p = 9.57E-06) and 8-gene (p = 2.84E-05; Figure 5B) models. For the 17-gene model, approximately 38.2% (n=97) of patients were placed in the low-risk group and were found to have a 10-year risk of relapse of only 7.8%. Similarly, 40.5% (n=103) and 21.3% (n=54) of patients were placed in the intermediate and high-risk groups with rates of relapse of 15.3% and 26.8% respectively. Very similar results were observed for the full-gene and 8-gene models (Table 7). Validation against the additional, independent, NKI dataset also had very similar results. The 17-gene and 8-gene models had AUC values of 0.688 and 0.699 respectively, nearly identical to the results for the previous independent dataset. Differences between risk groups in survival analysis were also significant for both 17-gene (p = 0.023) and 8-gene (p = 0.004, Figure 5C) models.

The linear relationship between risk group and rate of relapse continues if groups are broken down further (using training data) into five equal groups instead of the three groups defined above (Figure 6). This observation is consistent with the idea that the random forests relapse score (RFRS) is a quantitative, linear measure directly related to probability of relapse. Figure 7 shows the likelihood of relapse at 10 years, calculated for 50 RFRS intervals (from 0 to 1), with a smooth curve fitted, using a loess function and 95% confidence intervals representing error in the fit. The distribution of RFRS values observed in the training data is represented by short vertical marks just above the x axis, one for each patient.

In order to maximize the total size of our training dataset we allowed samples to be included from both untreated patients and those who received adjuvant hormonal therapy such as tamoxifen. Since outcomes likely differ between these two groups, and they may represent fundamentally different subpopulations, it is possible that performance of our predictive signatures is biased towards one group or the other. To assess this issue we performed validation against the independent test dataset, stratified by treatment status, using the 17-gene model. Both groups were found to have comparable AUC values with the slightly better value of 0.740 for hormone-treated versus 0.709 for untreated. Survival curves were also highly similar and significant with p-value of 0.004 and 3.76E-07 for treated and untreated respectively (Figure 13A and 13B). The difference in p-value appears more likely due to differences in the respective sample sizes than actual difference in survival curves.

The genes utilized in the RFRS model have only minimal overlap with those identified in other breast cancer outcome signatures. Specifically, the entire set of 100 genes (full-gene set before filtering) has only 6/65 genes in common with the gene expression panel proposed by van de Vijver, et al. N Engl J Med 347, 1999-2009 (2002)¹⁵, 2/21 with that proposed by Paik *et al., supra,* and and 4/77 with that proposed by Wang et al. Lancet 365:671-679 (2005)²⁰. The 17-gene and 8-gene optimized sets have only a single gene (AURKA) in common with the panel proposed by Paik *et al*., a single gene (FEN1) in common with Wang *et al.,* and none with that of van de Vijver *et al.* A Gene Ontology analysis using DAVID^{16,17} revealed that genes in the 17-gene list are involved in a wide range of biological processes known to be involved in breast cancer biology including cell cycle, hormone response, cell death, DNA repair, transcription regulation, wound healing and others (Figure 8). Since the 8-gene set is entirely contained in the 17-gene set it would be involved in many of the same processes.

While methods such as those proposed by Paik *et al*., and de Vijver, *et al.* (both *supra*)^{13, 15} exist to predict outcome in breast cancer, the RFRS is advantageous in several respects: (1) The signature was built from the largest and purest training dataset available to date; (2) Patients with HER2+ tumors were excluded, thus focusing only on patients without an existing clear treatment course; (3) The gene signature predicts relapse with equal success for both patients that went on to receive adjuvant hormonal therapy and those who did not (4) The gene signature was designed for robustness with (in most cases) several alternate genes available for each primary gene; (5) probe set sequences have been manually validated by alignment and manual assessment. These features, particularly the latter two, make this signature an especially strong candidate for efficient migration to multiple low-cost platforms for use in a clinical setting. Development of a panel for use in the clinic could take advantage of not only primary genes but also some number of alternate genes to increase the chance of a successful migration. Given the small but significant number of discrepencies observed between clinical and array based determination of ER status we also recommend inclusion of standard biomarkers such as ER, PR and HER2 on any design. Finally, we provide a list of consistently expressed genes, specific to breast tumor tissue, for use as control genes for those platforms that require them.

### Implementation of algorithm using 17-gene model as example:

The RFRS algorithm is implemented in the R programming language and can be applied to independent patient data. Input data is a tab-delimited text file of normalized expression values with 17 transcripts/genes as columns and patient(s) as rows. A sample patient data file (patient_data.txt) is presented in Appendix 1. A sample R program (RFRS_sample_code.R) for running the algorithm is presented in Appendix 2. The RFRS algorithm consists of a Random Forest of 100,001 decision trees. This is pre-computed, provided as an R data object (RF_model_17gene_optimized) based on the training set and is included in the working directory. Each node (branch) in each tree represents a binary decision based on transcript levels for transcripts described above. Based on these decisions, the patient is assigned to a terminal leaf of each decision tree, representing a vote for either "relapse" or no "relapse". The fraction of votes for "relapse" to votes for "no relapse" represents the RFRS - a measure of the probability of relapse. If RFRS is greater than or equal to 0.606 the patient is assigned to the "high risk" group, if greater than or equal to 0.333 and less than 0.606 the patient is assigned to "intermediate risk" group and if less than 0.333 the patient is assigned to "low risk" group. The patient's RFRS value is also used to determine a likelihood of relapse by comparison to a loess fit of RFRS versus likelihood of relapse for the training dataset. Pre-computed R data objects for the loess fit (RelapseProbabilityFit.Rdata) and summary plot (RelapseProbabilityPlot.Rdata) are loaded from file. The patient's estimated likelihood of relapse is determined, added to the summary plot, and output as a new report (see, Figure 9, for example).

### References cited in Examples section:

1 Desmedt, C. et al. Strong time dependence of the 76-gene prognostic signature for node-negative breast cancer patients in the TRANSBIG multicenter independent validation series. Clin Cancer Res 13, 3207-3214 (2007).
2 Ivshina, A. V. et al. Genetic reclassification of histologic grade delineates new clinical subtypes of breast cancer. Cancer Res 66, 10292-10301 (2006).
3 Loi, S. et al. Definition of clinically distinct molecular subtypes in estrogen receptor-positive breast carcinomas through genomic grade. J Clin Oncol 25, 1239-1246 (2007).
4 Miller, L. D. et al. An expression signature for p53 status in human breast cancer predicts mutation status, transcriptional effects, and patient survival. Proc Natl Acad Sci USA 102, 13550-13555 (2005).
5 Schmidt, M. et al. The humoral immune system has a key prognostic impact in node-negative breast cancer. Cancer Res 68, 5405-5413 (2008).
6 Sotiriou, C. et al. Gene expression profiling in breast cancer: understanding the molecular basis of histologic grade to improve prognosis. J Natl Cancer Inst 98, 262-272 (2006).
7 Symmans, W. F. et al. Genomic index of sensitivity to endocrine therapy for breast cancer. J Clin Oncol 28, 4111-4119 (2010).
8 Wang, Y. et al. Gene-expression profiles to predict distant metastasis of lymph-node-negative primary breast cancer. Lancet 365, 671-679 (2005).
9 Zhang, Y. et al. The 76-gene signature defines high-risk patients that benefit from adjuvant tamoxifen therapy. Breast Cancer Res Treat 116, 303-309 (2009).
10 Barrett, T. et al. NCBI GEO: archive for functional genomics data sets--10 years on. Nucleic Acids Res 39 (2011).
11 Dai, M. et al. Evolving gene/transcript definitions significantly alter the interpretation of GeneChip data. Nucleic Acids Res 33, e175, (2005).
12 Gong, Y. et al. Determination of oestrogen-receptor status and ERBB2 status of breast carcinoma: a gene-expression profiling study. Lancet Oncol 8, 203-211 (2007).
13 Paik, S. et al. A multigene assay to predict recurrence of tamoxifen-treated, node-negative breast cancer. N Engl J Med 351, 2817-2826 (2004).
14 Johannes, M. et al. Integration of pathway knowledge into a reweighted recursive feature elimination approach for risk stratification of cancer patients. Bioinformatics 26, 2136-2144 (2010).
15 van de Vijver, M. J. et al. A gene-expression signature as a predictor of survival in breast cancer. N Engl J Med 347, 1999-2009 (2002).
16 Huang da, W., Sherman, B. T. & Lempicki, R. A. Systematic and integrative analysis of large gene lists using DAVID bioinformatics resources. Nature protocols 4, 44-57 (2009).
17 Huang da, W., Sherman, B. T. & Lempicki, R. A. Bioinformatics enrichment tools: paths toward the comprehensive functional analysis of large gene lists. Nucleic Acids Res 37, 1-13 (2009).
18. Committee on the Review of Omics-Based Tests for Predicting Patient Outcomes in Clinical Trials, Board on Health Care Services, Board on Health Sciences Policy, Institute of Medicine. Evolution of Translational Omics: Lessons Learned and the Path Forward. Christine MM, Sharly JN, Gilbert SO, editors: The National Academies Press; 2012.
19. van de Vijver MJ, He YD, van't Veer LJ, Dai H, Hart AA, Voskuil DW, et al. A gene-expression signature as a predictor of survival in breast cancer. N Engl J Med 2002;347: 1999-2009.
20. Wang, Y. et al. Gene-expression profiles to predict distant metastasis of lymph-node-negative primary breast cancer. Lancet 365, 671-679 (2005).

**Table 1. 17-gene RFRS signature**

| **Primary Predictor** | | **Alternate 1** | | **Alternate 2** | |
|---|---|---|---|---|---|
| CCNB2 | 0.785 | MELK | 0.739 | GINS1 | 0.476 |
| TOP2A | 0.590 | MCM2 | 0.428 | CDK1 | 0.379 |
| RACGAP1 | 0.588 | LSM1 | 0.139 | SCD | 0.125 |
| CKS2 | 0.515 | NUSAP1 | 0.491 | ZWINT | 0.272 |
| AURKA | 0.508 | PRC1 | 0.499 | CENPF | 0.306 |
| FEN1 | 0.403 | FADD | 0.313 | SMC4 | 0.170 |
| EBP | 0.341 | RFC4 | 0.264 | NCAPG | 0.234 |
| TXNIP | 0.292 | N/A | N/A | N/A | N/A |
| SYNE2 | 0.270 | SCARB2 | 0.225 | PDLIM5 | 0.167 |
| DICER1 | 0.209 | CALD1 | 0.129 | SOX9 | 0.125 |
| AP1AR | 0.201 | PBX2 | 0.134 | WASL | 0.126 |
| NUP107 | 0.197 | FAM38A | 0.165 | PLIN2 | 0.110 |
| APOC1 | 0.176 | APOE | 0.121 | N/A | N/A |
| DTX4 | 0.164 | AQP1 | 0.141 | LMO4 | 0.120 |
| FMOD | 0.154 | RGS5 | 0.120 | PIK3R1 | 0.103 |
| MAPKAPK2 | 0.151 | MTUS1 | 0.136 | DHX9 | 0.136 |
| SUPT4H1 | 0.111 | PHB | 0.106 | CD44 | 0.105 |

**Table 2. 8-gene RFRS signature**

| **Primary Predictor** | | **Alternate 1** | | **Alternate 2** | |
|---|---|---|---|---|---|
| CCNB2 | 0.785 | MELK | 0.739 | TOP2A | 0.590 |
| RACGAP1 | 0.588 | TXNIP | 0.292 | APOC1 | 0.176 |
| CKS2 | 0.515 | NUSAP1 | 0.491 | FEN1 | 0.403 |
| AURKA | 0.508 | PRC1 | 0.499 | CENPF | 0.306 |
| EBP | 0.341 | FADD | 0.313 | RFC4 | 0.264 |
| SYNE2 | 0.270 | SCARB2 | 0.225 | PDLIM5 | 0.167 |
| DICER1 | 0.209 | FAM38A | 0.165 | FMOD | 0.154 |
| AP1AR | 0.201 | MAPKAPK2 | 0.151 | MTUS1 | 0.136 |

**Table 3.**

| **Top 25 RFRS Reference Genes** | | | | | | |
|---|---|---|---|---|---|---|
| **Probe set** | **Gene Symbol** | **Mean (exp)** | **S.D.** | **Fraction (exp)** | **COV** | **CDF** |
| 103910_at | MYL12B | 1017.5 | 195.8 | 1.00 | 0.192 | custom |
| 208672_s_at | SFRS3 | 1713.0 | 380.0 | 1.00 | 0.222 | standard |
| 200960_x_at | CLTA | 1786.2 | 397.5 | 1.00 | 0.223 | standard |
| 200893_at | TRA2B | 1403.7 | 312.8 | 1.00 | 0.223 | standard |
| 23787_at | MTCH1 | 1120.0 | 269.8 | 1.00 | 0.241 | custom |
| 221767_x_at | HDLBP | 1174.4 | 284.9 | 1.00 | 0.243 | standard |
| 23191_at | CYFIP1 | 1345.1 | 329.4 | 1.00 | 0.245 | custom |
| 211069_s_at | SUMO1 | 1111.6 | 276.2 | 1.00 | 0.248 | standard |
| 201385_at | DHX15 | 1529.4 | 383.5 | 1.00 | 0.251 | standard |
| 200014_s_at | HNRNPC | 1517.7 | 385.3 | 1.00 | 0.254 | standard |
| 200667_at | UBE2D3 | 1090.1 | 279.3 | 1.00 | 0.256 | standard |
| 9802_at | DAZAP2 | 1181.2 | 303.6 | 1.00 | 0.257 | custom |
| 200058_s_at | SNRNP200 | 1104.4 | 285.9 | 1.00 | 0.259 | standard |
| 91746_at | YTHDC1 | 965.1 | 250.7 | 1.00 | 0.260 | custom |
| 1315_at | COPB1 | 1118.2 | 291.9 | 1.00 | 0.261 | custom |
| 4714_at | NDUFB8 | 1219.0 | 325.5 | 1.00 | 0.267 | custom |
| 40189_at | SET | 1347.9 | 360.7 | 1.00 | 0.268 | standard |
| 221743_at | CELF1 | 1094.0 | 294.2 | 1.00 | 0.269 | standard |
| 208775_at | XFO1 | 940.7 | 256.1 | 1.00 | 0.272 | standard |
| 211270_x_at | PTBP1 | 973.1 | 266.8 | 1.00 | 0.274 | standard |
| 211185_s_at | SF3B1 | 1077.9 | 297.9 | 1.00 | 0.276 | standard |
| 10109_at | ARPC2 | 1357.4 | 375.9 | 1.00 | 0.277 | custom |
| 201336_at | VAMP3 | 959.2 | 267.4 | 1.00 | 0.279 | standard |
| 200028_s_at | STARD7 | 1087.9 | 303.4 | 1.00 | 0.279 | standard |
| 22872_at | SEC31A | 1040.4 | 290.5 | 1.00 | 0.279 | custom |

| **Top 15 RFRS Reference Genes (Set #2)** | | | | | | |
|---|---|---|---|---|---|---|
| 9927_at | MFN2 | 207.0 | 33.1 | 1.00 | 0.160 | custom |
| 26100_at | WIPI2 | 216.5 | 40.3 | 1.00 | 0.186 | custom |
| 201507_at | PFDN1 | 260.8 | 51.2 | 1.00 | 0.196 | standard |
| 7337_at | UBE3A | 225.3 | 46.5 | 0.99 | 0.207 | custom |
| 2976_at | GTF3C2 | 226.3 | 47.6 | 1.00 | 0.210 | custom |
| 10657_at | KHDRBS1 | 776.4 | 166.3 | 1.00 | 0.214 | custom |
| 201330_at | RARS | 502.6 | 117.1 | 1.00 | 0.233 | standard |
| 201319_at | MYL12A | 574.4 | 135.2 | 1.00 | 0.235 | standard |
| 3184_at | HNRNPD | 678.8 | 160.0 | 1.00 | 0.236 | custom |
| 10236_at | HNRNPR | 570.1 | 140.4 | 1.00 | 0.246 | custom |
| 200893_at | TRA2B | 1403.7 | 312.8 | 1.00 | 0.223 | standard |
| 221619_s_at | MTCH1* | 1401.9 | 342.6 | 1.00 | 0.244 | standard |
| 208923_at | CYFIP1* | 1339.2 | 333.6 | 1.00 | 0.249 | standard |
| 201385_at | DHX15 | 1529.4 | 383.5 | 1.00 | 0.251 | standard |
| 4714_at | NDUFB8 | 1219.0 | 325.5 | 1.00 | 0.267 | custom |

| **Onco*type* DX® (Genomic Health, Inc, Redwood City, CA) Reference Genes** | | | | | | |
|---|---|---|---|---|---|---|
| 213867_x_at | ACTB | 19566.3 | 4360.8 | 1.00 | 0.223 | standard |
| 200801_x_at | ACTB | 17901.0 | 3995.4 | 1.00 | 0.223 | standard |
| 2597_at | GAPDH | 11873.9 | 3810.3 | 1.00 | 0.321 | standard |
| 212581_x_at | GAPDH | 11930.9 | 4172.5 | 1.00 | 0.350 | standard |
| 217398_x_at | GAPDH | 6595.6 | 2460.2 | 1.00 | 0.373 | standard |
| 213453_x_at | GAPDH | 6695.2 | 2726.8 | 1.00 | 0.407 | standard |
| 60_at | ACTB | 3786.2 | 1622.3 | 1.00 | 0.428 | standard |
| 7037_at | TFRC | 781.8 | 466.6 | 1.00 | 0.597 | standard |
| 208691_at | TFRC | 1035.1 | 630.8 | 1.00 | 0.609 | standard |
| 207332_s_at | TFRC | 506.9 | 341.6 | 0.97 | 0.674 | standard |

| | | | | | | |
|---|---|---|---|---|---|---|
| RPLPO and GUS are also listed as reference genes for the Onco*type* DX® breast cancer assay. | | | | | | |

**Table 4- 100 probe sets including all primary, alternate, and excluded genes (k = 20 clusters)**

| **Gene (probe set)** | **EntrezID** | **CDF** | **VarImp** | **Predictor group** | **Predictor status** |
|---|---|---|---|---|---|
| CCNB2 (9133_at) | 9133 | custom | 0.785 | primary | predictor1 |
| MELK (9833_at) | 9833 | custom | 0.739 | alternate1 | predictor1 alternate1 |
| GINS1 (9837_at) | 9837 | custom | 0.476 | alternate2 | predictor1 alternate2 |
| RRM2 (6241_at) | 6241 | custom | 0.399 | alternate3 | predictor1 alternate3 |
| GINS2 (51659_at) | 51659 | custom | 0.354 | alternate4 | predictor1 alternate4 |
| CCNB1 (214710_s_at) | 891 | standard | 0.140 | alternate5 | predictor1 alternate5 |
| TOP2A (201291_s_at) | 7153 | standard | 0.590 | primary | predictor2 |
| MCM2 (4171_at) | 4171 | custom | 0.428 | alternate1 | predictor2 alternate1 |
| KIAA0101 (9768_at) | 9768 | custom | 0.409 | alternate2 | predictor2 alternate2 (excluded) |
| CDK1 (203213_at) | 983 | standard | 0.379 | alternate3 | predictor2 alternate3 |
| UBE2C (202954_at) | 11065 | standard | 0.365 | alternate4 | predictor2 alternate4 |
| TMEM97 (212281_s_at) | 27346 | standard | 0.147 | alternate5 | predictor2 alternate5 |
| DTL (218585_s_at) | 51514 | standard | 0.130 | alternate6 | predictor2 alternate6 |
| RACGAP1 (29127_at) | 29127 | custom | 0.588 | primary | predictor3 |
| LSM1 (27257_at) | 27257 | custom | 0.139 | alternate1 | predictor3 alternate1 |
| SCD (200832_s_at) | 6319 | standard | 0.125 | alternate2 | predictor3 alternate2 |
| HN1 (51155_at) | 51155 | custom | 0.104 | alternate3 | predictor3 alternate3 |
| CKS2 (1164_at) | 1164 | custom | 0.515 | primary | predictor4 |
| NUSAP1 (218039_at) | 51203 | standard | 0.491 | alternate1 | predictor4 alternate1 |
| PTTG1 (203554_x_at) | 9232 | standard | 0.408 | alternate2 | predictor4 alternate2 (excluded) |
| ZWINT (204026_s_at) | 11130 | standard | 0.272 | alternate3 | predictor4 alternate3 |
| TYMS (7298_at) | 7298 | custom | 0.269 | alternate4 | predictor4 alternate4 |
| MLF11P (218883_s_at) | 79682 | standard | 0.204 | alternate5 | predictor4 alternate5 |
| SQLE (209218_at) | 6713 | standard | 0.174 | alternate6 | predictor4 alternate6 |
| AURKA (208079_s_at) | 6790 | standard | 0.508 | primary | predictor5 |
| PRC1 (9055_at) | 9055 | custom | 0.499 | alternate1 | predictor5 alternate1 |
| CENPF (207828 s_at) | 1063 | standard | 0.306 | alternate2 | predictor5 alternate2 |
| ASPM (219918_s_at) | 259266 | standard | 0.293 | alternate3 | predictor5 alternate3 |
| NEK2 (204641_at) | 4751 | standard | 0.134 | alternate4 | predictor5 alternate4 |
| ECT2 (1894_at) | 1894 | custom | 0.105 | alternate5 | predictor5 alternate5 |
| FEN1 (204767_s_at) | 2237 | standard | 0.403 | primary | predictor6 |
| FADD (8772_at) | 8772 | custom | 0.313 | alternate1 | predictor6 alternate1 |
| SMC4 (10051_at) | 10051 | custom | 0.170 | alternate2 | predictor6 alternate2 |
| SLC35E3 (55508_at) | 55508 | custom | 0.151 | alternate3 | predictor6 alternate3 |
| TXNRD1 (7296_at) | 7296 | custom | 0.136 | alternate4 | predictor6 alternate4 |
| RAE1 (211318_s_at) | 8480 | standard | 0.132 | alternate5 | predictor6 alternate5 |
| ACBD3 (202323_s_at) | 64746 | standard | 0.129 | alternate6 | predictor6 alternate6 |
| ZNF274 (204937_s_at) | 10782 | standard | 0.122 | alternate7 | predictor6 alternate7 |
| FRG1 (2483_at) | 2483 | custom | 0.108 | alternate8 | predictor6 alternate8 (excluded) |
| LPCAT1 (201818_at) | 79888 | standard | 0.106 | alternate9 | predictor6 alternate9 |
| EBP (10682_at) | 10682 | custom | 0.341 | primary | predictor7 |
| RFC4 (204023_at) | 5984 | standard | 0.264 | alternate1 | predictor7 alternate1 |
| NCAPG (218662_s_at) | 64151 | standard | 0.234 | alternate2 | predictor7 alternate2 |
| RNASEH2A (10535_at) | 10535 | custom | 0.205 | alternate3 | predictor7 alternate3 |
| MED24 (9862_at) | 9862 | custom | 0.191 | alternate4 | predictor7 alternate4 |
| DONSON (29980_at) | 29980 | custom | 0.186 | alternate5 | predictor7 alternate5 |
| RMI1 (80010_at) | 80010 | custom | 0.184 | alternate6 | predictor7 alternate6 |
| PTGES (9536_at) | 9536 | custom | 0.164 | alternate7 | predictor7 alternate7 |
| C19orf60 (51200_at) | 55049 | standard | 0.151 | alternate8 | predictor7 alternate8 |
| ISYNA1 (222240_s_at) | 51477 | standard | 0.135 | alternate9 | predictor7 alternate9 |
| SKP2 (203625_x_at) | 6502 | standard | 0.130 | alternate10 | predictor7 alternate10 |
| DPP3 (218567_x_at) | 10072 | standard | 0.126 | alternate11 | predictor7 alternatell (excluded) |
| TYMP (204858_s_at) | 1890 | standard | 0.122 | alternate12 | predictor7 alternate12 |
| SNRPA1 (216977_x_at) | 6627 | standard | 0.116 | alternate13 | predictor7 alternate13 |
| DHCR7 (201791_s_at) | 1717 | standard | 0.113 | alternate14 | predictor7 alternate14 |
| TFPT (218996_at) | 29844 | standard | 0.105 | alternate15 | predictor7 alternate15 |
| CTTN (2017_at) | 2017 | custom | 0.102 | alternate16 | predictor7 alternate16 |
| MCM5 (216237_s_at) | 4174 | standard | 0.102 | alternate17 | predictor7 alternate17 |
| TXNIP (10628_at) | 10628 | custom | 0.292 | primary | predictor8 |
| SYNE2 (23224_at) | 23224 | custom | 0.270 | primary | predictor9 |
| SCARB2 (201646_at) | 950 | standard | 0.225 | alternate1 | predictor9 alternate1 |
| PDLIM5 (216804_s_at) | 10611 | standard | 0.167 | alternate2 | predictor9 alternate2 |
| TSC2 (7249_at) | 7249 | custom | 0.145 | alternate3 | predictor9 alternate3 |
| ELF1 (212420_at) | 1997 | standard | 0.119 | alternate4 | predictor9 alternate4 |
| DICER1 (23405_at) | 23405 | custom | 0.209 | primary | predictor10 |
| CALD1 (201616_s_at) | 800 | standard | 0.129 | alternate1 | predictor10 alternate1 |
| SOX9 (6662_at) | 6662 | custom | 0.125 | alternate2 | predictor10 alternate2 |
| FAM20B (202915_s_at) | 9917 | standard | 0.108 | alternate3 | predictor10 alternate3 |
| APH1A (218389_s_at) | 51107 | standard | 0.099 | alternate4 | predictor10 alternate4 |
| AP1AR (55435_at) | 55435 | custom | 0.201 | primary | predictor11 |
| PDCD6 (222380_s_at) | 10016 | standard | 0.154 | alternate1 | predictor11 alternate1 (excluded) |
| PBX2 (202876_s _at) | 5089 | standard | 0.134 | alternate2 | predictor11 alternate2 |
| WASL (205809_s_at) | 8976 | standard | 0.126 | alternate3 | predictor11 alternate3 |
| SLC11A2 (203123_s_at) | 4891 | standard | 0.119 | alternate4 | predictor11 alternate4 |
| KIAA0776 (212634_at) | 23376 | standard | 0.107 | alternate5 | predictor11 alternate5 (excluded) |
| C14orf101 (54916_at) | 54916 | custom | 0.101 | alternate6 | predictor11 alternate6 |
| NUP107 (57122_at) | 57122 | custom | 0.197 | primary | predictor12 |
| FAM38A (202771_at) | 9780 | standard | 0.165 | alternate1 | predictor12 alternate1 |
| PLIN2 (209122_at) | 123 | standard | 0.110 | alternate2 | predictor12 alternate2 |
| AIM1 (212543_at) | 202 | standard | 0.102 | alternate3 | predictor12 alternate3 |
| APOC1 (204416_x_at) | 341 | standard | 0.176 | primary | predictor13 |
| APOE (203382_s_at) | 348 | standard | 0.121 | alternate1 | predictor13 alternate1 |
| DTX4 (23220_at) | 23220 | custom | 0.164 | primary | predictor14 |
| AQP1 (358_at) | 358 | custom | 0.141 | alternate1 | predictor14 alternate1 |
| LMO4 (209205_s_at) | 8543 | standard | 0.120 | alternate2 | predictor14 alternate2 |
| TAF1D (218750_at) | 79101 | standard | 0.159 | primary | predictor15 (excluded) |
| SNORA25 (684959_at) | 684959 | custom | 0.127 | alternate1 | predictor15 alternate1 (excluded) |
| FMOD (202709_at) | 2331 | standard | 0.154 | primary | predictor16 |
| RGS5 (8490_at) | 8490 | custom | 0.120 | alternate1 | predictor16 alternate1 |
| PIK3R1 (212239_at) | 5295 | standard | 0.103 | alternate2 | predictor16 alternate2 |
| MBNL2 (203640_at) | 10150 | standard | 0.100 | alternate3 | predictor16 alternate3 |
| MAPKAPK2 (201461_s_at) | 9261 | standard | 0.151 | primary | predictor17 |
| MTUS1 (212093 s_at) | 57509 | standard | 0.136 | alternate1 | predictor17 alternate1 |
| DHX9 (212107_s_at) | 1660 | standard | 0.136 | alternate2 | predictor17 alternate2 |
| PPIF (201490_s_at) | 10105 | standard | 0.115 | alternate3 | predictor17 alternate3 |
| FOLR1 (211074_at) | 2348 | standard | 0.126 | primary | predictor18 (excluded) |
| KIAA1467 (57613_at) | 57613 | custom | 0.116 | primary | predictor19 (excluded) |
| SUPT4H1 (201483_s_at) | 6827 | standard | 0.111 | primary | predictor20 |
| PHB (200658_s_at) | 5245 | standard | 0.106 | alternate1 | predictor20 alternate1 |
| CD44 (204489_s_at) | 960 | standard | 0.105 | alternate2 | predictor20 alternate2 |

| | | | | | |
|---|---|---|---|---|---|
| Excluded genes are indicated by the notation "(excluded)" in the last column | | | | | |

**Table 5. 90 probe sets (failed probes excluded) including all primary and alternate genes (k = 8 clusters)**

| **Gene (probe set)** | **CDF** | **VarImp** | **predictor group** | **predictor status** |
|---|---|---|---|---|
| CCNB2 (9133_at) | custom | 0.785 | primary | predictor1 |
| MELK (9833_at) | custom | 0.739 | alternate1 | predictor1 alternate1 |
| TOP2A (201291_s_at) | standard | 0.590 | alternate2 | predictor1 alternate2 |
| GINS1 (9837_at) | custom | 0.476 | alternate3 | predictor1 alternate3 |
| MCM2 (4171_at) | custom | 0.428 | alternate4 | predictor1 alternate4 |
| RRM2 (6241_at) | custom | 0.399 | alternate5 | predictor1 alternate5 |
| CDK1 (203213_at) | standard | 0.379 | alternate6 | predictor1 alternate6 |
| UBE2C (202954_at) | standard | 0.365 | alternate7 | predictor1 alternate7 |
| GINS2 (51659_at) | custom | 0.354 | alternate8 | predictor1 alternate8 |
| NCAPG (218662_s_at) | standard | 0.234 | alternate9 | predictor1 alternate9 |
| TMEM97 (212281_s_at) | standard | 0.147 | alternate10 | predictor1 alternate10 |
| CCNB1 (214710_s_at) | standard | 0.140 | alternate11 | predictor1 alternate11 |
| DTL (218585_s_at) | standard | 0.130 | alternate12 | predictor1 alternate12 |
| RACGAP1 (29127_at) | custom | 0.588 | primary | predictor2 |
| TXNIP (10628_at) | custom | 0.292 | alternate1 | predictor2 alternate1 |
| APOC1 (204416_x_at) | standard | 0.176 | alternate2 | predictor2 alternate2 |
| LSM1 (27257_at) | custom | 0.139 | alternate3 | predictor2 alternate3 |
| SCD (200832_s_at) | standard | 0.125 | alternate4 | predictor2 alternate4 |
| HN1 (51155_at) | custom | 0.104 | alternate5 | predictor2 alternate5 |
| CKS2 (1164_at) | custom | 0.515 | primary1 | predictor3 |
| NUSAP1 (218039_at) | standard | 0.491 | alternate | predictor3 alternate1 |
| FEN1 (204767_s_at) | standard | 0.403 | alternate2 | predictor3 alternate2 |
| ZWINT (204026_s_at) | standard | 0.272 | alternate3 | predictor3 alternate3 |
| TYMS (7298_at) | custom | 0.269 | alternate4 | predictor3 alternate4 |
| MLF1IP (218883_s_at) | standard | 0.204 | alternate5 | predictor3 alternate5 |
| NUP107 (57122_at) | custom | 0.197 | alternate6 | predictor3 alternate6 |
| SQLE (209218_at) | standard | 0.174 | alternate7 | predictor3 alternate7 |
| SMC4 (10051_at) | custom | 0.170 | alternate8 | predictor3 alternate8 |
| SLC35E3 (55508_at) | custom | 0.151 | alternate9 | predictor3 alternate9 |
| APOE (203382_s_at) | standard | 0.121 | alternate10 | predictor3 alternate10 |
| SUPT4H1 (201483_s_at) | standard | 0.111 | alternate11 | predictor3 alternate11 |
| PLIN2 (209122_at) | standard | 0.110 | alternate12 | predictor3 alternate12 |
| PHB (200658_s_at) | standard | 0.106 | alternate13 | predictor3 alternate13 |
| AURKA (208079_s_at) | standard | 0.508 | primary | predictor4 |
| PRC1 (9055_at) | custom | 0.499 | alternate1 | predictor4 alternate1 |
| CENPF (207828_s_at) | standard | 0.306 | alternate2 | predictor4 alternate2 |
| ASPM (219918_s_at) | standard | 0.293 | alternate3 | predictor4 alternate3 |
| NEK2 (204641_at) | standard | 0.134 | alternate4 | predictor4 alternate4 |
| DHCR7 (201791_s_at) | standard | 0.113 | alternate5 | predictor4 alternate5 |
| ECT2 (1894_at) | custom | 0.105 | alternate6 | predictor4 alternate6 |
| EBP (10682_at) | custom | 0.341 | primary | predictor5 |
| FADD (8772_at) | custom | 0.313 | alternate1 | predictor5 alternate1 |
| RFC4 (204023_at) | standard | 0.264 | alternate2 | predictor5 alternate2 |
| RNASEH2A (10535_at) | custom | 0.205 | alternate3 | predictor5 alternate3 |
| MED24 (9862_at) | custom | 0.191 | alternate4 | predictor5 alternate4 |
| DONSON (29980_at) | custom | 0.186 | alternate5 | predictor5 alternate5 |
| RMI1 (80010_at) | custom | 0.184 | alternate6 | predictor5 alternate6 |
| PTGES (9536_at) | custom | 0.164 | alternate7 | predictor5 alternate7 |
| DTX4 (23220_at) | custom | 0.164 | alternate8 | predictor5 alternate8 |
| C19orf60 (51200_at) | standard | 0.151 | alternate9 | predictor5 alternate9 |
| TXNRD1 (7296_at) | custom | 0.136 | alternate10 | predictor5 alternate10 |
| ISYNA1 (222240_s_at) | standard | 0.135 | alternate11 | predictor5 alternate11 |
| RAE1 (211318_s_at) | standard | 0.132 | alternate12 | predictor5 alternate12 |
| SKP2 (203625_x_at) | standard | 0.130 | alternate13 | predictor5 alternate13 |
| ACBD3 (202323_s_at) | standard | 0.129 | alternate14 | predictor5 alternate14 |
| ZNF274 (204937_s_at) | standard | 0.122 | alternate15 | predictor5 alternate 15 |
| TYMP (204858_s_at) | standard | 0.122 | alternate16 | predictor5 alternate16 |
| SNRPA1 (216977_x_at) | standard | 0.116 | alternate17 | predictor5 alternate17 |
| LPCAT1 (201818_at) | standard | 0.106 | alternate18 | predictor5 alternate18 |
| TFPT (218996_at) | standard | 0.105 | alternate19 | predictor5 alternate19 |
| CTTN (2017_at) | custom | 0.102 | alternate20 | predictor5 alternate20 |
| MCM5 (216237_s_at) | standard | 0.102 | alternate21 | predictor5 alternate21 |
| SYNE2 (23224_at) | custom | 0.270 | primary | predictor6 |
| SCARB2 (201646_at) | standard | 0.225 | alternate1 | predictor6 alternate1 |
| PDLIM5 (216804_s_at) | standard | 0.167 | alternate2 | predictor6 alternate2 |
| TSC2 (7249_at) | custom | 0.145 | alternate3 | predictor6 alternate3 |
| AQP1 (358_at) | custom | 0.141 | alternate4 | predictor6 alternate4 |
| ELF1 (212420_at) | standard | 0.119 | alternate5 | predictor6 alternate5 |
| DICER1 (23405_at) | custom | 0.209 | primary | predictor7 |
| FAM38A (202771_at) | standard | 0.165 | alternate1 | predictor7 altemate1 |
| FMOD (202709_at) | standard | 0.154 | alternate2 | predictor7 alternate2 |
| CALD1 (201616_s_at) | standard | 0.129 | alternate3 | predictor7 alternate3 |
| SOX9 (6662_at) | custom | 0.125 | alternate4 | predictor7 alternate4 |
| RGS5 (8490_at) | custom | 0.120 | alternate5 | predictor7 alternate5 |
| FAM20B (202915_s_at) | standard | 0.108 | alternate6 | predictor7 alternate6 |
| CD44 (204489_s_at) | standard | 0.105 | alternate7 | predictor7 alternate7 |
| PIK3R1 (212239_at) | standard | 0.103 | alternate8 | predictor7 alternate8 |
| AIM1 (212543_at) | standard | 0.102 | alternate9 | predictor7 alternate9 |
| MBNL2 (203640_at) | standard | 0.100 | alternate10 | predictor7 alternate10 |
| APH1A (218389_s_at) | standard | 0.099 | alternate11 | predictor7 alternate11 |
| AP1AR (55435_at) | custom | 0.201 | primary | predictor8 |
| MAPKAPK2 (201461_s_at) | standard | 0.151 | alternate1 | predictor8 alternate1 |
| MTUS1 (212093_s_at) | standard | 0.136 | alternate2 | predictor8 alternate2 |
| DHX9 (212107_s_at) | standard | 0.136 | alternate3 | predictor8 alternate3 |
| PBX2 (202876_s_at) | standard | 0.134 | alternate4 | predictor8 alternate4 |
| WASL (205809_s_at) | standard | 0.126 | alternate5 | predictor8 alternate5 |
| LMO4 (209205_s_at) | standard | 0.120 | alternate6 | predictor8 alternate6 |
| SLC11A2 (203123_s_at) | standard | 0.119 | alternate7 | predictor8 alternate7 |
| PPIF (201490_s_at) | standard | 0.115 | alternate8 | predictor8 alternate8 |
| C14orf101 (54916_at) | custom | 0.101 | alternate9 | predictor8 alternate9 |

**Table 6.**

| **Study** | **GSE** | **Total samples** | **ER+/LN-/ untreated*/ outcome** | **Duplicates removed** | **ER+/HER-array** | **10 yr relapse** | **10 yr no relapse** |
|---|---|---|---|---|---|---|---|
| **Desmedt_2007¹** | GSE7390 | 198 | 135 | 135 | 116 | 42 | 60 |
| **Ivshina_2006²** | GSE4922 | 290 | 133 | 2 | 2 | 0 | 2 |
| **Loi_2007³** | GSE6532 | 327 | 170 | 43 | 40 | 10 | 5 |
| **Miller**_**2005⁴** | GSE3494 | 251 | 132 | 115 | 100 | 30 | 52 |
| **Schmidt_2008⁵** | GSE11121 | 200 | 200** | 200 | 155 | 25 | 46 |
| **Sotiriou**_**2006⁶** | GSE2990 | 189 | 113 | 48 | 45 | 12 | 15 |
| **Symmans_2010⁷** | GSE17705 | 298 | 175 | 110 | 102 | 12 | 41 |
| **Wang_2005⁸** | GSE2034 | 286 | 209 | 209 | 173 | 67 | 29 |
| **Zhang_2009⁹** | GSE12093 | 136 | 136 | 136 | 125 | 15 | 24 |
| **9 studies** | | **2175** | **1403** | **998** | **858** | **213** | **274** |

**Table 7. Comparison of validation results in independent test data for full-gene-set, 17-gene and 8-gene RFRS models**

| | | **Relapse-Free Survival** | | | | | | |
|---|---|---|---|---|---|---|---|---|
| **RFRS Performance** | | **Low risk** | | **Int risk** | | **High risk** | | |
| **Model** | **AUC** | **RR** | **N (%)** | **RR** | **N (%)** | **RR** | **N (%)** | **KM (p)** |
| Full-gene-set | 0.730 | 6.9 | 78 (30.7) | 15.8 | 133 (52.4) | 26.8 | 43 (16.9) | 6.54E-06 |
| 17-gene | 0.715 | 7.8 | 97 (38.2) | 15.3 | 103 (40.5) | 26.8 | 54 (21.3) | 9.57E-06 |
| 8-gene | 0.690 | 9.7 | 101 (39.8) | 13.9 | 105 (41.3) | 28.3 | 48 (18.9) | 2.84E-05 |
| **RR, relapse rate** | | | | | | | | |

**Appendix 1. Sample patient data tab-delimited text file: e.g., patient_data.txt)**

| | **TOP2A** | **MAPKAPK2** | **SUPT4H1** | **FMOD** | **APOC1** | **FEN1** | **AURKA** | **TXNIP** | **EBP** | **CKS2** | **DTX4** | **SYNE2** | **DICER1** | **RACGAP1** | **AP1AR** | **NUP107** | **CCNB2** |
|---|---|---|---|---|---|---|---|---|---|---|---|---|---|---|---|---|---|
| **GSM36893** | 7.0874 | 3.9958 | 7.6561 | 6.7689 | 10.268 | 8.8817 | 6.6811 | 8.3538 | 7.033 | 8.0512 | 6.0171 | 3.2419 | 6.272 | 10.0237 | 6.3404 | 8.9953 | 7.3143 |

### Appendix 2. RFRS algorithm code

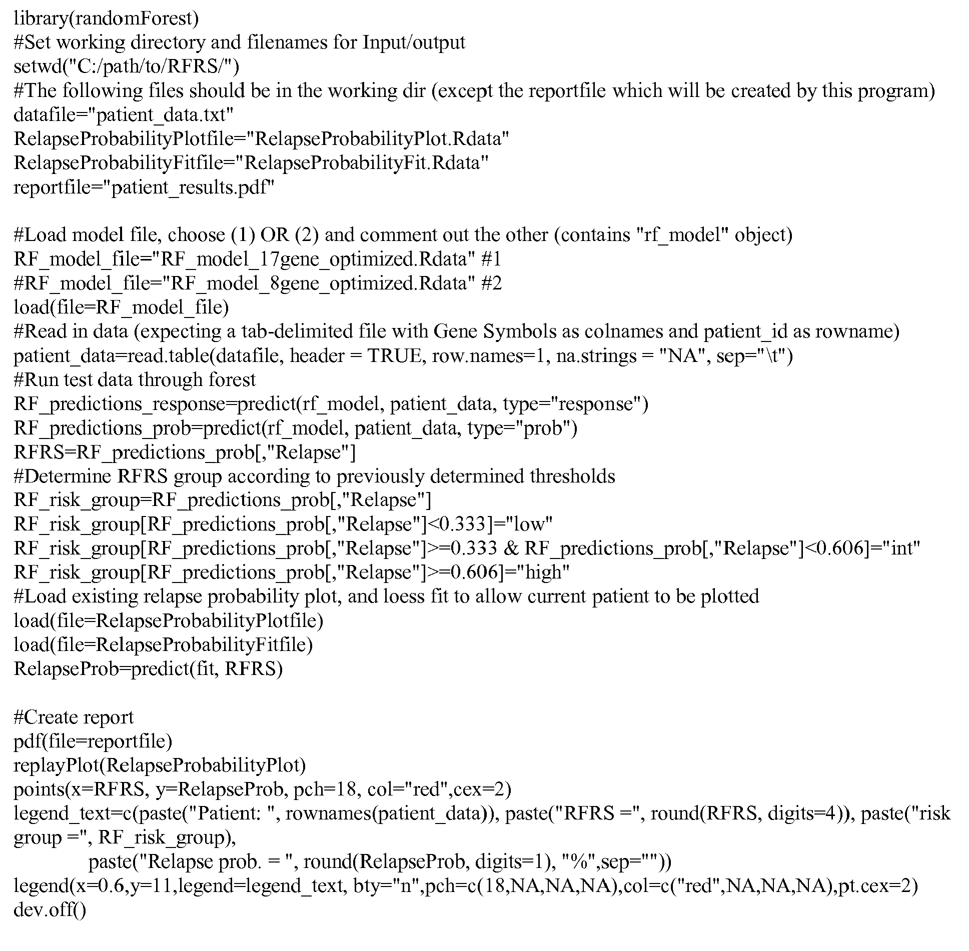

### Appendix 3. Probe sequences for top 100 probesets

CCNB2 probes (SEQ ID NO:1-9) 
ATGGAGCTGACTCTCATCGACTATG
ATATGGTGCATTATCATCCTTCTAA
AGTCCTCTGGTCTATCTCATGAAAC
CTTGCCTCCCCACTGATAGGAAGGT
CAAAAGCCGTCAAAGACCTTGCCTC
GATTTTGTACATAGTCCTCTGGTCT
GCCACTACACTTCTTAAGGCGAGCA
GATAGGAAGGTCCTAGGCTGCCGTG
ATCCTTCTAAGGTAGCAGCAGCTGC
TOP2A probes (SEQ ID NO:10-20) 
ACTCCGTAACAGATTCTGGACCAAC
GACCAACCTTCAACTATCTTCTTGA
GAAAGATGAACTCTGCAGGCTAAGA
ACAAGATGAACAAGTCGGACTTCCT
TGGCTCCTAGGAATGCTTGGTGCTG
GATATGATTCGGATCCTGTGAAGGC
AAAGAAAGAGTCCATCAGATTTGTG
GAATAATCAGGCTCGCTTTATCTTA
CTTGGTGCTGAATCTGCTAAACTGA
AAGAACAAGAGCTGGACACATTAAA
GAGACTTTTTGAACTCAGACTTAA
RACGAP1 probes (SEQ ID NO:21-25) 
GTACAACTCGTATTTATCTCTGATG
GAATGTTTGACTTCGTATTGACCCT
GGATGCTGAAATTTTTCCCATGGAA
ACTTCGTATTGACCCTTATCTGTAA
CAATATATCATCCTTTGGCATCCCA
CKS2 probes (SEQ ID NO:26-28) 
CGCTCTCGTTTCATTTCTGCAGCG
TATTCTTCTCTTTAGACGACCTCTT
TCTCTTTAGACGACCTCTTCCAAAA
AURKA probes (SEQ ID NO:29-39) 
CTACCTCCATTTAGGGATTTGCTTG
GTGTCTCAGAGCTGTTAAGGGCTTA
CCCTCAATCTAGAACGCTACACAAG
GAGGCCATGTGTCTCAGAGCTGTTA
TTAGGGATTTGCTTGGGATACAGAA
GTGCTCTACCTCCATTTAGGGATTT
AAATAGGAACACGTGCTCTACCTCC
GGGATACAGAAGAGGCCATGTGTCT
GAAGAGGCCATGTGTCTCAGAGCTG
CAGAGCTGTTAAGGGCTTATTTTTT
CATTGGAGTCATAGCATGTGTGTAA
FEN1 probes (SEQ ID NO:40-50 
[?]
GAACTTGCTATGTAATTTGTGTCTA
GATGGTGATGTTCACCTGGCAATCA
 
GAGCCACCAGGAAGGCGCATCTTAG
TTGACCCACCTTGAGAGAGAGCCAC
GGACACTAAGTCCATTGTTACATGA
GAAATGATTTCCTGGCTGGCCAACT
ACACTGGTTTTCATGCGCTGTTTTT
ACTGATTACTGGCTGTGTCTTGGGT
TGGACCTAGACTGTGCTTTTCTGTC
TTGGGTGGGCAGAAACTCGAACTTG
ACCTGGCAATCAGCTGAGTTGAGAC
EBP probes (SEQ ID NO:51-71) 
GAAGGCACTGCTGGGAGCCATTAGA
CAGGCTCATGGGCAGGCACAAGAAG
GTCTTAGTCGTGACCACATGGCTGT
CACAGATACAAGAGAAGCCAGGAGG
AAGGGGCTGTGTGAAGGCACTGCTG
AGAAGAACTGAGGAGTGGTGGACCA
GCCAGGAGGTCTATGATGGTGACGA
CCCACCTGGCATATACTGGCTGGCC
ACATGGCTGTTGTCAGGTCGTGCTG
TCTATGGGGATGTGCTCTACTTCCT
GCATGGAAACCATCACAGCTTGCCT
GAGTGGTGGACCAGGCTCGAACACT
TTGGAGGGACAAAGCTAATTGATCT
GATGCCAAGGCCACAAAAGCCAAGA
CCAGGCTCGAACACTGGCCGAGGAG
TGACAGAGCACCGCGACGGATTCCA
GGGAGCCATTAGAACACAGATACAA
TTTGTCTTCATGAATGCCCTGTGGC
GGAGACCAAGCCTTCTTATCTCAAC
TGCAGTGTGTGGGTTCATTCACCTG
CTCCGCTTCATTCTACAGCTTGTGG
TXNIP probes (SEQ ID NO:72-102) 
TGTGTCAGAGCACTGAGCTCCACCC
TACAAGTTCGGCTTTGAGCTTCCTC
AAAGGATGCGGACTCATCCTCAGCC
ACTTTGTTCACTGTCCTGTGTCAGA
GAAAGGGTTGCTGCTGTCAGCCTTG
AGATAGGGATATTGGCCCCTCACTG
GGCAATCTCCTGGGCCTTAAAGGAT
CTTAGCCTCTGACTTCCTAATGTAG
GCAAAGGGGTTTCCTCGATTTGGAG
AAATGGCCTCCTGGCGTAAGCTTTT
AAACCAACTCAGTTCCATCATGGTG
TTCCACCGTCATTTCTAACTCTTAA
GGTTTTCTCTTCATGTAAGTCCTTG
CGGAGTACCTGCGCTATGAAGACAC
CCCTGCATCCTCAACAACAATGTGC
GTGTTCTCCTACTGCAAATATTTTC
AATTGAGGCCTTTTCGATAGTTTCG
GGAGGTGGTCAGCAGGCAATCTCCT
CCAGCGCCCATGTTGTGATACAGGG
 
GAAAAACTCAGGCCCATCCATTTTC
TGAGGTGGTCTTTAACGACCCTGAA
TGTTCTTAGCACTTTAATTCCTGTC
AGCTCCACCCTTTTCTGAGAGTTAT
CACTCTCAGCCATAGCACTTTGTTC
GAAGCAGCTTTACCTACTTGTTTCT
GAAGTTACTCGTGTCAAAGCCGTTA
GGTGGATGTCAATACCCCTGATTTA
CCGAGCCAGCCAACTCAAGAGACAA
TGGATGCAGGGATCCCAGCAGTGCA
GATCCTGGCTTGCGGAGTGGCTAAA
GCTGAAACTGGTCTACTGTGTCTCT
SYNE2 probes (SEQ ID NO:103-113) 
TTTCTAAGACTTTTTCACATCCAAA
GTTTTACTCCAATCAGCTGGCAATT
GGCACCCTTAGCTGATGGAAACAAT
ATTTTGAGCTGCCGGTTATACACCA
TGTTCTGTTCAGTACCTAGCTCTGC
GTAAATGCCAAACTACCGACTTGAT
TACGCTTAGAATCAGTTTTACTCCA
GTTCAGAAACTCATAGGCACCCTTA
TGAGCAGTGGTGTCCATCACATATA
ATGTACAACTCAGATGTTTCTCATT
GCTCTGCTCTTTTATATTGCTTTAA
DICER1 probes (SEQ ID NO:114-142) 
AATTTCTTACTATACTTTTCATAAT
ATTTCACCTACCAAAGCTGTGCTGT
ACTAGCTCATTATTTCCATCTTTGG
AAATGATTTTTCACAACTAACTTGT
TTGCAGTCTGCACCTTATGGATCAC
TGATACATCTGTGATTTAGGTCATT
GGAGACGCCAATAGCAATATCTAGG
CTGATGCCACATAGTCTTGCATAAA
AGCTGTGCTGTTAATGCCGTGAAAG
GAAGTGCGCCAATGTTGTCTTTTCT
GTGAAACCTTCATGGATAGTCTTTA
TTTACTAAAGTCCTCCTGCCAGGTA
GGACATCAACCACAGACAATTTAAA
TGTTGCATGCATATTTCACCTACCA
ATAAACCTTAGACATATCACACCTA
TAGTCTTTAATCTCTGATCTTTTTG
GAGACAGCGTGATACTTACAACTCA
GACCATTGTATTTCCACTAGCAGT
CTGCAGCAGCAGGTTACATAGCAAA
GCCGTGAAAGTTTAACGTTTGCGAT
AACTGCCGTAATTTTGATACATCTG
TATTTACCATCACATGCTGCAGCTG
AACGTTTGCGATAAACTGCCGTAAT
GGAAATTTGCATTGAGACCATTGTA
GCACCTTATGGATCACAATTACCTT
AGAAGCAAAACACAGCACCTTTACC
 
CCCTTAGTCTCCTCACATAAATTTC
TGTGTAAGGTGATGTTCCCGGTCGC
CTGCCAGGTAGTTCCCACTGATGGA
AP1AR probes (SEQ ID NO:143-153) 
GCCTTCCTTTACCTTGTAGTACAAG
TTTTTCCTCTTGCAACAATGACGGT
GTCAATTTACAAGGCCAGGGATAGA
TTCCACTTCATTTTACATGCCACTA
GTGCTAGACAATTACTGTTCTTTTC
AATATCTATAACTGCATTTTGTGCT
GATAGAAAACACTCCATAATTGCTT
CATTGATTTTATTAAGCCTTCCTTT
TACATGCCACTATATTGACTTTAAT
TCTGGTATGAAAGGCTCCATTGATT
GCTTTCCTTGATTTTGCTGAGGATT
NUP107 probes (SEQ ID NO:154-163) 
GGATATCAGCGTTTCTCTGTGTGCT
GAAAGCTTTGTCTGCCAATGTTGTG
CAGAGAGTCCTCTCTAATGCTCCTA
GATATTGCACAGTACTGGTCAGTAT
GACCAGGGACTTGACCCATTAGGGT
AGATATGGTATCCTCTGAGCGCCAC
AATGCTCCTAGACCAGGGACTTGAC
ATCGTGACACTTTCAACATGTAGGG
TTGGATGCCCTAACTGCTGATGTGA
GTGTTTTCTGCTTCATACGATATTG
APOC1 probes (SEQ ID NO:164-174) 
AAGGGTGACATCCAGGAGGGGCCTC
CAGGAGGGGCCTCTGAAATTTCCCA
GATGCGGGAGTGGTTTTCAGAGACA
CAGCAAGGATTCAGGAGTGCCCCTC
GTGAACTTTCTGCCAAGATGCGGGA
CAAGGCTCGGGAACTCATCAGCCGC
AACACACTGGAGGACAAGGCTCGGG
GACGTCTCCAGTGCCTTGGATAAGC
CCAAGCCCTCCAGCAAGGATTCAGG
TCATCAGCCGCATCAAACAGAGTGA
GTTCTGTCGATCGTCTTGGAAGGCC
DTX4 probes (SEQ ID NO:175-180) 
ATCGCCACCTGGTGCTCATGAGGTG
ACTCGTCTTGGTATTGCACTGTTGT
ATTCTCTTCCCATTTTTGTACATTT
TGCTCCGTGAAAGGACATCGCCACC
GGAGACAAACCTCGTCAGATGCTCA
TGAAGTCTTTGGTGTTGCTCCGTGA
TAF1D probes (SEQ ID NO:181-191) 
TGATTGTTGCCATGTGAGAGTTTTA
ACTCCTAATGTTTGGTGCTATGTTT
 
GTATGGGTCATTTCAAAGAGGGCTT
TGGTGCTATGTTTTCCTGAGGAGAT
AAGTTTCTCTAGTGTTTTCTGTGGA
GTATTTTTGGCTCGAAGTTTCTCTA
GAAGCCATAGCACTCCTAATGTTTG
AAGAGGGCTTATGAGGCTGTGAAAC
CCCAGAGCTCTTAACGCTGTGACCA
GAGGCTGTGAAACCCAGAGCTCTTA
ATTTCTCTTCTTCAGGGCAAACTTG
FMOD probes (SEQ ID NO:192-202) 
GCTGGGGAGCACTTAATTCTTCCCA
GGAGCTCCGATGTGAGGGGCAAGGC
TCTGGCTGGGGTCCGTGAAGCCCAG
GCCAAACCAGCTCATTTCAACAAAG
ATGTGAACACCATCATGCCTTTATA
TGCCATCACATCCCTGATACTGTGT
TTTGGACTACGTTCTTGGCTCCAGA
GCAGCCAAATCTTGCCTGTGCTGGG
GCTTTGAAGCACCTTCCCTGAGAAG
TCTGCTTTCACATCTCTGAGCTATA
TAATGTTGCCTGGGGCTTAACCCAC
MAPKAPK2 probes (SEQ ID NO:203-213) 
GCTGAAGAGGCGGAAGAAAGCTCGG
CTCCTGCCCACGGGAGGACAAGCAA
CCTGCCCACGGGAGGACAAGCAATA
GGACAAGCAATAACTCTCTACAGGA
AACTCTCTACAGGAATATATTTTTT
GTTGACTACGAGCAGATCAAGATAA
AATGCGCGTTGACTACGAGCAGATC
CACAATGCGCGTTGACTACGAGCAG
GCGCGTTGACTACGAGCAGATCAAG
AAGCAATAACTCTCTACAGGAATAT
AGACAGAACTGTCCACATCTGCCTC
FOLR1 probes (SEQ ID NO:214-224) 
AATCTTTGAGACAAGCATATGCTAC
CGGCCGTGCGTACTTAGACATGCAT
CCATTCGCAGTTTCACTGTACCGGC
GTGCGTACTTAGACATGCATGGCTT
GGAGCGAGCGACCAAAGGAACCATA
GCATATGCTACTGGCAGGATCAACC
AACCATAACTGATTTAATGAGCCAT
GACATGCATGGCTTAATCTTTGAGA
GAGCGACCAAAGGAACCATAACTGA
CAAGTAGGAGAGGAGCGAGCGACCA
AATGAGCCATTCGCAGTTTCACTGT
KIAA1467 probes (SEQ ID NO:225-235) 
TCTCTAATCCCATCCTGAGGTTGCC
GGAAGCTTCATCTGACCAATGTGGG
AAATGCAAGGGTCTTACCCTCCTCT
 
CCACCCACCCAGGTGTCTAAGATAG
GCAAAGCCAATATGACCACTACTGA
ATCCCCTGAATGTGAATTGCTATCC
AGATAGGACATGCTCCTTTCTTTCT
TTGCTATCCTTATTGCCCTATTAAA
TGGTATGGTGAAACTAATCCCCTGA
TTGCCATCCCCCAAATGTGTGGTAT
CTTGTGAAATGTGTCCCTAAGCCTC
SUPT4H1 probes (SEQ ID NO:236-246) 
TACCCTCCAATTCAGACTCAGCTGA
CAGAACTTCAAATACTTCCTACCCT
CCTGCCCCAAGGAATCGTGCGGGAG
GACAGCTGGGTCTCCAAGTGGCAGC
ATCTTCTTTGGACTACAGGTGGGGT
TAGGATGCTGATTTTCCTACCCGTG
GTATATGACTGCACTAGCTCTTCCT
GAGAGCAGCACATCATTTTATCATT
GTCGAGGAGTGGCCTACAAATCCAG
TGCAAGGCTGCCAGCATCTTTGCTC
ATATGCGGTGTCAGTCACTGGTCGC
MELK probes (SEQ ID NO:247-257) 
AAGACTGTTATGATCGCTTTGATTT
GCCCATCTGTCATTATGTTACTGTC
AGGGCGATGCCTGGGTTTACAAAAG
AGCTCTTAACTATGTCTCTTTGTAA
GATTCTTCCATCCTGCCGGATGAGT
GAATCTAAATCAAGCCCATCTGTCA
GAGCTATCTTAAGACCAATATCTCT
GGAAGACATCCTATCTAGCTGCAAG
GTGTGGGTGTGATACAGCCTACATA
ATGTGGTGGGTATCAGGAGGCAGCG
GGAGGCAGCGGCTTAAGGGCGATGC
MCM2 probes (SEQ ID NO:258-268) 
TTGTGCTTCTCACCTTTGGGTGGGA
GGATGCCTGCGTGTGGTTTAGGTGT
TAGCAGGATGTCTGGCTGCACCTGG
TCTCCACTCAGTACCTTGGATCAGA
GAGTCATGCGGATTATCCACTCGCC
CTGGCATGACTGTTTGTTTCTCCAA
CCCCACTCTCTTATTTGTGCATTCG
AGCACTTGATGAACTCGGGGTACTA
GCCAGTGTGTCTTACTTGGTTGCTG
CCCTCTTGGCGTGAGTTGCGTATTC
TTGGTTGCTGAACATCTTGCCACCT
LSM1 probes (SEQ ID NO:269-278) 
GAAGGACCGAGGTCTTTCCATTCCT
GAGTACTAATCTTTTGCCCAGAGGC
AGTGAAAGTGACATCCTGGCCACCT
ACAGTGGCATAGACTCCTTCACACA
 
ACAGGGACAGTCTTCATTTACTTGT
TCCATTCCTCGAGCAGATACTCTTG
GCACCAGCAACTACTTCTTTATATT
AAAAGGAGAGTGACACACCCCTCCA
CACCTCACGCATTTGATCACAGACT
CCTTCACACATCACTGTGGCACCAG
NUSAP1 probes (SEQ ID NO:279-289) 
CCTTCACCTCAGTGGAGCTTCTGAG
GGCTTTGCTTAGTATCATGTCCATG
TGTACCTTCGTTCAAATATCCTCAT
CATCTGTCACTCACTATATTCACAA
GTTTTATACTGCTCAAGATCGTCAT
GGGATAGAAAGGCCACCTCTTCACT
AACTGCAGTCTTCTGCTAGCCAATA
ACTCATTCTAACATTGCTTACTTAA
CACCTCTTCACTCTCTATAGAATAT
GCTACATAGCCCTATCGAAATGCGA
TCCTCATGTAATTGCCATCTGTCAC
PRC1 probes (SEQ ID NO:290-300) 
TTGCACATGTCACTACTGGGGAGGT
CCTCTCAATCACTACTCTTCTTGAA
GTTCTCAAAAGCTTACCAGTGTGGA
GTGTTCAGTTCTGTTACACAGTGCA
GAGCTGTCTTTGTCGTGGAGATCTG
ACACAGTGCATTGCCCTTTGTTGGG
ACACATGCTTGTCGGAACGCTTTCT
ACTTGGTGTTAGCCACGCTGTTTAC
GTGTCCGAAGTTGAGATGGCCTGCC
GGGAGTCTGTTTGTTCCAATGGGTT
GGAGATCTGGAACTTTGCACATGTC
FADD probes (SEQ ID NO:301-311) 
GATGAGCAGTCACACTGTTACTCCA
GCACTCTCTAAATCTTCCTTGTGAG
GGATTATGGGTCCTGCAATTCTACA
GAAAGGATGTTTTGTCCCATTTCCT
AATTGCCAAGGCAGCGGGATCTCGT
TCCTCTCTGAGACTGCTAAGTAGGG
TGCTCAACCACTGTGGCGTTCTGCT
TGATTGACACACAGCACTCTCTAAA
CTGGACACTAGGGTCAGGCGGGGTG
AGAGGCCCAGGAATCGGAGCGAAGC
GGGGCAGTGATGGTTGCCAGGACGA
RFC4 probes (SEQ ID NO:312-322) 
TCATGCAGCAACTCAGCTCGTCAAT
ATGTTCAAAATTCCGCTTCAAGCCT
AAAGCGCTACTCGATTAACAGGTGG
ACTCATCAGCCTTTGTGCAACTGTG
GAACATTTGCAACTCATCAGCCTTT
TCAACAGCAGCGATTACTAGACATT
 
ACCCCTGACCTCTAGATGTTCAAAA
GTGATGCAGCAGTTATCTCAGAATT
GATGGAGTATTTGCTGCCTGTCAGA
AAGCCATTACATTTCTTCAAAGCGC
TCAGCTCGTCAATCAACTCCATGAT
SCARB2 probes (SEQ ID NO:323-333) 
GTGACAATCATTTTGCTGACAGAAT
AAGGGCATTTTCTTTGATTCTCAAA
GGAGCCATCATATGTCACAGTGTTC
AGAGAAACGTGTGCCCTATACTTCC
GAAATCCATCTATCTACAGCCTAAG
TAGCTCACTGTCACTCACTGAATAG
GAGACACCACTTTTCAAAGGACTTC
AGTTCTTTCCAGTGTTTTGTAGCTC
GGACTTCTTGGTTTCAGCATAACCT
GAGAAGCCTATACATTTAGCTGACA
TGCCCTATACTTCCTGTGACAATCA
CALD1 probes (SEQ ID NO:334-344) 
CTTCCCCCACTAAGGTTTGAGACAG
GACGCAGGACGAGCTCAGTTGTAGA
GACGTATCCAGCAAGCGGAACCTCT
TTCAATATCCCAGTAAACCCATGTA
AGCAGTGATACCAACCACATCTGAA
CTTGAGACCAGGAGACGTATCCAGC
ACTGATCATCATAACTCTGTATCTG
GAACCCAAGCTCAAGACGCAGGACG
GCAAGCGGAACCTCTGGGAAAAGCA
GCGGAATGTGTGCAGTATCTAGAAA
TCTGTGGATAAGGTCACTTCCCCCA
PDCD6 probes (SEQ ID NO:345-355) 
GGTTGGTGCAGCAGTCATTAAAAGT
GAGTCAAGGCCAGACTAGATCAGCC
TTCTCATGGAGCTTCCTTTCTAGAG
CAAAGGGGCGTGTCATGTGCCTCAT
CAAGGCCAGACTAGATCAGCCTAAG
CATGGAGCTTCCTTTCTAGAGGGGA
CTCTATTCTCATGGAGCTTCCTTTC
ATTTGAGTAGATTTGGCCTCTATTC
GACTTTCAAAGGGGCGTGTCATGTG
TTGGCCTCTATTCTCATGGAGCTTC
GATTCTAATAGGTTGGTGCAGCAGT
FAM38A probes (SEQ ID NO:356-366) 
GCTACGGCATCATGGGGCTGTACGT
ATCATGGGGCTGTACGTGTCCATCG
CATTATGTTCGAGGAGCTGCCGTGC
GCTGGCGCCCGAGAGGGAAGGAGCC
GCTGGTCATCGGCAAGTTCGTGCGC
GAGGAGTTGTACGCCAAGCTCATCT
CGCTCACCGGAGACCATGATCAAGT
 
GCGGATTCTTCAGCGAGATCTCGCA
TTCGTGCGCGGATTCTTCAGCGAGA
TCCCCCACGTGTACTGTAGAGTTTT
AGATCTCGCACTCCATTATGTTCGA
APOE probes (SEQ ID NO:367-377) 
GGCCCCTGGTGGAACAGGGCCGCGT
TGGTGGAAGACATGCAGCGCCAGTG
GAAGCGCCTGGCAGTGTACCAGGCC
AGCAGGCCCAGCAGATACGCCTGCA
GTGCCCAGCGACAATCACTGAACGC
TGGGGCCCCTGGTGGAACAGGGCCG
AAGCGCCTGGCAGTGTACCAGGCCG
GCCCAGCGACAATCACTGAACGCCG
GCGCGCGCGGATGGAGGAGATGGGC
GCGACAATCACTGAACGCCGAAGCC
CCCTGGTGGAACAGGGCCGCGTGCG
AQP1 probes (SEQ ID NO:378-399) 
[?]
CATAAGTCCTTTCAATTCCACCAGG
GCTAGACAATGATTTGGCCAGGCCT
CAGTGCATCACATCTGCACACTCTC
CTGACCTTGGAATCGTCCCTATATC
TGGAATCGTCCCTATATCAGGGCCT
GCAGCCCCTAAGTGCAAACACAGCA
TCTGCATATATGTCTCTTTGGAGTT
GAAGGCTGGATTCTATCTACATAAG
GCCCTTAACTATCACCAGTGCATCA
CACCACTGTGCACTTAGCCATGATG
ACCACGAGGCTGATTCCTCTCATTT
TGCAAAGTGGCAGGGACCGGCAGAG
GCAAACACAGCATGGGTCCAGAAGA
GCATATATGTCTCTTTGGAGTTGGA
AGACGTGGTCTAGACCAGGGCTGCT
ACTTACTGCCTGACCTTGGAATCGT
GGCCTAGTAACCAAGGCCCTGTCTC
GCATGGGTCCAGAAGACGTGGTCTA
GCATCTGTCTGCTCTGCATATATGT
TCTCAGTTTCTGCCTGGGCAATGGC
TTACTGCCTGACCTTGGAATCGTCC
GCAGGAACTTCTAGCTCATTTAACA
SNORA25 probes (SEQ ID NO:400-405) 
[?]
ACTCCTAATGTTTGGTGCTATGTTT
TGGTGCTATGTTTTCCTGAGGAGAT
GAAGCCATAGCACTCCTAATGTTTG
AAGAGGGCTTATGAGGCTGTGAAAC
CCCAGAGCTCTTAACGCTGTGACCA
GAGGCTGTGAAACCCAGAGCTCTTA
RGS5 probes (SEQ ID NO:406-438) 
TGCTCCATTGGAGTAGTCTCCCACC
GGTAGAGGCCTTCTAGGTGAGACAC
 
TACTTATCTACTGTCCGAAGGCCTT
CCTGCATTTCCCATTAATCTACATA
AATGCTGAGAAATTTGCCACTGGAG
TATACAGTTTAATAAGCCTCTTGCA
ATTTAAAATATTGATCCTTCCCTTG
ATCTCACTTGTTTAGTTCTGATCC
ATTTGGGTCCAACTTCAATAATGTA
GACTGTGGGTCAAATGTTTCCATTT
AAATGAAACTGTTGCTCCATTGGAG
GTATCTGTAACCACAATCACACATA
GGACCACCTTCATGTTAGTTGGGTA
TTGCAAGTTACTTGTTCTCTCACCT
CTTTTTGCCCACACTGCTTTGGATA
AGATCACCCCTCTAATTATTTCTGA
TATTTCCTCCATAATAACCCTGCAT
GGGATGTTGCTTACTCTTTTTGCCC
GTACTATGTGACTCATGCTTCTGGA
GTTCTCTCACCTGAGGTATTTTTTT
GCCACTGGAGACAAGCAATCTGAAT
TCATCCTGTGAGTTATTTCCTCCAT
TGCAACTAGCAACTCATCTTCGGAA
CTGCCCATAGTCACCAAATTCTGTT
TGGAAAAGGATTCTCTGCCTCGCTT
GCTAATTGTCCTATGATGCTATTAT
TTCCTCTTCTCCCTTTGCAAGAGGA
ATGACATTTATCTTCAAAACACCAA
GAGTAGTCTCCCACCTAAATATCAA
TTCCCACAGCAGCTTTGCTCAGTGA
CTCGCTTTGTGCGCTCTGAGTTTTA
ATCCATTTGTAAGCATTTATCCCAT
ATGTATTTATGCTGCTAGACTGTGG
MTUS1 probes (SEQ ID NO:439-449) 
TCTTCACCACAGACACCTTCTTGTG
GAGCCTAACACTATCCTGTAATTCA
GTCCCTGTCTATACATTCTCTGTAT
TAACCTTTGTAATGTTCTTCACCAC
ACTCTGCTCAGCCCTGTAACAGGGT
TTTTACTTACCCATGTGAGCCTAAC
TTCATTGCCTTTTTCACCTAAGCAT
TTCTCTGTATCTTTTGGGGGTAACT
AGGAAGAGCTTTGACTTGTCCCTGT
GTTTTTCAGTGTTCAGCCATGTCAG
ATTATGATCATCTACCACCAACTCT
PHB probes (SEQ ID NO:450-460) 
GCAGGGGATGGCCTGATCGAGCTGC
TGAGCGACGACCTTACAGAGCGAGC
GACCTTCGGGAAGGAGTTCACAGAA
GAGTTCACAGAAGCGGTGGAAGCCA
CAGCCCCGATGATTCTTAACACAGC
GCAGGTGAGCGACGACCTTACAGAG
CAGGGGATGGCCTGATCGAGCTGCG
 
GAGCAACAGAAAAGGCGGCCATCA
TCCTGGATGACGTGTCCTTGACACA
TCGGGAAGGAGTTCACAGAAGCGGT
TGGATGACGTGTCCTTGACACATCT
GINS1 probes (SEQ ID NO:461-470) 
TGTTGAACTTGTATCCTTCAGCCTT
TAATATTGAGTCTTCTGGCCTATAA
GGTCTGTCTTCCTAGGTATTAATGT
AGTTTTCAGTGTACAGGTCTACCAT
GCCTTGCTAAACTGTGAGTTCTCAT
GGCCTATAAACAAGGTCTGTCTTCC
GTAGTCACAGTTACACGGCAGGCTG
GTTGGGCACCTTGATTGAGATTGCA
AATTCTAACCACTTGTTGCTAGTAA
AGGTCTACCATGTCAGCATTTCATA
KIAA0101 probes (SEQ ID NO:471-490) 
AATGGTGCCATATTGTCACTCCTTC
ACCAGCCCAGGCAACATAGCGTAAA
GTGTTTGTTCCAATTAGCTTTGTTG
TAGGTTGTCCCCTAAAGATTCTGAA
TGCTTAGATTGTTGTACTGCTGCCA
TTAAACGGTTGATAATGCCTCTACA
TATTCTACCCTCTTTTTGGCAAGG
CAAGTCATTGCATTGTGTTCTAATT
CATAGCGTAAACCCTATCTCTAAAA
AACCTTGGATGGATATCTTCTCTTT
ATTGTTGTACTGCTGCCATTTTTAT
CACAGTGGCTTCTCAGGAGGCTGAG
GGATAGAATCATGGTGGGCACAGTG
TCTCCTTGTTTACCCTGGTATTCTA
AAGTGTCTAGTTCTTGCTAAAATCA
TGGAGAATTCTTTAGGTTGTCCCCT
GGAGGGAGGTTTGCTTGAGTCCAGG
TGGCAAGGAGGACAAATACGCAATG
TCATCTTTGAATAACGTCTCCTTGT
GATAATGCCTCTACAACAACAAGAA
SCD probes (SEQ ID NO:491-501) 
TGAACTTGATACGTCCGTGTGTCCC
GGGCAGTTTTGAGGCATGACTAATG
AAAAGCGAGGTGGCCATGTTATGCT
TAACTATAAGGTGCCTCAGTTTTCC
AGATGCTGTCATTAGTCTATATGGT
GGAATTCTCAAGACCTGAGTATTTT
CTGACCTACCTCAAAGGGCAGTTTT
ACAACGCATTGCCACGGAAACATAC
AGCATTTTGGGATCCTTCAGCACAG
GAAGCTAATTGTACTAATCTGAGAT
ATGTCCACCATGAACTTGATACGTC
PTTG1 probes (SEQ ID NO:502-512) 
CATTCTGTCGACCCTGGATGTTGAA
TTGAGAGTTTTGACCTGCCTGAAGA
AATTGCCACCTGTTTGCTGTGACAT
GTGCCTCTCATGATCCTTGACGAGG
TGCAGTCTCCTTCAAGCATTCTGTC
CCTGCCTCAGATGATGCCTATCCAG
AAAACAGCCAAGCTTTTCTGCCAAA
GGGAATCCAATCTGTTGCAGTCTCC
TGAAGAGCACCAGATTGCGCACCTC
AAGCAAAAAGCTCTGTTCCTGCCTC
TTCCCTTCAATCCTCTAGACTTTGA
CENPF probes (SEQ ID NO:513-523) 
GGTCAAAGTTGCTCAGCGGAGCCCA
TGCACAGAAGTTAGCGCTATCCCCA
TACCCCTGGGAGGTGCCAGTCATTG
GTTTGGAAGCACTGATCACCTGTTA
GAAGGCACTTTGTGTGTCAGTACCC
GATCACCTGTTAGCATTGCCATTCC
GAGCCCAGTAGATTCAGGCACCATC
GTACTCTTTAGATCTCCCATGTGTA
TGAGGGTCAAGCGAGGCCGACTTGT
TTGCCATTCCTCTACTGCAATGTAA
CGAAATCCGTCCCAGTCAATAATCT
SMC4 probes (SEQ ID NO:524-544) 
GGACAGTGTTTCAACAAGCCTAGGC
GCATCTAAGGGACTTTGTTGAACTT
GATGGCCTCTGATTTACACTGGTTC
AGAAGTCTGCCCTAGCTGTTAAATT
GAGTTAATTGTTCCTTTCTTCAGTG
TAGACAGCTTGGATCCTTTCTCTGA
GGTTTACCAGGATGTAGTCCCACTG
GAAAACACTTAGTTCATTGGCTTTA
GCGTATTTTTACACTATTGGCTCAA
ATTTACACAGCTAGATTTGGAAGAT
GGATGAGATTGATGCAGCCCTTGAT
ATTGATGCAGCCCTTGATTTTAAAA
AGTTCATAATAATTTCTCTTCGAAA
GGAAGGACTTTCGGTATTGTATTAG
CCTTTCTTCAGTGGGCCATTGTTTT
TTAGTATTTGCTCTTCACCACTACA
GATACCTTGAGTAATGTTTGCCTAT
CACTCCCCTTTACTTCATGGATGAG
AAGCCTAGGCTATCTCGTAAGTTGA
GGACGCCGAACTCGAGCTTGTAGAC
AATATCCCACTATAGTTGCTTCATG
NCAPG probes (SEQ ID NO:545-555) 
CCCAATTTCTCAATGAAGATCTAAG
GATTATGTCCAGTTATTTGCTTTAA
GGTGGAATCCTTTAAGATTATGTCC
AAGACGATGGAGGTGGAATCCTTTA
 
GAGCCAAAACCGCAGCACTAGAAAA
GAGACTACCAAGACGAGCCAAAACC
TTCCAGAACCAGAATCAGAAATGAA
CCAAGACGAGCCAAAACCGCAGCAC
GGACGAACAGGAGGTGTCAGACTGC
GAAGATGAGACTACCAAGACGAGCC
GTGTCAGACTGCTGAAGCCGACTCT
PDLIM5 probes (SEQ ID NO:556-566) 
CTTGCTTTGTATGCTCAGTGTGTTG
GCCCTCTTTGGTACTATATGCCATG
ACACCTGGCATGACACTTGCTTTGT
GAATTTCCCATAGAAGCTGGTGACA
GAAGCTGGTGACATGTTCCTGGAAG
CAAGAAGGACAAGCCCCTGTGTAAG
TGACATGTTCCTGGAAGCTCTGGGC
ATATGCCATGGATGTGAATTTCCCA
GCCCCTGTGTAAGAAACATGCTCAT
GGAAGGTCAGACCTTTTTCTCCAAG
TTATTATGCCCTCTTTGGTACTATA
SOX9 probes (SEQ ID NO:567-588) 
GAGAGGACCAACCAGAATTCCCTTT
AAGCATGTGTCATCCATATTTCTCT
CTACCTGGAGGGGATCAGCCCACTG
AGTTGAACAGTGTGCCCTAGCTTTT
GGAGAATCGTGTGATCAGTGTGCTA
GTAGTGTATCACTGAGTCATTTGCA
TGGGCTGCCTTATATTGTGTGTGTG
TGTTTTCTGCCACAGACCTTTGGGC
TGTTCTCTCCGTGAAACTTACCTTT
AAATGCTCTTATTTTTCCAACAGCT
CCTAGCTTTTCTTGCAACCAGAGTA
GAATTCCCTTTGGACATTTGTGTTT
GCCAACCTTGGCTAAATGGAGCAGC
ATTACTGCTGTGGCTAGAGAGTTTG
TTGGAGTGAGGGAGGCTACCTGGAG
ATATGGCATCCTTCAATTTCTGTAT
CAGCCCACTGACAGACCTTAATCTT
ATCAGTGGCCAGGCCAACCTTGGCT
TTTTCCAACAGCTAAACTACTCTTA
GCAACTCGTACCCAAATTTCCAAGA
ACATGACCTATCCAAGCGCATTACC
GTAAAAGCTTTGGTTTGTGTTCGTG
PBX2 probes (SEQ ID NO:589-599) 
TAGTTCTCTCCTCACTTGTAAACTT
GTATATGTATCTTCCTCAATTTCCC
GGAGGCAGTGAAGGGCTTGCCCTGC
CATCTTCCCCTGTGAGTGACATGTC
AGGTTGGAAGTGTGATGGGTGGGGG
GGTATCTTTTTGTCACACCAAAATC
CCCCTCCCATTAAAGATCCGGGCAG
 
AAAGTAACATCAACACTGTCCCATC
GATCCCCTCAGACATTCTCAGGATT
GACTGTCAGAGTGGGGAACCCCTCC
GGGTTGGGGTGCTTGTATATGTATC
PLIN2 probes (SEQ ID NO:600-610) 
TATGTTCTCATTCTATGGCCATTGT
GAGTCTCAGAATGCTCAGGACCAAG
TGTGGCCAGACAGATGACACCTTTT
GTCTGCTCTGGTGTGATCTGAAAAG
GCTTTATCTCATGATGCTTGCTTGT
GGGGTAGAAACTGGTGTCTGCTCTG
CAGGAGACCCAGCGATCTGAGCATA
GAAAAGGCGTCTTCACTGCTTTATC
TATGGCCATTGTGTTGCCTCTGTTA
ATCACTAGTGCATGCTGTGGCCAGA
AACATCTTCATGTGGGCTGGGGTAG
LMO4 probes (SEQ ID NO:611-621) 
CCCTTCCCGCATTTATTGGTGTATT
ACCTTTGTAGCTAGCACCAGTGCCA
TTCATCTCAGATTTGTTCATCACAG
GTCTTCAGTAGACAAGTCACCTTTG
TTAAGGACTCCATGAACCTGGGCTA
TAATGTTGCTACTCCCATGGCAAAG
GTTTTTGTCCTAATGTTGCTACTCC
CAGAGGACATCTTGGGGAGGGGGAG
CACCTTCTTTAGTCTTGATTGCCCT
CCATTGCACCTTCTTTAGTCTTGAT
GATGTGGCTTTTGTGATATTCTATC
PIK3R1 probes (SEQ ID NO:622-632) 
CACGGTCAGTTGTAACTTTGCCTTC
GACTATCCAACTTAACATGAAACTT
GAGATAGCATTAGCTGCCCAGGATG
AATGGAGCTATGTCTTGTTTTAAGT
GAGAGGGAGGATGTCACGGTCAGTT
AGTTGGTCTTTTGACGAGAGGGAGG
GTGCCTCCTTGACATTTCGTTCAAG
GAAACTTGTCACCATGAGATAGCAT
AAAGCTACAATCTGTTCAATGTTTT
CTGCCCAGGATGCTGCTATATATAT
AAAAACTCATTTATACCTGTGTATT
DHX9 probes (SEQ ID NO:633-643) 
TGACCGAGCAGCAGAGTGTAACATC
GTAACATCGTAGTAACTCAGCCCAG
ATCAGTGCGGTTTCTGTGGCAGAGC
GACTTTATCCAGAATGACCGAGCAG
TAGAGGGGCTACTGGATGTGGGAAA
CTCAGCCCAGAAGAATCAGTGCGGT
GGCTTATCCTGAAGTTCGCATTGTT
TGGGAAAACCACACAGGTTCCCCAG
 
TGTACTGTAGGTGTGCTCCTGAGAA
GCGTGATGTTGTTCAGGCTTATCCT
GGAGGACTTACCCAGTTCAAGAATA
CD44 probes (SEQ ID NO:644-654) 
GGATGGCTTCTAACAAAAACTACAC
GTGTGCTATGGATGGCTTCTAACAA
TAGTTACACATCTTCAACAGACCCC
AGGGTGAAGCTATTTATCTGTAGTA
TTAGGGCCCAATTAATAATCAGCAA
CTTCCATAGCCTAATCCCTGGGCAT
CACATATGTATTCCTGATCGCCAAC
CAGACCCCCTCTAGAAATTTTTCAG
TTGAATGGGTCCATTTTGCCCTTCC
CAGGGTTAATAGGGCCTGGTCCCTG
TTAAACCCTGGATCAGTCCTTTGAT
RRM2 probes (SEQ ID NO:655-671) 
GTATTCAGTATTTGAACGTCGTCCT
GTCTTGCATTGTGAGGTACAGGCGG
TTTTACCTTGGATGCTGACTTCTAA
GTACAGGCGGAAGTTGGAATCAGGT
GACCCTTTAGTGAGCTTAGCACAGC
CCTGGCTGGCTGTGACTTACCATAG
GAACGTCGTCCTGTTTATTGTTAGT
CTCACAACCAGTCCTGTCTGTTTAT
GAAGTGTTACCAACTAGCCACACCA
ATGTGAGGATTAACTTCTGCCAGCT
CTAGCCACACCATGAATTGTCCGTA
CAGCCTCACTGCTTCAACGCAGATT
TTAGGATTCTGTCTCTCATTAGCTG
GTGCTGGTAGTATCACCTTTTGCCA
TATGGTCCTTATATGTGTACAACAT
GAAGATGTGCCCTTACTTGGCTGAT
TAAACAGTCCTTTAACCAGCACAGC
CDK1 probes (SEQ ID NO:672-682) 
TGAAGTATTTTTATGCTCTGAATGT
CAAAGATCAAGGGCTGTCCGCAACA
GATGAATATTTTTCTACTGGTATTT
GACATAGTGTTTATTAGCAGCCATC
GAAAGCTTTTTGTCTAAGTGAATTC
GTGAATTCTTATGCCTTGGTCAGAG
TGTTAACTATACAACCTGGCTAAAG
AAATGTTCTCATCAGTTTCTTGCCA
TGCTAAGTTCAAGTTTCGTAATGCT
AAGGGCTGTCCGCAACAGGGAAGAA
CTTATCTTGGCTTTCGAGTCTGAGT
HN1 probes (SEQ ID NO:683691) 
GGCCTCTAATATCTTTGGGACACCT
GAAGGAACTCCTCTGAAGCAAGCTC
GACTTGGAGTCATCTGGACTGCAGA
 
AGAGTGAAGAGAAGCCCGTGCCTGC
GACCCCAACAGCAGGAATAGCTCCC
GTGGTGGATCCAATTTTTCATTAGG
CATCCAGAAGAAATCCCCCTGGCGG
ACAACCACCACCTTCAAGGGAGTCG
GCAAGCTCCGGAGACTTCTTAGATC
ZWINT probes (SEQ ID NO:692-702) 
GATGTACCTTTTTTGTCAACTCTTA
TAGTGATACCTTGATCTTTCCCACT
GTTTCATTGACCTCTAGTGATACCT
GTACAGCCTAGTGTTAACATTCTTG
GATTGGCTTTTGTCATCCACTATTG
AACATTTCTCGATCACTGGTTTCAG
TTTCCCACTTTCTGTTTTCGGATTG
GGCCTCCTATGATGCAGACATGGTG
TCTTGGTATCTTTTTGTGCCTTATC
AGGAGCTGGGACTGGTTTGAACACA
CAGATGGGGAGGGGGTACTGGCCTT
ASPM probes (SEQ ID NO:703-713) 
ATAGAGCCTCTGATGTACGAAGTAG
CAGTCTCTACAAACTTACAGCTCAT
AATCCCCTGCAAGCTATTCAAATGG
GAAGAAATCACAAATCCCCTGCAAG
GTGATGGATACGCTTGGCATTCCTT
GCATTCCTTTTATCCCAGAAACACC
GTTGTTTGTTGGCTATTTTACTGAA
GGAGCTTTTGCAGATATACCGAGAA
TCAGATATGCTGTGCAAGTCTTGCT
GTTGTTGACCGTATTTACAGTCTCT
GTTGTAATCGCAGTATTCCTTGTAT
SLC35E3 probes (SEQ ID NO:714-723) 
AGTAGCTCTCTGCTTGCTGATAGAT
ATTTTAGTTTAGCTTCCTGATTTAT
GATGGTTTCCCAGTGTGAGATTTGT
ATGGTTTGGTTGGTCCCAGCAAAGT
GTTCTCGGTGTTCAAACTCTTCTAA
TGCAAATATGCTGTGGGTTCTCGGT
AAGGAATTGCTGTTACTGTACTGCA
TAAATCTAGTGTTTCTATTTTAGTT
ATATACCATCCCATATATATGTGGG
CTGCTTGCTGATAGATGGTTTCCCA
RNASEH2A probe (SEQ ID NO:724-731) 
ATGCCAGAGACATACCAGGCGCAGC
GGAAGATCACATCCTACTTCCTCAA
ACTGATTATGGCTCAGGCTACCCCA
TGCAGCAAAGTTTTCCCGGGATTGA
CTCAATGAAGGGTCCCAAGCCCGTC
TCGTGGACACCGTAGGGATGCCAGA
GAGGACTCAGCATCCGAGAATCAGG
 TCTTCCCACCGATATTTCCTGGAAC
TSC2 probes (SEQ ID NO:732-738) 
TGGCCTCACAGGTGCATCATAGCCG
GGGCAACGACTTTGTGTCCATTGTC
CCCTGATGCCCACCAAGGACGTGGA
AGCACCGCTGCGACAAGAAGCGCCA
TCAACTTTGTCCACGTGATCGTCAC
GTGAGGACTTCAAGCTTGGCACCAT
TGGACTACGAGTGCAACCTGGTGTC
FAM20B probes (SEQ ID NO:739-749) 
GTATTAATAAGGCATTGCCCCCTGT
TGTAAGGCTGCATTGTGGGTTTGGG
GTTTTGTAACACTGTCCTACTTTAT
AGTCGTTGCAGGGTTTGGATCAGCT
GGATCAGCTGTAAGTTAGGTATGCC
AAGGCTGTTACAATCAAGTCGTTGC
AGATGAGTCCTATACGTGGCAATTT
TCTGAAGCCAGCATTATCTTCCAAA
GCCCCCTGTTTGCACTCAGGGTTAA
CGTGGCAATTTTTCAATGTCATCTG
TCAGAACTCATGGCCATTTCCTGCC
WASL probes (SEQ ID NO:750-760) 
GAGATACTTGTCAAGTTGCTCTTAA
TGGGCCGTCGACAAAGGAAATCTGA
AATTTCGAAAAGCAGTTACAGACCT
AATCTACCCATGGCTACAGTTGATA
GTGGGAACAAGAGCTATACAATAAC
TAGTCCTAGAGGATATTTTCATACC
GATCCCCCAAATGGTCCTAATCTAC
AGAAAAGACGAGATCCCCCAAATGG
TACCCATGGCTACAGTTGATATAAA
TTCATACCTTTGCTGGAGATACTTG
ATACCTTTGCTGGAGATACTTGTCA
AIM1 probes (SEQ ID NO:761-771) 
GCCTGTGCTGAACTGATCTCTTAAA
AATACTGGTGCTCTTGTCACAGGTA
AAAATGCTGATCTTCTCTGGAGTCT
TGCTCTTCCAACAGTGGGTTCTAGC
ACAACTGACAAGACACCAGCCCATA
TTAGGCCTTTTGTGCATACCATTAC
GGTGCATGTACAACAGCATCCAACA
TCTTTTTGTCCTCATCACTCAATAC
GCAATCTTGGAATCCTCAACTGCAG
GGTAGAACAGCTTGTTTCTTTTCCA
GCATCCAACATATCTGTCTTGTTCC
MBNL2 probes (SEQ ID NO:772-782) 
TTCAGCCCTTTAATAATGGAGCATC
TTTACTATGATATCCATTTTCCAGA
 
GAGACTAACTCTCCACTTGTATGGG
GGGAACTACATTTCACTCTTGGTTT
ACCTGTAACCCCAAGCAAATATAGA
AACTCTCCACTTGTATGGGAACTAC
TCAGGATATAACAGCACTTCACCGA
ATTTCACTCTTGGTTTTCAGGATAT
TAACAGCACTTCACCGAAATATTCT
TATTAGCACACAACTATTTTCAGCC
AAGTTTGTTTATATTCAGAAGTCTG
PPIF probes SEQ ID NO:783-793)) 
GCTGAAGGCAGATGTCGTCCCAAAG
TGGCAAGCATGTTGTGTTCGGTCAC
GCCTGAAACGATACGTGTGCCCACT
TAATGCTGGTCCTAACACCAACGGC
CCGCTTTCCTGACGAGAACTTTACA
GTGGCCAGGGTGCTGGCATGGTGGC
AGAAGGGCTTCGGCTACAAAGGCTC
AAGTCCATCTACGGAAGCCGCTTTC
TGTCCTGTCCATGGCTAATGCTGGT
GTTCGGTCACGTCAAAGAGGGCATG
GACTGTGGCCAGTTGAGCTAATCTG
GINS2 probes (SEQ ID NO:794-803) 
TACAGCAGGAGTGGCCATGTGGTCC
GATGAGGTACTCGTGGTTCTGGAGC
GGCAGATGGTGCAGCCAACAATGCT
AACAATGCTGACCGGTGCTTATCCT
GGACATTCTTCAATTCCACATCTGT
GGGCTGAATTTAGACTCTCTCACAG
CAGCCTCTGGAGAGTACTCAGTCTC
AAATAAGTCATTCTCCCTAGCAGAG
CTCTAAGCCCTGATCCACAATAAAA
GGAGCTCTAGAAACACTTCTGATGC
UBE2C probes (SEQ ID NO:804-814) 
TATAAGCTCTCGCTAGAGTTCCCCA
GAGCTCTGGAAAAACCCCACAGCTT
GGAAAAGTGGTCTGCCCTGTATGAT
GGGTAACATATGCCTGGACATCCTG
CAATGCGCCCACAGTGAAGTTCCTC
GGGTAGGGACCATCCATGGAGCAGC
GCCTTCCCTGAATCAGACAACCTTT
TCCATCCAGAGCCTTCTAGGAGAAC
ATGATGTCAGGACCATTCTGCTCTC
AGATGGTCTGTCCTTTTTGTGATTT
TGATAGTCCCTTGAACACACATGCT
TYMS probes (SEQ ID NO:815-825) 
GAGGGTATCTGACAATGCTGAGGTT
GCATTTCAATCCCACGTACTTATAA
ATCTGTCCGTGACCTATCAGTTATT
GTACAATCCGCATCCAACTATTAAA
 
AAATGGCTGTTTAGGGTGCTTTCAA
TCACAAGCTATTCCCTCAAATCTGA
AACTGTGCCAGTTCTTTCCATAATA
GAGGGAGCTGAGTAACACCATCGAT
GGGGTTGGGCTGGATGCCGAGGTAA
AAAGCTCAGGATTCTTCGAAAAGTT
GAACTAGGTCAAAAATCTGTCCGTG
NEK2 probes (SEQ ID NO:826-837) 
ATTAATACCATGACATCTTGCTTAT
GCTGTAGTGTTGAATACTTGGCCCC
GCCATGCCTTTCTGTATAGTACACA
TGAGCTGTCTGTCATTTACCTACTT
GTAGCACTCACTGAATAGTTTTAAA
TTGGTTGGGCTTTTAATCCTGTGTG
CTCTGTAGTTCAAATCTGTTAGCTT
GATATTTCGGAATTGGTTTTACTGT
AAATATTCCATTGCTCTGTAGTTCA
TGAATACTTGGCCCCATGAGCCATG
GGTATGCTTACAATTGTCATGTCTA
TXNRD1 probes (SEQ ID NO:839-844) 
ACCTGTATTTCTCAGTTGCAGCACT
CCCATGCATCTGCCTGGCATTTAGG
GCAATTGAGGCAGTTGACCATATTC
TCCTCATCTCATTTGGCTGTGTAAA
CCTGCCAGCAGTTCTTGAAGCTTCT
TCCAAGTCCACCAGTCTCTGAAATT
TGGCATTTAGGCAGCAGAGCCCCTG
GGAGTGGAATGTTCTATCCCCACAA
MED24 probes (SEQ ID NO:845-854) 
CAGCCCAGGAGTAGTCTTACCTCTG
CTTACCTCTGAGGAACTTTCTAGAT
GGCTGCTAAAGCCATTGCTGCACTC
GATGAGGCATCGTGCCTCACATCCG
TAAAGCCATTGCTGCACTCTGAGGG
GTGAGGGAAATCTACCTTCGTTCAT
TGGTGCAAGAGCCTCTAGCGGCTTC
TTCTTCTTTCAAAATTTCCTCTCCA
AACTGGTGAAGGTGTCAGCCATGTC
CATCCGCTCCACATGGTGCAAGAGC
ELF1 probes (SEQ ID NO:855-865) 
AGATGACATGGTTGTTGCCCCAGTC
AAATATGCAGACTCACCGGGAGCCT
CATGTTCCTGGTGCTGATATTCTCA
TTATGCCGGTCTAGCCTGTGTGGAA
TCACCCATGTGTCCGTCACATTAGA
GATATTCTCAATAGTTATGCCGGTC
GATGAACGACAGCTTGGTGATCCAG
TTGGTGATCCAGCTATTTTTCCTGC
GGCACTCCTCAATATGGATTCCCCT
 
GCTGCCTGATACGTGAATCTTCTTG
GACATCACCCTTACAGTTGAAGCTT
APH1A probes (SEQ ID NO:866-876) 
GTGCATGTTTGGGAACTGGCATTAC
TTCTCAGTACTCCCTCAAGACTGGA
ACTCCAGAGCTGCAGTGCCACTGGA
GTGCCACTGGAGGAGTCAGACTACC
ATAGATGAGCTCTGAGTTTCTCAGT
GTCAGACTACCATGACATCGTAGGG
TCTTCTAACCTCCTTGGGCTATATT
CAGGCCTGAGGGGGAACCATTTTTG
GGACATCTTGGTCTTTTTCTCAGGC
TGCTGAGGGTGGAGTGTCCCATCCT
GAGGTATATTGGAACTCTTCTAACC
SLC11A2 probes (SEQ ID NO:877-887) 
ACTGACCATACATTTTTCTTAGCCC
GACATTTTTACATACCGAGCCTGAG
TTCATCTGAGCCCCCAAAAGCATTA
TTCTTAGCCCCTCAAGTAATATAGC
AAACTGGTCATAAAGGCACTCTGTG
CCTTCCAGAGTCCTGGCTGATTGGT
GGGACTGACATCTTAAGCTCTCACC
TACACTACTGTGTTTCACTGACCAT
TGATTGGTGTTCGCTGTTCATCTGA
GGACTTCTCATTTTTGGAGCTTTCC
GAATGACAATTCCCCTAACCATTCC
CCNB1 probes (SEQ ID NO:888-898) 
TTTGCACTTCCTTCGGAGAGCATCT
CTTCCAGTTATGCAGCACCTGGCTA
CAACATTACCTGTCATATACTGAAG
TGGACACCAACTCTACAACATTACC
TGGCACCATGTGCCATCTGTACATA
ACTATGACATGGTGCACTTTCCTCC
GGAACTAACTATGTTGGACTATGAC
GAATCTCTTCTTCCAGTTATGCAGC
GCACCTGGCTAAGAATGTAGTCATG
TCCTCCTTCTCAAATTGCAGCAGGA
AGATTGGAGAGGTTGATGTCGAGCA
TMEM97 probes (SEQ ID NO:899-909) 
ATTCCATCAGCTTTCTCTAAGTCTT
ATAGAGGGTCTCTTCACGTTGATGC
ATCCACTGTGTGCATAGAGGGTCTC
TCAGCTTTCTCTAAGTCTTTGCTCA
AAGTTCAACCTTAAAATGATGTTAG
TCTCTTCACGTTGATGCTTGGCATT
GCCAGGCATAACATATCCACTGTGT
TCACGTTGATGCTTGGCATTCCATC
GTGTGCATAGAGGGTCTCTTCACGT
TACAGCCAGGCATAACATATCCACT
 CCATCAGCTTTCTCTAAGTCTTTGC
MLF1IP probes (SEQ ID NO:910-920) 
AGGCCATAATCATCTTTTCTGGTTA
AAATAGCATCAGTTTGTCCAATAGT
ATGTTGACACCTTAATCGGTCCCAG
GAAGCTCCTTGACCAGGGATGAGAA
CAACCATCAGTTAGAGAAGCTCCTT
AAGAACACTTCTGGGAGCCGAAAGC
GTGCCTATAGGAAGACTAGTCTCAT
GCCATCTGCGAAATATCAACCATCA
AAACGTATGATTCATCCAGCCTTCC
ATCGGTCCCAGGTATGAGCTATAAT
GGAGCCGAAAGCCATCTGCGAAATA
ECT2 probes (SEQ ID NO:921-931) 
AGACTGTTTGTACCCTTCATGAAAT
TCACAATAGCCTTTTTATAGTCAGT
GTAAATGACTCTTTGCTACATTTTA
GCATGTTCAACTTTTTATTGTGGTC
GGTAATTTTATCCACTAGCAAATCT
GCATAGATATGCGCATGTTCAACTT
GAAGTTGCCATCAGTTTTACTAATC
CAGTTTTACTAATCTTCTGTGAAAT
TAGCTGTTTCAGAGAGAGTACGGTA
TTCCTATTTCTTTAGGGAGTGCTAC
GTATGTGCCACTTCTGAGAGTAGTA
RAE1 probes (SEQ ID NO:932-942) 
AGCCTTGTTGGGTTGTCAGCCATGG
AACAGTTAGATCAGCCCATCTCAGC
ATGGCACCCTTGCAACTGTGGGATC
AGAAGTAGTGGCTGGAGACTCTGGC
CTGCCTCATCTCTGTACGAATTTGG
GATGGTAGATTCAGCTTCTGGGACA
CTCAGCTTGCTGTTTCAATCACAAT
AATGGAATCGCGTTCCATCCTGTTC
TGGATTTCAACCCCTGGAGAAAACG
ACGAATTTGGGTCCCAGCCTTGTTG
CATATTTGCATACGCTTCCAGCTAC
DONSON probes (SEQ ID NO:943-949) 
GGAAATCACATAGCAGTTACCCCAT
GTGGTGCTGAGAGACTACATTTATA
ATACCGTTACTTGGGAAATCATCTT
CAACTGCTGTATTTAACATCTGCCT
GATATCTATATCCCTACAACCTAAT
TAACTGTGGTTTGCACCCTAACACT
GTTACCCCATGGCATTGTGACTAAT
KIAA0776 probes (SEQ ID NO:950-960) 
CCACCTCATACACACACAATTCAGT
GTCATATAGTAAGCATTTTCCCCCA
 
CATATTTCAGGTTTGTTCTCTTTCC
AAAGTAGTCACTATACAACTCCCCT
AAGACCTTGTTCTCAAATCTAGGAA
GAAGGATTCATTTGTTGCGAGTGCC
GTTGCGAGTGCCAGTATACTTAAAT
TTTTCCAATCCATTTATCCCTGGGG
CCTGATTTTCACACAAATACTATAT
GTAAATTGGGTTCTTCATGGAAGTT
GGAAATCATCTGTGACGGAAGAGTA
>DTL_probe1 (SEQ ID NO:961) 
ATGATTTTGTTTGTATCCCTACCCA
DTL probes  (SEQ ID NO:962-971)
GGAAGCCATAGAATTGCTCTGGTCA
AGCACACCATAGCCTTAACTGAATA
TGGGTGCCAAAGGTCAACTGTAATG
GACCAATATCTGCCAGTAACGCTGT
AATTGGGATACATTTGGCTGTCAGA
TAACGCTGTTTATCTCACTTGCTTT
GACAACTTTTTAATTCCTTTGATCT
GTCTACTGGGTATAACATGTCTCAC
GGAAATCTGCCTAATCTGCTTATAT
GCTCTGGTCAAAACCAAGCACACCA
SQLE probes (SEQ ID NO:972-982) 
GATTCCCTGCATCAACTAAGAAAAG
TTGGTGGCGAATGTGTTGCGGGTCC
GCGTGTTCTGTAATATTTCCTCTAA
TCTCCTAACCCTCTAGTTTTAATTG
TCTCAGTAGTGGTGCTGTATTGTAC
AATTGGACACTTCTTTGCTGTTGCA
CTTGGATTACAAAACCTCGAGCCCT
CTGTTGGGCTGCTTTCTGTATTGTC
ATCTATGCCGTGTATTTTTGCTTTA
TTGCTGTTGCAATCTATGCCGTGTA
ATAGCATAGTACCATACCACTTATA
ACBD3 probes (SEQ ID NO:983-993) 
GAACGCAGAGCGACTCGAGGTGTCC
GGCAAAGCATTTCATCCAACTTATG
GCCTGGAGGAGTTGTACGGCCTGGC
GCAGCAGCCGGAGATGGCGGCGGTG
TTAAATAGGTGTTGCCATCTCTTTT
GACACTTGTCCTGAGGTTGGATTCT
GGCAGCCCTGGGAAACATGTCTAAA
TCAACATATGTTGCGTCCCACAAAA
TGGCACTGCGCTTCTTCAAAGAAAA
TAAGCAAGTTCTTATGGGCCCATAT
GGCCCATATAATCCAGACACTTGTC
RMI1 probes (SEQ ID NO:994-1004) 
CCCTGAACATGCCTGAGCTTGTCAT
TCCCTTTCTGAATTAGCTGTACATA
 
GCATTTATCTATGTCTTTAGGTGTC
GGTAATTTCCTTCTAATATGTTGGT
TACCATTCTTCCACTGTGCTGTTAT
GTAGATCAGAACATCAGGCTTTCAG
ATGCCTGAGCTTGTCATAATATGTT
ATGTTGGTACTGTCTATGGCCATAC
TTAGGTGTCATTGTTCCCTTTCTGA
AGAGGGACTGTTTACCATTCTTCCA
GCTGTACATATAAGCCTTCCTTTGG
C14orf101 probes (SEQ ID NO:1005-1015) 
CAGAAAGCACCGAATGACCCACAGC
TTCCGTCTGTACTCTCAGAAAGCAC
GAAGTGCTGTTATCGGAAACCATCA
GACTTCCAGTCTTTCACCAGATGAC
GTATCATGGTCCAGCAGTACTGTTT
TATCCCGTGTTACCAAATTACCATT
GAAACCATCAGACATTTCCGTCTGT
ATGAAAAACCTGCTCATCGTTCAGC
TTAAAACTAAGTCATCTCCCAGATA
GCAAAATTCTGTTTATCCCGTGTTA
CTCATCGTTCAGCTTCCAAAATTCT
ZNF274 probes (SEQ ID NO:1016-1026) 
CCTTTTCAGCTTGACCCTGCAATAT
AATCTGCACTGATATTACATCCACA
ACCTCATAGCTCTCAAGCCAGTTGA
AGGAGACTGCCCAGCACATAATGAA
GATATTGTTTGTTCACTCATTTAGT
ACATGCACAGGCCTGCTTGTGAATC
GCCAGTTGAAGAAACCTTGCCTTTT
AAGATTTCCCATTCACTTGATATTG
TACATCCACAGTACCACAGTATTTA
GAAGAAACAGCCTACCTCATAGCTC
GAGCGCCCATATGCATGCAACAAAT
PTGES probes (SEQ ID NO:1027-1048) 
TGGATGTCTTTGCTGCAGTCTTCTC
GGTCTTGGGTTCCTGTATGGTGGAA
CAAAGGAACTTTCTGGTCCCTTCAG
TTGGCCACCAGACCATGGGCCAAGA
CAAAGGGCAGTGGGTGGAGGACCGG
TCTCCTAGACCCGTGACCTGAGATG
CGTGGCTATACCTGGGGACTTGATG
CAGCCACTCAAAGGAACTTTCTGGT
GGTTTGGAAACTGCAAATGTCCCCT
AGGTTTGAGTCCCTCCAAAGGGCAG
GGCCCACCGGAACGACATGGAGACC
TCTCTGGGCACAGTGGGCCTGTGTG
CTCTGGGCACAGTGGGCCTGTGTGT
TTTGGATGTCTTTGCTGCAGTCTTC
ACCTGGGGACTTGATGTTCCTTCCA
GGCTATACCTGGGGACTTGATGTTC
 
CTCCTAGACCCGTGACCTGAGATGT
TGGAGGACCGGGAGCTTTGGGTGAC
CACCAGACCATGGGCCAAGAGCCGC
GCAGTGGGTGGAGGACCGGGAGCTT
TTTCTGGTCCCTTCAGTATCTTCAA
CACCGGAACGACATGGAGACCATCT
FRG1 probes (SEQ ID NO:1049-1053) 
GGCTCGGAAAGATGGATTTTTGCAT
GCAGTTTTCGGCTGTCAAATTATCT
GGGCGTTCAGATGCAATTGGACCAA
TAGTCCTCCAGAGCAGTTTTCGGCT
ATTGCCCTGAAGTCTGGCTATGGAA
C19orf60 probes (SEQ ID NO:1054-1069) 
GCACGGTGGCCCTGCTGCAGTTGAT
GGCGATCAGCGAGGTTCTCCAGGAC
GGACCTTAGGTTTGATGCGGAATCT
GGTTCTCCAGGACCTTAGGTTTGAT
CAGCGAGGTTCTCCAGGACCTTAGG
AATTAAAACCATGGAGGCGATCAGC
CCTTAGGTTTGATGCGGAATCTGCC
CGCTGTACGGATGCAGCAGCTGAAA
TCTGCCGAGTGATGGCGGCTCCCCA
CATGGAGGCGATCAGCGAGGTTCTC
GTTTGATGCGGAATCTGCCGAGTGA
ACTGCGCTGCTGACCTTCCTGCAGT
ATTAAAACCATGGAGGCGATCAGCG
CCAGGACCTTAGGTTTGATGCGGAA
AGTGCACGGGGTGACCCAGGCCTTC
ATCTGCCGAGTGATGGCGGCTCCCC
LPCAT1 probes (SEQ ID NO:1070-1080) 
TGTGTGTGAGACAGGACGCAGCGGG
CAGACCCGTGGGCAGGTGGGGCATG
GTTGAGTTAAACCCCTTGTGTGTGA
TCCCTTCCGCAGGTCTGCAGATGAA
AATTTCAGGGCTCTTGGCGTGTTGG
TGAAATGCCACTGCGCATTTTCAGA
TCTTTTCTCTTCGTGGCGACTTAGA
GCCTTTGGTAGCTAACAGTCACTGA
AGAAATCCTAGTGCAGCCTTTGGTA
TGAATGGATGTTTGTTCCTCCTGAT
GAGTTGGCGGATATTCGGAACTGTG
ISYNA1 probes (SEQ ID NO:1081-1091) 
TACCTCGGAGCTGATGCTGGGCGGA
ACCAATGGCTGCACCGGTGATGCCA
CAACACGTGTGAGGACTCGCTGCTG
GCCTCAAGCGAGTTGGACCCGTGGC
GCCACCTACCCTATGTTGAACAAGA
GGAACCAACACACTGGTGCTGCACA
GTGAGCTTCTGCACTGACATGGACC
 
AACCACATGCTCCTGGAACACAAAA
GGGCATCTGCAAGAGGAGCCCCCAA
CAAAATGGAGCGCCCAGGGCCCAGC
CCAGCGCAGCTGCATCGAGAACATC
SKP2 probes (SEQ ID NO:1092-1102) 
AAATTGATGACTTGTTCGTATGTTC
GAAGTGCCTTTATCTGCTTAGACCT
TGCCCTCAAACATACAGAACTTCCA
CTCTGACATCGGATGCCCTCAAACA
AGCTATTTTGCCAACATGTCAGAGT
AGAACTTCCAAACTCAAGTCCAGCC
AAGTCCAGCCATAAGCTATTTTGCC
AGAGCTGGGGTTAGGATCCGGTTGG
TAGGATCCGGTTGGACTCTGACATC
AAAGCTAACACCAGTCATTTATATT
GATGATGCTTCAATTTCTTAATAGT
DPP3 probes (SEQ ID NO:1103-1113) 
AAACGTTCTCACCAAATCCAATGCT
ATACGAGGCGTCAGCTGCTGGCCTC
AGGAGCTTGGACCTTGGTACTACCT
GATGCCCGATTCTGGAAGGGCCCCA
CAGACCAAGGCTGCAAGTGGCCCTC
GCTTACCATCCTGTCTACCAGATGA
CTCTGTGATCTCATTTCATCTGCAC
GTGGCACGTGACAGCTAGGGTTCAA
TGAGCGTTTCCCAGAGGATGGACCC
TGAGGGTGGTGACACAACCCCTTCC
TCATCTGCACTGCCATACGTGGAGT
TYMP probes (SEQ ID NO:1114-1124) 
CCTGTGCTCGGGAAGTCCCGCAGAA
CCTTGGCCGCTTCGAGCGGATGCTG
CCGCTTCGAGCGGATGCTGGCGGCG
TGGCCCGAGCCCTGTGCTCGGGAAG
CCGAGCCCTGTGCTCGGGAAGTCCC
TGCTCGGGAAGTCCCGCAGAACGCC
CTGCTGGTCGACGTGGGTCAGAGGC
CTGGTCGACGTGGGTCAGAGGCTGC
GCCCGCCAGACTTAAGGGACCTGGT
CAGGCCCGCCAGACTTAAGGGACCT
ACTTAAGGGACCTGGTCACCACGCT
SNRPA1 probes (SEQ ID NO:1125-1135) 
GGTTGCTGCAGTCTGGTCAGATCCC
AGCTGACGGCGGAGCTGATCGAGCA
GTGGGCCATCTCCAGGGGATGTAGA
GTCAGATCCCTGGCAGAGAACGCAG
TAGCAAATGCTTCAACTCTGGCTGA
TGATCGAGCAGGCGGCGCAGTACAC
AAGGTTCCGCAAGTCAGAGTACTGG
TGGCATCTCTCAAATCGCTGACTTA
 
TCCAGGTGCTGGTTTGCCAACTGAC
TCCGGGGGTGATCTGAACCCTCTGG
AACGCAGATCAGGGCCCACTGATGA
DHCR7 probes (SEQ ID NO:1136-1146) 
TCTCCAGCGAGGAGGTCTCAGTCCC
GCGTGCACGGTGTTGAACTGGGACA
CTATGCTCCGAGTAGAGTTCATCTT
CTCCTTGGTAGCGTGCACGGTGTTG
TGACTGTGCAGACTCTGGCTCGAGC
AGGTGTAGGCAGGTGGGCTCTGCTT
GAAAGGGGCTTTCATGTCGTTTCCT
TCTTCCTCATCCCTAGGGTGTTGTG
GAACTCTTTTTAAACTCTATGCTCC
GTCTGCAGACCTCAGAGAGGTCCCA
GAACTGGGACACTGGGGAGAAAGGG
TFPT probes (SEQ ID NO:1147-1157) 
AAAGTACCAGGCACTAGGTCGGCGC
GCGCTGCCGGGAGATCGAGCAGGTG
TGGCCCCGGTGCAGATTAAGGTTGA
CAGCCAGTTCACCATTGTGCTGGAG
GCCGAGCAGGAAATGCGCTGACTCC
CCTGGATTCCAGTTGGGTTTCTCGG
GCTGGACTCCTACGGGGATGACTAC
AACGAGCGGGTCCTGAACAGGCTCC
TCGGGGTCCAGACAAACTGCTGCCC
GGTTCCTCATGAGAGTGCTGGACTC
GGCGGCGCCAGCGGGAATTAAATCG
CTTN probes (SEQ ID NO:1158-1174) 
TGTGTCTTTCCAGAAGGTCACGTGG
CAAAGATGGGGTGCCAAGACGGTGC
TCGCCCAGGATGACGCGGGGGCCGA
GTGGAAATGTCTCGGGACTTGGGTC
CGTGAACAGCCTTTTATCTCCAAGC
GAAACTCATCTCCTTCCTGAGGAGC
GAATTTCGTGAACAGCCTTTTATCT
CCAGGACACCGCTGTCCTGGCATTT
CAGCCTTTTATCTCCAAGCGGAAAG
TTCCTCATTGGATTACTGTGTTTTA
GAAGGTCACGTGGAAATGTCTCGGG
CTGGGAGACCGACCCTGATTTTGTG
AATCAGTCCCCAATGCCTGGAAATT
GCCTGGAAATTCCTCATTGGATTAC
CCTGAGGTGCATTTTCTCATCATCC
CATCCTTGCTTTACCACAATGAGCA
ATTTGTGGCCACTCACTTTGTAGGA
MCM5 probes (SEQ ID NO:1175-1185)

GCATCGCATGCAGCGCAAGGTTCTC
GAGGAAGGAGCTGTAGTGTCCTGCT
CTGGGAAGTGTGCTTTTGGCATCCG

CGGCGAGATCCAGCATCGCATGCAG
CCAGCATCGCATGCAGCGCAAGGTT
CTGCCTGCCATTGACAATGTTGCTG
GCGAGATCCAGCATCGCATGCAGCG
GTTCTGGGAAGTGTGCTTTTGGCAT
GAAGGAGCTGTAGTGTCCTGCTGCC
TCGCATGCAGCGCAAGGTTCTCTAC
TTGACAATGTTGCTGGGACCTCTGC

### Appendix 4. Probe sequences for 17-gene and 8-gene panel of Tables 1 and 2.

CCNB2 probes SEQ ID NO:1-9 
ATGGAGCTGACTCTCATCGACTATG
ATATGGTGCATTATCATCCTTCTAA
AGTCCTCTGGTCTATCTCATGAAAC
CTTGCCTCCCCACTGATAGGAAGGT
CAAAAGCCGTCAAAGACCTTGCCTC
GATTTTGTACATAGTCCTCTGGTCT
GCCACTACACTTCTTAAGGCGAGCA
GATAGGAAGGTCCTAGGCTGCCGTG
ATCCTTCTAAGGTAGCAGCAGCTGC
TOP2A probes (SEQ ID NO:10-17 and SEQ ID NO:19-20) 
ACTCCGTAACAGATTCTGGACCAAC
GACCAACCTTCAACTATCTTCTTGA
GAAAGATGAACTCTGCAGGCTAAGA
ACAAGATGAACAAGTCGGACTTCCT
TGGCTCCTAGGAATGCTTGGTGCTG
GATATGATTCGGATCCTGTGAAGGC
AAAGAAAGAGTCCATCAGATTTGTG
GAATAATCAGGCTCGCTTTATCTTA
AAGAACAAGAGCTGGACACATTAAA
GAGACTTTTTTGAACTCAGACTTAA
RACGAP1 probes SEQ ID NO:21-25) 
GTACAACTCGTATTTATCTCTGATG
GAATGTTTGACTTCGTATTGACCCT
GGATGCTGAAATTTTTCCCATGGAA
ACTTCGTATTGACCCTTATCTGTAA
CAATATATCATCCTTTGGCATCCCA
CKS2 probes (SEQ ID NO:26-28) 
CGCTCTCGTTTCATTTCTGCAGCG
TATTCTTCTCTTTAGACGACCTCTT
TCTCTTTAGACGACCTCTTCCAAAA
AURKA probes (SEQ ID NO:29-39) 
CTACCTCCATTTAGGGATTTGCTTG
GTGTCTCAGAGCTGTTAAGGGCTTA
CCCTCAATCTAGAACGCTACACAAG
GAGGCCATGTGTCTCAGAGCTGTTA
TTAGGGATTTGCTTGGGATACAGAA
GTGCTCTACCTCCATTTAGGGATTT
AAATAGGAACACGTGCTCTACCTCC
GGGATACAGAAGAGGCCATGTGTCT
GAAGAGGCCATGTGTCTCAGAGCTG
CAGAGCTGTTAAGGGCTTATTTTTT
CATTGGAGTCATAGCATGTGTGTAA
FEN1 probes (SEQ ID NO:40-50) 
GAACTTGCTATGTAATTTGTGTCTA
GATGGTGATGTTCACCTGGCAATCA
 
GAGCCACCAGGAAGGCGCATCTTAG
TTGACCCACCTTGAGAGAGAGCCAC
GGACACTAAGTCCATTGTTACATGA
GAAATGATTTCCTGGCTGGCCAACT
ACACTGGTTTTCATGCGCTGTTTTT
ACTGATTACTGGCTGTGTCTTGGGT
TGGACCTAGACTGTGCTTTTCTGTC
TTGGGTGGGCAGAAACTCGAACTTG
ACCTGGCAATCAGCTGAGTTGAGAC
EBP probes (SEQ ID NO:51-71) 
GAAGGCACTGCTGGGAGCCATTAGA
CAGGCTCATGGGCAGGCACAAGAAG
GTCTTAGTCGTGACCACATGGCTGT
CACAGATACAAGAGAAGCCAGGAGG
AAGGGGCTGTGTGAAGGCACTGCTG
AGAAGAACTGAGGAGTGGTGGACCA
GCCAGGAGGTCTATGATGGTGACGA
CCCACCTGGCATATACTGGCTGGCC
ACATGGCTGTTGTCAGGTCGTGCTG
TCTATGGGGATGTGCTCTACTTCCT
GCATGGAAACCATCACAGCTTGCCT
GAGTGGTGGACCAGGCTCGAACACT
TTGGAGGGACAAAGCTAATTGATCT
GATGCCAAGGCCACAAAAGCCAAGA
CCAGGCTCGAACACTGGCCGAGGAG
TGACAGAGCACCGCGACGGATTCCA
GGGAGCCATTAGAACACAGATACAA
TTTGTCTTCATGAATGCCCTGTGGC
GGAGACCAAGCCTTCTTATCTCAAC
TGCAGTGTGTGGGTTCATTCACCTG
CTCCGCTTCATTCTACAGCTTGTGG
TXNIP probes (SEQ ID NO:72-102) 
TGTGTCAGAGCACTGAGCTCCACCC
TACAAGTTCGGCTTTGAGCTTCCTC
AAAGGATGCGGACTCATCCTCAGCC
ACTTTGTTCACTGTCCTGTGTCAGA
GAAAGGGTTGCTGCTGTCAGCCTTG
AGATAGGGATATTGGCCCCTCACTG
GGCAATCTCCTGGGCCTTAAAGGAT
CTTAGCCTCTGACTTCCTAATGTAG
GCAAAGGGGTTTCCTCGATTTGGAG
AAATGGCCTCCTGGCGTAAGCTTTT
AAACCAACTCAGTTCCATCATGGTG
TTCCACCGTCATTTCTAACTCTTAA
GGTTTTCTCTTCATGTAAGTCCTTG
CGGAGTACCTGCGCTATGAAGACAC
CCCTGCATCCTCAACAACAATGTGC
GTGTTCTCCTACTGCAAATATTTTC
AATTGAGGCCTTTTCGATAGTTTCG
GGAGGTGGTCAGCAGGCAATCTCCT
CCAGCGCCCATGTTGTGATACAGGG
 
GAAAAACTCAGGCCCATCCATTTTC
TGAGGTGGTCTTTAACGACCCTGAA
TGTTCTTAGCACTTTAATTCCTGTC
AGCTCCACCCTTTTCTGAGAGTTAT
CACTCTCAGCCATAGCACTTTGTTC
GAAGCAGCTTTACCTACTTGTTTCT
GAAGTTACTCGTGTCAAAGCCGTTA
GGTGGATGTCAATACCCCTGATTTA
CCGAGCCAGCCAACTCAAGAGACAA
TGGATGCAGGGATCCCAGCAGTGCA
GATCCTGGCTTGCGGAGTGGCTAAA
GCTGAAACTGGTCTACTGTGTCTCT
SYNE2 probes (SEQ ID NO:103-113) 
TTTCTAAGACTTTTTCACATCCAAA
GTTTTACTCCAATCAGCTGGCAATT
GGCACCCTTAGCTGATGGAAACAAT
ATTTTGAGCTGCCGGTTATACACCA
TGTTCTGTTCAGTACCTAGCTCTGC
GTAAATGCCAAACTACCGACTTGAT
TACGCTTAGAATCAGTTTTACTCCA
GTTCAGAAACTCATAGGCACCCTTA
TGAGCAGTGGTGTCCATCACATATA
ATGTACAACTCAGATGTTTCTCATT
GCTCTGCTCTTTTATATTGCTTTAA
DICER1 probes SEQ ID NO:114-142) 
AATTTCTTACTATACTTTTCATAAT
ATTTCACCTACCAAAGCTGTGCTGT
ACTAGCTCATTATTTCCATCTTTGG
AAATGATTTTTCACAACTAACTTGT
TTGCAGTCTGCACCTTATGGATCAC
TGATACATCTGTGATTTAGGTCATT
GGAGACGCCAATAGCAATATCTAGG
CTGATGCCACATAGTCTTGCATAAA
AGCTGTGCTGTTAATGCCGTGAAAG
GAAGTGCGCCAATGTTGTCTTTTCT
GTGAAACCTTCATGGATAGTCTTTA
TTTACTAAAGTCCTCCTGCCAGGTA
GGACATCAACCACAGACAATTTAAA
TGTTGCATGCATATTTCACCTACCA
ATAAACCTTAGACATATCACACCTA
TAGTCTTTAATCTCTGATCTTTTTG
GAGACAGCGTGATACTTACAACTCA
GACCATTGTATTTTCCACTAGCAGT
CTGCAGCAGCAGGTTACATAGCAAA
GCCGTGAAAGTTTAACGTTTGCGAT
AACTGCCGTAATTTTGATACATCTG
TATTTACCATCACATGCTGCAGCTG
AACGTTTGCGATAAACTGCCGTAAT
GGAAATTTGCATTGAGACCATTGTA
GCACCTTATGGATCACAATTACCTT
AGAAGCAAAACACAGCACCTTTACC
 
CCCTTAGTCTCCTCACATAAATTTC
TGTGTAAGGTGATGTTCCCGGTCGC
CTGCCAGGTAGTTCCCACTGATGGA
AP1AR probes (SEQ ID NO:143-153) 
GCCTTCCTTTACCTTGTAGTACAAG
TTTTTCCTCTTGCAACAATGACGGT
GTCAATTTACAAGGCCAGGGATAGA
TTCCACTTCATTTTACATGCCACTA
GTGCTAGACAATTACTGTTCTTTTC
AATATCTATAACTGCATTTTGTGCT
GATAGAAAACACTCCATAATTGCTT
CATTGATTTTATTAAGCCTTCCTTT
TACATGCCACTATATTGACTTTAAT
TCTGGTATGAAAGGCTCCATTGATT
GCTTTCCTTGATTTTGCTGAGGATT
NUP107 probes (SEQ ID NO:154-163) 
GGATATCAGCGTTTCTCTGTGTGCT
GAAAGCTTTGTCTGCCAATGTTGTG
CAGAGAGTCCTCTCTAATGCTCCTA
GATATTGCACAGTACTGGTCAGTAT
GACCAGGGACTTGACCCATTAGGGT
AGATATGGTATCCTCTGAGCGCCAC
AATGCTCCTAGACCAGGGACTTGAC
ATCGTGACACTTTCAACATGTAGGG
TTGGATGCCCTAACTGCTGATGTGA
GTGTTTTCTGCTTCATACGATATTG
APOC1 probes (SEQ ID NO:164-174) 
AAGGGTGACATCCAGGAGGGGCCTC
CAGGAGGGGCCTCTGAAATTTCCCA
GATGCGGGAGTGGTTTTCAGAGACA
CAGCAAGGATTCAGGAGTGCCCCTC
GTGAACTTTCTGCCAAGATGCGGGA
CAAGGCTCGGGAACTCATCAGCCGC
AACACACTGGAGGACAAGGCTCGGG
GACGTCTCCAGTGCCTTGGATAAGC
CCAAGCCCTCCAGCAAGGATTCAGG
TCATCAGCCGCATCAAACAGAGTGA
GTTCTGTCGATCGTCTTGGAAGGCC
DTX4 probes (SEQ ID NO:175-180) 
ATCGCCACCTGGTGCTCATGAGGTG
ACTCGTCTTGGTATTGCACTGTTGT
ATTCTCTTCCCATTTTTGTACATTT
TGCTCCGTGAAAGGACATCGCCACC
GGAGACAAACCTCGTCAGATGCTCA
TGAAGTCTTTGGTGTTGCTCCGTGA
FMOD probes (SEQ ID NO:192-202) 
GCTGGGGAGCACTTAATTCTTCCCA
GGAGCTCCGATGTGAGGGGCAAGGC
 
TCTGGCTGGGGTCCGTGAAGCCCAG
GCCAAACCAGCTCATTTCAACAAAG
ATGTGAACACCATCATGCCTTTATA
TGCCATCACATCCCTGATACTGTGT
TTTGGACTACGTTCTTGGCTCCAGA
GCAGCCAAATCTTGCCTGTGCTGGG
GCTTTGAAGCACCTTCCCTGAGAAG
TCTGCTTTCACATCTCTGAGCTATA
TAATGTTGCCTGGGGCTTAACCCAC
MAPKAPK2 probes (SEQ ID NO:203-213) 
GCTGAAGAGGCGGAAGAAAGCTCGG
CTCCTGCCCACGGGAGGACAAGCAA
CCTGCCCACGGGAGGACAAGCAATA
GGACAAGCAATAACTCTCTACAGGA
AACTCTCTACAGGAATATATTTTTT
GTTGACTACGAGCAGATCAAGATAA
AATGCGCGTTGACTACGAGCAGATC
CACAATGCGCGTTGACTACGAGCAG
GCGCGTTGACTACGAGCAGATCAAG
AAGCAATAACTCTCTACAGGAATAT
AGACAGAACTGTCCACATCTGCCTC
SUPT4H1 probes (SEQ ID NO:236-246)

TACCCTCCAATTCAGACTCAGCTGA
CAGAACTTCAAATACTTCCTACCCT
CCTGCCCCAAGGAATCGTGCGGGAG
GACAGCTGGGTCTCCAAGTGGCAGC
ATCTTCTTTGGACTACAGGTGGGGT
TAGGATGCTGATTTTCCTACCCGTG
GTATATGACTGCACTAGCTCTTCCT
GAGAGCAGCACATCATTTTATCATT
GTCGAGGAGTGGCCTACAAATCCAG
TGCAAGGCTGCCAGCATCTTTGCTC
ATATGCGGTGTCAGTCACTGGTCGC

### Appendix 5. Probe sequences for top 25 reference probesets (set #1) and top 15 reference probesets (set #2). Overlapping probesets listed only once.

MYL12B probes (SEQ ID NO:1186-1189) 
GTTACATTGTCTTACTCTCTTTTAC
GTTACATTGTCTTACTCTCTTTTAC
GAGGCCCCAGGGCCAATCAATTTCA
GTACCATTCAGGAAGATTACCTAAG
SFRS3 probes (SEQ ID NO:1190-1200) 
GAAACACAGGCCATCAGGGAAAACG
GAAAAATCCAACTCTCATCCTGGGC
CATCCTGGGCAGAGGTTGCCTAGTT
GATACATGGCTGTTCGTGACATTCT
AATGTCCTGCCAGTTTAAGGGTACA
GGGTACATTGTAGAGCCGAACTTTG
GAGCCGAACTTTGAGTTACTGTGCA
TACTTTACAATGTTCCCTTAAGCAA
GATAATAAACCTCTAAACCTGCCCA
AACCTGCCCAGCGGAAGTGTGTTTT
TACTTTTTTTTCCATAGCTGGGATA
CLTA probes: (SEQ ID NO:1201-1211) 
CAAGAGTAGCCTCAACCTGTGCTTC
CAGGGTGGCAGATGAAGCTTTCTAC
ACAACCCTTCGCTGACGTGATTGGT
ACCATCCTTGCTACAGCCTAGAACA
TGACATTGACGAGTCGTCCCCAGGC
TAACCCCAAGTCTAGCAAGCAGGCC
GCAAGCAGGCCAAAGATGTCTCCCG
GCCACCCTGTGGAAACACTACATCT
ATCTGCAATATCTTAATCCTACTCA
GAAGCTCTTCACAGTCATTGGATTA
TGTTTGTGATTGCATGTTTCCTTCC
TRA2B probes: (SEQ ID NO:1212-1222) 
TACTTTTCTTTCTAACATATCAATG
ATACCATACTTATATACCTGCAACT
ATGCTCTGTAACTCTGTACTGCTAG
AATACAGCCAGTGCTTAATGCTTAT
AATGTGGATTTGTCGGCTTTTATGT
GCAAGTGACAATACATTCCACCACA
AATACACTCTTGTTCTTCTAGCTTT
AAACCGGGTGCTTCAAAGTACATGA
GGAACACTATACCTGTCATGGATGA
GGATGAACTGAAGACTTTGCCTGTT
GGAGGCCCAATTTCACTCAAATGTT
MTCH1 probes: (SEQ ID NO:1223-1232) 
GTTTTTCTCAACACTACTTTTCTGA
GCTCAGCTGGGAGCATCATTCTCCT
GCTCAGCTGGGAGCATCATTCTCCT
GAGAATGGCTTATGGGGGCCCAGGT
 
GTTTAATGGTGATGCCTCGCGTACA
TCTCTAGTCCTACCCAGTTTTAAAG
GCCTCGCGTACAGGATCTGGTTACC
GTTGGGCAGATCAGTGTCTCTAGTC
CACCATCATGTCTAGGCCTATGCTA
GACCTCATCTCCCGCAAATAAATGT
HDLBP probes (SEQ ID NO:1233-1243) 
AACGCCCGCAGCACAACGAAGAGGC
CCGCAGCACAACGAAGAGGCCAATG
AAGAGGCCAATGGGCACTCTTCCAG
CACTCTTCCAGAGGCTTTGTGGTGC
TCCAGAGGCTTTGTGGTGCGGGACC
ACCTGCTCCACTGTTTAACACTAAA
AACCAAGGTCATGAGCATTCGTGCT
TAAGATAACAGACTCCAGCTCCTGG
TAGGATTCCACTTCCTGTGTCATGA
CCACTTCCTGTGTCATGACCTCAGG
GACCTCAGGAAATAAACGTCCTTGA
CYFIP1 probes: (SEQ ID NO:1244-1254) 
TGGGATGTTCTGGCAGCTGTGTCAT
TTGTTGCCATCACGTTCCTACAAAA
GCCTTTCTCTCCGTAAACTATTTAG
AATAGTGAACTTGATTCCCCTGCTT
ATGCTGCTGGGTTCATTCATTCATT
CTGCTTCCACTAAATCCAGTTGTGA
GCACTCCGTAACTCAACATGGCATG
GAGAATATTGGCTGCTGATTGTTGC
GTTTAGGGATCTTTCTGATGGTCTT
TTTTCAGTATCTCTGTACCTGTTAA
CTTAGTTCTAAGTCATTGTTCCCAT
SUMO1 probes: (SEQ ID NO:1255-1265) 
AAATCTTGTCAGAAGATCCCAGAAA
AAAGTTCTAATTTTCATTAGCAATT
ATTTGTACTTTTTGGCCTGGGATAT
GCCTGGGATATGGGTTTTAAATGGA
AATGGACATTGTCTGTACCAGCTTC
CATTGTCTGTACCAGCTTCATTAAA
AATGACCTTTCCTTAACTTGAAGCT
GACCTTTCCTTAACTTGAAGCTACT
GAGGGTCTGGACCAAAAGAAGAGGA
AGGTGAGAGTAATGACTAACTCCAA
CTAACTCCAAAGATGGCTTCACTGA
DHX15 probes: (SEQ ID NO:1266-1276) 
AAGTTCGAGTTGTGCTCTTCACGTT
TGTGCTCTTCACGTTGGTTCGATAA
CACGTTGGTTCGATAATGGCCTTTA
GTAAATATTCCATTCTGATTTCATA
ATTAAACATTTATGCCTCCCTTTTG
CCTCCCTTTTGTGTTGACACTGTAG
 
GTTGACACTGTAGCTCATACTGGAA
GTGATTATCGACCATGGTATGCATG
GGTATGCATGATCGTTGTAATTGTT
TTTTTTGTTTCAGTACCAGAGGCAC
GTACCAGAGGCACTGACTTCAATAA
HNRNPC probes: (SEQ ID NO:1277-1287) 
AATAATCTCTTGTTATGCAGGGAGT
TATGCAGGGAGTACAGTTCTTTTCA
TCTTTTCATTCATACATAAGTTCAG
TAAGTTCAGTAGTTGCTTCCCTAAC
GTTGCTTCCCTAACTGCAAAGGCAA
ACTGCAAAGGCAATCTCATTTAGTT
GAGTAGCTCTTGAAAGCAGCTTTGA
AGAAGTATGTGTGTTACACCCTCAC
TGCTGTGTGGGGCAGTTCAACACAA
GTTGGCATGTCAAATGCATCCTCTA
ACAGCCTGATGTTTGGGACCTTTTT
UBE2D3 probes: (SEQ ID NO:1288-1298) 
GTTGGGATTTGCTTCATTGTTTGAC
TGCACAGTCTGTTACAGGTTGACAC
TTGACACATTGCTTGACCTGATTTA
TAGTGTAGCTTTAATGTGCTGCACA
GTGCTGCACATGATACTGGCAGCCC
ACTGGCAGCCCTAGAGTTCATAGAT
GTTCATAGATGGACTTTTGGGACCC
GGGACCCAGCAGTTTTGAAATGTGT
GCAGCCCCTGTCTAACTGAAATTTC
CTAACTGAAATTTCTCTTCACCTTG
CTTCACCTTGTACACTTGACAGCTG
DAZAP2 probes: (SEQ ID NO:1299-1324) 
AGAGTGTCTGATGCGGCCACTCATT
TGAAGCCGCCCTAAGGATTTTCCTT
GGGGAACTTCTTCATGGGTGGTTCA
TTGTGTGTTCTGTACATGTGATGTT
CTCCCAATGCTGCTCAGCTTGCAGT
GAGGAGGATGCATTTCAAAAGCTTG
GATGTCGTGCAAACTGTACTGTGAA
ATAGGTTGTCTCTGCATACACGAAC
GATTCTTTACTTAGCTTGTTTTTAG
ATTTATATCCCATCTAGAATTCAGC
TGCAGTCATGCAGGGAGCCAACGTC
GTGGTGCACTTAACTTGTGGAATTT
GTTTGACTGTACCATTGACTGTTAT
GATGAAGTTGCATTACACCTCACTG
AAGTTCAGCGTTGTATGTCTCTCTC
AATACTGTACCATACTGGTCTTTGC
TCTTTCTGGTGCCCAAACTTTCAGG
TACACGAACCTAACCCAAATTTGCT
GAATTCAGCTAGGTGCTGCTGCTGC
ACGTCCTCGTAACTCAGCGGAAGGG
 
CTCTCTCTACACTGTGGTGCACTTA
AATGACTTGAGTCCAGTGAAATCTC
TAGCAGTACCTCCCTAAAGCATTTT
ACACCTCACTGCAAGGATTCTTTAC
GGCTCCCCAGAATTCCTAGACTGGG
CTCTGTTTCCTTTGATGACGCTTTG
SNRNP200 probes: (SEQ ID NO:1325-1335) 
GAAGTCACAGGCCCTGTCATTGCGC
GATGCCAAGTCCAATAGCCTCATCT
CCCACAACTACACTCTGTACTTCAT
GGAGTACAAATTCAGCGTGGATGTG
GATTCAGATTGAGTCCTGAGGCATT
GTAGGAATCCTGGTTGTGGGGACCA
ACTCTGGATCCAGTGACAGCAGGTG
ACAGCAGGTGTCATGGGTCAAGCAT
AATCATATATAGCATTTTCAGGCAT
GGCATGTTCCTGGTAGTTCTTTTGA
CTGGTAGTTCTTTTGAGTCTGACAT
YTHDC1 probes: (SEQ ID NO:1336-1346) 
GTATGATGGTTTGACTGTATGGCAG
GGATCTTGATTGATAACTGCCATGA
GTGTGTTCATCCTAGAGTTATTTTT
CCTTCCCCTCCAAATTGTATACATT
TGTTGTAGCAGCCTCTTGTTTTTTT
GCGTGGCAGCGGAAGACGATTCCCA
AATTCCTATGTTCAGTAGCGTGGTT
AACTGCCATGATATTTTGCTTTGAT
ACATGTAGTTGCACACGGTTCAGTA
TTTCCTCAGTCTTCAATGACGAGAG
ATGTTCACAACTTGCGTGCGTGGCA
COPB1 probes: (SEQ ID NO:1347-1368) 
AGTCCTTGAAGCTTTACAGTTAATT
ACCTTTATGCTCGTTCCATATTTGG
TATGGCAGCCAACCTTTATGCTCGT
GTTTCATGTACCAAGACCCTTTTCA
GTTTGTCTTTTGTCTTAACAGTTCT
GAATGCTGTCCTCAAAGTATATAAT
TGCTGTCCTCAAAGTATATAATGTT
GATGCACTTGCAAATGTCAGCATTG
ACCAAGACCCTTTTCACAGTACAAT
GAATACTTTTCAGCCAATAATTTAT
GACCCTTTTCACAGTACAATAAACA
TAATGTTTCATGTACCAAGACCCTT
CTGCTGTTACCGGCCATATAAGAAT
CATGTACCAAGACCCTTTTCACAGT
AAATGACTACTTACAGCACATATTA
GGTATGGGCTTACTGGACTCCAACA
TAACAGTTCTGAATGCTGTCCTCAA
GTCTTTTGTCTTAACAGTTCTGAAT
GAAGCCAATTCACCAGGGACCAGAT
 
ACTCCAACATCTTTTGTACTCTTTC
AGTTCTGAATGCTGTCCTCAAAGTA
GCCCTTTCTGGTTACTGTGGCTTTA
NDUFB8 probes: (SEQ ID NO:1369-1385) 
GAGATCTGAGGAGGCTTCGTGGGCT
GCACTGGCACCTAGACATGTACAAC
CGGGTGGTTCACTATGAGATCTGAG
TGGTATAGCTGGGACCAGCCGGGCC
TGGGTCCTCTAACTAGGACTCCCTC
CCCTGACCGCTCACAGCATGAGAGA
GCCGGGCCTGAGGTTGAACTGGGGT
CGGTGATCCCTCCAAAGAACCAGAG
TACGAACCTTACCCGGATGATGGCA
ACACCTGTTTCTTGGCATGTCATGT
AACCGTGTGGATACATCCCCCACAC
TCATGTGCTGGGTGGGGGACGTGTA
TCTAACTAGGACTCCCTCATTCCTA
GGACTCCCTCATTCCTAGAAATTTA
CATGACCAAGGACATGTTCCCGGGG
GATCCCTCCAAAGAACCAGAGCGGG
CCAGAGCGGGTGGTTCACTATGAGA
SET probes: (SEQ ID NO:1386-1401) 
ATTGGCCTTTTACCTGGATATAAAT
ACCATCCAACAGACCTGGTGCTCTA
CCATCCAACAGACCTGGTGCTCTAA
TGCTCTAATGCCAAGTTATACACGG
ATAGGCTCTCAGTAAGAAGTCTGAT
GGTATAAAGCTCTCAAATGTGACCA
AAGCTCTCAAATGTGACCATGTGAA
TAATGGACTCAGCTCTGTCTGCTCA
AATGCCATTGTGCAGAGAAGCACCC
GAAGCACCCTAATGCATAAGCTTTT
CTAATGCATAAGCTTTTTAATGCTG
AATTAAATGCCACTTTTTCAGAGGT
CCACTTTTTCAGAGGTGAATTAATG
TAAATGGAACTATTCCATCAATAGG
CACTGTATACCGATCAGGAATCTTG
ATACCGATCAGGAATCTTGCTCCAA
CELF1 probes: SEQ ID NO:1402-1412) 
TTGCCACTATGACCAAACGCACAGT
AAACGCACAGTCTGTTCTGCAGCAA
CTGCAGCAACAACGGGATTCAATCA
TCAACTCAGTCGTGATTCAGCCGTA
TCAGCCGTAGAAATGCTTTTCCTTT
TTATCTTGTTTGAGCTTTTCCTTTC
GAACTTGTGTTGTACTCTGTAGAAA
GTCCCAATGGGGAACCTAAATCTGT
GTTTTAATTGCACAGACACATGGAC
AAAGTCATTTTGTATCTGCCAAGTG
ATCTGCCAAGTGTGGTACCTTCCTT
XPO1 probes: SEQ ID NO:1413-1423) 
TAGGGAGCATTTTCCTTCTAGTCTA
GCATTGTCTGAAGTTAGCACCTCTT
GCACCTCTTGGACTGAATCGTTTGT
GAATCGTTTGTCTAGACTACATGTA
GATCATGTGCATATCATCCCATTGT
ATCATCCCATTGTAAAGCGACTTCA
GTGTGTGCTGTCGCTTGTCGACAAC
GTCGCTTGTCGACAACAGCTTTTTG
ATTTGTGAGCCTTCATTAACTCGAA
GTTAGAATAGGCTGCATCTTTTTAA
ACAACTCTGGCTTTTGAGATGACTT
PTBP1 probes: (SEQ ID NO:1424-1434) 
TTCACCTGCAGTCGCCTAGAAAACT
AAACTTGCTCTCAAACTTCAGGGTT
AAGTCTCATTTCTGTGTTTTGCCTG
CCTCTGATGCTGGGACCCGGAAGGC
ATACCTGTTGTGAGACCCGAGGGGC
CGGCGCGGTTTTTATGGTGACACA
TCCAGGCTCAGTATTGTGACCGCGG
TGCCTTACCCGATGGCTTGTGACGC
TGTTCGCTGTGGACGCTGTAGAGGC
GTTGGCCAGTCTGTACCTGGACTTC
GAATAAATCTTCTGTATCCTCAAAA
SF3B1 probes: (SEQ ID NO:1435-1445) 
GTTTACAGGGTCTGTTTCACCCAGC
TTCACCCAGCCCGGAAAGTCAGAGA
CAACTCCATCTACATTGGTTCCCAG
CTCATAGCACATTACCCAAGAATCT
GAACACCTATATTCGTTATGAACTT
TTAATGCACAGCTACTTCACACCTT
CACACCTTAAACTTGCTTTGATTTG
AATAACCTGTCTTTGTTTTTGATGT
GTAAATGCCAGTAGTGACCAAGAAC
TACACTATACTGGAGGGATTTCATT
GATTTAGAACTCATTCCTTGTGTTT
ARPC2 probes: SEQ ID NO:1446-1468) 
ACTGGATAATCGTAGCTTTTAATGT
GTAGCTTTTAATGTTGCGCCTCTTC
GTGACAACATTGGCTACATTACCTT
TGCGCCTCTTCAGGTTCTTAAGGGA
GCTGTGCTTGCAAAGACTTCATAGT
ATCTTCCGGCATCCAAGGATTCCAT
GAGCTGAAAGACACAGACGCCGCTG
AAAGAAGGACGCAGAGCCAGCCACA
ATCTGCAGAAACGAGCTGTGCTTGC
GTCTCTTTGCTATATGACCTTGAAA
GAGGAAGCGGCTGGCAACTGAAGGC
CTCTTTTCCAAGCTGTTTCGCTTTG
 
CGTTTTCATCCCGCTAATCTTGGGA
GTTTCGCTTTGCAATATATTACTGG
GGAACACTTGCTACTGGATAATCGT
GAAGCGAAATTGTTTTGCCTCTGTC
GAGTCACAGTAGTCTTCAGCACAGT
GGAAGCGGCTGGCAACTGAAGGCTG
TGCAGTCATAACTTGTTTTCTCCTA
TCCTCTTTAGCCACAGGGAACCTCC
GACCTTGAAAATCTTCCGGCATCCA
GGATTCCATTGTGCATCAAGCTGGC
TTCATCCCGCTAATCTTGGGAATAA
VAMP3 probes: (SEQ ID NO:1469-1479) 
GAGACTCAACATCAGGATCCACAGC
ACAGACTTTATCGCTCTGTGGCTCA
AAGCAGCAACAGCTGAGGCGCACCA
GCTTCCATTTCTTTAACGTCTGTTC
TCTGTTCCCTTAACATCGCTGAAAT
GAAGAGATGCCTTGCGGTGTGGCCA
GACTCAGAAACCTTGGTACTCGCCC
ACTGGCTCCTGCATTAACCCAGAAA
TAACCCAGAAATACCTCGCTTCTAT
CTCGCTTCTATCTGTGCACTTAGCT
GGGAACTTACCCACTGTAATCACCT
STARD7 probes: (SEQ ID NO:1480-1490) 
TTGTGCCAAGGAAGTAGCTGCCCCA
CCTTCTCCGCGTCATTGTTGGAAGA
AGGAGAGATGCATCGAGCAGTCCCA
GCTGCTTTTCATTTATTACTTCTTC
CTTCTTCTTTCCAGGACCTGACAGA
TTATGTCCAAACTTAGCACCTGCAA
TGTGCGTCTGCGAGCGCACACACAT
AGGAGTTGCGGTTGCTCCATGTTCT
GCTCCATGTTCTGACTTAGGGCAAT
CTGCACTTGGGGTCTGTCTGTACAG
GTCTGTACAGTTACTCATGTCATTG
SEC31A probes: (SEQ ID NO:1491-1511) 
TTCAGTGAGACCTCTGCTTTCATGC
AGCATGTTTGCATAGCAACCAGTCA
GTTGCCAGTGATGATTTTCCTATTC
ATTTCTGCTGATATACTCACCTTAG
GCTGCCTTTCTTCAGCAACAGACCC
AGCAACCAGTCAAGAGCATTTACAC
TGAAGGTGCCCCAGGGGCTCCTATT
AGTATGGTTTCCTGAAGTATTCTGA
TAAAACTAAATTTCTTTCATGTCCT
TGCTCAGAACCCTGGTGCTTTATTT
GCGTACAGCAACCTCTTGGTCAAAC
TTCTCTTCCACTCAATATTGCCATT
GGACTAGTCCTCATTAGCATGTTTG
CATACCCACATAGTTAGCACCAGCA
 
GAGCATTTACACTATTTCTGCTGAT
AGAAGGATTGACCATGCATACCCAC
GCACCAGCAACTTCAGTGAGACCTC
TTGAGGATCTTATTCAGCGCTGCCT
GCCAGTTCTCAAAGTTGTTCTCACC
TACTCACCTTAGAACTGCTCAGAAC
GTATTTCCTGGATTACACATAGTAT
MFN2 probes: (SEQ ID NO:1512-1532) 
GTCTATGAGCGTCTGACCTGGACCA
GTTACTCCTGTATCATTGCTCATAA
AGCCTCTGTGCACTGTTTGGTGGCC
TGTATTTAAAGCCCTCAGTCTGTCC
GCCTGAATGGACAGGGGCCACTTCA
ATCACTGTCACACAATTCCAATGGA
GACCTTTGCTCATCTGTGTCAGCAA
CCCGGCGTGTGCCGGGCCTGAATGG
GCTGGAGCGCAAGACGTGCTGACAC
AGGTGATGTCCTGTTCACATACCTG
GCCTTCAAGCGCCAGTTTGTGGAGC
CCTCCATGGGCATTCTTGTTGTTGG
TCATGGTTTCCATGGTTACCGGCCT
CCCAGCCATCACTCATCTTTGAGGA
GCGAAGTGATGGACTCTGCCAGGTG
GCCACTTCACAGCATGTCAGGGAAA
GTCCTGTTGTGTGGGGCGAAGTGAT
GTGCTGACACAGTGAGTTTTCTCTG
GCAGCTTGTCATCAGCTACACTGGC
GTGTCAGCAAGTTGACGTCACCCGG
CTGCCAGGTGGACATGCTGTGGGTG
WIPI2 probes: (SEQ ID NO:1533-1564) 
CAATGAGATCTTGGACTCTGCCTCT
AGATCCCGCGGTTGTTGGTGGGTGC
TCTACTTCACTCTTCCTGTTGAAAA
CAGACTCTGCATTCCAAACCAAGGC
GACTGACTGAACTTGACCTGTGACC
AGCAACAGAGAGTAGGCGGCTGGGC
TGCCTGGACTCGCTGGAGCAAAGGA
CCGCCCATGATTCTTCGGACTGACT
GCCCCTTAGTCACTCAGACATACGG
CGCCGACGGGTACCTGTACATGTAC
GTCATGTGCCTTTCTATTTTCATCT
TAGGGGAGCTAGAAGCCACTTTCCA
GGGCTTCCTACCTGTGTGAGAGGTC
TCGCTTCCCTTTTCATATTTACAGA
ACTTGAAAGGTTGCCTGGACTCGCT
GCATGAACGTGCCAAGCCAGCATAG
CCCTGCGCCTGGATGAGGACAGCGA
CAGAACTCAAGTGTGGTGGCCGTCT
AATTGGATCGCTCTGGGATTTCTTC
GGACAGCGAGGTTCTTTCTGATACT
CCCACCAGGTGTGCTGGGCAGACTT
 
AAATGATCTGTTCTTCTACTTCACT
AAACAACCTCAAGTACCTCAGACTC
GGGCAGACTTCAGCTGGGACAGAAG
TTCGGGAAAGTGCTCATGGCCTCCA
CAAGCTTCAGTATTTGCCTCGCTTC
GCGTAAGGAAACCGTGGCGTCGCGC
CCACAAAAACATCTGCTCGCTAGCC
TCTGCTTGTCAAGGCCAGTTCTGCA
GCGAGTGTGCCCTGATGAAGCAGCA
GAGGTCGTAGCGGGAGACAGCAACA
TGGGACAGAAGTCCGATCTCCCTAG
PFDN1 probes: (SEQ ID NO:1565-1575) 
GGCAGTCTGCCTAAAGATTCCTTTC
GCCTTCTCCCATACATTCCAAAAGG
GTTCAACAGTAAGCAGCACCTCCAA
TCTCCTTTCGGCCAGTATCATAAGA
TGGACGCCATAATCCTGAGGCTCCT
GGCTCCTAGAGGCTGAGGGGGCAAC
TGAGGGGGCAACGGTGTGATCCAGC
GCAAGCCAGTTGTCAAACACAGCCA
GTGAGAGAGGCAGTGGCCGTCCTCC
TTCCTGTACCTTTGACTAACGCTCA
CTTCCGGGCCTGCATGCAGTAGACA
UBE3A probes: (SEQ ID NO:1576-1621) 
ATCAGCCATTTTATCGAGGCACGTG
TAGCTAATGTGCTGAGCTTGTGCCT
TAGACCACGTAACCTTCAAGTATGT
GAACTACTCTCCCAAGGAAAATATT
TAAGGAAGCGCGGTCCCGCATGAG
GCCATCATCTTGTTGAATCAGCCAT
TACAACGGGCACAGACAGAGCACCT
TTTACTTCCGGAATACTCAAGCAAA
TATGGTGACCAATGAATCTCCCTTA
GATTGTTTTAACTGATTACTGTAGA
TTCCTAGTCTTCTGTGTATGTGATG
AGGATGTCTTTCAGGATTATTTTAA
TAATTACTTACTTATTACCTAGATT
GACTACAGGAGACGACGGGGCCTTT
GACAGAACTGTTTGTTATGTACCAT
ACTGTGCCTTGTGTTACTTAATCAT
GCGACGAACGCCGGGATTTCGGCGG
GTATAGCCCCACAGATTAAATTTAA
TTGCCACCATTTGTAGACCACGTAA
GAAGACAATGCTTTCCATATTGTGA
GCTTTAATGTGCTTTTACTTCCGGA
ATTTTTTTGCGTGAAAGTGTTACAT
CGGATAAGGAAGCGCGGTCCCGCA
CTGGGCTCGGGGTGACTACAGGAGA
AAAGATGGCTACTGTGCCTTGTGTT
AAGGCCATCACGTATGCCAAAGGAT
GACTCTTCTTGCAGTTTACAACGGG
 
GAGACATTGATATATCCTTTTGCTA
GATTACTGTAGATCAACCTGATGAT
GGCTCGGGGTGACTACAGGAGACGA
GCCTCGTTTTCCGGATAAGGAAGCG
CCATTTGTAGACCACGTAACCTTCA
TTCGTGTTGCCATCATCTTGTTGAA
TTACCTACATCTCATACTTGCTTTA
GAGCTTGTGCCTTGGTGATTGATTG
CAAGGCTTTTCGGAGAGGTTTTCAT
GGGTGACTACAGGAGACGACGGGGC
TGTTACATATTCTTTCACTTGTATG
GATAAGGTAACATGGGGTTTTTCTG
GAATTACATTGTATAGCCCCACAGA
GATATATCCTTTTGCTACAAGCTAT
TGACGGTGGCTATACCAGGGACTCT
GAAACTATTACTCCTAAGAATTACA
GCTGGCGACGAACGCCGGGATTTCG
ATGCAGCTTTCAAATCATTGGGGGG
GAGGCACGTGATCAGTGTTGCAACA
GTF3C2 probes: (SEQ ID NO:1622-1653) 
GGGCAGGAGCCTCGCAATATGTGGC
GGCTCCTCAGCCTAAGACTATGGCT
AGAAACACTCAGGCCTGACCTAGGC
TAACCATCATGTATGCCCACGAGGG
TAACCATCATGTATGCCCACGAGGG
GACCCCTCTGAGTGTGGTCAGTGCC
TCCCTGTGATTGCCCTGTTAAGTAT
TCCCTGTGATTGCCCTGTTAAGTAT
TGCTCCTGCTTACGAAGTATTCCCA
TGCTCCTGCTTACGAAGTATTCCCA
GATTGCTTGTGACAACGGCTGCATC
GGCTCCTGTCTGACTATTCCAGGAT
CCCTACCGATAGAACAGTGGCTCAG
GCATGAAGGCTCCTGTCTGACTATT
TGCATCTGGGACCTCAAGTTCTGCC
CCACCAACACCTAGCTGCTGGATAT
CACAGACACCCTACCGATAGAACAG
TGGCCTGCTCAGACGGGAAAGTACT
GTATCTGCATGAAGGCTCCTGTCTG
CAAGGAATACCACAGACACCCTACC
AACCATAGCTATCATGTGTTTCCCA
ATAGCTATCATGTGTTTCCCAAATC
ACAGGGCCCACTTTGTCTATGGGAT
TTCCCAATCACTGGTCATCTGACCC
TTCCCAATCACTGGTCATCTGACCC
GGAAATCTAGTCATCTTCCCTGTGA
GGAAATCTAGTCATCTTCCCTGTGA
GACATGAATGAGACACACCCACTGA
GCAACTCTGCAGGTGGGGTCTATGC
AAGTACTGCTATTCAGTCTACCCCA
TATTACTGCCTTCTGAAACTTCCTC
TATTACTGCCTTCTGAAACTTCCTC
KHDRBS1 probes: SEQ ID NO:1654-1674) 
GTTACTGATTTCTTGTATCTCCCAG
GCTACATGTGTAAGTCTGCCTAAAT
AATCTAGCCCCAGACATACTGTGTT
CCTCCCATTTTGTTCTCGGAAGATT
GTCCATTTGAGATTCTGCACTCCAT
CCCCTCCTGCTAGGCCAGTGAAGGG
TAATTGGATTTGTACCGTCCTCCCA
GTCAAGTATGTCTCAACACTAGCAT
GAAAAGTTCACTTGGACGCTGGGGC
TTGTCAATATATCGAACTGTTCCCA
TAACTCTGCATTCTGGCTTCTGTAT
TGTCTAAGTGTTTTTCTTCGTGGTC
AGGCCTCCTGAATTGAGTTTGATGC
GACTGGAATGGGACCAGGCCGTCGC
GATGCAGAGCTTTTTAGCCATGAAG
ATACAGAGAGCACCCATATGGACGT
GTAGATGCTTTTTTCTTTGTTGTTT
TGACTTTTTCATTACGTGGGTTTTG
GTATCTCCCAGGATTCCTGTTGCTT
TTGCTTTACCCACAACAGACAAGTA
CCTTATTCCATTCTTAACTCTGCAT
RARS probes: (SEQ ID NO:1675-1685) 
GTTGAATGACTACATCTTCTCCTTT
AGCTGCTTACTTGTTGTATGCCTTC
GTATGCCTTCACTAGAATCAGGTCT
AATCAGGTCTATTGCACGTCTGGCC
GGAAACTAGGCCGGTGCATTTTACG
GAGCTGGCAACTGCTTTCACAGAGT
GAACATGTGGCGTATCTTGTGTGAA
CTGGCCCAAGGGTGTAATCCCTCAC
AATCCCTCACAGGTTTGAACCCTGT
TTTTCCCAAGTGGCCATTGGCCCTG
GCTTTTTTTCAATCTTGTGGGCACA
MYL12A probes: (SEQ ID NO:1686-1696) 
GCAACTGGCACCATACAGGAAGATT
CAAATTCCAGCCAACGTCCTTGTTG
AACGTCCTTGTTGCACTTTGGGTAT
GCACTTTGGGTATTCTGAGATTTTC
TCTTGCCATTCCCTTAGGCTTTAGC
GGCTTTAGCAGCTTTGCATTTCCTG
TTGCATTTCCTGTTGTATTTATTCT
TATTCTCAGCCATTTTGGGCATATG
CAGACTGGAAACGGGACTTTCTATT
CTTCTCCCCCAATAACTGTGGGTCT
TCAGAGAAAGTTAGTTCGGCTCGAT
HNRNPD probes: (SEQ ID NO:1697-1728) 
GATAGTTAATGTTTTATGCTTCCAT
TATTCCATTTGCAACTTATCCCCAA
 
GCAAAAGTACCCCTTTGCACAGATA
GAAATGCGGCTAGTTCAGAGAGATT
AATTTTTTGTATCAAGTCCCTGAAT
GACAGGCTTGCCGAAATTGAGGACA
AACAGCCAAGGTTACGGTGGTTATG
GTGTCCTCCCTGTCCAAATTGGGAA
GATTCATTTGAAGGTGGCTCCTGCC
ATAATACTTCCTTATGTAGCCATTA
AATGTCAATTTGTTTGTTGGTTGTT
GAGTGGTTATGGGAAGGTATCCAGG
GACTACACTGGTTACAACAACTACT
GGTGGTCATCAAAATAGCTACAAAC
AAGTTTGGAAGACAGGCTTGCCGAA
GGAACCAGGGATATAGTAACTATTG
GAGCTGTGGTGGACTTCATAGATGA
AGTCCCTGAATGGAAGTATGACGTT
AAAAGCCCAGTGTGACAGTGTCATG
GAAGTTTAATTCTGAGTTCTCATTA
GGAGGATATGACTACACTGGTTACA
GAGAGATTTTTAGAGCTGTGGTGGA
TTATTCCATTTGCAACTTATCCCCA
AGGTTACGGTGGTTATGGAGGATAT
ATTTGCTTTCATTGTTTTATTTCTT
AGAAATTTGCTTTCATTGTTTTATT
CCTTTCCCCCAGTATTGTAGAGCAA
GGTATCCAGGCGAGGTGGTCATCAA
GTATGACGTTGGGTCCCTCTGAAGT
GTATGACGTTGGGTCCCTCTGAAGT
AACAACTACTATGGATATGGTGATT
TGTGCTTTTTAGAACAAATCTGGAT
TARDBP probes: (SEQ ID NO:1729-1739) 
GAGAGCGCGTGCAGAGACTTGGTGG
TGGCGAGATGTGTCTCTCAATCCTG
TCTCTCAATCCTGTGGCTTTGGTGA
GTTTTTGTTCTTAGATAACCCACAT
TGAAATGATACTTGTACTCCCCCTA
CTTTGTCAACTGCTGTGAATGCTGT
GAATGCTGTATGGTGTGTGTTCTCT
GGACTGAGCTTGTGGTGTGCTTTGC
GCAGAGTTCACCAGTGAGCTCAGGT
GTTCTAATGTCTGTTAGCTACCCAT
AAGAATGCTGTTTGCTGCAGTTCTG
HNRNPR probes: (SEQ ID NO:1740-1760)

AAGCTAGTGCTTTGTCTTAGTAGTT
GGGGCAATCGTGGGGGCAATGTAGG
TCGTTTCAGGCTTCATTTTAGCTTC
TCACACCTTTTTGAAATCTGCCCTA
ACCCTCCAGATTACTACGGCTATGA
ATTGTTATAACTTCACACCTTTTTG
TGGATATGGCTACCCTCCAGATTAC
AAACAAGCTGGGCACACTGTTAAAT

GCTCTTGGACATTATTGGGCTTGCA
CATGATTTTGCAGAACCTTTGGTTT
CAATGCTTTTATCGTTTCAGGCTTC
GTTCCCGTGGATCTCGGGGCAATCG
GATTCCAAGCGTCGTCAGACCAACA
TCAACAGCAGAGAGGCCGTGGTTCC
GGCTATGAAGATCCCTACTACGGCT
AAAGCCGTGACAATTTGTTCTTTGA
TCACAGAGGGGGGCACCTTTGGGAC
ACCTTTGGGACCACCAAGAGGCTCT
GTATTTCCAATTTCTTGTTCATGTA
GTCGTCAGACCAACAACCAACAGAA
TGGGCTTGCAGAGTTCCCTTATTCT

## Claims

1. A method of evaluating the likelihood of a relapse for a patient that has a lymph node-negative, estrogen receptor-positive, HER2-negative breast cancer, the method comprising:
(a) detecting in a breast tumor sample from the patient the levels of expression of the 17 genes identified in Table 1 as primary predictors, wherein at least one alternate gene listed in Table 1 can be detected in place of the corresponding primary gene listed in Table 1; or
(b) detecting in a breast tumor sample from the patient the levels of expression of the 8 genes identified in Table 2 as primary predictors, wherein at least one alternate gene listed in Table 2 can be detected in place of the corresponding primary gene listed in Table 2;
correlating the levels of expression with the likelihood of a relapse.

2. The method of claim 1, wherein:
(a) the detecting step of 1(a) comprises detecting at least one alternate gene in addition to the primary gene listed in Table 1; or
(b) the detecting step of 1(b) comprises detecting at least one alternate gene in addition to the primary gene listed in Table 2.

3. The method of claim 1 or 2, further comprising detecting the level of expression of at least one reference gene identified in Table 3.

4. The method of claim 1, 2, or 3, wherein:
(a) the detecting step comprises detecting the level of expression of RNA, optionally wherein detecting the level of expression of RNA comprises (i) a quantitative PCR reaction; or (ii) hybridizing a nucleic acid obtained from the sample to an array that comprises probes to all of the 17 genes identified in Table 1 as primary predictors, and/or to one or more corresponding alternates of said genes as listed in Table 1; or hybridizing a nucleic acid obtained from the sample to an array that comprises probes to all of the 8 genes identified in Table 2 as primary predictors, and/or to one or more corresponding alternates of said genes as listed in Table 2; or
(b) the detecting step comprises detecting the level of protein expression.

5. Use of a microarray for determining a likelihood of relapse for a patient that has a lymph node-negative, estrogen receptor-positive, HER2-negative breast cancer by detecting the levels of expression of the 17 genes set forth in Table 1 as primary predictors, wherein at least one alternate gene listed in Table 1 can be detected in place of the corresponding primary gene listed in Table 1; or detecting the level of expression of the 8 genes set forth in Table 2 as primary predictors, wherein at least one alternate gene listed in Table 2 can be detected in place of the corresponding primary gene listed in Table 2, wherein said microarray comprises probes for detecting:
(a) the panel of all 17 genes identified in Table 1 as primary predictors, and/or one or more corresponding alternates of said genes as listed in Table 1;
(b) the panel of all 8 genes identified in Table 2 as primary predictors, and/or one or more corresponding alternates of said genes as listed in Table 2; or
(c) the panel of (a) or (b) and at least one gene identified in Table 3.

6. Use of a kit for determining a likelihood of relapse for a patient that has a lymph node-negative, estrogen receptor-positive, HER2-negative breast cancer by detecting the levels of expression of the 17 genes set forth in Table 1 as primary predictors, wherein at least one alternate gene listed in Table 1 can be detected in place of the corresponding primary gene listed in Table 1; or detecting the level of expression of the 8 genes set forth in Table 2 as primary predictors, wherein at least one alternate gene listed in Table 2 can be detected in place of the corresponding primary gene listed in Table 2, wherein said kit comprises primers and probes for detecting expression of all 17 genes listed in Table 1, and/or one or more corresponding alternates of said genes as listed in Table 1; or primers and probes for detecting expression of all 8 genes as listed in Table 2, and/or one or more corresponding alternates of said genes as listed in Table 2.

7. The kit of claim 6, further comprising primers and probes for detecting expression of at least one reference gene identified in Table 3.

8. A computer-implemented method for evaluating the likelihood of a relapse for a patient that has a lymph node-negative, estrogen receptor-positive, HER2-negative breast cancer, the method comprising:
receiving, at one or more computer systems, information describing the level of expression of the 17 genes identified in Table 1 as primary predictors, or one or more alternate genes listed in Table 1 in place of the corresponding primary predictor listed in Table 1, in a breast tumor tissue sample obtained from the patient;
performing, with one or more processors associated with the computer system, a random forest analysis in which the level of expression of each gene in the analysis is assigned to a terminal leaf of each decision tree, representing a vote for either "relapse" or no "relapse";
generating, with the one or more processors associated with the one or more computer systems, a random forest relapse score (RFRS), wherein if the RFRS is greater than or equal to 0.606 the patient is assigned to a high risk group, if greater than or equal to 0.333 and less than 0.606 the patient is assigned to an intermediate risk group and if less than 0.333 the patient is assigned to low risk group.

9. The computer-implemented method of claim 8, further comprising generating, with the one or more processors associated with the one or more computer systems, a likelihood of relapse by comparison of the RFRS score for the patient to a loess fit of RFRS versus likelihood of relapse for a training dataset.

10. A non-transitory computer-readable medium storing program code for evaluating the likelihood of a relapse for a patient that has a lymph node-negative, estrogen receptor-positive, HER2-negative breast cancer, the computer-readable medium comprising:
code for receiving information describing the level of expression of the 17 genes identified in Table 1 as primary predictors, or one or more alternate genes listed in Table 1 in place of the corresponding primary predictor listed in Table 1, in a breast tumor tissue sample obtained from the patient;
code for performing a random forest analysis in which the level of expression of each gene in the analysis is assigned to a terminal leaf of each decision tree, representing a vote for either "relapse" or no "relapse"; and
code for generating a random forest relapse score (RFRS), wherein if the RFRS is greater than or equal to 0.606 the patient is assigned to a high risk group, if greater than or equal to 0.333 and less than 0.606 the patient is assigned to an intermediate risk group and if less than 0.333 the patient is assigned to low risk group.

11. The computer-readable medium of claim 10, further comprising code for generating a likelihood of relapse by comparison of the RFRS score for the patient to a loess fit of RFRS versus likelihood of relapse for a training dataset.

12. A computer-implemented method for evaluating the likelihood of a relapse for a patient that has a lymph node-negative, estrogen receptor-positive, HER2-negative breast cancer, the method comprising:
receiving, at one or more computer systems, information describing the level of expression of the 8 genes identified in Table 2 as primary predictors, or one or more alternate genes listed in Table 2 in place of the corresponding primary predictor listed in Table 2, in a breast tumor tissue sample obtained from the patient;
performing, with one or more processors associated with the computer system, a random forest analysis in which the level of expression of each gene in the analysis is assigned to a terminal leaf of each decision tree, representing a vote for either "relapse" or no "relapse";
generating, with the one or more processors associated with the one or more computer systems, a random forest relapse score (RFRS), wherein if the RFRS is greater than or equal to 0.606 the patient is assigned to a high risk group, if greater than or equal to 0.333 and less than 0.606 the patient is assigned to an intermediate risk group and if less than 0.333 the patient is assigned to low risk group.

13. The computer-implemented method of claim 12, further comprising generating, with the one or more processors associated with the one or more computer systems, a likelihood of relapse by comparison of the RFRS score for the patient to a loess fit of RFRS versus likelihood of relapse for a training dataset.

14. A non-transitory computer-readable medium storing program code for evaluating the likelihood of a relapse for a patient that has a lymph node-negative, estrogen receptor-positive, HER2-negative breast cancer, the computer-readable medium comprising:
code for receiving information describing the level of expression of the 8 genes identified in Table 2 as primary predictors, or one or more alternate genes listed in Table 2 in place of the corresponding primary predictor listed in Table 2, in a breast tumor tissue sample obtained from the patient;
code for performing a random forest analysis in which the level of expression of each gene in the analysis is assigned to a terminal leaf of each decision tree, representing a vote for either "relapse" or no "relapse"; and
code for generating a random forest relapse score (RFRS), wherein if the RFRS is greater than or equal to 0.606 the patient is assigned to a high risk group, if greater than or equal to 0.333 and less than 0.606 the patient is assigned to an intermediate risk group and if less than 0.333 the patient is assigned to low risk group.

15. The non-transitory computer-readable medium storing program code of claim 14, further comprising code for generating a likelihood of relapse by comparison of the RFRS score for the patient to a loess fit of RFRS versus likelihood of relapse for a training dataset.

## Patentansprüche

1. Verfahren zum Beurteilen der Wahrscheinlichkeit eines Rezidivs bei einem Patienten mit lymphknotennegativem, östrogenrezeptorpositivem, HER2-negativem Brustkrebs, wobei das Verfahren umfasst:
(a) Nachweisen der Expressionshöhen der 17 Gene, die in Tabelle 1 als primäre Vorläufer identifiziert sind, in einer Brusttumorprobe des Patienten, wobei zumindest ein alternatives Gen, das in Tabelle 1 angeführt ist, anstatt des entsprechenden primären Gens, das in Tabelle 1 angeführt ist, nachgewiesen werden kann; oder
(b) Nachweisen der Expressionshöhen der 8 Gene, die in Tabelle 2 als primäre Vorläufer identifiziert sind, in einer Brusttumorprobe des Patienten, wobei zumindest ein alternatives Gen, das in Tabelle 2 angeführt ist, anstatt des entsprechenden primären Gens, das in Tabelle 2 angeführt ist, nachgewiesen werden kann;
Korrelieren der Expressionshöhen mit der Wahrscheinlichkeit eines Rezidivs.

2. Verfahren nach Anspruch 1, wobei:
(a) der Nachweisschritt von 1(a) das Nachweisen zumindest eines alternativen Gens zusätzlich zum primären Gen, das in Tabelle 1 angeführt ist, umfasst; oder
(b) der Nachweisschritt von 1(b) das Nachweisen zumindest eines alternativen Gens zusätzlich zum primären Gen, das in Tabelle 2 angeführt ist, umfasst.

3. Verfahren nach Anspruch 1 oder 2, das ferner das Nachweisen der Expressionshöhe zumindest eines Referenzgens, das in Tabelle 3 angeführt ist, umfasst.

4. Verfahren nach Anspruch 1, 2 oder 3, wobei:
(a) der Nachweisschritt das Nachweisen der Expressionshöhe von RNA umfasst, optional wobei das Nachweisen der Expressionshöhe von RNA (i) eine quantitative PCR-Reaktion; oder (ii) das Hybridisieren einer aus der Probe erhaltenen Nukleinsäure mit einem Array, umfassend Sonden für alle 17 Gene, die in Tabelle 1 als primäre Vorläufer identifiziert sind, und/oder für eine oder mehrere entsprechende Alternativen der Gene, wie in Tabelle 1 angeführt; oder das Hybridisieren einer aus der Probe erhaltenen Nukleinsäure mit einem Array, umfassend Sonden für alle 8 Gene, die in Tabelle 2 als primäre Vorläufer identifiziert sind, und/oder für einen oder mehrere entsprechende Alternativen der Gene, die in Tabelle 2 angeführt sind; umfasst; oder
(b) der Nachweisschritt das Nachweisen der Proteinexpressionshöhe umfasst.

5. Verwendung eines Mikroarrays zum Bestimmen einer Wahrscheinlichkeit eines Rezidivs bei einem Patienten mit lymphknotennegativem, östrogenrezeptorpositivem, HER2-negativem Brustkrebs durch Nachweisen der Expressionshöhe der 17 Gene, die in Tabelle 1 als primäre Vorläufer identifiziert sind, wobei zumindest ein alternatives Gen, das in Tabelle 1 angeführt ist, anstatt des entsprechenden primären Gens, das in Tabelle 1 angeführt ist, nachgewiesen werden kann; oder Nachweisen der Expressionshöhe der 8 Gene, die in Tabelle 2 als primäre Vorläufer identifiziert sind, wobei zumindest ein alternatives Gen, das in Tabelle 2 angeführt ist, anstatt des entsprechenden primären Gens, das in Tabelle 2 angeführt ist, nachgewiesen werden kann, wobei das Mikroarray Sonden zum Nachweisen:
(a) des Felds aller 17 Gene, die in Tabelle 1 als primäre Vorläufer identifiziert sind, und/oder einer oder mehrerer entsprechender Alternativen der Gene, wie in Tabelle 1 angeführt;
(b) des Felds aller 8 Gene, die in Tabelle 2 als primäre Vorläufer identifiziert sind, und/oder einer oder mehrerer entsprechender Alternativen der Gene, wie in Tabelle 2 angeführt; oder
(c) des Felds von (a) oder (b) und zumindest eines Gens, das in Tabelle 3 identifiziert ist,
umfasst.

6. Verwendung eines Kits zum Bestimmen einer Wahrscheinlichkeit eines Rezidivs bei einem Patienten mit lymphknotennegativem, östrogenrezeptorpositivem, HER2-negativem Brustkrebs durch Nachweisen der Expressionshöhen der 17 Gene, die in Tabelle 1 als primäre Vorläufer identifiziert sind, wobei zumindest ein alternatives Gen, das in Tabelle 1 angeführt ist, anstatt des entsprechenden primären Gens, das in Tabelle 1 angeführt ist, nachgewiesen werden kann; oder Nachweisen der Expressionshöhen der 8 Gene, die in Tabelle 2 als primäre Vorläufer identifiziert sind, wobei zumindest ein alternatives Gen, das in Tabelle 2 angeführt ist, anstatt des entsprechenden primären Gens, das in Tabelle 2 angeführt ist, nachgewiesen werden kann, wobei das Kit Primer und Sonden zum Nachweisen der Expression aller 17 Gene, die in Tabelle 1 angeführt sind, und/oder einer oder mehrerer entsprechender Alternativen der Gene, wie in Tabelle 1 angeführt; oder Primer und Sonden zum Nachweisen der Expression aller 8 Gene, wie in Tabelle 2 angeführt, und/oder einer oder mehrerer entsprechender Alternativen der Gene, wie in Tabelle 2 angeführt, umfasst.

7. Kit nach Anspruch 6, das ferner Primer und Sonden zum Nachweisen der Expression zumindest eines Referenzgens, das in Tabelle 3 identifiziert ist, umfasst.

8. Computerimplementiertes Verfahren zum Beurteilen der Wahrscheinlichkeit eines Rezidivs bei einem Patienten mit lymphknotennegativem, östrogenrezeptorpositivem, HER2-negativem Brustkrebs, wobei das Verfahren umfasst:
Empfangen einer Information, die die Expressionshöhe der 17 Gene, die in Tabelle 1 als primäre Vorläufer identifiziert sind, oder eines oder mehrerer alternativer Gene, die in Tabelle 1 angeführt sind, anstatt des entsprechenden primären Vorläufers, der in Tabelle 1 angeführt ist, in einer Brusttumorgewebeprobe des Patienten auf einem oder mehreren Computersystemen;
Durchführen einer Random-Forest-Analyse mit einem oder mehreren Prozessoren, die mit dem Computersystem assoziiert sind, wobei die Expressionshöhe jedes Gens bei der Analyse einem letzten Blatt jedes Entscheidungsbaums zugeordnet wird, das ein Votum für "Rezidiv" oder kein "Rezidiv" darstellt;
Generieren eines Random-Forest-Rezidivpunktewerts (RFRS) mit dem einen oder den mehreren Prozessoren, die mit dem einen oder den mehreren Computersystemen assoziiert sind, wobei, wenn der RFRS größer gleich 0,606 ist, der Patient einer Gruppe mit hohem Risiko zugeordnet wird, wenn er größer gleich 0,333 und kleiner als 0,606 ist, der Patient einer Gruppe mit mittlerem Risiko zugeordnet wird, und wenn er kleiner als 0,333 ist, der Patient einer Gruppe mit geringem Risiko zugeordnet wird.

9. Computerimplementiertes Verfahren nach Anspruch 8, das ferner das Generieren einer Wahrscheinlichkeit eines Rezidivs mit dem einen oder den mehreren Prozessoren, die mit dem einen oder den mehreren Computersystemen assoziiert sind, durch Vergleichen des RFRS-Punktewerts für den Patienten mit einer Loess-Anpassung des RFRS versus Wahrscheinlichkeit eines Rezidivs für einen Schulungsdatensatz umfasst.

10. Nichtflüchtiges computerlesbares Medium, auf dem ein Programmcode zum Beurteilen der Wahrscheinlichkeit eines Rezidivs bei einem Patienten mit lymphknotennegativem, östrogenrezeptorpositivem, HER2-negativem Brustkrebs gespeichert ist, wobei das computerlesbare Medium umfasst:
einen Code zum Empfangen einer Information, die die Expressionshöhe der 17 Gene, die in Tabelle 1 als primäre Vorläufer identifiziert sind, oder eines oder mehrerer alternativer Gene, die in Tabelle 1 angeführt sind, anstatt des entsprechenden primären Vorläufers, der in Tabelle 1 angeführt ist, in einer Brusttumorgewebeprobe des Patienten;
einen Code zum Durchführen einer Random-Forest-Analyse, wobei die Expressionshöhe jedes Gens bei der Analyse einem letzten Blatt jedes Entscheidungsbaums zugeordnet wird, das ein Votum für "Rezidiv" oder kein "Rezidiv" darstellt; und
einen Code Generieren eines Random-Forest-Rezidivpunktewerts (RFRS), wobei, wenn der RFRS größer gleich 0,606 ist, der Patient einer Gruppe mit hohem Risiko zugeordnet wird, wenn er größer gleich 0,333 und kleiner als 0,606 ist, der Patient einer Gruppe mit mittlerem Risiko zugeordnet wird, und wenn er kleiner als 0,333 ist, der Patient einer Gruppe mit geringem Risiko zugeordnet wird.

11. Computerlesbares Medium nach Anspruch 10, das ferner einen Code zum Generieren einer Wahrscheinlichkeit eines Rezidivs durch Vergleichen des RFRS-Punktewerts für den Patienten mit einer Loess-Anpassung des RFRS versus Wahrscheinlichkeit eines Rezidivs für einen Schulungsdatensatz umfasst.

12. Computerimplementiertes Verfahren zum Beurteilen der Wahrscheinlichkeit eines Rezidivs bei einem Patienten mit lymphknotennegativem, östrogenrezeptorpositivem, HER2-negativem Brustkrebs, wobei das Verfahren umfasst:
Empfangen einer Information, die die Expressionshöhe der 8 Gene, die in Tabelle 2 als primäre Vorläufer identifiziert sind, oder eines oder mehrerer alternativer Gene, die in Tabelle 2 angeführt sind, anstatt des entsprechenden primären Vorläufers, der in Tabelle 2 angeführt ist, in einer Brusttumorgewebeprobe des Patienten auf einem oder mehreren Computersystemen;
Durchführen einer Random-Forest-Analyse mit einem oder mehreren Prozessoren, die mit dem Computersystem assoziiert sind, wobei die Expressionshöhe jedes Gens bei der Analyse einem letzten Blatt jedes Entscheidungsbaums zugeordnet wird, das ein Votum für "Rezidiv" oder kein "Rezidiv" darstellt;
Generieren eines Random-Forest-Rezidivpunktewerts (RFRS) mit dem einen oder den mehreren Prozessoren, die mit dem einen oder mehreren Computersystemen assoziiert sind, wobei, wenn der RFRS größer gleich 0,606 ist, der Patient einer Gruppe mit hohem Risiko zugeordnet wird, wenn er größer gleich 0,333 und kleiner als 0,606 ist, der Patient einer Gruppe mit mittlerem Risiko zugeordnet wird, und wenn er kleiner als 0,333 ist, der Patient einer Gruppe mit geringem Risiko zugeordnet wird.

13. Computerimplementiertes Verfahren, das ferner das Generieren einer Wahrscheinlichkeit eines Rezidivs durch Vergleichen des RFRS-Punktewerts für den Patienten mit einer Loess-Anpassung des RFRS versus Wahrscheinlichkeit eines Rezidivs für einen Schulungsdatensatz mit dem einen oder den mehreren Prozessoren, die mit dem einen oder den mehreren Computersystemen assoziiert sind, umfasst.

14. Nichtflüchtiges computerlesbares Medium, auf dem ein Programm zum Beurteilen der Wahrscheinlichkeit eines Rezidivs bei einem Patienten mit lymphknotennegativem, östrogenrezeptorpositivem, HER2-negativem Brustkrebs gespeichert ist, wobei computerlesbare Medium umfasst:
einen Code zum Empfangen einer Information, die die Expressionshöhe der 8 Gene, die in Tabelle 2 als primäre Vorläufer identifiziert sind, oder eines oder mehrerer alternativer Gene, die in Tabelle 2 angeführt sind, anstatt des entsprechenden primären Vorläufers, der in Tabelle 2 angeführt ist, in einer Brusttumorgewebeprobe des Patienten;
einen Code zum Durchführen einer Random-Forest-Analyse, wobei die Expressionshöhe jedes Gens bei der Analyse einem letzten Blatt jedes Entscheidungsbaums zugeordnet wird, das ein Votum für "Rezidiv" oder kein "Rezidiv" darstellt; und
einen Code zum Generieren eines Random-Forest-Rezidivpunktewerts (RFRS) wobei, wenn der RFRS größer gleich 0,606 ist, der Patient einer Gruppe mit hohem Risiko zugeordnet wird, wenn er größer gleich 0,333 und kleiner als 0,606 ist, der Patient einer Gruppe mit mittlerem Risiko zugeordnet wird, und wenn er kleiner als 0,333 ist, der Patient einer Gruppe mit geringem Risiko zugeordnet wird.

15. Nichtflüchtiges computerlesbares Medium, auf dem ein Programmcode nach Anspruch 14 gespeichert ist, das ferner einen Code zum Generieren einer Wahrscheinlichkeit eines Rezidivs durch Vergleichen des RFRS-Punktewerts für den Patienten mit einer Loess-Anpassung des RFRS versus Wahrscheinlichkeit eines Rezidivs für einen Schulungsdatensatz umfasst.

## Revendications

1. Procédé d'évaluation de la probabilité de récidive pour un patient atteint d'un cancer du sein HER2-négatif, à récepteurs d'oestrogène sans envahissement ganglionnaire, le procédé comprenant :
(a) la détection dans un échantillon de tumeur du sein prélevé sur le patient du taux d'expression des 17 gènes identifiés dans le Tableau 1 en tant que facteurs de prédiction principaux, au moins un gène suppléant énuméré dans le Tableau 1 pouvant être détecté à la place du gène principal correspondant énuméré dans le Tableau 1 ; ou
(b) la détection dans un échantillon de tumeur du sein prélevé sur le patient du taux d'expression des 8 gènes identifiés dans le Tableau 2 en tant que facteurs de prédiction principaux, au moins un gène suppléant énuméré dans le Tableau 2 pouvant être détecté à la place du gène principal correspondant énuméré dans le Tableau 2 ;
la corrélation des taux d'expression avec la probabilité d'une rechute.

2. Procédé de la revendication 1, dans lequel :
(a) l'étape de détection de 1(a) comprend la détection d'au moins un gène suppléant en plus du gène principal énuméré dans le Tableau 1 ; ou
(b) l'étape de détection de 1(b) comprend la détection d'au moins un gène suppléant en plus du gène principal énuméré dans le Tableau 2.

3. Procédé de la revendication 1 ou 2, comprenant en outre la détection du taux d'expression d'au moins un gène de référence identifié dans le Tableau 3.

4. Procédé de la revendication 1, 2 ou 3, dans lequel :
(a) l'étape de détection comprend la détection du taux d'expression d'ARN, la détection du taux d'expression d'ARN comprenant éventuellement (i) une réaction par PCR quantitative ; ou (ii) l'hybridation d'un acide nucléique obtenu à partir de l'échantillon sur une matrice qui comprend des sondes pour l'ensemble des 17 gènes identifiés dans le Tableau 1 en tant que facteurs de prédiction principaux, et/ou pour au moins un suppléant correspondant desdits gènes tels qu'énumérés dans le Tableau 1 ; ou l'hybridation d'un acide nucléique obtenu à partir de l'échantillon sur une matrice qui comprend des sondes pour l'ensemble des 8 gènes identifiés dans le Tableau 2 en tant que facteurs de prédiction principaux, et/ou pour au moins un suppléant correspondant desdits gènes tels qu'énumérés dans le Tableau 2 ; ou
(b) l'étape de détection comprend la détection du taux d'expression protéique.

5. Utilisation d'un microréseau pour déterminer la probabilité de récidive pour un patient atteint d'un cancer du sein HER2-négatif, à récepteurs d'oestrogène sans envahissement ganglionnaire par la détection du taux d'expression des 17 gènes indiqués dans le Tableau 1 en tant que facteurs de prédiction principaux, au moins un gène suppléant énuméré dans le Tableau 1 pouvant être détecté à la place du gène principal correspondant énuméré dans le Tableau 1 ; ou par la détection du taux d'expression des 8 gènes indiqués dans le Tableau 2 en tant que facteurs de prédiction principaux, au moins un gène suppléant énuméré dans le Tableau 2 pouvant être détecté à la place du gène principal correspondant énuméré dans le Tableau 2, ledit microréseau comprenant des sondes destinées à détecter
(a) le panel de l'ensemble des 17 gènes identifiés dans le Tableau 1 en tant que facteurs de prédiction principaux, et/ou au moins suppléant correspondant desdits gènes tels qu'énumérés dans le Tableau 1 ;
(b) le panel de l'ensemble des 8 gènes identifiés dans le Tableau 2 en tant que facteurs de prédiction principaux, et/ou au moins suppléant correspondant desdits gènes tels qu'énumérés dans le Tableau 2 ; ou
(c) le panel de (a) ou (b) et d'au moins un gène identifié dans le Tableau 3.

6. Utilisation d'un kit pour déterminer une probabilité de récidive pour un patient atteint d'un cancer du sein HER2-négatif, à récepteurs d'oestrogène sans envahissement ganglionnaire par la détection du taux d'expression des 17 gènes indiqués dans le Tableau 1 en tant que facteurs de prédiction principaux, au moins un gène suppléant énuméré dans le Tableau 1 pouvant être détecté à la place du gène principal correspondant énuméré dans le Tableau 1 ; ou par la détection du taux d'expression des 8 gènes indiqués dans le Tableau 2 en tant que facteurs de prédiction principaux, au moins un gène suppléant énuméré dans le Tableau 2 pouvant être détecté à la place du gène principal correspondant énuméré dans le Tableau 2, ledit kit comprenant des amorces et des sondes destinées à détecter l'expression de l'ensemble des 17 gènes énumérés dans le Tableau 1, et/ou au moins un suppléant desdits gènes tels qu'énumérés dans le Tableau 1 ; ou des amorces et des sondes destinés à détecter l'expression de l'ensemble des 8 gènes tels qu'énumérés dans le Tableau 2, et/ou au moins un suppléant correspondant desdits gènes tels qu'énumérés dans le Tableau 2.

7. Kit de la revendication 6, comprenant en outre des amorces et des sondes destinées à détecter l'expression d'au moins un gène de référence identifié dans le Tableau 3.

8. Procédé mis en oeuvre par ordinateur permettant d'évaluer la probabilité de récidive pour un patient atteint d'un cancer du sein HER2-négatif, à récepteurs d'oestrogène sans envahissement ganglionnaire, le procédé comprenant :
la réception, au niveau d'au moins un système informatique, d'informations décrivant le taux d'expression des 17 gènes identifiés dans le Tableau 1 en tant que facteurs de prédiction principaux, ou d'au moins un gène suppléant énuméré dans le Tableau 1 à la place du facteur de prédiction principal correspondant énuméré dans le Tableau 1, dans un échantillon de tissu tumoral du sein prélevé sur le patient ;
la réalisation, avec au moins un processeur associé au système informatique, d'une analyse de forêt aléatoire dans laquelle le taux d'expression de chaque gène dans l'analyse est attribué à une feuille terminale de chaque arbre de décision, représentant un vote soit en faveur de « récidive », soit en faveur de « non-récidive » ;
la génération, avec l'au moins un processeur associé à l'au moins un système informatique, d'un score de récidive par forêt aléatoire (RFRS), où si le RFRS est supérieur ou égal à 0,606, le patient est classé dans un groupe à haut risque, s'il est supérieur ou égal à 0,333 et inférieur à 0,606, le patient est classé dans un groupe de risque intermédiaire, et s'il est inférieur à 0,333, le patient est classé dans un groupe de faible risque.

9. Procédé mis en oeuvre par ordinateur de la revendication 8, comprenant en outre la génération, avec l'au moins un processeur associé à l'au moins un système informatique, d'une probabilité de récidive par comparaison du score RFRS pour le patient à un ajustement de loess du RFRS par rapport à la probabilité de récidive pour un ensemble de données d'apprentissage.

10. Support lisible par ordinateur non transitoire stockant un code de programme destiné à évaluer la probabilité de récidive pour un patient atteint d'un cancer du sein HER2-négatif, à récepteurs d'oestrogène sans envahissement ganglionnaire, le support lisible par ordinateur comprenant :
un code pour recevoir des informations décrivant le taux d'expression des 17 gènes identifiés dans le Tableau 1 en tant que facteurs de prédiction principaux, d'au moins un gène suppléant énuméré dans le Tableau 1 à la place du facteur de prédiction principal correspondant énuméré dans le Tableau 1, dans un échantillon de tissu tumoral du sein prélevé sur le patient ;
un code pour effectuer une analyse de forêt aléatoire dans laquelle le taux d'expression de chaque gène dans l'analyse est attribué à une feuille terminale de chaque arbre de décision, représentant un vote soit en faveur de « récidive », soit en faveur de « non-récidive » ; et
un code pour générer un score de récidive par forêt aléatoire (RFRS), où si le RFRS est supérieur ou égal à 0,606, le patient est classé dans un groupe à haut risque, s'il est supérieur ou égal à 0,333 et inférieur à 0,606, le patient est classé dans un groupe de risque intermédiaire, et s'il est inférieur à 0,333, le patient est classé dans un groupe de faible risque.

11. Support lisible par ordinateur de la revendication 10, comprenant en outre un code pour générer une probabilité de récidive par comparaison du score RFRS pour le patient à un ajustement de loess du RFRS par rapport à la probabilité de récidive pour un ensemble de données d'apprentissage.

12. Procédé mis en oeuvre par ordinateur permettant d'évaluer la probabilité de récidive pour un patient atteint d'un cancer du sein HER2-négatif, à récepteurs d'oestrogène sans envahissement ganglionnaire, le procédé comprenant :
la réception, au niveau d'au moins un système informatique, d'informations décrivant le taux d'expression des 8 gènes identifiés dans le Tableau 2 en tant que facteurs de prédiction principaux, ou d'au moins un gène suppléant énuméré dans le Tableau 2 à la place du facteur de prédiction principal correspondant énuméré dans le Tableau 2, dans un échantillon de tissu tumoral du sein prélevé sur le patient ;
la réalisation, avec au moins un processeur associé au système informatique, d'une analyse de forêt aléatoire dans laquelle le taux d'expression de chaque gène dans l'analyse est attribué à une feuille terminale de chaque arbre de décision, représentant un vote soit en faveur de « récidive », soit en faveur de « non-récidive » ;
la génération, avec l'au moins un processeur associé à l'au moins un système informatique, d'un score de récidive par forêt aléatoire (RFRS), où si le RFRS est supérieur ou égal à 0,606, le patient est classé dans un groupe à haut risque, s'il est supérieur ou égal à 0,333 et inférieur à 0,606, le patient est classé dans un groupe de risque intermédiaire, et s'il est inférieur à 0,333, le patient est classé dans un groupe de faible risque.

13. Procédé mis en oeuvre par ordinateur de la revendication 12, comprenant en outre la génération, avec l'au moins un processeur associé à l'au moins un système informatique, d'une probabilité de récidive par comparaison du score RFRS pour le patient à un ajustement de loess du RFRS par rapport à la probabilité de récidive pour un ensemble de données d'apprentissage.

14. Support lisible par ordinateur non transitoire stockant un code de programme destiné à évaluer la probabilité de récidive pour un patient atteint d'un cancer du sein HER2-négatif, à récepteurs d'oestrogène sans envahissement ganglionnaire, le support lisible par ordinateur comprenant :
un code pour recevoir des informations décrivant le taux d'expression des 8 gènes identifiés dans le Tableau 2 en tant que facteurs de prédiction principaux, d'au moins un gène suppléant énuméré dans le Tableau 2 à la place du facteur de prédiction principal correspondant énuméré dans le Tableau 2, dans un échantillon de tissu tumoral du sein prélevé sur le patient ;
un code pour effectuer une analyse de forêt aléatoire dans laquelle le taux d'expression de chaque gène dans l'analyse est attribué à une feuille terminale de chaque arbre de décision, représentant un vote soit en faveur de « récidive », soit en faveur de « non-récidive » ; et
un code pour générer un score de récidive par forêt aléatoire (RFRS), où si le RFRS est supérieur ou égal à 0,606, le patient est classé dans un groupe à haut risque, s'il est supérieur ou égal à 0,333 et inférieur à 0,606, le patient est classé dans un groupe de risque intermédiaire, et s'il est inférieur à 0,333, le patient est classé dans un groupe de faible risque.

15. Support lisible par ordinateur non transitoire stockant un code de programme de la revendication 14, comprenant un code pour générer une probabilité de récidive par comparaison du score RFRS pour le patient à un ajustement de loess du RFRS par rapport à la probabilité de récidive pour un ensemble de données d'apprentissage.
